# EUROPEAN PATENT SPECIFICATION

(11) **EP 3 886 894 B1**
(45) Date of publication and mention of the grant of the patent: **13.03.2024**
(21) Application number: 19824223.2
(22) Date of filing: 29.11.2019
(51) Int. Cl.: C12N 5/0783, C07K 14/05, A61K 35/17, A61K 39/00, A61P 35/00

(54) **METHODS FOR DOSING AND TREATMENT OF B CELL MALIGNANCIES IN ADOPTIVE CELL THERAPY**
VERFAHREN ZUR DOSIERUNG UND BEHANDLUNG VON B-ZELL-MALIGNOMEN IN DER ADOPTIVEN ZELLTHERAPIE
MÉTHODES DE DOSAGE ET DE TRAITEMENT DE MALIGNITÉS DE LYMPHOCYTES B AU MOYEN D'UNE THÉRAPIE CELLULAIRE ADOPTIVE

(30) Priority: 30.11.2018 US 201862774168 P; 03.12.2018 US 201862774858 P; 14.05.2019 US 201962847897 P; 30.05.2019 US 201962854957 P; 05.11.2019 US 201962931143 P
(43) Date of publication of application: 06.10.2021
(73) Proprietor: Juno Therapeutics, Inc., Seattle, WA 98109-4703 (US)
(72) Inventor: GILLENWATER, Heidi, Seattle, Washington 98109-4703 (US); ALBERTSON, Tina, Seattle, Washington 98109 (US); DUBOVSKY, Jason A., Seattle, Washington 98109 (US); THORPE, Jerill, Seattle, Washington 98109 (US)
(74) Representative: Carpmaels & Ransford LLP
(86) International application number: PCT/US2019/063883
(87) International publication number: WO 2020/113188

(56) References cited:
- WO-A1-2015/157384
- WO-A2-2017/214207

## Description

### Field

The present disclosure relates in some aspects to adoptive cell therapy involving the administration of doses of cells for treating subjects with disease and conditions such as certain B cell malignancies, and related methods, compositions, uses and articles of manufacture. The cells generally express recombinant receptors such as chimeric antigen receptors (CARs). In some embodiments, the disease or condition is a chronic lymphocytic leukemia (CLL), such as relapsed or refractory CLL. In some embodiments, the disease or condition is a small lymphocytic lymphoma (SLL). In some embodiments, the subject is of a specific group or subset of CLL or SLL subjects, such as heavily pretreated or poor-prognosis subjects.

### Background

Chronic lymphocytic leukemia (and small lymphocytic lymphoma) is an indolent cancer in which immature lymphocytes are found in the blood and bone marrow and/or in the lymph nodes. Chronic lymphocytic leukemia and small lymphocytic lymphoma are the same disease, but in CLL cancer cells are found mostly in the blood and bone marrow. In SLL, cancer cells are found mostly in the lymph nodes. CLL is considered incurable. Patients eventually relapse or become refractory to available therapies. B cell receptor antagonists have shown improved outcomes for patients with relapsed/refractory CLL, however complete remission rates remain low, and patients who progress on or after therapy have poor outcomes. Effective therapies for patients with CLL and SLL who have failed B cell receptor antagonist therapy are needed. Provided are methods and uses that meet such needs. WO 2017/214207 A2 describes methods for treatment of B cell malignancies using adoptive cell therapy. WO 2015/157384 A1 describes defined composition gene modified T-cell products.

### Summary

The invention is defined in the appended claims. Any references herein to methods of treatment are intended to refer to products for use in said methods.

The invention provides a first composition comprising one of CD4+ T cells and CD8+ T cells for use with a second composition comprising the other of CD4+ T cells and CD8+ T cells in a method of treating a subject having a chronic lymphocytic leukemia (CLL) or small lymphocytic lymphoma (SLL), wherein the method comprises administering to the subject a dose of engineered T cells comprising CD4+ and CD8+ T cells, comprising a chimeric antigen receptor (CAR) that specifically binds to CD19, wherein:
(i) the subject has relapsed following remission after treatment with, become refractory to or failed treatment with and/or is intolerant to ibrutinib and venetoclax;
(ii) the administration comprises administering a plurality of separate comporistions, wherein the plurarlity of separate compositions comprises the first composition comprising one of CD4+ T cells and CD8+ Tcells and the second composition comprising the other of CD4+ T cells and CD8+ T cells; and
(iii) the dose of engineered T cells comprises a defined ratio of CD4+ cells expressing the CAR to CD8+ cells expressing the CAR, optionally wherein the ratio is between approximately 1:3 and approximately 3:1.

The invention further provides a plurality of separate compositions comprising a first composition comprising one of CD4+ T cells and CD8+ T cells and a second composition comprising the other of CD4+ T cells and CD8+ T cells for use in a method of treating subject having chronic lymphocytic leukemia (CLL) or small lymphocytic lymphoma (SLL), wherein the method comprises administering to the subject a dose of engineered T cells omprising CD4+ and CD8+ T cells omprising a chimeric antigen receptor (CAR) that specifically binds to CD19, wherein:
(i) the subject has relapsed following remission after treatment with, become refractory to or failed treatment with and/or is intolerant to ibrutinib and venetoclax;
(ii) the administration comprises administering the plurality of separate compositions, wherein the plurality of separate compositions comprises the first composition comprising one of CD4+ T cells and CD8+ T cells and the second composition comprising the other of CD4+ Tcells and CD8+ T cells; and
(iii) the dose of engineered T cells comprises a defined ratio of CD4+ cells expressing the CAR to CD8+ cells expressing the CAR, optionally wherein the ratio is between approximately 1:3 and approximately 3: 1.

In some embodiments, the dose of cells or cells administered in connection with any embodiments of the provided compositions for use contains CD4⁺ T cells or a subtype or phenotype thereof and/or CD8⁺ T cells or a subtype thereof. In some embodiments, the CD8⁺ cells or subtype or phenotype are present at a particular dose or amount or number; in some embodiments the CD4⁺ cells or subtype or phenotype are present at a particular dose or amount or number. In some embodiments, the CD8⁺ cells or subtype or phenotype thereof and the CD4⁺ cells or subtype or phenotype thereof, are administered at a defined ratio, at or about 1:1, or between at or about 1:3 and at or about 3: 1. In some embodiments, the dose or administration contains or is of a particular amount or number of one population of the cells and the ratio is a defined ratio.

In some embodiments, the dose of engineered T cells is enriched for CD4+ and CD8+ primary human T cells.

In some embodiments, the dose of engineered T cells comprises at or about 2.5 × 10⁷ total CAR-expressing cells to at or about 1.5 × 10⁸ total CAR-expressing cells.

In some embodiments, the dose of cells enriched in CD4+ and CD8+ engineered T cells comprises greater than or greater than about 70%, greater than or greater than about 75%, greater than or greater than about 80%, greater than or greater than about 85%, greater than or greater than about 90%, greater than or greater than about 95% or greater than or greater than about 98% CD4+ and CD8+ primary human T cells.

In some embodiments, the dose of CD4⁺ and CD8⁺ engineered T cells comprises a defined ratio of CD4⁺ cells expressing the CAR to CD8⁺ cells expressing the CAR that is or is approximately 1:1. In some embodiments, the dose of engineered T cells comprises at or about 2.5 × 10⁷ total CAR-expressing cells to at or about 1.0 × 10⁸ total CAR-expressing cells. In some embodiments, the dose of engineered T cells comprises or about 2.5 × 10⁷ total CAR-expressing cells. In some embodiments, the dose of engineered T cells comprises at or about 5 × 10⁷ total cells or total CAR-expressing cells. In some embodiments, the engineered T cells comprises at or about 1 × 10⁸ total cells or total CAR-expressing cells.

In some embodiments, the CAR comprised by the CD4⁺ T cells and/or the CAR comprised by the CD8⁺ T cells comprises a CAR that is the same and/or wherein the CD4⁺ T cells and/or the CD8⁺ T cells are genetically engineered to express a CAR that is the same.

In particular embodiments, the first composition comprises the CD8⁺ T cells and the second composition comprises the CD4+ T cells. In some embodiments, the initiation of the administration of the first composition is carried out prior to the initiation of the administration of the second composition.

In some embodiments, the administration of the first composition and the administration of the second composition are carried out on the same day, are carried out no more than 36 hours apart, no more than 24 hours apart, no more than 12 hours apart, no more than 6 hours apart, no more than 4 hours apart, no more than 2 hours apart, or no more than 1 hour apart or no more than 30 minutes apart. In some embodiments, the administration of the first composition and the administration of the second composition are carried out between at or about 0 and at or about 48 hours, between at or about 0 and at or about 36 hours, between at or about 0 and at or about 24 hours, between at or about 0 and at or about 12 hours, between at or about 0 and at or about 6 hours, between at or about 0 and at or about 2 hours, between at or about 0 and at or about 1 hours, between at or about 0 and at or about 30 minutes, between at or about 30 minutes and at or about 48 hours, between at or about 30 minutes and at or about 36 hours, between at or about 30 minutes and at or about 24 hours, between at or about 30 minutes and at or about 12 hours, between at or about 30 minutes and at or about 6 hours, between at or about 30 minutes and at or about 4 hours, between at or about 30 minutes and at or about 2 hours, between at or about 30 minutes and at or about 1 hour, between at or about 1 hours and at or about 48 hours, between at or about 1 hour and at or about 36 hours, between at or about 1 hour and at or about 24 hours, between at or about 1 hour and at or about 12 hours, between at or about 1 hour and at or about 6 hours, between at or about 1 hour and at or about 4 hours, between at or about 1 hour and at or about 2 hours, between at or about 2 hours and at or about 48 hours, between at or about 2 hours and at or about 36 hours, between at or about 2 hours and at or about 24 hours, between at or about 2 hours and at or about 12 hours, between at or about 2 hours and at or about 6 hours, between at or about 2 hours and at or about 4 hours, between at or about 4 hours and at or about 48 hours, between at or about 4 hours and at or about 36 hours, between at or about 4 hours and at or about 24 hours, between at or about 4 hours and at or about 12 hours, between at or about 4 hours and at or about 6 hours, between at or about 6 hours and at or about 48 hours, between at or about 6 hours and at or about 36 hours, between at or about 6 hours and at or about 24 hours, between at or about 6 hours and at or about 12 hours, between at or about 12 hours and at or about 48 hours, between at or about 12 hours and at or about 36 hours, between at or about 12 hours and at or about 24 hours, between at or about 24 hours and at or about 48 hours, between at or about 24 hours and at or about 36 hours or between at or about 36 hours and at or about 48 hours.

In some embodiments, the administration of the first composition and the administration of the second composition are carried out on the same day, are carried out between about 0 and about 12 hours apart, between about 0 and about 6 hours apart or between about 0 to 2 hours apart; or the initiation of administration of the first composition and the initiation of administration of the second composition are carried out between about 1 minute and about 1 hour apart or between about 5 minutes and about 30 minutes apart. In some embodiments, the first composition and second composition are administered no more than 2 hours, no more than 1 hour, no more than 30 minutes, no more than 15 minutes, no more than 10 minutes or no more than 5 minutes apart.

In some embodiments, the first composition and second composition are administered no more than 2 hours, no more than 1 hour, no more than 30 minutes, no more than 15 minutes, no more than 10 minutes or no more than 5 minutes apart.

In some embodiments of any of the provided compositions for use, the subject has CLL or is suspected of having CLL; or the subject is identified or selected as having CLL. In some embodiments, the CLL is relapsed or refractory CLL.

In some embodiments, the subject has SLL or is suspected of having SLL; or the subject is identified or selected as having SLL. In some embodiments, the SLL is a relapsed or refractory SLL.

In some embodiments, prior to the administration of the dose of engineered T cells, the subject has been treated with one or more prior therapies for the CLL or SLL, other than another dose of cells expressing CAR or a lymphodepleting therapy. In some embodiments, the one or more prior therapy comprises at least two prior therapies, optionally three, four, five, six, seven, eight, nine or more.

In some embodiments, at or immediately prior to the time of the administration of the dose of cells, the subject has relapsed following remission after treatment with, or become refractory to, failed and/or was intolerant to treatment with the one or more prior therapies for the CLL or SLL. In the invention, the subject has relapsed following remission after treatment with, or become refractory to, failed and/or was intolerant to treatment with two or more prior therapies comprising ibrutinib and venetoclax. In some embodiments, at or immediately prior to the time of the administration of the dose of cells, the subject has relapsed following remission after treatment with, or become refractory to, failed and/or was intolerant to treatment with three or more prior therapies. In some embodiments, the prior therapies are selected from a kinase inhibitor, optionally an inhibitor of Bruton's tyrosine kinase (BTK); a combination therapy comprising fludarabine and rituximab; radiation therapy; and hematopoietic stem cell transplantation (HSCT).

In some embodiments, at or prior to the administration of the dose of cells: the subject is or has been identified as having one or more cytogenetic abnormalities, optionally associated with high-risk CLL, optionally selected from among: complex karyotype or cytogenetic abnormalities, del 17p, unmutated IGVH gene, and TP53 mutation; the subject is or has been identified as having high-risk CLL.

In some embodiments, the subject is or has been identified as having an ECOG status of 0 or 1; and/or the subject does not have an ECOG status of >1. In some embodiments, at or immediately prior to the administration of the dose of engineered cells or the lymphodepleting therapy the subject does not have a Richter's transformation of the CLL or SLL.

In some embodiments, the subject is an adult and/or is over at or about 50, 60, or 70 years of age.

In some embodiments, the T cells are primary T cells obtained from a subject. In some embodiments, the T cells are autologous to the subject. In some embodiments, the dose of engineered cells are viable cells.

In some embodiments, prior to the administration of the dose of engineered cells, lymphodepleting therapy is administered to the subject. In some embodiments, the subject has been preconditioned with a lymphodepleting therapy. In some embodiment, the lymphodepleting therapy comprises the administration of fludarabine and/or cyclophosphamide. In some embodiments, the lymphodepleting therapy comprises administration of cyclophosphamide at about 200-400 mg/m², optionally at or about 300 mg/m², inclusive, and/or fludarabine at about 20-40 mg/m², optionally 30 mg/m², daily for 2-4 days, optionally for 3 days. In some embodiments, the lymphodepleting therapy comprises administration of cyclophosphamide at or about 300 mg/m² and fludarabine at about 30 mg/m²daily for 3 days, optionally wherein the dose of cells is administered at least at or about 2-7 days after the lymphodepleting therapy or at least at or about 2-7 days after the initiation of the lymphodepleting therapy.

In some embodiments, at or prior to the administration of the dose of cells: the subject is or has been identified as having one or more cytogenetic abnormalities, optionally associated with high-risk CLL or SLL, optionally selected from among: complex karyotype or cytogenetic abnormalities, del 17p, unmutated IGVH gene, and TP53 mutation; and/or the subject is or has been identified as having high-risk CLL or SLL. In some embodiments, at or immediately prior to the time of the administration of the dose of cells, the subject has relapsed following remission after treatment with, or become refractory to, failed and/or was intolerant to treatment with two or more prior therapies. In some embodiments, at or prior to the administration of the dose of cells, the subject is or has been identified as having a standard-risk CLL or SLL. In some embodiments, at or immediately prior to the time of the administration of the dose of cells, the subject has relapsed following remission after treatment with, or become refractory to, failed and/or was intolerant to treatment with three or more prior therapies. In some embodiments, at or prior to the administration of the dose of cells, the subject is or has been identified as being intolerant to an inhibitor of Bruton's tyrosine kinase (BTK) and has received an inhibitor of BTK for a duration of less than at or about 6 months, and/or is ineligible for treatment with an inhibitor of BTK.

In some embodiments, (i) the subject is or has been identified as having high-risk CLL or SLL, and at or immediately prior to the time of the administration of the dose of cells, the subject has relapsed following remission after treatment with, or become refractory to, failed and/or was intolerant to treatment with one or more prior therapies other than the inhibitor of BTK; or (ii) the subject is or has been identified as having a standard-risk CLL or SLL, and at or immediately prior to the time of the administration of the dose of cells, the subject has relapsed following remission after treatment with, or become refractory to, failed and/or was intolerant to treatment with two or more prior therapies other than the inhibitor of BTK.

In some embodiments, the administration of the cell dose and/or the lymphodepleting therapy is carried out via outpatient delivery. In some embodiments, the dose of cells is administered parenterally, optionally intravenously.

In some embodiments, the response in at least 35%, at least 40 %, at least 50%, at least 60% or at least 70% of subjects treated is complete remission (CR) in the subjects treated according to the method. In some embodiments, the duration of the response until progression is durable for greater than 3 months or greater than 6 months. In some embodiments, in the subjects treated according to the method, greater than 50%, greater than 60%, or greater than 70% had undetectable minimal residual disease (MRD) for at least one month, at least two months, at least three months or at least 6 month after administering the dose of cells.

In some embodiments, of the subjects treated according to the method, no more than 10% of subjects exhibit a cytokine release syndrome (CRS) higher than grade 2. In some embodiments, of a plurality of subjects treated according to the method, no more than 10%, no more than 20%, no more than 30% or no more than 40% of the subjects exhibit neurotoxicity higher than grade 2.

In some embodiments, the CAR comprises an extracellular antigen-binding domain specific for CD 19, a transmembrane domain, a cytoplasmic signaling domain derived from a costimulatory molecule, which optionally is a 4-1 BB, and a cytoplasmic signaling domain derived from a primary signaling ITAM-containing molecule, which optionally is a CD3zeta; the CAR comprises, in order, an extracellular antigen-binding domain specific for CD 19, a transmembrane domain, a cytoplasmic signaling domain derived from a costimulatory molecule, and a cytoplasmic signaling domain derived from a primary signaling ITAM-containing molecule.

In some embodiments, the antigen-binding domain is an scFv.

In some embodiments, the scFv comprises a CDRL1 sequence of RASQDISKYLN (SEQ ID NO: 35), a CDRL2 sequence of SRLHSGV (SEQ ID NO: 36), and/or a CDRL3 sequence of GNTLPYTFG (SEQ ID NO: 37) and/or a CDRH1 sequence of DYGVS (SEQ ID NO: 38), a CDRH2 sequence of VIWGSETTYYNSALKS (SEQ ID NO: 39), and/or a CDRH3 sequence of YAMDYWG (SEQ ID NO: 40); the scFv comprises a variable heavy chain region of FMC63 and a variable light chain region of FMC63 and/or a CDRL1 sequence of FMC63, a CDRL2 sequence of FMC63, a CDRL3 sequence of FMC63, a CDRH1 sequence of FMC63, a CDRH2 sequence of FMC63, and a CDRH3 sequence of FMC63 or binds to the same epitope as or competes for binding with any of the foregoing; the scFv comprises a VH set forth in SEQ ID NO:41 and a VL set forth in SEQ ID NO: 42, optionally wherein the VH and VL are separated by a flexible linker, optionally wherein the flexible linker is or comprises the sequence set forth in SEQ ID NO:24; and/or the scFv is or comprises the sequence set forth in SEQ ID NO:43.

In some embodiments, the costimulatory signaling region is a signaling domain of CD28 or 4-1BB. In some embodiments, the costimulatory signaling region is a signaling domain of 4-1BB.

In some embodiments, the costimulatory domain comprises SEQ ID NO: 12 or a variant thereof having at least 85%, 86%, 87%, 88%, 89%, 90%, 91%, 92%, 93%, 94%, 95%, 96%, 97%, 98%, 99% or more sequence identity thereto. In some embodiments, the primary signaling domain is a CD3zeta signaling domain. In some embodiments, the primary signaling domain comprises SEQ ID NO: 13 or 14 or 15 having at least 85%, 86%, 87%, 88%, 89%, 90%, 91%, 92%, 93%, 94%, 95%, 96%, 97%, 98%, 99% or more sequence identity thereto.

In some embodiments, the CAR further comprises a spacer between the transmembrane domain and the scFv. In some embodiments, the spacer is a polypeptide spacer that comprises or consists of all or a portion of an immunoglobulin hinge or a modified version thereof, optionally an IgG4 hinge, or a modified version thereof. In some embodiments, the spacer is about 15 amino acids or less, and does not comprise a CD28 extracellular region or a CD8 extracellular region. In some embodiments, the spacer is at or about 12 amino acids in length.

In some embodiments, the spacer has or consists of the sequence of SEQ ID NO: 1, a sequence encoded by SEQ ID NO: 2, SEQ ID NO: 30, SEQ ID NO: 31, SEQ ID NO: 32, SEQ ID NO: 33, SEQ ID NO: 34, or a variant of any of the foregoing having at least 85%, 86%, 87%, 88%, 89%, 90%, 91%, 92%, 93%, 94%, 95%, 96%, 97%, 98%, 99% or more sequence identity thereto; and/or comprises or consists of the formula X₁PPX₂P, where X₁ is glycine, cysteine or arginine and X₂ is cysteine or threonine.

In some of any of the embodiments, the subject is a human subject.

### Brief Description of the Drawings

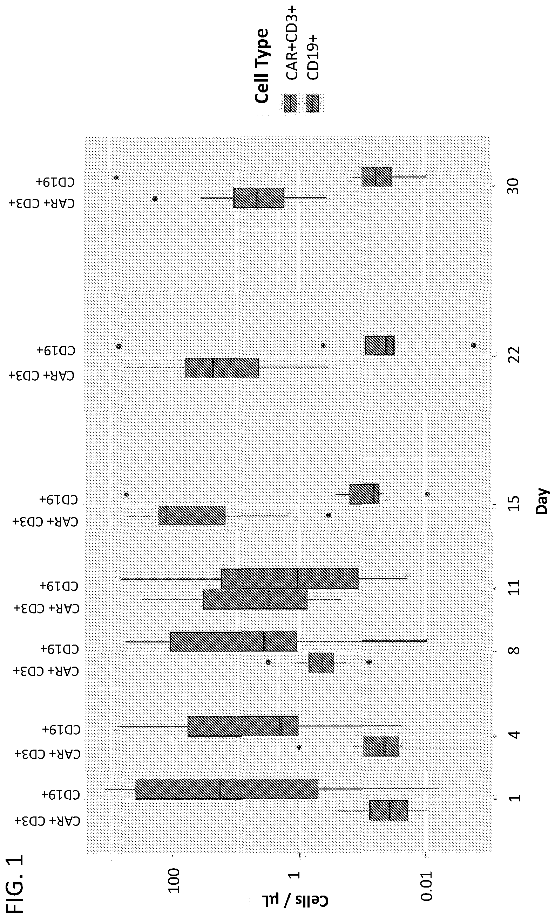

FIG. 1 shows a box plot of the frequency of CAR T cells in whole blood as measured by flow cytometry. The boxes extend from the 1^{st} to the 3^{rd} quartile, with the median shown as a horizontal line. The whiskers extend to 1.5 times the inter-quartile range, with measurements outside this range plotted as individual points.

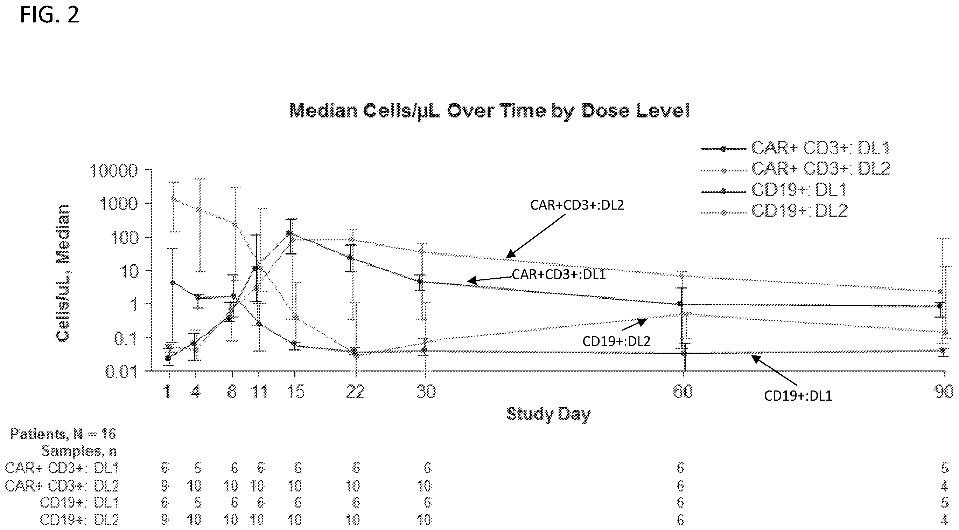

FIG. 2 shows a graph of the median cells/µl over time by dose level. Patients, N = 16; samples, n. Upper error bar represents the third quartile; lower error bar represents the first quartile. The dose was given on day 1.

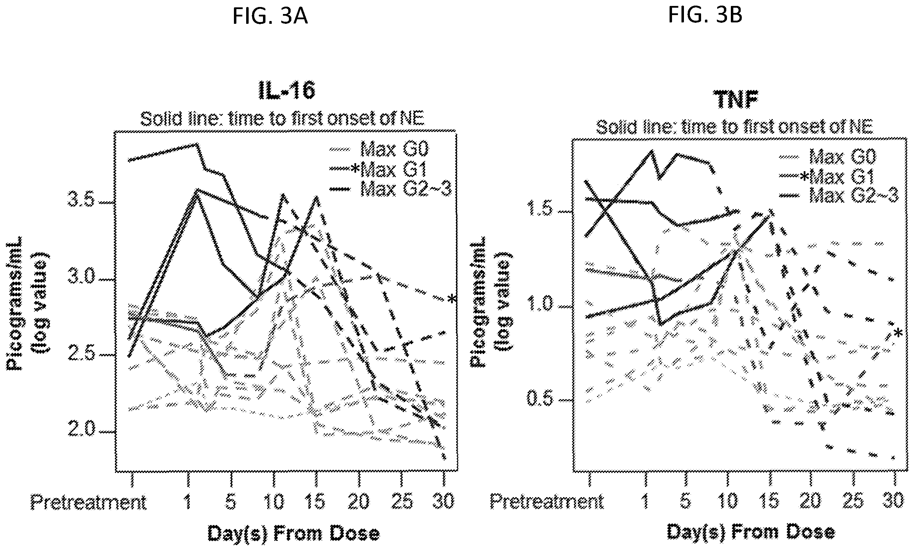

**FIG. 3A** and **FIG. 3B** show graphs of the biomarker analysis of IL-16 and TNFα in patients over time. Patients, N = 13. Thirty-nine cytokines were analyzed in 13 patients within the first thirty days of treatment.

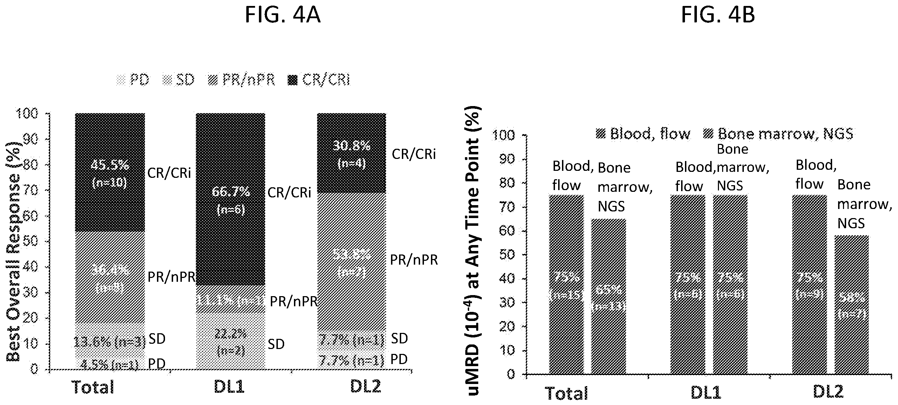

**FIG. 4A** show results of the best overall response in subjects with R/R CLL (N=22) administered anti-CD19 CAR+ T cells at DL1 (5 × 10⁷ CAR-expressing T cells) or DL2 (1 × 10⁸ CAR-expressing T cells). **FIG. 4B** shows results of undetectable minimal residual disease (uMRD) in blood by flow cytometry or in bone marrow by next generation sequencing (NGS) at any time point following administration to subjects with R/R CLL of anti-CD19 CAR-expressing T cells at DL1 (5 × 10⁷ CAR-expressing T cells) or DL2 (1 × 10⁸ CAR-expressing T cells).

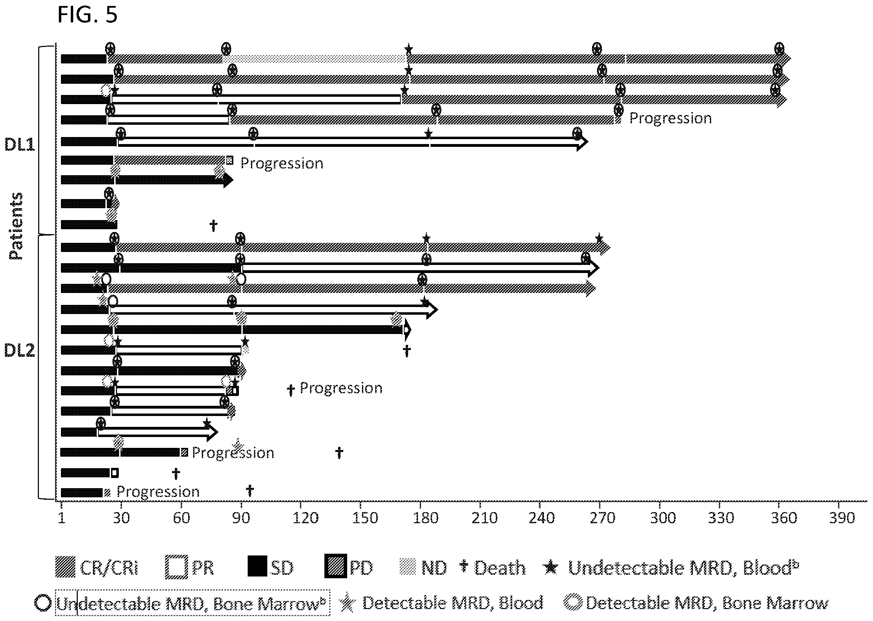

**FIG. 5** shows a swimmer plot of the duration of response over time in subjects with R/R CLL (N=22) administered anti-CD19 CAR+ T cells at DL1 (5 × 10⁷ CAR-expressing T cells) or DL2 (1 × 10⁸ CAR-expressing T cells)..

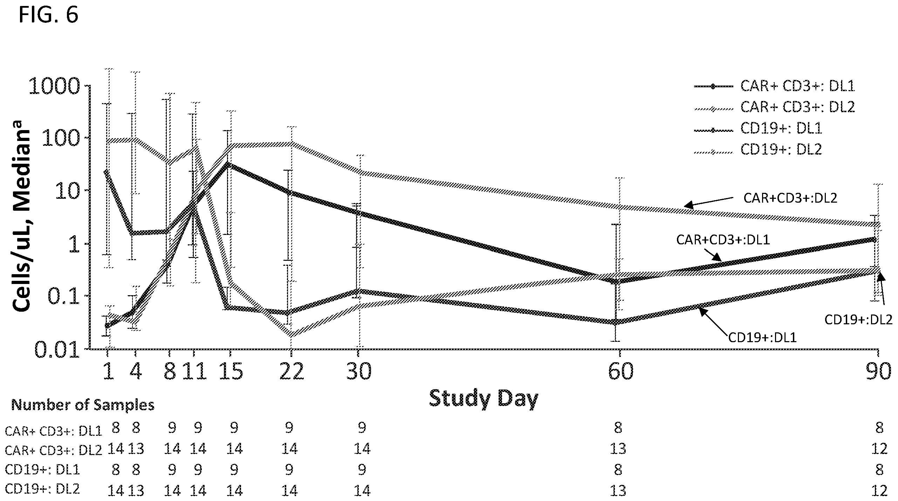

**FIG. 6** shows a graph of the median cells/µl over time by dose levels in subjects with R/R CLL administered anti-CD19 CAR+ T cells at DL1 (5 × 10⁷ CAR-expressing T cells) or DL2 (1 × 10⁸ CAR-expressing T cells). Upper error bar represents the third quartile, lower error bar represents the first quartile. The dose of anti-CD19 CAR+ T cells was given on day 1.

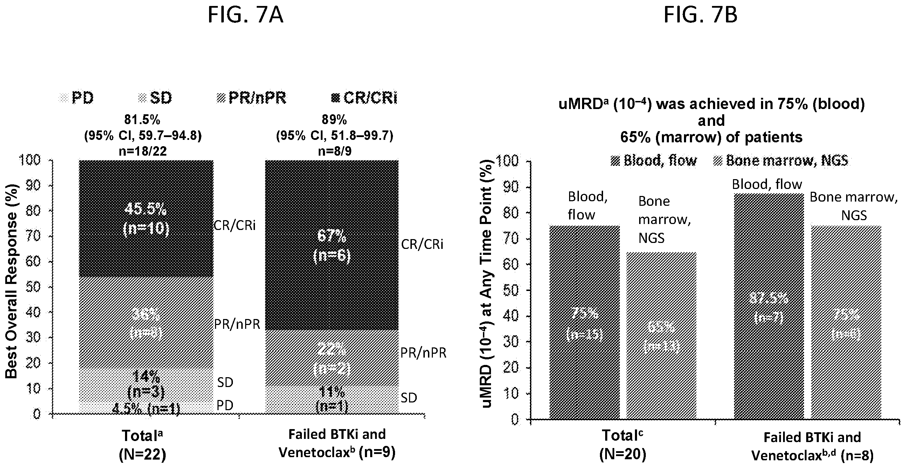

**FIG. 7A** show results of the best overall response in subjects with R/R CLL (N=22) or subjects who have failed prior treatment with both a Bruton's tyrosine kinase inhibitor (BTKi) and venetoclax (N=9). **FIG. 7B** shows results of undetectable minimal residual disease (uMRD) in blood by flow cytometry or in bone marrow by next generation sequencing (NGS) at any time point following administration in subjects with R/R CLL (N=20) or subjects who have failed prior treatment with both a BTKi and venetoclax (N=8). ^{a}Evaluable for response defined as having a pretreatment assessment and ≥1 postbaseline assessment; evaluable for MRD was defined as patients with detectable MRD at baseline. One subject was not evaluable for response. ^{b}Failed venetoclax defined as discontinuation due to PD or <PR after ≥3 months of therapy. ^{c}Two subjects were not evaluable for MRD. ^{d}One subject was not evaluable for MRD. CI, confidence interval; CRi, complete response with incomplete blood count recovery; NGS, next-generation sequencing; nPR, nodular partial response; PD, progressive disease; PR, partial response; SD, stable disease; uMRD, undetectable minimal residual disease.

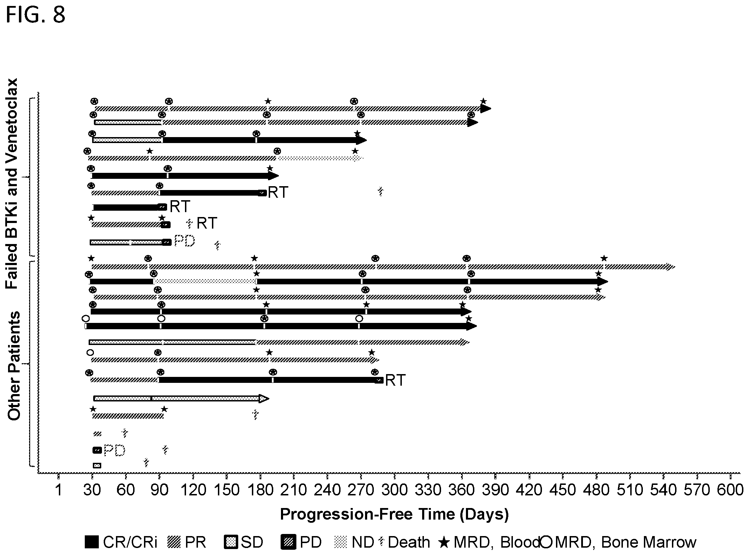

**FIG. 8** shows a swimmer plot of the duration of response over time in individual subjects with R/R CLL who have failed prior treatment with both a BTKi and venetoclax, and the other treated subjects. *MRD non-evaluable. There were 7 on-study deaths: 5 subjects died from disease progression; 1 subject had grade 5 respiratory failure (DL1) unrelated to CAR+T cells therapy treatment; 1 subject had septic shock, acute kidney injury, and pneumonia (DL2), unrelated to CAR+T cells therapy treatment. No deaths occurred within the first 30 days. ND, not done; RT, Richter Transformation.

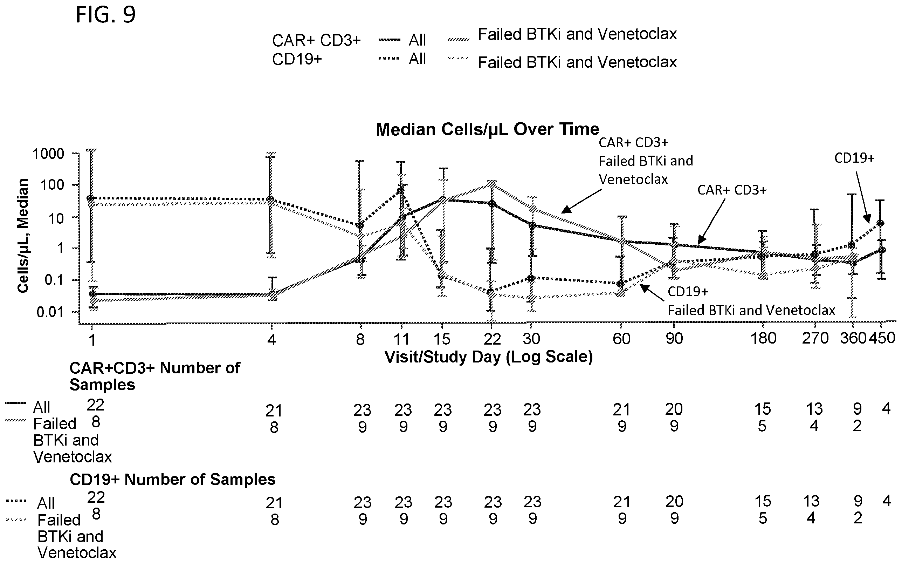

**FIG. 9** shows a graph of the median cells/µL over time by dose levels in evaluable treated subjects and subjects who have failed prior treatment with both a BTKi and venetoclax. Upper error bar represents the third quartile, Lower error bar represents the first quartile. CAR+T cells therapy was given on Day 1. AUC₀₋₂₉, area under the curve from days 0 to 29; Cₘₐₓ, maximum concentration; PK/PD, pharmacokinetic/pharmacodynamic; Q, quartile; Tₘₐₓ, time to maximum concentration.

### Detailed Description

Embodiments of the invention fall within some of the instances disclosed below. Any references herein to methods of treatment are intended to refer to products for use in said methods.

Disclosed herein are methods and uses of engineered cells (e.g., T cells) and/or compositions thereof, for the treatment of subjects having a disease or condition, which generally is or includes a cancer or a tumor, such as a leukemia or a lymphoma, most particularly chronic lymphocytic leukemia (CLL), or small lymphocytic lymphoma (SLL). In some disclosures, the methods and uses provide for or achieve improved response and/or more durable responses or efficacy and/or a reduced risk of toxicity or other side effects, e.g., in particular groups of subjects treated, as compared to certain alternative methods. In some instances disclosed herein, the methods are advantageous by virtue of the administration of specified numbers or relative numbers of the engineered cells, the administration of defined ratios of particular types of the cells, treatment of particular patient populations, such as those having a particular risk profile, staging, and/or prior treatment history, and/or combinations thereof.

Also disclosed are methods that include assessing particular parameters, e.g., expression of specific biomarkers or analytes, that can be correlated with development of toxicity, and methods for treatment, e.g., intervention therapy, to prevent and/or ameliorate toxicities. Also disclosed are methods that involve assessing particular parameters, e.g., expression of specific biomarkers or analytes, that can be correlated with an outcome, such as a therapeutic outcome, including a response, such as a complete response (CR) or a partial response (PR); or a safety outcome, such as a development of a toxicity, for example, neurotoxicity or CRS, after administration of an immunotherapy and/or cell therapy. Also disclosed are methods to assess the likelihood of response and/or likelihood of risk of toxicity, based on assessment of the parameters, such as expression of biomarkers or analytes. Also disclosed are compositions for use in cell therapy. Also disclosed are articles of manufacture and kits, e.g., for use in the methods disclosed herein. In some instances disclosed herein, the articles of manufacture and kits optionally contain instructions for using, according to the methods disclosed herein.

In particular, among instances disclosed herein are methods of treating subjects with CLL or SLL. In some disclosures, CLL is considered an incurable disease, and subjects eventually relapse or become refractory to available therapies or treatments. In some of any instances disclosed herein, the subjects have a high risk disease. In some instances disclosed herein, the subjects have a high risk CLL or SLL. In some instances disclosed herein, existing treatment strategies for high risk and very high risk subjects may include fludarabine, cyclophosphamide, and rituximab (FCR), Bruton's tyrosine kinase (BTK) inhibitors (e.g. ibrutinib), and/or allogeneic stem cell transplantation. (Puiggros et al., BioMed Research International, Volume 2014 (2014), Article ID 435983). Many of the existing therapies include oral-targeted drugs, which have, for some patients with CLL, improved treatment outcomes. Nonetheless, some patients prove intolerant or resistant to therapy and/or fail to achieve complete response with undetectable MRD (uMRD). In some disclosures, subjects who have progressive disease after treatment with available therapies have poor outcomes. For instance, in some disclosures, subjects treated for CLL exhibit poor long-term outcomes. For example, in some instances disclosed herein, refractory (R/R) high-risk CLL subjects exhibit poor survival after ibrutinib discontinuation (Jain et al. (2015) Blood 125(13):2062-2067). There is a need for improved methods of treating CLL, and in some disclosures, for those appropriate for treating high and/or very high-risk CLL and/or subjects having relapsed or become refractory to multiple prior therapies.

In some instances disclosed herein, the methods include administration of cells to a subject selected or identified as having a certain prognosis or risk of CLL. Chronic lymphocytic leukemia (CLL) is a generally a variable disease. Some subjects with CLL may survive without treatment while others may require immediate intervention. In some instances disclosed herein, subjects with CLL may be classified into groups that may inform disease prognosis and/or recommended treatment strategy. In some instances disclosed herein, these groups may be "low risk," "intermediate risk," "high risk," and/or "very high risk" and patients may be classified as such depending on a number of factors including, but not limited to, genetic abnormalities and/or morphological or physical characteristics. In some instances disclosed herein, subjects treated in accord with the method are classified or identified based on the risk of CLL. In some instances disclosed herein, the subject is one that has high risk CLL.

In some instances disclosed herein, the disclosed methods and uses provide for or achieve improved or more durable responses or efficacy as compared to certain alternative methods, such as in particular groups of subjects treated, such as in patients with a leukemia, such as CLL or SLL, including those with high-risk disease. In some instances disclosed herein, the methods are advantageous by virtue of administering T cell therapy, such as a composition including cells for adoptive cell therapy, e.g., such as a CAR-expressing T cells, e.g. anti-CD19 CAR+ T cells. In some instances disclosed herein, the methods also include, prior to the T cell therapy, a lymphodepleting therapy, e.g. such as cyclophosphamide, fludarabine, or combinations thereof.

In some instances, the disclosures are based on observations, such as those described in the Examples disclosed herein, that the disclosed methods can be used to achieve a high response rate with high durability, compared to certain available methods for cell therapy, without an increased risk of toxicity. In some instances disclosed herein, the disclosed methods permit prolonged persistence of adoptively transferred cells for cell therapy, and/or low rate of developing toxicity in the subject. In some instances disclosed herein, the methods can be used to select subjects for treatment with cell therapy that are likely or more likely to respond to the therapy and/or to determine appropriate doses or dosing regimen for higher response rate and/or more durable response, while minimizing the risk of toxicity. Such methods can inform rational strategies to facilitate the safe and effective clinical application of adoptive cell therapy, such as CAR-T cell therapy.

In some instances disclosed herein, the disclosed methods achieve a high response rate in a heavily pretreated population of subjects with high-risk CLL (or SLL), all of whom have received one or more prior therapies including ibrutinib. In some instances disclosed herein, the treated subjects include subjects that have relapsed following initial remission on ibrutinib or who are refractory or intolerant to treatment with ibrutinib. In particular instances disclosed herein, the treated subjects include subjects that have relapsed following remission or are refractory or intolerant to one or more further prior therapy in addition to ibrutinib, such as 1, 2, 3, 4, 5 or more prior therapies. In some instances disclosed herein, the subjects have relapsed or are refractory to both a prior treatment of ibrutinib and venetoclax. In some instances disclosed herein, subjects that are refractory to such treatment have progressed following one or more prior therapy. In some instances disclosed herein, subjects treated, including those treated with one or more prior therapies (e.g. ibrutinib and/or venetoclax), include those with a high-risk cytogenetics, including TP53 mutation, complex karyotype (i.e. at least three chromosomal alterations) and del17(p). In some instances disclosed herein, subjects for treatment in accordance with the instances disclosed herein include subjects that have failed both a BTK inhibitor (e.g., ibrutinib) and venetoclax. As demonstrated herein, results from an ongoing clinical trial demonstrate a high overall response rate (ORR) of greater than 65% of subjects treated across dose-levels, including complete remission (CR) with incomplete blood count recovery (CRi) in greater than 35% of subjects treated. Of such subjects, all have been previously treated with ibrutinib and approximately half have been previously treated with ibrutinib and venetoclax. In some disclosures, the results are associated with achievement of undetectable MRD (uMRD); achievement of uMRD has been reported to correlate with improved outcomes (Kovacs et al. (2016) J. Clin. Oncol., 34:3758-3765; Thompson and Wierda (2016) Blood, 127:279-286). In some instances disclosed herein, the disclosed methods result in a high percentage of sustained responses that continue without progression for greater than 1 month, greater than 3 months, greater than six months or more.

Such results achieved in high risk subjects are superior compared to certain other alternative therapies. In particular, CLL is generally considered to be incurable and patients often eventually relapse or become refractory to available therapies (Dighiero and Hamblin (2008) The Lancet, 371:1017-1029). In some instances disclosed herein, CR and uMRD are inadequate and/or subjects progress or have poor outcomes following treatment with certain other agents, such as single-agent ibrutinib, venetoclax-Rituximab, Bendamustine-Rituximab or both ibrutinib and venetoclax. Further, reports have indicated that certain other CAR T-cell therapies may not achieve such durable response rates.

In some instances disclosed herein, the methods and uses include administering to the subject cells expressing genetically engineered (recombinant) cell surface receptors in adoptive cell therapy, which generally are chimeric receptors such as chimeric antigen receptors (CARs), recognizing an antigen expressed by, associated with and/or specific to the leukemia or lymphoma and/or cell type from which it is derived. In particular instances disclosed herein, the targeted antigen is CLL. The cells are generally administered in a composition formulated for administration; the methods generally involve administering one or more doses of the cells to the subject, which dose(s) may include a particular number or relative number of cells or of the engineered cells, and/or a defined ratio or compositions of two or more sub-types within the composition, such as CD4 vs.CD8 T cell.

In particular instances disclosed herein, methods are carried out with a therapeutic T cell product involving the separate administration of CD4+ and CD8+ CAR T cell compositions administered at a particular or precise number as a flat dose and/or as a defined ratio of CD4+ and CD8+ CAR T cells. In some instances disclosed herein, methods include producing or engineering the CAR T cell composition by a process that includes the separate isolation, selection or enrichment of CD4+ and CD8+ T cells from a biological sample. In some instances disclosed herein, methods for producing a CAR-T cell composition that includes enrichment of CD4+ and CD8+ T cells avoids the risk of including tumor cells in the CAR-T cell product or during the manufacturing of the CAR-T cell product. Compared to other diseases, CLL is a cancer in which the tumor cells are found in the periphery, which, in some contexts may interfere with and/or impact the efficacy of a CAR-T product that may include such cells or be derived from an initial composition containing such cells.

The section headings used herein are for organizational purposes only and are not to be construed as limiting the subject matter described.

### I. METHODS AND USES OF CELL THERAPY WITH GENETICALLY ENGINEERED CELLS

In some instances disclosed herein, the methods and uses disclosed herein include administering to the subject cells expressing genetically engineered (recombinant) cell surface receptors in adoptive cell therapy, which generally are chimeric receptors such as chimeric antigen receptors (CARs), recognizing an antigen expressed by, associated with and/or specific to the leukemia or lymphoma and/or cell type from which it is derived. The cells are generally administered in a composition formulated for administration; the methods generally involve administering one or more doses of the cells to the subject, which dose(s) may include a particular number or relative number of cells or of the engineered cells, and/or a defined ratio or compositions of two or more sub-types within the composition, such as CD4 vs.CD8 T cells.

In some instances disclosed herein, the cells, populations, and compositions are administered to a subject having the particular disease or condition to be treated, e.g., via adoptive cell therapy, such as adoptive T cell therapy. In some instances disclosed herein, the methods involve treating a subject having a lymphoma or a leukemia, such as a chronic lymphocytic leukemia (CLL) or small lymphocytic lymphoma (SLL) with a dose of antigen receptor-expressing cells (e.g. CAR-expressing cells).

In some instances disclosed herein, the disclosed methods involve treating a specific group or subset of subjects, e.g., subjects identified as having high-risk disease, e.g., high-risk CLL. In some disclosures, the methods treat subjects having a form of aggressive and/or poor prognosis CLL, such as CLL that has relapsed or is refractory (R/R) to standard therapy and has a poor prognosis. In some instances disclosed herein, the subject has failed one or more prior therapies. In some instances disclosed herein, the subject is ineligible for other prior therapy. In some instances disclosed herein, the subject has failed a prior therapy with a Bruton's Tyrosine Kinase inhibitor (BTKi), such as ibrutinib. In some instances disclosed herein, the subject has failed ibrutinib and venetoclax. In some instances disclosed herein, the overall response rate (ORR; also known in some cases as objective response rate) to available therapies, to a standard of care, or to a reference therapy for the disease and/or patient population for which the therapy is indicated, is less than 40% and/or the complete response (CR; also known in some cases as complete remission) is less than 20%.

In some instances disclosed herein, the methods, uses and articles of manufacture involve, or are used for treatment of subjects involving, selecting or identifying a particular group or subset of subjects, e.g., based on specific types of disease, diagnostic criteria, prior treatments and/or response to prior treatments. In some instances disclosed herein, the methods involve treating a subject having relapsed following remission after treatment with, or become refractory to, one or more prior therapies; or a subject that has relapsed or is refractory (R/R) to one or more prior therapies, e.g., one or more lines of standard therapy. In some instances disclosed herein, the methods involve treating subjects having chronic lymphocytic leukemia. In some instances disclosed herein, the methods involve treating subjects having small lymphocytic lymphoma. In some instances disclosed herein, the methods involve treating a subject that has an Eastern Cooperative Oncology Group Performance Status (ECOG) of 0-1. In some instances disclosed herein, the methods treat a poor-prognosis population of CLL patients or subject thereof that generally responds poorly to therapies or particular reference therapies, such as one having high-risk cytogenetics (i.e., Del(17p), TP53 mutation, mutated IGHV, and complex karyotype).

In some instances disclosed herein, the antigen receptor (e.g. CAR) specifically binds to a target antigen associated with the disease or condition, such as associated with CLL. In some instances disclosed herein, the antigen receptor binds to a target antigen associated with SLL. In some instances disclosed herein, the antigen associated with the disease or disorder is CD 19.

In some instances disclosed herein, the methods include administration of the cells or a composition containing the cells to a subject, tissue, or cell, such as one having, at risk for, or suspected of having the disease, condition or disorder. In some instances disclosed herein, the subject is the subject is an adult. In some instances disclosed herein, the subject is over at or about 50, 60, or 70 years of age.

In some instances disclosed herein, the subject has been previously treated with a therapy or a therapeutic agent targeting the disease or condition, e.g., CLL or SLL, prior to administration of the cells expressing the recombinant receptor. In some instances disclosed herein, the subject has been previously treated with a hematopoietic stem cell transplantation (HSCT), e.g., allogeneic HSCT or autologous HSCT. In some instances disclosed herein, the subject has had poor prognosis after treatment with standard therapy and/or has failed one or more lines of previous therapy, for example at least at or about 1, 2, 3, 4 or more lines of previous therapy. In some instances disclosed herein, the subject has been treated or has previously received at least or about at least or about 1, 2, 3, or 4 other therapies for treating the CLL other than a lymphodepleting therapy and/or the dose of cells expressing the antigen receptor. In some instances disclosed herein, the subject has been previously treated with chemotherapy or radiation therapy. In some disclosures, the subject is refractory or non-responsive to the other therapy or therapeutic agent. In some instances disclosed herein, the subject has persistent or relapsed disease, e.g., following treatment with another therapy or therapeutic intervention, including chemotherapy or radiation. In some instances disclosed herein, the subjects have a relapsed or refractory (R/R) chronic lymphocytic leukemia (CLL) and had failed or are ineligible of a Bruton's Tyrosine Kinase inhibitor (BTKi) therapy.

In some instances disclosed herein, the subject has been previously treated with a therapy or a therapeutic agent targeting the disease or condition, e.g. CLL, prior to administration of the cells expressing the recombinant antigen receptor. In some instances disclosed herein, the therapeutic agent is a kinase inhibitor, such as an inhibitor of Bruton's tyrosine kinase (Btk), for example, ibrutinib. In some instances disclosed herein, the therapeutic agent is an inhibitor of B-cell lymphoma-2 (Bcl-2), for example, venetoclax. In some instances disclosed herein, the therapeutic agent is an antibody (e.g. monoclonal antibody) that specifically binds to an antigen expressed by the cells of the CLL or NHL, e.g. an antigen from any one or more of CD20, CD19, CD22, ROR1, CD45, CD21, CD5, CD33, Igkappa, Iglambda, CD79a, CD79b or CD30. In some instances disclosed herein, the therapeutic agent is an anti-CD20 antibody, e.g., rituximab. In some instances disclosed herein, the therapeutic agent is a depleting chemotherapy that is a combination therapy that includes rituximab, e.g., a combination therapy of fludarabine and rituximab or a combination therapy of anthracycline and rituximab. In some instances disclosed herein, the subject has been previously treated with hematopoietic stem cell transplantation (HSCT), e.g., allogenic HSCT or autogenic HSCT. In some instances disclosed herein, the subject has been treated or has previously received at least or about at least or about 1, 2, 3, or 4 other therapies for treating the CLL other than the lymphodepleting therapy and/or the dose of cells expressing the antigen receptor. In some instances disclosed herein, the subject has been previously treated with chemotherapy or radiation therapy.

In some disclosures, the subject is refractory or non-responsive to the other therapy or therapeutic agent. In some instances disclosed herein, the subject has persistent or relapsed disease, e.g., following treatment with another therapy or therapeutic intervention, including chemotherapy or radiation.

In some instances disclosed herein, the subject is one that is eligible for a transplant, such as is eligible for a hematopoietic stem cell transplantation (HSCT), e.g., allogeneic HSCT. In some such instances disclosed herein, the subject has not previously received a transplant, despite being eligible, prior to administration of the engineered cells (e.g. CAR-T cells) or a composition containing the cells to the subject as disclosed herein.

In some instances disclosed herein, the subject is one that is not eligible for a transplant, such as is not eligible for a hematopoietic stem cell transplantation (HSCT), e.g., allogeneic HSCT. In some instances disclosed herein, such a subject is administered the engineered cells (e.g. CAR-T cells) or a composition containing the cells according to the instances disclosed herein herein.

In some instances disclosed herein, the methods include administration of cells to a subject selected or identified as having high-risk CLL. In some instances disclosed herein, the subject exhibits one or more cytogenetic abnormalities, such as associated with high-risk CLL. In some disclosures, the population to be treated includes subjects having an Eastern Cooperative Oncology Group Performance Status (ECOG) that is anywhere from 0-1.

In some of the instances disclosed herein, the subjects to be treated have failed two or more prior therapies. In some of the instances disclosed herein, the subject to be treated has failed three of more prior therapies. In some instances disclosed herein, the prior therapies include any of a therapy with an inhibitor of Bruton's tyrosine kinase (BTK), such as ibrutinib; venetoclax; a combination therapy comprising fludarabine and rituximab; radiation therapy; and hematopoietic stem cell transplantation (HSCT). In some instances disclosed herein, the subject or patient has previously received but has relapsed following remission, is refractory to, has failed and/or is intolerant to treatment with ibrutinib and/or venetoclax. In some instances disclosed herein, the subject or patient has previously received but has relapsed following remission, is refractory to, has failed and/or is intolerant to treatment with ibrutinib and venetoclax.

In some instances disclosed hereinare methods of treating subjects selected or identified as having relapsed following remission or who are refractory to prior treatment with ibrutinib and venetoclax for treating the CLL or SLL. In some disclosures, the selected or identified subjects are administered a CAR T-cell therapy, e.g. anti-CD19 CAR-T cell therapy, in accord with the disclosed methods.

In some instances disclosed herein, the subject has never achieved a complete response (CR), never received autologous stem cell transplant (ASCT), is refractory to 1 or more second line therapy, has primary refractory disease, and/or has an ECOG performance score that is between 0 and 1.

In some disclosures, subjects to be treated in accordance with the instances disclosed herein include subjects with a diagnosis of CLL or SLL. In some instances disclosed herein, subjects with CLL include those with CLL diagnosis with indication of treatment based on the International Workshop on Chronic Lymphocytic Leukemia (iwCLL) guidelines and clinical measurable disease (bone marrow involvement by > 30% lymphocytes, peripheral blood lymphocytosis > 5×10⁹/L, and/or measurable lymph nodes and/or hepatic or splenomegaly. In some instances disclosed herein, subjects with SLL include those with SLL diagnosis is based on lymphadenopathy and/or splenomegaly and < 5×10⁹ CD19+ CD5+ clonal B lymphocytes/L [< 5000/µL] in the peripheral blood at diagnosis with measurable disease defined as at least one lesion > 1.5 cm in the greatest transverse diameter, and that is biopsy-proven SLL.

In some disclosures, the subjects are either ineligible for treatment with Bruton's tyrosine kinase inhibitor (BTKi, e.g., ibrutinib) due to a requirement for full-dose anticoagulation or history or arrhythmia, or had failed treatment after having been previously administered BTKi as determined by stable disease (SD) or progressive disease (PD) as best response, PD after previous response, or discontinuation due to intolerance (e.g. unmanageable toxicity). In some disclosures, the subjects are treated in accordance with the instances disclosed herein if they had high risk disease (as determined by complex cytogenetic abnormalities (e.g., complex karyotype), del(17p), TP53 mutation, unmutated IGVH) and had failed greater than or equal to (e.g., at least) 2 prior therapies; or if they had standard-risk disease and had failed greater than or equal to (e.g., at least) 3 prior therapies. In some disclosures, subjects to be treated in accordance with the instances disclosed herein exclude subjects with active untreated CNS disease, ECOG >1, or Richter's transformation.

In some disclosures, disclosed are compositions, methods and uses for administration of a defined composition of the cell therapy, at particular doses, that are associated with a high response rate and/or high durability of response, and low levels and/or incidence of toxicity. In some instances disclosed herein, the composition or dose administered is a flat and/or fixed dose, such as a precise flat dose, of cells and/or of one or more cells having a particular phenotype, such as a particular number of such cells or a number that is within a particular range and/or degree of variability or variance as compared to a target number. In some instances disclosed herein, the composition or dose administered contains a defined ratio of CD4⁺ and CD8⁺ cells (e.g., 1:1 ratio of CD4⁺:CD8⁺ CAR⁺ T cells) and/or contains a ratio that is within a certain degree of variability from such ratio, such as no more than ± 10%, such as no more than ± 8%, such as a degree of variability or variance of no more than ± 10%, such as no more than ± 8%. In some instances disclosed herein, the CD4⁺ and CD8⁺ cells are individually formulated and administered. In some instances disclosed herein, the administered cells exhibit consistent activity and/or function, e.g., cytokine production, apoptosis and/or expansion. In some instances disclosed herein, the disclosedcompositions exhibit highly consistent and defined activity, and low variability between cells, e.g., in terms of cell number, cell function and/or cell activity, in the composition or between preparations. In some instances disclosed herein, the consistency in activity and/or function, e.g., low variability between preparations of compositions, allows improved efficacy and/or safety. In some instances disclosed herein, administration of the defined compositions resulted in low product variability and low toxicity, e.g., CRS or neurotoxicity, compared to administration of cell compositions with high heterogeneity. In some instances disclosed herein, the defined, consistent composition also exhibits consistent cell expansion. Such consistency can facilitate the identification of dose, therapeutic window, evaluation of dose response and identification of factors of the subject that may correlate with safety or toxicity outcomes.

In some instances disclosed herein, in a certain cohort of subjects receiving a single infusion of a particular dose level, the subjects in some cohorts can achieve an overall response rate (ORR, in some cases also known as objective response rate) of more than 80%, a complete response (CR) rate of more than 50% at 3 months. In some instances disclosed herein, subjects receiving a defined dose show improved safety outcomes. In some disclosures, the rate of severe CRS or severe NT is low. In some instances disclosed herein, particular factors of the subject, e.g., certain biomarkers (e.g. TNF-alpha or IL-16), can be used to predict the risk of toxicity. In some disclosures, the instances disclosed herein can be used to achieve high response rate with low risk of toxicity.

In some instances disclosed herein, no more than 25%, no more than 20%, no more than 15%, no more than 10% or no more than 5% of subjects treated using the disclosed compositions, articles of manufacture, kits, methods and uses are administered an agent (e.g. tocilizumab and/or dexamethasone) to ameliorate, treat or prevent a toxicity, either prior to or subsequent to administration of the cell therapy. In some instances disclosed herein, the subject is not administered any prophylaxis treatment prior to receiving the engineered cells (e.g. CAR-T cells).

In some instances disclosed herein, the disclosuresprovide an advantage, e.g., permits administration of the cell therapy on an outpatient basis. In some disclosures, the administration of the cell therapy, e.g. dose of T cells in accord with the instances disclosed herein, can be performed on an outpatient basis or does not require admission to the subject to the hospital, such as admission to the hospital requiring an overnight stay. In some instances disclosed herein, such outpatient administration can allow increased access and decreased costs, while maintaining a high, durable response rate with low toxicity. In some disclosures, outpatient treatment can be advantageous for patients who already are otherwise immunocompromised by prior treatments, e.g. post-lymphodepletion, and are at a greater risk for exposures at a hospital stay or in an in-patient setting. In some disclosures, outpatient treatments also increases options for treatment for subjects who may not have access to in-patient, hospital settings, or transplant centers, thereby expanding access to the treatment.

In some instances disclosed herein, the methods and uses provide for or achieve a higher response rate and/or more durable responses or efficacy and/or a reduced risk of toxicity or other side effects that can be associated with cell therapy, such as neurotoxicity (NT) or cytokine release syndrome (CRS). In some disclosures, the disclosed observations indicated a low rate of severe NT (sNT) or severe CRS (sCRS), and a high rate of patients without any toxicities, e.g., NT or CRS.

In some instances disclosed herein, at least at least 45%, at least 50%, at least 55%, at least 60%, at least 65%, at least 70%, or at least 75% or more of the subjects treated according to the disclosed methods, and/or with the disclosed articles of manufacture or compositions, achieve a complete response (CR). In some instances disclosed herein, at least 75%, at least 80%, or at least 90% of the subjects treated according to the disclosed methods, and/or with the disclosed articles of manufacture or compositions, achieve an objective response (OR). In some instances disclosed herein, at least 35%, at least 45%, at least 50%, at least 55%, at least 60% or more of the subjects treated according to the disclosed methods, and/or with the disclosed articles of manufacture or compositions, achieve a CR or OR by one month, by two months or by three months. In some instances disclosed herein, at least at least 45%, at least 50%, at least 55%, at least 60%, at least 65%, at least 70%, or at least 75% or more of subjects that had failed prior treatment with a Bruton's Tyrosine Kinase inhibitor (BTKi) and venetoclax treated according to the disclosed methods, and/or with the disclosed articles of manufacture or compositions, achieve a complete response (CR). In some instances disclosed herein, at least 75%, at least 80%, or at least 90% of the subjects that had failed prior treatment with a BTKi and venetoclax treated according to the disclosed methods, and/or with the disclosed articles of manufacture or compositions, achieve an objective response (OR). In some instances disclosed herein, at least 35%, at least 45%, at least 50%, at least 55%, at least 60% or more of the subjects that had failed prior treatment with a BTKi and venetoclax treated according to the disclosed methods, and/or with the disclosed articles of manufacture or compositions, achieve a CR or OR by one month, by two months or by three months. In some instances disclosed herein, in the subjects treated according to the method, greater than 50%, greater than 60%, or greater than 70% had undetectable minimal residual disease (MRD) for at least one month, at least two months, at least three months or at least 6 month after administering the dose of cells. In some instances disclosed herein, in the subjects that had failed prior treatment with a BTKi and venetoclax treated according to the methods, and/or with the disclosed articles of manufacture or compositions, greater than 50%, greater than 60%, or greater than 70% had undetectable minimal residual disease (MRD) for at least one month, at least two months, at least three months or at least 6 month after administering the dose of cells.

In some instances disclosed herein, by three months after initiation of administration of the cell therapy, at least 55%, at least 60%, at least 65%, at least 70%, at least 75%, at least 80%, at least 85% or more of the subjects treated according to the disclosed methods, and/or with the disclosed articles of manufacture or compositions, remain in response, such as remain in CR or OR and/or have undetectable MRD. In some instances disclosed herein, such response, such as CR or OR, is durable for at least three months. In some instances disclosed herein, by three months after initiation of administration of the cell therapy, at least 55%, at least 60%, at least 65%, at least 70%, at least 75%, at least 80%, at least 85% or more of the subjects that had failed prior treatment with a BTKi and venetoclax treated according to the disclosed methods, and/or with the disclosed articles of manufacture or compositions, remain in response, such as remain in CR or OR and/or have undetectable MRD. In some instances disclosed herein, such response, such as CR or OR, is durable for at least three months.

In some instances disclosed herein, the resulting response observed in such subjects by the treatment in accord with the disclosed methods, and/or with the disclosed articles of manufacture or compositions, is associated with or results in a low risk of any toxicity or a low risk of severe toxicity in a majority of the subjects treated. In some instances disclosed herein, greater than or greater than about 30%, 35%, 40%, 50%, 55%, 60% or more of the subjects treated according to the disclosed methods and/or with the disclosed articles of manufacture or compositions do not exhibit any grade of CRS or any grade of neurotoxicity (NT). In some instances disclosed herein, greater than or greater than about 50%, 60%, 70%, 80% or more of the subjects treated according to the disclosed methods and/or with the disclosed articles of manufacture or compositions do not exhibit severe CRS or grade 3 or higher CRS. In some instances disclosed herein, greater than or greater than about 50%, 60%, 70%, 80% or more of the subjects treated according to the disclosed methods, and/or with the disclosed articles of manufacture or compositions, do not exhibit severe neurotoxicity or grade 3 or higher neurotoxicity, such as grade 4 or 5 neurotoxicity.

In some instances disclosed herein, the resulting response observed in such subjects that had failed prior treatment with a BTKi and venetoclax by the treatment in accord with the disclosed methods, and/or with the disclosed articles of manufacture or compositions, is associated with or results in a low risk of any toxicity or a low risk of severe toxicity in a majority of the subjects treated. In some instances disclosed herein, greater than or greater than about 30%, 35%, 40%, 50%, 55%, 60% or more of the subjects that had failed prior treatment with a BTKi and venetoclax treated according to the disclosed methods and/or with the disclosed articles of manufacture or compositions do not exhibit any grade of CRS or any grade of neurotoxicity (NT). In some instances disclosed herein, greater than or greater than about 50%, 60%, 70%, 80% or more of the subjects that had failed prior treatment with a BTKi and venetoclax treated according to the disclosed methods and/or with the disclosed articles of manufacture or compositions do not exhibit severe CRS or grade 3 or higher CRS. In some instances disclosed herein, greater than or greater than about 50%, 60%, 70%, 80% or more of the subjects that had failed prior treatment with a BTKi and venetoclax treated according to the disclosed methods, and/or with the disclosed articles of manufacture or compositions, do not exhibit severe neurotoxicity or grade 3 or higher neurotoxicity, such as grade 4 or 5 neurotoxicity.

In some instances disclosed herein, at least at or about 45%, 50%, 60%, 65%, 70%, 75%, 80%, 85%, 90%, or 95% of subjects treated according to the method and/or with the disclosed articles of manufacture or compositions do not exhibit early onset CRS or neurotoxicity and/or do not exhibit onset of CRS earlier than 1 day, 2 days, 3 days or 4 days following initiation of the administration. In some instances disclosed herein, at least at or about 45%, 50%, 60%, 65%, 70%, 75%, 80%, 85%, 90%, or 95% of subjects treated according to the methods, and/or with the disclosed articles of manufacture or compositions, do not exhibit onset of neurotoxicity earlier than 3 days, 4 days, 5 days, six days or 7 days following initiation of the administration. In some disclosures, the median onset of neurotoxicity among subjects treated according to the methods, and/or with the disclosed articles of manufacture or compositions, is at or after the median peak of, or median time to resolution of, CRS in subjects treated according to the method. In some instances disclosed herein, the median onset of neurotoxicity among subjects treated according to the method is greater than at or about 8, 9, 10, or 11 days.

In some instances disclosed herein, at least at or about 45%, 50%, 60%, 65%, 70%, 75%, 80%, 85%, 90%, or 95% of subjects that had failed prior treatment with a BTKi and venetoclax, treated according to the method and/or with the disclosed articles of manufacture or compositions compositions that had failed prior treatment with a BTKi and venetoclax,do not exhibit early onset CRS or neurotoxicity and/or do not exhibit onset of CRS earlier than 1 day, 2 days, 3 days or 4 days following initiation of the administration. In some instances disclosed herein, at least at or about 45%, 50%, 60%, 65%, 70%, 75%, 80%, 85%, 90%, or 95% of subjects that had failed prior treatment with a BTKi and venetoclax, treated according to the methods, and/or with the disclosed articles of manufacture or compositions, do not exhibit onset of neurotoxicity earlier than 3 days, 4 days, 5 days, six days or 7 days following initiation of the administration. In some disclosures, the median onset of neurotoxicity among subjects that had failed prior treatment with a BTKi and venetoclax, treated according to the methods, and/or with the disclosed articles of manufacture or compositions, is at or after the median peak of, or median time to resolution of, CRS in subjects treated according to the method. In some instances disclosed herein, the median onset of neurotoxicity among subjects that had failed prior treatment with a BTKi and venetoclax, treated according to the method is greater than at or about 8, 9, 10, or 11 days.

In some instances disclosed herein, such results are observed following administration of from or from about 2.5 × 10⁷ to at or about 1.5 × 10⁸, such as from about 5 × 10⁷ to at or about 1 × 10⁸ total recombinant receptor-expressing T cells (e.g. CAR+ T cells), such as a dose of T cells including CD4⁺ and CD8⁺ T cells administered at a defined ratio as described herein, e.g. at or about a 1:1 ratio, and/or at a precise or flat or fixed number of CAR⁺ T cells, or precise or flat or fixed number of a particular type of CAR⁺ T cells such as CD4⁺CAR⁺ T cells and/or CD8⁺CAR⁺ T cells, and/or a number of any of such cells that is within a specified degree of variance, such as no more than, + or - (plus or minus, in some cases indicated as ±), 5, 6, 7, 8, 9, 10, 11, 12, 13, 14, or 15% as compared to such precise or flat or fixed number. In some instances disclosed herein, such flat or fixed number of cells is at or about 2.5 × 10⁷ total CAR⁺ T cells or of CD8⁺ and/or CD4⁺ CAR⁺ T cells, 5×10⁷ total CAR⁺ T cells or of CD8⁺ and/or CD4⁺ CAR⁺ T cells, or 1 × 10⁸ total CAR⁺ T cells or of CD8⁺ and/or CD4⁺ CAR⁺ T cells. In some instances disclosed herein, the number of cells in the dose includes or consists of or consists essentially of 2.5 × 10⁷ CAR⁺ T cells (optionally 1.25 × 10⁷ CD4⁺CAR⁺ T cells and 1.25 × 10⁷ CD8⁺CAR⁺ T cells); in some instances disclosed herein, it includes or consists of or consists essentially of 5 × 10⁷ CAR⁺ T cells (optionally 2.5 × 10⁷ CD4⁺CAR⁺ T cells and 2.5 × 10⁷ CD8⁺CAR⁺ T cells); in some instances disclosed herein, it includes 1 × 10⁸ CAR+ T cells (optionally 0.5 × 10⁸ CD4⁺CAR⁺ T cells and 0.5 × 10⁸ CD8⁺CAR⁺ T cells). In some disclosures, the number of cells administered, is within a certain degree of variance of such numbers in the aforementioned instances disclosed herein, such as within plus or minus (±) 5, 6, 7, 8, 9, or 10%, such as within plus or minus 8%, as compared to such number(s) of cells. In some disclosures, the dose is within a range in which a correlation is observed (optionally a linear relationship) between the number of such cells (e.g., of total CAR⁺ T cells or of CD8⁺ and/or CD4⁺ CAR⁺ T cells) and one or more outcomes indicative of therapeutic response, or duration thereof (e.g., likelihood of achieving a remission, a complete remission, and/or a particular duration of remission) and/or duration of any of the foregoing. In some disclosures, it is found that the higher dose of cells administered can result in greater response without or without substantially impacting or affecting the incidence or risk of toxicity (e.g. CRS or neurotoxicity), or degree of incidence or risk of toxicity, in the subject e.g. severe CRS or severe neurotoxicity.

In some instances disclosed herein, the subject to be treated in accordance with the disclosures have adequate organ function. For example, in some disclosures, the subjects exhibit one or more of the following: serum creatinine ≤ 1.5 × age-adjusted upper limit of normal (ULN) or calculated creatinine clearance (Cockcroft and Gault) > 30 mL/min; alanine aminotransferase (ALT) ≤ 5 × ULN and total bilirubin < 2.0 mg/dL (or < 3.0 mg/dL for subjects with Gilbert's syndrome or leukemic infiltration of the liver); adequate pulmonary function, defined as ≤ Common Terminology Criteria for Adverse Events (CTCAE) Grade 1 dyspnea and saturated oxygen (SaO₂) ≥ 92% on room air; and/or adequate cardiac function, defined as left ventricular ejection fraction (LVEF) ≥ 40% as assessed by echocardiogram (ECHO) or multiple uptake gated acquisition (MUGA) scan performed within 30 days prior to assessment of the subjects for administration of the engineered cell composition.

In some instances disclosed herein, the disclosed methods can achieve a high or a particular rate of response (such as a rate of response among a population as assessed after a certain period post-administration, such as one month or three months), e.g., ORR (such as a 1-month or 3-month ORR) of at or about 75% or more, 80% or more, 85% or more, and CR rate (such as a 1-month or 3-month CR rate) of at or about 30% or more, 35% or more, 40% or more, 45% or more, 50% or more, 55% or more, 60% or more, 65% or more, 70% or more, 71% or more, 72% or more, 73% or more, 74% or more or approximately 75% or more. In some instances disclosed herein, such rates of response and durability are received following only a single administration or dose of such therapy. Treatment of such subjects by the disclosed methods, and/or with the disclosed articles of manufacture or compositions, in some instances disclosed herein, also result in the subjects achieving the high rate of response, yet not exhibiting higher incidence of developing toxicities, such as neurotoxicity or CRS, even at a higher cell dosage.

Thus, in some instances disclosed herein, the disclosed methods, articles of manufacture and/or compositions, can offer advantages over other available methods or solutions or approaches for treatment such as for adoptive cell therapy. In particular, among the instances disclosed herein are those that offer an advantage for subjects with high-risk CLL, by achieving a durable response at a high rate, with reduced incidence of toxicities or side effects.

### A. Method of Treatment

Any references herein to methods of treatment are intended to refer to products for use in said methods.

Disclosed herein are methods of treatment that involve administering engineered cells or compositions containing engineered cells, such as engineered T cells. Also disclosed are methods and uses of engineered cells (e.g., T cells) and/or compositions thereof, including methods for the treatment of subjects having a disease or condition such as a leukemia or a lymphoma, e.g., a chronic lymphocytic leukemia (CLL) or a small lymphocytic lymphoma (SLL), that involves administration of the engineered cells and/or compositions thereof. In some instances disclosed herein, the disclosed methods and uses can achieve improved response and/or more durable responses or efficacy and/or a reduced risk of toxicity or other side effects, e.g., in particular groups of subjects treated, as compared to certain alternative methods. In some disclosures, also disclosed are methods of administering engineered cells or compositions containing engineered cells, such as engineered T cells, to a subject, such as a subject that has a disease or disorder. In some disclosures, also disclosed are uses of engineered cells or compositions containing engineered cells, such as engineered T cells for treatment of a disease or disorder. In some disclosures, also disclosed are uses of engineered cells or compositions containing engineered cells, such as engineered T cells for the manufacture of a medicament for the treatment of a disease or disorder. In some disclosures, also disclosed are methods of administering engineered cells or compositions containing engineered cells, such as engineered T cells, for use in treatment of a disease or disorder, or for administration to a subject having a disease or disorder. In some disclosures, the uses of the engineered cells or compositions containing engineered cells, such as engineered T cells are in accord with any of the methods described herein.

The engineered cells expressing a recombinant receptor, such as a chimeric antigen receptor (CAR), or compositions comprising the same, described herein are useful in a variety of therapeutic, diagnostic and prophylactic indications. For example, the engineered cells or compositions comprising the engineered cells are useful in treating a variety of diseases and disorders in a subject. Such methods and uses include therapeutic methods and uses, for example, involving administration of the engineered cells, or compositions containing the same, to a subject having a disease, condition, or disorder, such as a tumor or cancer. In some instances disclosed herein, the engineered cells or compositions comprising the same are administered in an effective amount to effect treatment of the disease or disorder. Uses include uses of the engineered cells or compositions in such methods and treatments, and in the preparation of a medicament in order to carry out such therapeutic methods. In some instances disclosed herein, the engineered cells or compositions comprising the engineered cells are for use in treating a variety of diseases and disorders in a subject, for example, in accordance with the therapeutic methods. In some instances disclosed herein, the methods are carried out by administering the engineered cells, or compositions comprising the same, to the subject having or suspected of having the disease or condition. In some instances disclosed herein, the methods thereby treat the disease or condition or disorder in the subject.

General methods for administration of cells for adoptive cell therapy are known and may be used in connection with the disclosed methods and compositions. For example, adoptive T cell therapy methods are described, e.g., in US Patent Application Publication No. 2003/0170238 to Gruenberg et al; US Patent No. 4,690,915 to Rosenberg; Rosenberg (2011) Nat Rev Clin Oncol. 8(10):577-85). See, e.g., Themeli et al. (2013) Nat Biotechnol. 31(10): 928-933; Tsukahara et al. (2013) Biochem Biophys Res Commun 438(1): 84-9; Davila et al. (2013) PLoS ONE 8(4): e61338.

The disease or condition that is treated can be any in which expression of an antigen is associated with and/or involved in the etiology of a disease condition or disorder, e.g. causes, exacerbates or otherwise is involved in such disease, condition, or disorder. Exemplary diseases and conditions can include diseases or conditions associated with malignancy or transformation of cells (e.g. cancer), autoimmune or inflammatory disease, or an infectious disease, e.g. caused by a bacterial, viral or other pathogen. Exemplary antigens, which include antigens associated with various diseases and conditions that can be treated, are described above. In particular instances disclosed herein, the chimeric antigen receptor or transgenic TCR specifically binds to an antigen associated with the disease or condition.

Among the diseases, conditions, and disorders are tumors, including solid tumors, hematologic malignancies, and melanomas, and including localized and metastatic tumors, infectious diseases, such as infection with a virus or other pathogen, e.g., HIV, HCV, HBV, CMV, HPV, and parasitic disease, and autoimmune and inflammatory diseases. In some instances disclosed herein, the disease, disorder or condition is a tumor, cancer, malignancy, neoplasm, or other proliferative disease or disorder. Such diseases for treatment according to the disclosed methods herein include but are not limited to leukemia, lymphoma, e.g., chronic lymphocytic leukemia (CLL) and small lymphocytic lymphoma (SLL).

In some instances disclosed herein, the Eastern Cooperative Oncology Group (ECOG) performance status indicator can be used to assess or select subjects for treatment, e.g., subjects who have had poor performance from prior therapies (see, e.g., Oken et al. (1982) Am J Clin Oncol. 5:649-655). The ECOG Scale of Performance Status describes a patient's level of functioning in terms of their ability to care for themselves, daily activity, and physical ability (e.g., walking, working, etc.). In some instances disclosed herein, an ECOG performance status of 0 indicates that a subject can perform normal activity. In some disclosures, subjects with an ECOG performance status of 1 exhibit some restriction in physical activity but the subject is fully ambulatory. In some disclosures, patients with an ECOG performance status of 2 is more than 50% ambulatory. In some instances disclosed herein, the subject with an ECOG performance status of 2 may also be capable of self-care; see e.g., Sørensen et al., (1993) Br J Cancer 67(4) 773-775. The criteria reflective of the ECOG performance status are described in **Table 1** below:

| **Table 1. ECOG Performance Status Criteria** | |
|---|---|
| **Grade** | **ECOG performance status** |
| 0 | Fully active, able to carry on all pre-disease performance without restriction |
| 1 | Restricted in physically strenuous activity but ambulatory and able to carry out work of a light or sedentary nature, e.g., light house work, office work |
| 2 | Ambulatory and capable of all self-care but unable to carry out any work activities; up and about more than 50% of waking hours |
| 3 | Capable of only limited self-care; confined to bed or chair more than 50% of waking hours |
| 4 | Completely disabled; cannot carry on any self-care; totally confined to bed or chair |
| 5 | Dead |

Antigens targeted by the receptors (e.g. CAR) in some instances disclosed herein include antigens associated with a B cell malignancy, such as any of a number of known B cell marker. In some instances disclosed herein, the antigen is or includes CD20, CD19, CD22, ROR1, CD45, CD21, CD5, CD33, Igkappa, Iglambda, CD79a, CD79b or CD30. In some instances disclosed herein, the antigen is CD19.

In some instances disclosed herein, the cell therapy, e.g., adoptive T cell therapy, is carried out by autologous transfer, in which the cells are isolated and/or otherwise prepared from the subject who is to receive the cell therapy, or from a sample derived from such a subject. Thus, in some disclosures, the cells are derived from a subject, e.g., patient, in need of a treatment and the cells, following isolation and processing are administered to the same subject.

In some instances disclosed herein, the cell therapy, e.g., adoptive T cell therapy, is carried out by allogeneic transfer, in which the cells are isolated and/or otherwise prepared from a subject other than a subject who is to receive or who ultimately receives the cell therapy, e.g., a first subject. In such instances disclosed herein, the cells then are administered to a different subject, e.g., a second subject, of the same species. In some instances disclosed herein, the first and second subjects are genetically identical. In some instances disclosed herein, the first and second subjects are genetically similar. In some instances disclosed herein, the second subject expresses the same HLA class or supertype as the first subject.

The cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some instances disclosed herein, they are administered by parenteral, intrapulmonary, and intranasal, and, if desired for local treatment, intralesional administration. Parenteral infusions include intramuscular, intravenous, intraarterial, intraperitoneal, or subcutaneous administration. In some instances disclosed herein, a given dose is administered by a single bolus administration of the cells. In some instances disclosed herein, it is administered by multiple bolus administrations of the cells, for example, over a period of no more than 3 days, or by continuous infusion administration of the cells. In some instances disclosed herein, administration of the cell dose or any additional therapies, e.g., the lymphodepleting therapy, intervention therapy and/or combination therapy, is carried out via outpatient delivery.

For the prevention or treatment of disease, the appropriate dosage may depend on the type of disease to be treated, the type of cells or recombinant receptors, the severity and course of the disease, whether the cells are administered for preventive or therapeutic purposes, previous therapy, the subject's clinical history and response to the cells, and the discretion of the attending physician. The compositions and cells are in some instances disclosed herein suitably administered to the subject at one time or over a series of treatments.

In some instances disclosed herein, the methods comprise administration of a chemotherapeutic agent, e.g., a conditioning chemotherapeutic agent.

Preconditioning subjects with immunodepleting (e.g., lymphodepleting) therapies in some disclosures can improve the effects of adoptive cell therapy (ACT).

Thus, in some instances disclosed herein, the methods include administering a preconditioning agent, such as a lymphodepleting or chemotherapeutic agent, such as cyclophosphamide, fludarabine, or combinations thereof, to a subject prior to the initiation of the cell therapy. For example, the subject may be administered a preconditioning agent at least 2 days prior, such as at least 3, 4, 5, 6, or 7 days prior, to the initiation of the cell therapy. In some instances disclosed herein, the subject is administered a preconditioning agent no more than 7 days prior, such as no more than 6, 5, 4, 3, or 2 days prior, to the initiation of the cell therapy.

In some instances disclosed herein, the subject is preconditioned with cyclophosphamide at a dose between or between about 20 mg/kg and 100 mg/kg, such as between or between about 40 mg/kg and 80 mg/kg. In some disclosures, the subject is preconditioned with or with about 60 mg/kg of cyclophosphamide. In some instances disclosed herein, the cyclophosphamide can be administered in a single dose or can be administered in a plurality of doses, such as given daily, every other day or every three days. In some instances disclosed herein, the cyclophosphamide is administered once daily for one or two days. In some instances disclosed herein, where the lymphodepleting agent comprises cyclophosphamide, the subject is administered cyclophosphamide at a dose between or between about 100 mg/m² and 500 mg/m², such as between or between about 200 mg/m² and 400 mg/m², or 250 mg/m² and 350 mg/m², inclusive. In some instances, the subject is administered about 300 mg/m² of cyclophosphamide. In some instances disclosed herein, the cyclophosphamide can be administered in a single dose or can be administered in a plurality of doses, such as given daily, every other day or every three days. In some instances disclosed herein, cyclophosphamide is administered daily, such as for 1-5 days, for example, for 3 to 5 days. In some instances, the subject is administered about 300 mg/m² of cyclophosphamide, daily for 3 days, prior to initiation of the cell therapy.

In some instances disclosed herein, where the lymphodepleting agent comprises fludarabine, the subject is administered fludarabine at a dose between or between about 1 mg/m² and 100 mg/m², such as between or between about 10 mg/m² and 75 mg/m², 15 mg/m² and 50 mg/m², 20 mg/m² and 40 mg/m², or 24 mg/m² and 35 mg/m², inclusive. In some instances, the subject is administered about 30 mg/m² of fludarabine. In some instances disclosed herein, the fludarabine can be administered in a single dose or can be administered in a plurality of doses, such as given daily, every other day or every three days. In some instances disclosed herein, fludarabine is administered daily, such as for 1-5 days, for example, for 3 to 5 days. In some instances, the subject is administered about 30 mg/m² of fludarabine, daily for 3 days, prior to initiation of the cell therapy.

In some instances disclosed herein, the lymphodepleting agent comprises a combination of agents, such as a combination of cyclophosphamide and fludarabine. Thus, the combination of agents may include cyclophosphamide at any dose or administration schedule, such as those described above, and fludarabine at any dose or administration schedule, such as those described above. For example, in some disclosures, the subject is administered 60 mg/kg (~2 g/m²) of cyclophosphamide and 3 to 5 doses of 25 mg/m² fludarabine prior to the first or subsequent dose.

Following administration of the cells, the biological activity of the engineered cell populations in some instances disclosed herein is measured, e.g., by any of a number of known methods. Parameters to assess include specific binding of an engineered or natural T cell or other immune cell to antigen, in vivo, e.g., by imaging, or ex vivo, e.g., by ELISA or flow cytometry. In certain instances disclosed herein, the ability of the engineered cells to destroy target cells can be measured using any suitable known methods, such as cytotoxicity assays described in, for example, Kochenderfer et al., J. Immunotherapy, 32(7): 689-702 (2009), and Herman et al. J. Immunological Methods, 285(1): 25-40 (2004). In certain instances disclosed herein, the biological activity of the cells is measured by assaying expression and/or secretion of one or more cytokines, such as CD 107a, IFNγ, IL-2, and TNF.

In certain instances disclosed herein, the engineered cells are further modified in any number of ways, such that their therapeutic or prophylactic efficacy is increased. For example, the engineered CAR or TCR expressed by the population can be conjugated either directly or indirectly through a linker to a targeting moiety. The practice of conjugating compounds, e.g., the CAR or TCR, to targeting moieties is known. See, for instance, Wadwa et al., J. Drug Targeting 3: 1 1 1 (1995), and U.S. Patent 5,087,616.In some instances disclosed herein, the cells are administered as part of a combination treatment, such as simultaneously with or sequentially with, in any order, another therapeutic intervention, such as an antibody or engineered cell or receptor or agent, such as a cytotoxic or therapeutic agent. The cells in some instances disclosed herein are co-administered with one or more additional therapeutic agents or in connection with another therapeutic intervention, either simultaneously or sequentially in any order. In some contexts, the cells are co-administered with another therapy sufficiently close in time such that the cell populations enhance the effect of one or more additional therapeutic agents, or vice versa. In some instances disclosed herein, the cells are administered prior to the one or more additional therapeutic agents. In some instances disclosed herein, the cells are administered after the one or more additional therapeutic agents. In some instances disclosed herein, the one or more additional agent includes a cytokine, such as IL-2, for example, to enhance persistence.

### B. Dosing

In some instances disclosed herein, a dose of cells is administered to subjects in accord with the disclosed methods, and/or with the disclosed articles of manufacture or compositions. In some instances disclosed herein, the size or timing of the doses is determined as a function of the particular disease or condition in the subject. In some instances disclosed herein, the size or timing of the doses for a particular disease in view of the disclosed description may be empirically determined.

In certain instances disclosed herein, the cells, or individual populations of sub-types of cells, are administered to the subject at a range of about one million to about 100 billion cells and/or that amount of cells per kilogram of body weight, such as, e.g., 1 million to about 50 billion cells (e.g., about 5 million cells, about 25 million cells, about 500 million cells, about 1 billion cells, about 5 billion cells, about 20 billion cells, about 30 billion cells, about 40 billion cells, or a range defined by any two of the foregoing values), such as about 10 million to about 100 billion cells (e.g., about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, or a range defined by any two of the foregoing values), and in some instances disclosed herein about 100 million cells to about 50 billion cells (e.g., about 120 million cells, about 250 million cells, about 350 million cells, about 450 million cells, about 650 million cells, about 800 million cells, about 900 million cells, about 3 billion cells, about 30 billion cells, about 45 billion cells) or any value in between these ranges and/or per kilogram of body weight. Dosages may vary depending on attributes particular to the disease or disorder and/or patient and/or other treatments. In some instances disclosed herein, such values refer to numbers of recombinant receptor-expressing cells; in other instances disclosed herein, they refer to number of T cells or PBMCs or total cells administered. In some instances disclosed herein, the number of cells is the number of such cells that are viable cells.

In some instances disclosed herein, the dose of cells is a flat dose of cells or fixed dose of cells such that the dose of cells is not tied to or based on the body surface area or weight of a subject.

In some instances disclosed herein, the dose of genetically engineered cells comprises from at or about 1 × 10⁵ to at or about 5 × 10⁸ total CAR-expressing T cells, from at or about 1 × 10⁵ to at or about 2.5 × 10⁸ total CAR-expressing T cells, from at or about 1 × 10⁵ to at or about 1 × 10⁸ total CAR-expressing T cells, from at or about 1 × 10⁵ to at or about 5 × 10⁷ total CAR-expressing T cells, from at or about 1 × 10⁵ to at or about 2.5 × 10⁷ total CAR-expressing T cells, from at or about 1 × 10⁵ to at or about 1 × 10⁷ total CAR-expressing T cells, from at or about 1 × 10⁵ to at or about 5 × 10⁶ total CAR-expressing T cells, from at or about 1 × 10⁵ to at or about 2.5 × 10⁶ total CAR-expressing T cells, from at or about 1 × 10⁵ to at or about 1 × 10⁶ total CAR-expressing T cells, from at or about 1 × 10⁶ to at or about 5 × 10⁸ total CAR-expressing T cells, from at or about 1 × 10⁶ to at or about 2.5 × 10⁸ total CAR-expressing T cells, from at or about 1 × 10⁶ to at or about 1 × 10⁸ total CAR-expressing T cells, from at or about 1 × 10⁶ to at or about 5 × 10⁷ total CAR-expressing T cells, from at or about 1 × 10⁶ to at or about 2.5 × 10⁷ total CAR-expressing T cells, from at or about 1 × 10⁶ to at or about 1 × 10⁷ total CAR-expressing T cells, from at or about 1 × 10⁶ to at or about 5 × 10⁶ total CAR-expressing T cells, from at or about 1 × 10⁶ to at or about 2.5 × 10⁶ total CAR-expressing T cells, from at or about 2.5 × 10⁶ to at or about 5 × 10⁸ total CAR-expressing T cells, from at or about 2.5 × 10⁶ to at or about 2.5 × 10⁸ total CAR-expressing T cells, from at or about 2.5 × 10⁶ to at or about 1 × 10⁸ total CAR-expressing T cells, from at or about 2.5 × 10⁶ to at or about 5 × 10⁷ total CAR-expressing T cells, from at or about 2.5 × 10⁶ to at or about 2.5 × 10⁷ total CAR-expressing T cells, from at or about 2.5 × 10⁶ to at or about 1 × 10⁷ total CAR-expressing T cells, from at or about 2.5 × 10⁶ to at or about 5 × 10⁶ total CAR-expressing T cells, from at or about 5 × 10⁶ to at or about 5 × 10⁸ total CAR-expressing T cells, from at or about 5 × 10⁶ to at or about 2.5 × 10⁸ total CAR-expressing T cells, from at or about 5 × 10⁶ to at or about 1 × 10⁸ total CAR-expressing T cells, from at or about 5 × 10⁶ to at or about 5 × 10⁷ total CAR-expressing T cells, from at or about 5 × 10⁶ to at or about 2.5 × 10⁷ total CAR-expressing T cells, from at or about 5 × 10⁶ to at or about 1 × 10⁷ total CAR-expressing T cells, from at or about 1 × 10⁷ to at or about 5 × 10⁸ total CAR-expressing T cells, from at or about 1 × 10⁷ to at or about 2.5 × 10⁸ total CAR-expressing T cells, from at or about 1 × 10⁷ to at or about 1 × 10⁸ total CAR-expressing T cells, from at or about 1 × 10⁷ to at or about 5 × 10⁷ total CAR-expressing T cells, from at or about 1 × 10⁷ to at or about 2.5 × 10⁷ total CAR-expressing T cells, from at or about 2.5 × 10⁷ to at or about 5 × 10⁸ total CAR-expressing T cells, from at or about 2.5 × 10⁷ to at or about 2.5 × 10⁸ total CAR-expressing T cells, from at or about 2.5 × 10⁷ to at or about 1 × 10⁸ total CAR-expressing T cells, from at or about 2.5 × 10⁷ to at or about 5 × 10⁷ total CAR-expressing T cells, from at or about 5 × 10⁷ to at or about 5 × 10⁸ total CAR-expressing T cells, from at or about 5 × 10⁷ to at or about 2.5 × 10⁸ total CAR-expressing T cells, from at or about 5 × 10⁷ to at or about 1 × 10⁸ total CAR-expressing T cells, from at or about 1 × 10⁸ to at or about 5 × 10⁸ total CAR-expressing T cells, from at or about 1 × 10⁸ to at or about 2.5 × 10⁸ total CAR-expressing T cells, or from at or about 2.5 × 10⁸ to at or about 5 × 10⁸ total CAR-expressing T cells. In some instances disclosed herein, the number of cells is the number of such cells that are viable cells.

In some instances disclosed herein, the dose of genetically engineered cells comprises from at or about 2.5 × 10⁷ to at or about 1.5 × 10⁸ total CAR-expressing T cells, such as 5 × 10⁷ to 1 × 10⁸ total CAR-expressing T cells. In some instances disclosed herein, the dose of genetically engineered cells comprises at least or at least about 2.5 × 10⁷ CAR-expressing cells, at least or at least about 5 × 10⁷ CAR-expressing cells, or at least or at least about 1 × 10⁸ CAR-expressing cells. In some instances disclosed herein, the dose of T cells comprises: at or about 2.5 × 10⁷ CAR-expressing T cells. In some instances disclosed herein, the dose of T cells comprises at or about 1 × 10⁸ CAR-expressing T cells. In some instances disclosed herein, the dose of T cells comprises at or about 5 × 10⁷ CAR-expressing T cells. In some instances disclosed herein, the number of cells is the number of such cells that are viable cells.

In some instances disclosed herein, the number is with reference to the total number of CD3⁺, CD8⁺, or CD4⁺ and CD8⁺, in some instances disclosed herein also recombinant receptor-expressing (e.g. CAR⁺) cells. In some instances disclosed herein, the number of cells is the number of such cells that are viable cells.

In some instances disclosed herein, the cell therapy comprises administration of a dose comprising a number of cell from or from about 1 × 10⁵ to or to about 5 × 10⁸ CD3⁺, CD8⁺, or CD4⁺ and CD8⁺ total T cells or CD3⁺, CD8⁺, or CD4⁺ and CD8⁺ recombinant receptor (e.g. CAR+)-expressing cells, from or from about 5 × 10⁵ to or to about 1 × 10⁷ CD3⁺, CD8⁺, or CD4⁺ and CD8⁺ total T cells or CD3⁺, CD8⁺, or CD4⁺ and CD8⁺ recombinant receptor (e.g. CAR+)-expressing cells, or from or from about 1 × 10⁶ to or to about 1 × 10⁷ CD3⁺, CD8⁺, or CD4⁺ and CD8⁺ total T cells or CD3⁺, CD8⁺, or CD4⁺ and CD8⁺ recombinant receptor (e.g. CAR+)-expressing cells, each inclusive. In some instances disclosed herein, the cell therapy comprises administration of a dose comprising a number of cell from or from about 1 × 10⁵ to or to about 5 × 10⁸ total CD3⁺/CAR⁺, CD8⁺/CAR⁺ or CD4⁺/CD8⁺/CAR⁺ cells, from or from about 5 × 10⁵ to or to about 1 × 10⁷ total CD3⁺/CAR⁺, CD8⁺/CAR⁺, or CD4⁺/CD8⁺/CAR⁺ cells, or from or from about 1 × 10⁶ to or to about 1 × 10⁷ total CD3⁺/CAR⁺, CD8⁺/CAR⁺, or CD4⁺/CD8⁺/CAR⁺ cells, each inclusive. In some instances disclosed herein, the number of cells is the number of such cells that are viable cells.

In some instances disclosed herein, the dose of genetically engineered cells comprises from or from about 2.5 × 10⁷ to 1.5 × 10⁸ total CD3+/CAR⁺, CD8⁺/CAR⁺, or CD4⁺/CD8⁺/CAR⁺ T cells, such as 5 × 10⁷ to 1 × 10⁸ total CD3+/CAR⁺, CD8⁺/CAR⁺, or CD4⁺/CD8⁺/CAR⁺ T cells. In some instances disclosed herein, the dose of genetically engineered cells comprises at least or at least about 2.5 × 10⁷ CD3+/CAR⁺, CD8⁺/CAR⁺, or CD4⁺/CD8⁺/CAR⁺ T cells, at least or at least about 5 × 10⁷ CD3+/CAR⁺, CD8⁺/CAR⁺, or CD4⁺/CD8⁺/CAR⁺ T cells, or at least or at least about 1 × 10⁸ CD3+/CAR⁺, CD8⁺/CAR⁺, or CD4⁺/CD8⁺/CAR⁺ T cells. In some instances disclosed herein, the dose of genetically engineered cells comprises at or about 2.5 × 10⁷ CD3+/CAR⁺, CD8⁺/CAR⁺, or CD4⁺/CD8⁺/CAR⁺ T cells, at or about 5 × 10⁷ CD3+/CAR⁺, CD8⁺/CAR⁺, or CD4⁺/CD8⁺/CAR⁺ T cells, or at or about 1 × 10⁸ CD3+/CAR⁺, CD8⁺/CAR⁺, or CD4⁺/CD8⁺/CAR⁺ T cells. In some instances disclosed herein, the number of cells is the number of such cells that are viable cells.

In some instances disclosed herein, the dose of T cells comprises: at or about 5 × 10⁷ recombinant receptor (e.g. CAR)-expressing T cells or at or about 2.5 × 10⁷ recombinant receptor (e.g. CAR)-expressing CD8⁺ T cells. In some instances disclosed herein, the dose of T cells comprises: at or about 1 × 10⁸ recombinant receptor (e.g. CAR)-expressing T cells or at or about 5 × 10⁷ recombinant receptor (e.g. CAR)-expressing CD8⁺ T cells. In some instances disclosed herein, the dose of T cells comprises: at or about 1.5 × 10⁸ recombinant receptor (e.g. CAR)-expressing T cells or at or about 0.75 × 10⁸ recombinant receptor (e.g. CAR) -expressing CD8⁺ T cells. In some instances disclosed herein, the number of cells is the number of such cells that are viable cells.

In some instances disclosed herein, the T cells of the dose include CD4⁺ T cells, CD8⁺ T cells or CD4⁺ and CD8⁺ T cells. In some instances disclosed herein, for example, where the subject is human, the CD8⁺ T cells of the dose, including in a dose including CD4⁺ and CD8⁺ T cells, includes between about 2.5 × 10⁷ and 1 × 10⁸ total recombinant receptor (e.g., CAR)-expressing CD8⁺cells, or a fraction thereof such as present at a ratio of 1:3 to 3:1 CD4+ cells to CD8+ T cell, optionally at or about 1:1.

In some instances disclosed herein, the patient is administered multiple doses, and each of the doses or the total dose can be within any of the foregoing values. In some instances disclosed herein, the dose of cells comprises the administration of from or from about 1 × 10⁵ to or to about 5 × 10⁸ total recombinant receptor (e.g. CAR)-expressing T cells or total T cells, from or from about 1 × 10⁵ to or to about 1.5 × 10⁸ total recombinant receptor (e.g. CAR)-expressing T cells or total T cells, from or from about 1 × 10⁵ to or to about 1 × 10⁸ total recombinant receptor (e.g. CAR)-expressing T cells or total T cells, from or from about 5 × 10⁵ to or to about 1 × 10⁷ total recombinant receptor (e.g. CAR)-expressing T cells or total T cells, or from or from about 1 × 10⁶ to or to about 1 × 10⁷ total recombinant receptor (e.g. CAR)-expressing T cells or total T cells, each inclusive.

In some instances disclosed herein, the dose of cells is a flat dose of cells or fixed dose of cells such that the dose of cells is not tied to or based on the body surface area or weight of a subject.

In some instances disclosed herein, the dose of genetically engineered cells comprises from or from about 1 × 10⁵ to 5 × 10⁸ total CAR-expressing T cells, 1 × 10⁵ to 2.5 × 10⁸ total CAR-expressing T cells, 1 × 10⁵ to 1 × 10⁸ total CAR-expressing T cells, 1 × 10⁵ to 5 × 10⁷ total CAR-expressing T cells, 1 × 10⁵ to 2.5 × 10⁷ total CAR-expressing T cells, 1 × 10⁵ to 1 × 10⁷ total CAR-expressing T cells, 1 × 10⁵ to 5 × 10⁶ total CAR-expressing T cells, 1 × 10⁵ to 2.5 × 10⁶ total CAR-expressing T cells, 1 × 10⁵ to 1 × 10⁶ total CAR-expressing T cells, 1 × 10⁶ to 5 × 10⁸ total CAR-expressing T cells, 1 × 10⁶ to 2.5 × 10⁸ total CAR-expressing T cells, 1 × 10⁶ to 1 × 10⁸ total CAR-expressing T cells, 1 × 10⁶ to 5 × 10⁷ total CAR-expressing T cells, 1 × 10⁶ to 2.5 × 10⁷ total CAR-expressing T cells, 1 × 10⁶ to 1 × 10⁷ total CAR-expressing T cells, 1 × 10⁶ to 5 × 10⁶ total CAR-expressing T cells, 1 × 10⁶ to 2.5 × 10⁶ total CAR-expressing T cells, 2.5 × 10⁶ to 5 × 10⁸ total CAR-expressing T cells, 2.5 × 10⁶ to 2.5 × 10⁸ total CAR-expressing T cells, 2.5 × 10⁶ to 1 × 10⁸ total CAR-expressing T cells, 2.5 × 10⁶ to 5 × 10⁷ total CAR-expressing T cells, 2.5 × 10⁶ to 2.5 × 10⁷ total CAR-expressing T cells, 2.5 × 10⁶ to 1 × 10⁷ total CAR-expressing T cells, 2.5 × 10⁶ to 5 × 10⁶ total CAR-expressing T cells, 5 × 10⁶ to 5 × 10⁸ total CAR-expressing T cells, 5 × 10⁶ to 2.5 × 10⁸ total CAR-expressing T cells, 5 × 10⁶ to 1 × 10⁸ total CAR-expressing T cells, 5 × 10⁶ to 5 × 10⁷ total CAR-expressing T cells, 5 × 10⁶ to 2.5 × 10⁷ total CAR-expressing T cells, 5 × 10⁶ to 1 × 10⁷ total CAR-expressing T cells, 1 × 10⁷ to 5 × 10⁸ total CAR-expressing T cells, 1 × 10⁷ to 2.5 × 10⁸ total CAR-expressing T cells, 1 × 10⁷ to 1 × 10⁸ total CAR-expressing T cells, 1 × 10⁷ to 5 × 10⁷ total CAR-expressing T cells, 1 × 10⁷ to 2.5 × 10⁷ total CAR-expressing T cells, 2.5 × 10⁷ to 5 × 10⁸ total CAR-expressing T cells, 2.5 × 10⁷ to 2.5 × 10⁸ total CAR-expressing T cells, 2.5 × 10⁷ to 1 × 10⁸ total CAR-expressing T cells, 2.5 × 10⁷ to 5 × 10⁷ total CAR-expressing T cells, 5 × 10⁷ to 5 × 10⁸ total CAR-expressing T cells, 5 × 10⁷ to 2.5 × 10⁸ total CAR-expressing T cells, 5 × 10⁷ to 1 × 10⁸ total CAR-expressing T cells, 1 × 10⁸ to 5 × 10⁸ total CAR-expressing T cells, 1 × 10⁸ to 2.5 × 10⁸ total CAR-expressing T cells, or 2.5 × 10⁸ to 5 × 10⁸ total CAR-expressing T cells. In some instances disclosed herein, the number of cells is the number of such cells that are viable cells.

In some instances disclosed herein, the dose of genetically engineered cells comprises from at or about 2.5 × 10⁷ to at or about 1.5 × 10⁸ total CAR-expressing T cells, such as 5 × 10⁷ to 1 × 10⁸ total CAR-expressing T cells. In some instances disclosed herein, the dose of genetically engineered cells comprises at least or at least about 2.5 × 10⁷ CAR-expressing cells, at least or at least about 5 × 10⁷ CAR-expressing cells, or at least or at least about 1 × 10⁸ CAR-expressing cells. In some instances disclosed herein, the dose of T cells comprises: at or about 2.5 × 10⁷ CAR-expressing T cells. In some instances disclosed herein, the dose of T cells comprises at or about 1 × 10⁸ CAR-expressing T cells. In some instances disclosed herein, the dose of T cells comprises at or about 5 × 10⁷ CAR-expressing T cells. In some instances disclosed herein, the number of cells is the number of such cells that are viable cells.

In some instances disclosed herein, the number is with reference to the total number of CD3⁺, CD8⁺, or CD4⁺ and CD8⁺, in some instances disclosed herein also recombinant receptor-expressing (e.g. CAR⁺) cells. In some instances disclosed herein, the number of cells is the number of such cells that are viable cells.

In some instances disclosed herein, the cell therapy comprises administration of a dose comprising a number of cell from or from about 1 × 10⁵ to or to about 5 × 10⁸ CD3⁺, CD8⁺, or CD4⁺ and CD8⁺ total T cells or CD3⁺, CD8⁺, or CD4⁺ and CD8⁺ recombinant receptor (e.g. CAR+)-expressing cells, from or from about 5 × 10⁵ to or to about 1 × 10⁷ CD3⁺, CD8⁺, or CD4⁺ and CD8⁺ total T cells or CD3⁺, CD8⁺, or CD4⁺ and CD8⁺ recombinant receptor (e.g. CAR+)-expressing cells, or from or from about 1 × 10⁶ to or to about 1 × 10⁷ CD3⁺, CD8⁺, or CD4⁺ and CD8⁺ total T cells or CD3⁺, CD8⁺, or CD4⁺ and CD8⁺ recombinant receptor (e.g. CAR+)-expressing cells, each inclusive. In some instances disclosed herein, the cell therapy comprises administration of a dose comprising a number of cell from or from about 1 × 10⁵ to or to about 5 × 10⁸ total CD3⁺/CAR⁺, CD8⁺/CAR⁺ or CD4⁺/CD8⁺/CAR⁺ cells, from or from about 5 × 10⁵ to or to about 1 × 10⁷ total CD3⁺/CAR⁺, CD8⁺/CAR⁺, or CD4⁺/CD8⁺/CAR⁺ cells, or from or from about 1 × 10⁶ to or to about 1 × 10⁷ total CD3⁺/CAR⁺, CD8⁺/CAR⁺, or CD4⁺/CD8⁺/CAR⁺ cells, each inclusive. In some instances disclosed herein, the number of cells is the number of such cells that are viable cells.

In some instances disclosed herein, the dose of genetically engineered cells comprises from or from about 2.5 × 10⁷ to 1.5 × 10⁸ total CD3+/CAR⁺, CD8⁺/CAR⁺, or CD4⁺/CD8⁺/CAR⁺ T cells, such as 5 × 10⁷ to 1 × 10⁸ total CD3+/CAR⁺, CD8⁺/CAR⁺, or CD4⁺/CD8⁺/CAR⁺ T cells. In some instances disclosed herein, the dose of genetically engineered cells comprises at least or at least about 2.5 × 10⁷ CD3⁺/CAR⁺, CD8⁺/CAR⁺, or CD4⁺/CD8⁺/CAR⁺ T cells, at least or at least about 5 × 10⁷ CD3+/CAR⁺, CD8⁺/CAR⁺, or CD4⁺/CD8⁺/CAR⁺ T cells, or at least or at least about 1 × 10⁸ CD3+/CAR⁺, CD8⁺/CAR⁺, or CD4⁺/CD8⁺/CAR⁺ T cells. In some instances disclosed herein, the dose of genetically engineered cells comprises at or about 2.5 × 10⁷ CD3+/CAR⁺, CD8⁺/CAR⁺, or CD4⁺/CD8⁺/CAR⁺ T cells, at or about 5 × 10⁷ CD3+/CAR⁺, CD8⁺/CAR⁺, or CD4⁺/CD8⁺/CAR⁺ T cells, or at or about 1 × 10⁸ CD3+/CAR⁺, CD8⁺/CAR⁺, or CD4⁺/CD8⁺/CAR⁺ T cells. In some instances disclosed herein, the number of cells is the number of such cells that are viable cells.

In some instances disclosed herein, the dose of T cells comprises: at or about 5 × 10⁷ recombinant receptor (e.g. CAR)-expressing T cells or at or about 2.5 × 10⁷ recombinant receptor (e.g. CAR)-expressing CD8⁺ T cells. In some instances disclosed herein, the dose of T cells comprises: at or about 1 × 10⁸ recombinant receptor (e.g. CAR)-expressing T cells or at or about 5 × 10⁷ recombinant receptor (e.g. CAR)-expressing CD8⁺ T cells. In some instances disclosed herein, the dose of T cells comprises: at or about 1.5 × 10⁸ recombinant receptor (e.g. CAR)-expressing T cells or at or about 0.75 × 10⁸ recombinant receptor (e.g. CAR) -expressing CD8⁺ T cells. In some instances disclosed herein, the number of cells is the number of such cells that are viable cells.

In some instances disclosed herein, the patient is administered multiple doses, and each of the doses or the total dose can be within any of the foregoing values. In some instances disclosed herein, the dose of cells comprises the administration of from or from about 1 × 10⁵ to or to about 5 × 10⁸ total recombinant receptor (e.g. CAR)-expressing T cells or total T cells, from or from about 1 × 10⁵ to or to about 1.5 × 10⁸ total recombinant receptor (e.g. CAR)-expressing T cells or total T cells, from or from about 1 × 10⁵ to or to about 1 × 10⁸ total recombinant receptor (e.g. CAR)-expressing T cells or total T cells, from or from about 5 × 10⁵ to or to about 1 × 10⁷ total recombinant receptor (e.g. CAR)-expressing T cells or total T cells, or from or from about 1 × 10⁶ to or to about 1 × 10⁷ total recombinant receptor (e.g. CAR)-expressing T cells or total T cells, each inclusive.

In some instances disclosed herein, the T cells of the dose include CD4⁺ T cells, CD8⁺ T cells or CD4⁺ and CD8⁺ T cells.

In some instances disclosed herein, the dose of cells, e.g., recombinant receptor-expressing T cells, is administered to the subject as a single dose or is administered only one time within a period of two weeks, one month, three months, six months, 1 year or more.

In the context of adoptive cell therapy, administration of a given "dose" encompasses administration of the given amount or number of cells as a single composition and/or single uninterrupted administration, e.g., as a single injection or continuous infusion, and also encompasses administration of the given amount or number of cells as a split dose or as a plurality of compositions, disclosed in multiple individual compositions or infusions, over a specified period of time, such as over no more than 3 days. Thus, in some contexts, the dose is a single or continuous administration of the specified number of cells, given or initiated at a single point in time. In some contexts, however, the dose is administered in multiple injections or infusions over a period of no more than three days, such as once a day for three days or for two days or by multiple infusions over a single day period.

Thus, in some disclosures, the cells of the dose are administered in a single pharmaceutical composition. In some instances disclosed herein, the cells of the dose are administered in a plurality of compositions, collectively containing the cells of the dose.

In some instances disclosed herein, cells of the dose may be administered by administration of a plurality of compositions or solutions, such as a first and a second, optionally more, each containing some cells of the dose. In some disclosures, the plurality of compositions, each containing a different population and/or sub-types of cells, are administered separately or independently, optionally within a certain period of time. For example, the populations or sub-types of cells can include CD8⁺ and CD4⁺ T cells, respectively, and/or CD8⁺- and CD4⁺-enriched populations, respectively, e.g., CD4⁺ and/or CD8⁺ T cells each individually including cells genetically engineered to express the recombinant receptor. In some instances disclosed herein, the administration of the dose comprises administration of a first composition comprising a dose of CD8⁺ T cells or a dose of CD4+ T cells and administration of a second composition comprising the other of the dose of CD4+ T cells and the CD8⁺ T cells.

In some instances disclosed herein, the administration of the composition or dose, e.g., administration of the plurality of cell compositions, involves administration of the cell compositions separately. In some disclosures, the separate administrations are carried out simultaneously, or sequentially, in any order. In particular instances disclosed herein, the separate administrations are carried out sequentially by administering, in any order, a first composition comprising a dose of CD8⁺ T cells or a dose of CD4⁺ T cells and a second composition comprising the other of the dose of CD4⁺ T cells and the CD8⁺ T cells. In some instances disclosed herein, the dose comprises a first composition and a second composition, and the first composition and second composition are administered within 48 hours of each other, such as no more than 36 hours of each other or not more than 24 hours of each other. In some instances disclosed herein, the first composition and second composition are administered at or about 0 to at or about 12 hours apart, from at or about 0 to at or about 6 hours apart or from at or about 0 to at or about 2 hours apart. In some instances disclosed herein, the initiation of administration of the first composition and the initiation of administration of the second composition are carried out no more than at or about 2 hours, no more than at or about 1 hour, or no more than at or about 30 minutes apart, no more than at or about 15 minutes, no more than at or about 10 minutes or no more than at or about 5 minutes apart. In some instances disclosed herein, the initiation and/or completion of administration of the first composition and the completion and/or initiation of administration of the second composition are carried out no more than at or about 2 hours, no more than at or about 1 hour, or no more than at or about 30 minutes apart, no more than at or about 15 minutes, no more than at or about 10 minutes or no more than at or about 5 minutes apart.

In some composition, the first composition, e.g., first composition of the dose, comprises CD4⁺ T cells. In some composition, the first composition, e.g., first composition of the dose, comprises CD8⁺ T cells. In some instances disclosed herein, the first composition is administered prior to the second composition. In particular instances disclosed herein, the CD8+ T cells are administered prior to the CD4+ T cells.

In some instances disclosed herein, the dose or composition of cells includes a defined or target ratio of CD4⁺ cells expressing a recombinant receptor (e.g. CAR) to CD8⁺ cells expressing a recombinant receptor (e.g. CAR) and/or of CD4⁺ cells to CD8⁺ cells, which ratio optionally is approximately 1: 1 or is between approximately 1:3 and approximately 3:1, such as approximately 1:1. In some disclosures, the administration of a composition or dose with the target or desired ratio of different cell populations (such as CD4⁺:CD8⁺ ratio or CAR⁺CD4⁺:CAR⁺CD8⁺ ratio, e.g., 1:1) involves the administration of a cell composition containing one of the populations and then administration of a separate cell composition comprising the other of the populations, where the administration is at or approximately at the target or desired ratio. In some disclosures, administration of a dose or composition of cells at a defined ratio leads to improved expansion, persistence and/or antitumor activity of the T cell therapy.

In some instances disclosed herein, the dose or composition of cells includes a defined or target ratio of CD4⁺ cells expressing a recombinant receptor to CD8⁺ cells expressing a recombinant receptor and/or of CD4⁺ cells to CD8⁺ cells, which ratio optionally is approximately 1:1. In some disclosures, the administration of a composition or dose with the target or desired ratio of different cell populations (such as CD4⁺:CD8⁺ ratio or CAR⁺CD4⁺:CAR⁺CD8⁺ ratio, e.g., 1:1) involves the administration of a cell composition containing one of the populations and then administration of a separate cell composition comprising the other of the populations, where the administration is at or approximately at the target or desired ratio. In some disclosures, administration of a dose or composition of cells at a defined ratio leads to improved expansion, persistence and/or antitumor activity of the T cell therapy.

In particular instances disclosed herein, the numbers and/or concentrations of cells refer to the number of recombinant receptor (e.g., CAR)-expressing cells or the number of recombinant receptor (e.g., CAR)-expressing T cell or CD3+ T or a CD4+ and/or CD8+ T cell subset thereof. In some instances disclosed herein, the number and/or concentration of cells refers to such number of cells that are viable cells.

In some instances disclosed herein, the dose of genetically engineered cells is or is about 5 × 10⁷ CD3+ CAR+ viable cells, that includes a separate dose of at or about 2.5 × 10⁷ CD4+ CAR+ viable cells and at or about 2.5 × 10⁷ CD8+CAR+ viable cells. In some instances disclosed herein, the dose of genetically engineered cells is or is about 1 × 10⁸ CD3+CAR+ viable cells, that includes a separate dose of at or about 5 × 10⁷ CD4+CAR+ viable cells and at or about 5 ×10⁷ CD8+CAR+ viable cells. In some instances disclosed herein, the dose of genetically engineered cells is or is about 1.5 × 10⁸ CD3+CAR+ viable cells, that includes a separate dose of at or about 0.75 × 10⁸ CD4+CAR+ viable cells and at or about 0.75 ×10⁸ CD8+CAR+ viable cells.

### C. Response, Efficacy, and Survival

In some instances disclosed herein, the administration effectively treats the subject despite the subject having failed, having become refractory to and/or resistant to another therapy. In some instances disclosed herein, the administration effectively treats the subject despite the subject having become resistant to another therapy. In some instances disclosed herein, at least 30% of subjects treated according to the method achieve complete remission (CR); and/or at least about 75% of the subjects treated according to the method achieve an objective response (OR). In some instances disclosed herein, at least or about at least 35%, 40%, 45%, 50%, 55%, 60% or more of subjects treated according to the method achieve CR and/or at least or about at least 50%, 60%, 70%, or 80% achieve an objective response (OR). In some instances disclosed herein, at least 30% of subjects that have failed both a prior BTK inhibitor (e.g., ibrutinib) therapy and venetoclax, treated according to the method achieve complete remission (CR); and/or at least about 75% of the subjects that have failed both a prior BTK inhibitor (e.g., ibrutinib) therapy and venetoclax, treated according to the method achieve an objective response (OR). In some instances disclosed herein, at least or about at least 35%, 40%, 45%, 50%, 55%, 60% or more of subjects that have failed both a prior BTK inhibitor (e.g., ibrutinib) therapy and venetoclax, treated according to the method achieve CR and/or at least or about at least 50%, 60%, 70%, or 80% achieve an objective response (OR). In some instances disclosed herein, criteria assessed for effective treatment includes overall response rate (ORR; also known in some cases as objective response rate), complete response (CR; also known in some cases as complete response), complete remission with incomplete blood count recovery (CRi), stable disease (SD), and/or partial disease (PD).

In some instances disclosed herein, the duration of the response before progression is for greater than 1 month, greater than 2 months, greater than 3 months, greater than 6 months or more. In some instances disclosed herein, at least 35%, 40%, 45%, 50%, 55%, 60% or more of subjects treated according to the methods disclosed herein achieve complete remission (CR; also known in some cases as complete response) at or about 3 months or at or about 6 months after administration of the cell therapy.

In some disclosures, the administration in accord with the disclosed methods, and/or with the disclosed articles of manufacture or compositions, generally reduces or prevents the expansion or burden of the disease or condition in the subject. For example, where the disease or condition is a tumor, the methods generally reduce tumor size, bulk, metastasis, percentage of blasts in the bone marrow or molecularly detectable cancer and/or improve prognosis or survival or other symptom associated with tumor burden.

Disease burden can encompass a total number of cells of the disease in the subject or in an organ, tissue, or bodily fluid of the subject, such as the organ or tissue of the tumor or another location, e.g., which would indicate metastasis. For example, tumor cells may be detected and/or quantified in the blood or bone marrow in the context of certain hematological malignancies. Disease burden can include, in some instances disclosed herein, the mass of a tumor, the number or extent of metastases and/or the percentage of blast cells present in the bone marrow.

In some instances disclosed herein, a subject has leukemia. The extent of disease burden can be determined by assessment of residual leukemia in blood or bone marrow.

In some disclosures, response rates in subjects, such as subjects with CLL, are based on the International Workshop on Chronic Lymphocytic Leukemia (IWCLL) response criteria (Hallek, et al., Blood 2008, Jun 15; 111(12): 5446-5456). In some disclosures, response rates in subjects, such as subjects with CLL, are based on the International Workshop on Chronic Lymphocytic Leukemia (IWCLL) response criteria (Hallek et al., Blood 2018 131 (25): 2745-2760). In some disclosures, these criteria are described as follows: complete remission (CR; also known in some cases as complete response), which in some disclosures requires the absence of peripheral blood clonal lymphocytes by immunophenotyping, absence of lymphadenopathy, absence of hepatomegaly or splenomegaly, absence of constitutional symptoms and satisfactory blood counts; complete remission with incomplete marrow recovery (CRi), which in some disclosures is described as CR above, but without normal blood counts; partial remission (PR; also known in some cases as partial response), which in some disclosures is described as ≥ 50% fall in lymphocyte count, ≥ 50% reduction in lymphadenopathy or ≥ 50% reduction in liver or spleen, together with improvement in peripheral blood counts; progressive disease (PD), which in some disclosures is described as ≥ 50% rise in lymphocyte count to > 5 ×10⁹/L, ≥ 50% increase in lymphadenopathy, ≥ 50% increase in liver or spleen size, Richter's transformation, or new cytopenias due to CLL; and stable disease, which in some disclosures is described as not meeting criteria for CR, CRi, PR or PD.

In some instances disclosed herein, the subjects exhibits a CR or OR if, within 1 month of the administration of the dose of cells, lymph nodes in the subject are less than at or about 20 mm in size, less than at or about 10 mm in size or less than at or about 10 mm in size.

In some instances disclosed herein, an index clone of the CLL is not detected in the bone marrow of the subject (or in the bone marrow of greater than 50%, 60%, 70%, 80%, 90% or more of the subjects treated according to the methods. In some instances disclosed herein, an index clone of the CLL is assessed by IgH deep sequencing. In some instances disclosed herein, the index clone is not detected at a time that is at or about or at least at or about 1, 2, 3, 4, 5, 6, 12, 18 or 24 months following the administration of the cells.

In some instances disclosed herein, a subject exhibits morphologic disease if there are greater than or equal to 5% blasts in the bone marrow, for example, as detected by light microscopy, such as greater than or equal to 10% blasts in the bone marrow, greater than or equal to 20% blasts in the bone marrow, greater than or equal to 30% blasts in the bone marrow, greater than or equal to 40% blasts in the bone marrow or greater than or equal to 50% blasts in the bone marrow. In some instances disclosed herein, a subject exhibits complete or clinical remission if there are less than 5% blasts in the bone marrow.

In some instances disclosed herein, a subject has leukemia. The extent of disease burden can be determined by assessment of residual leukemia in blood or bone marrow.

In some instances disclosed herein, a subject exhibits morphologic disease if there are greater than or equal to 5% blasts in the bone marrow, for example, as detected by light microscopy, such as greater than or equal to 10% blasts in the bone marrow, greater than or equal to 20% blasts in the bone marrow, greater than or equal to 30% blasts in the bone marrow, greater than or equal to 40% blasts in the bone marrow or greater than or equal to 50% blasts in the bone marrow. In some instances disclosed herein, a subject exhibits complete or clinical remission if there are less than 5% blasts in the bone marrow.

In some instances disclosed herein, a subject may exhibit complete remission, but a small proportion of morphologically undetectable (by light microscopy techniques) residual leukemic cells are present. A subject is said to exhibit minimum residual disease (MRD) if the subject exhibits less than 5% blasts in the bone marrow and exhibits molecularly detectable cancer. In some instances disclosed herein, molecularly detectable cancer can be assessed using any of a variety of molecular techniques that permit sensitive detection of a small number of cells. In some disclosures, such techniques include PCR assays, which can determine unique Ig/T-cell receptor gene rearrangements or fusion transcripts produced by chromosome translocations. In some instances disclosed herein, flow cytometry can be used to identify cancer cell based on leukemia-specific immunophenotypes. In some instances disclosed herein, molecular detection of cancer can detect as few as 1 leukemia cell in 100,000 normal cells. In some instances disclosed herein, a subject exhibits MRD that is molecularly detectable if at least or greater than 1 leukemia cell in 100,000 cells is detected, such as by PCR or flow cytometry. In some instances disclosed herein, the disease burden of a subject is molecularly undetectable or MRD⁻, such that, in some instances disclosed herein, no leukemia cells are able to be detected in the subject using PCR or flow cytometry techniques.

In some instances disclosed herein, an index clone of the leukemia, e.g. CLL, is not detected in the bone marrow of the subject (or in the bone marrow of greater than 50%, 60%, 70%, 80%, 90% or more of the subjects treated according to the methods. In some instances disclosed herein, an index clone of the leukemia, e.g. CLL, is assessed by IGH deep sequencing. In some instances disclosed herein, the index clone is not detected at a time that is at or about or at least at or about 1, 2, 3, 4, 5, 6, 12, 18 or 24 months following the administration of the cells.

In some disclosures MRD is detected by flow cytometry. Flow cytometry can be used to monitor bone marrow and peripheral blood samples for cancer cells. In particular disclosures, flow cytometry is used to detect or monitor the presence of cancer cells in bone marrow. In some disclosures, multiparameter immunological detection by flow cytometry is used to detect cancer cells (see for example, Coustan-Smith et al., (1998) Lancet 351:550-554). In some disclosures, multiparameter immunological detection by mass cytometry is used to detect cancer cells. In some instances disclosed herein, 1, 2, 3, 4, 5, 6, 7, 8, 9, 10, 11, 12, 13, 14, 15, 20, 25, 30, 35, 40, 45 or 50 parameters can be used to detect cancer cells. The antigens used for detection are selected based on the cancer being detected (Foon and Todd (1986) Blood 68:1-31). In some instances disclosed herein, MRD is described as subjects who have no evidence of CLL in peripheral blood or marrow, i.e., CR or PR based on residual lymphadenopathy or splenomegaly. In some disclosures, MRD is measured via flow cytometry of peripheral blood and IgHV deep sequencing of bone marrow.

In some instances disclosed herein, bone marrow is harvested by bone marrow aspirates or bone marrow biopsies, and lymphocytes are isolated for analysis. Monoclonal and/or polyclonal antibodies conjugated to a fluorochrome (*e.g*., fluorescein isothiocyanate (FITC), phycoerythrin, peridinin chlorophyll protein, or biotin) can be used to detect epitopes, such as terminal deoxynucleotidyl transferase (TdT), CD3, CD10, CD11c, CD13, CD14, CD33, CD19, CD20, CD21, CD22, CD23, CD34, CD45, CD56, CD79b, IgM, and/or KORSA3544, on isolated lymphocytes. Labeled cells can then be detected using flow cytometry, such as multiparameter flow cytometry, or mass cytometry, to detect multiple epitopes.

Lymphoid cells can be identified and gated based on a light-scatter dot plot and then secondarily gated to identify cell populations expressing the immunophenotypic features of interest. Exemplary epitopes are set forth in **Table 2** below. Other immunologic classification of leukemias and lymphomas are provided by Foon and Todd (Blood (1986) 68(1): 1-31). In some disclosures, flow cytometric assessment of MRD can be achieved by quantifying live lymphocytes bearing one or more CLL immunophenotypes (e.g., low forward/side scatter; CD3^{neg}; CD5⁺; CD14^{neg}; CD19⁺; CD23⁺; CD45⁺; CD56^{neg}).

| **Table 2. Exemplary Immnunophenotype and Cytogentics Characteristics** | | |
|---|---|---|
| **Disease** | **Immunophenotype** | **Cytogenetics** |
| Chronic Lymphocytic Leukemia (CLL) | Pan-B+; CD5+; CD23+; CD79b/CD22 weak; FMC7-; sIg weak | Trisomy 12 |
| | | del(13)(q14.3) |
| | | del 11q22-q23 |
| | | del 17p13 (p53) |
| | | t(11;14)(q13;q32) BCL1/IgH rearrangement |
| | | t(14;19)(q32;q13) |
| | | IgH deletion (14q32) |
| | | del(6q) |
| | | +8q24 |
| | | +3 |
| | | +18 |
| | | del 6q21 |
| Small lymphocytic lymphoma (SLL) | Pan-B+; CD5+; CD23+; CD10-; sIgM+ faint | del(6)(q21-23) |
| +: positive in >90% of the cases | | |
| +/-: positive in more than 50% of the cases | | |
| -/+: positive in less than 50% of cases | | |
| -: positive in <10% of the cases | | |
| Pan-B markers: e.g., CD19, CD20, CD79a | | |
| sIG: surface immunoglobulins | | |
| cyIg: cytoplasmic immunoglobulins | | |

In some disclosures, deep sequencing of the immunoglobulin heavy chain (IGH) locus of harvested B cells can be used to detect minimal residual disease (MRD). Clonal presence of a particular IgG rearrangement can provide a marker to detect the presence of B cell malignancies, such as CLL and/or residual presence of malignant cells thereof. In some disclosures cells such as a population containing or suspected of containing B cells are harvested and isolated from blood. In some disclosures, cells are harvested and isolated from bone marrow, e.g., from bone marrow aspirates or bone marrow biopsies and/or from other biological samples. In some disclosures, polymerase chain reaction (PCR) amplification of the complementarity determining region 3 (CDR3) is achieved using primers to highly conserved sequences within the V and J regions of the gene locus, which may be used to identify clonal populations of cells for purposes of assessing minimal residual disease. Other methods for detecting clonal populations, such as single cell sequencing approaches, including those providing information regarding number of cells of a particular lineage and/or expressing a particular variable chain such as variable heavy chain or binding site thereof, such as a clonal population, may be used. In some disclosures, the IGH DNA is amplified using a degenerate primers or primers recognizing regions of variable chains shared among different cell clones, such as those recognizing consensus V and degenerate consensus J region of the IGH sequence. An exemplary sequence of the V region is ACACGGCCTCGTGTATTACTGT (SEQ ID NO: 57). An exemplary degenerate consensus sequence of the J region is ACCTGAGGAGACGGTGACC (SEQ ID NO: 58).

The PCR product or sequencing result in some disclosures is specific to the rearranged allele and serves as a clonal marker for MRD detection. Following PCR amplification of the CDR3 region, PCR products can be sequenced to yield patient-specific oligonucleotides constructed as probes for allele-specific PCR for sensitive detection of MRD following treatment of B-cell malignancies with CAR-T cell therapy, e.g. CD19 CAR- T cell therapy. In instances disclosed herein where a PCR product is not generated using the consensus primers, V region family-specific primers for the framework region 1 can be used instead.

In some disclosures, persistence of PCR-detectable tumor cells such as cells of the B cell malignancy such as the CLL, such as detectable IGH sequences corresponding to the malignant or clonal IGH sequences, after treatment is associated with increased risk of relapse. In some disclosures, patients who are negative for malignant IGH sequences following treatment (in some disclosures, even in the context of other criteria indicating progressive disease or only a partial response, such as persistence of enlarged lymph nodes or other criteria that may in some contexts be associated with disease or lack of complete response) may be deemed to have increased likelihood to enter into CR or durable CR or prolonged survival, compared to patients with persistent malignant IGH sequences. In some instances disclosed herein, such prognostic and staging determinations are particularly relevant for treatments in which clearance of malignant cells is observed within a short period of time following administration of the therapy, e.g., in comparison to resolution of other clinical symptoms such as lymph node size or other staging criteria. For example, in some such disclosures, absence of detectable IGH or minimal residual disease in a sample such as the bone marrow may be a preferred readout for response or likelihood of response or durability thereof, as compared to other available staging or prognostic approaches. In some disclosures, results from MRD, e.g., IGH deep sequencing information, may inform further intervention or lack thereof. For example, the methods and other instances disclosed herein in some contexts provide that a subject deemed negative for malignant IGH may in some disclosures be not further treated or not be further administered a dose of the therapy disclosed, or that the subject be administered a lower or reduced dose. Conversely, it may be disclosed or specified that a subject exhibiting MRD via IGH deep sequencing be further treated, e.g., with the therapy initially administered at a similar or higher dose or with a further treatment. In some disclosures, the disease or condition persists following administration of the first dose and/or administration of the first dose is not sufficient to eradicate the disease or condition in the subject.

In some instances disclosed herein, the method reduces the burden of the disease or condition, e.g., number of tumor cells, size of tumor, duration of patient survival or event-free survival, to a greater degree and/or for a greater period of time as compared to the reduction that would be observed with a comparable method using an alternative dosing regimen, such as one in which the subject receives one or more alternative therapeutic agents and/or one in which the subject does not receive a dose of cells and/or a lymphodepleting agent in accord with the disclosed methods, and/or with the disclosed articles of manufacture or compositions. In some instances disclosed herein, the burden of a disease or condition in the subject is detected, assessed, or measured. Disease burden may be detected in some disclosures by detecting the total number of disease or disease-associated cells, e.g., tumor cells, in the subject, or in an organ, tissue, or bodily fluid of the subject, such as blood or serum. In some disclosures, survival of the subject, survival within a certain time period, extent of survival, presence or duration of event-free or symptom-free survival, or relapse-free survival, is assessed. In some instances disclosed herein, any symptom of the disease or condition is assessed. In some instances disclosed herein, the measure of disease or condition burden is specified.

In some instances disclosed herein, following treatment by the method, the probability of relapse is reduced as compared to other methods, for example, methods in which the subject receives one or more alternative therapeutic agents and/or one in which the subject does not receive a dose of cells and/or a lymphodepleting agent in accord with the disclosed methods, and/or with the disclosed articles of manufacture or compositions.

In some instances disclosed herein, the pharmacokinetics of administered cells, e.g., adoptively transferred cells are determined to assess the availability, e.g., bioavailability of the administered cells. Methods for determining the pharmacokinetics of adoptively transferred cells may include drawing peripheral blood from subjects that have been administered engineered cells, and determining the number or ratio of the engineered cells in the peripheral blood. Approaches for selecting and/or isolating cells may include use of chimeric antigen receptor (CAR)-specific antibodies (e.g., Brentjens et al., Sci. Transl. Med. 2013 Mar; 5(177): 177ra38) Protein L (Zheng et al., J. Transl. Med. 2012 Feb; 10:29), epitope tags, such as Strep-Tag sequences, introduced directly into specific sites in the CAR, whereby binding reagents for Strep-Tag are used to directly assess the CAR (Liu et al. (2016) Nature Biotechnology, 34:430; international patent application Pub. No. WO2015095895) and monoclonal antibodies that specifically bind to a CAR polypeptide (see international patent application Pub. No. WO2014190273). Extrinsic marker genes may in some instances disclosed herein be utilized in connection with engineered cell therapies to permit detection or selection of cells and, in some instances disclosed herein, also to promote cell suicide. A truncated epidermal growth factor receptor (EGFRt) in some instances disclosed herein can be co-expressed with a transgene of interest (a CAR or TCR) in transduced cells (see e.g. U.S. Patent No. 8,802,374). EGFRt may contain an epitope recognized by the antibody cetuximab (Erbitux^{®}) or other therapeutic anti-EGFR antibody or binding molecule, which can be used to identify or select cells that have been engineered with the EGFRt construct and another recombinant receptor, such as a chimeric antigen receptor (CAR), and/or to eliminate or separate cells expressing the receptor. See U.S. Patent No. 8,802,374 and Liu et al., Nature Biotech. 2016 April; 34(4): 430-434).

In some instances disclosed herein, the number of CAR⁺ T cells in a biological sample obtained from the patient, e.g., blood, can be determined at a period of time after administration of the cell therapy, e.g., to determine the pharmacokinetics of the cells. In some instances disclosed herein, number of CAR⁺ T cells, optionally CAR⁺ CD8⁺ T cells and/or CAR⁺ CD4⁺ T cells, detectable in the blood of the subject, or in a majority of subjects so treated by the method, is greater than 1 cells per µL, greater than 5 cells per µL or greater than per 10 cells per µL.

### D. Toxicity

In some instances disclosed herein, the disclosed methods are designed to or include features that result in a lower rate and/or lower degree of toxicity, toxic outcome or symptom, toxicity-promoting profile, factor, or property, such as a symptom or outcome associated with or indicative of cytokine release syndrome (CRS) or neurotoxicity, for example, compared to administration of an alternative cell therapy, such as an alternative CAR⁺ T cell composition and/or an alternative dosing of cells, e.g. a dosing of cells that is not administered at a defined ratio.

In some instances disclosed herein, the disclosed methods do not result in a high rate or likelihood of toxicity or toxic outcomes, or reduces the rate or likelihood of toxicity or toxic outcomes, such as neurotoxicity (NT), cytokine release syndrome (CRS), such as compared to certain other cell therapies. In some instances disclosed herein, the methods do not result in, or do not increase the risk of, severe NT (sNT), severe CRS (sCRS), macrophage activation syndrome, tumor lysis syndrome, fever of at least at or about 38 degrees Celsius for three or more days and a plasma level of CRP of at least at or about 20 mg/dL. In some instances disclosed herein, greater than or greater than about 30%, 35%, 40%, 50%, 55%, 60% or more of the subjects treated according to the disclosed methods do not exhibit any grade of CRS or any grade of neurotoxicity. In some instances disclosed herein, no more than 50% of subjects treated (e.g. at least 60%, at least 70%, at least 80%, at least 90% or more of the subjects treated) exhibit a cytokine release syndrome (CRS) higher than grade 2 and/or a neurotoxicity higher than grade 2. In some instances disclosed herein, at least 50% of subjects treated according to the method (e.g. at least 60%, at least 70%, at least 80%, at least 90% or more of the subjects treated) do not exhibit a severe toxic outcome (e.g. severe CRS or severe neurotoxicity), such as do not exhibit grade 3 or higher neurotoxicity and/or does not exhibit severe CRS, or does not do so within a certain period of time following the treatment, such as within a week, two weeks, or one month of the administration of the cells.. In some instances disclosed herein, greater than or greater than about 30%, 35%, 40%, 50%, 55%, 60% or more of the subjects that had failed both a prior BTK inhibitor (e.g., ibrutinib) therapy and venetoclax, treated according to the disclosed methods do not exhibit any grade of CRS or any grade of neurotoxicity. In some instances disclosed herein, no more than 50% of subjects treated (e.g. at least 60%, at least 70%, at least 80%, at least 90% or more of the subjects treated that had failed both a prior BTK inhibitor (e.g., ibrutinib) therapy and venetoclax) exhibit a cytokine release syndrome (CRS) higher than grade 2 and/or a neurotoxicity higher than grade 2. In some instances disclosed herein, at least 50% of subjects that had failed both a prior BTK inhibitor (e.g., ibrutinib) therapy and venetoclax, treated according to the method (e.g. at least 60%, at least 70%, at least 80%, at least 90% or more of the subjects treated) do not exhibit a severe toxic outcome (e.g. severe CRS or severe neurotoxicity), such as do not exhibit grade 3 or higher neurotoxicity and/or does not exhibit severe CRS, or does not do so within a certain period of time following the treatment, such as within a week, two weeks, or one month of the administration of the cells. In some instances disclosed herein, parameters assessed to determine certain toxicities include adverse events (AEs), treatment-emergent adverse events, dose-limiting toxicities (DLTs), CRS, neurologic events and NT.

Administration of adoptive T cell therapy, such as treatment with T cells expressing chimeric antigen receptors, can induce toxic effects or outcomes such as cytokine release syndrome and neurotoxicity. In some instances disclosed herein, such effects or outcomes parallel high levels of circulating cytokines, which may underlie the observed toxicity.

In some disclosures, the toxic outcome is or is associated with or indicative of cytokine release syndrome (CRS) or severe CRS (sCRS). CRS, e.g., sCRS, can occur in some instances disclosed herein following adoptive T cell therapy and administration to subjects of other biological products. See Davila et al., Sci Transl Med 6, 224ra25 (2014); Brentjens et al., Sci. Transl. Med. 5, 177ra38 (2013); Grupp et al., N. Engl. J. Med. 368, 1509-1518 (2013); and Kochenderfer et al., Blood 119, 2709-2720 (2012); Xu et al., Cancer Letters 343 (2014) 172-78.

Typically, CRS is caused by an exaggerated systemic immune response mediated by, for example, T cells, B cells, NK cells, monocytes, and/or macrophages. Such cells may release a large amount of inflammatory mediators such as cytokines and chemokines. Cytokines may trigger an acute inflammatory response and/or induce endothelial organ damage, which may result in microvascular leakage, heart failure, or death. Severe, life-threatening CRS can lead to pulmonary infiltration and lung injury, renal failure, or disseminated intravascular coagulation. Other severe, life-threatening toxicities can include cardiac toxicity, respiratory distress, neurologic toxicity and/or hepatic failure.

CRS may be treated using anti-inflammatory therapy such as an anti-IL-6 therapy, e.g., anti-IL-6 antibody, e.g., tocilizumab, or antibiotics or other agents as described. Outcomes, signs and symptoms of CRS are known and include those described herein. In some instances disclosed herein, where a particular dosage regimen or administration effects or does not effect a given CRS-associated outcome, sign, or symptom, particular outcomes, signs, and symptoms and/or quantities or degrees thereof may be specified.

In the context of administering CAR-expressing cells, CRS typically occurs 6-20 days after infusion of cells that express a CAR. See Xu et al., Cancer Letters 343 (2014) 172-78. In some instances disclosed herein, CRS occurs less than 6 days or more than 20 days after CAR T cell infusion. The incidence and timing of CRS may be related to baseline cytokine levels or tumor burden at the time of infusion. Commonly, CRS involves elevated serum levels of interferon (IFN)-γ, tumor necrosis factor (TNF)-α, and/or interleukin (IL)-2. Other cytokines that may be rapidly induced in CRS are IL-1β, IL-6, IL-8, and IL-10.

Exemplary outcomes associated with CRS include fever, rigors, chills, hypotension, dyspnea, acute respiratory distress syndrome (ARDS), encephalopathy, ALT/AST elevation, renal failure, cardiac disorders, hypoxia, neurologic disturbances, and death. Neurological complications include delirium, seizure-like activity, confusion, word-finding difficulty, aphasia, and/or becoming obtunded. Other CRS-related outcomes include fatigue, nausea, headache, seizure, tachycardia, myalgias, rash, acute vascular leak syndrome, liver function impairment, and renal failure. In some disclosures, CRS is associated with an increase in one or more factors such as serum-ferritin, d-dimer, aminotransferases, lactate dehydrogenase and triglycerides, or with hypofibrinogenemia or hepatosplenomegaly. Other exemplary signs or symptoms associated with CRS include hemodynamic instability, febrile neutropenia, increase in serum C-reactive protein (CRP), changes in coagulation parameters (for example, international normalized ratio (INR), prothrombin time (PTI) and/or fibrinogen), changes in cardiac and other organ function, and/or absolute neutrophil count (ANC).

In some instances disclosed herein, outcomes associated with CRS include one or more of: persistent fever, e.g., fever of a specified temperature, e.g., greater than at or about 38 degrees Celsius, for two or more, e.g., three or more, e.g., four or more days or for at least three consecutive days; fever greater than at or about 38 degrees Celsius; elevation of cytokines, such as a max fold change, e.g., of at least at or about 75, compared to pre-treatment levels of at least two cytokines (e.g., at least two of the group consisting of interferon gamma (IFNγ), GM-CSF, IL-6, IL-10, Flt-3L, fracktalkine, and IL-5, and/or tumor necrosis factor alpha (TNFα)), or a max fold change, e.g., of at least at or about 250 of at least one of such cytokines; and/or at least one clinical sign of toxicity, such as hypotension (e.g., as measured by at least one intravenous vasoactive pressor); hypoxia (e.g., plasma oxygen (POz) levels of less than at or about 90%); and/or one or more neurologic disorders (including mental status changes, obtundation, and seizures).

Exemplary CRS-related outcomes include increased or high serum levels of one or more factors, including cytokines and chemokines and other factors associated with CRS. Exemplary outcomes further include increases in synthesis or secretion of one or more of such factors. Such synthesis or secretion can be by the T cell or a cell that interacts with the T cell, such as an innate immune cell or B cell.

In some instances disclosed herein, the CRS-associated serum factors or CRS-related outcomes include inflammatory cytokines and/or chemokines, including interferon gamma (IFN-γ), TNF-a, IL-1β, IL-2, IL-6, IL-7, IL-8, IL-10, IL-12, sIL-2Ra, granulocyte macrophage colony stimulating factor (GM-CSF), macrophage inflammatory protein (MIP)-1, tumor necrosis factor alpha (TNFα), IL-6, and IL-10, IL-1β, IL-8, IL-2, MIP-1, Flt-3L, fracktalkine, and/or IL-5. In some instances disclosed herein, the factor or outcome includes C reactive protein (CRP). In addition to being an early and easily measurable risk factor for CRS, CRP also is a marker for cell expansion. In some instances disclosed herein, subjects that are measured to have high levels of CRP, such as ≥ 15 mg/dL, have CRS. In some instances disclosed herein, subjects that are measured to have high levels of CRP do not have CRS. In some instances disclosed herein, a measure of CRS includes a measure of CRP and another factor indicative of CRS.

In some instances disclosed herein, one or more inflammatory cytokines or chemokines are monitored before, during, or after CAR treatment. In some instances disclosed herein, TNF-alpha or IL-16 is assessed or monitored in a subject, such as in accord with the methods herein.

CRS criteria that appear to correlate with the onset of CRS to predict which patients are more likely to be at risk for developing sCRS have been developed (see Davilla et al. Science translational medicine. 2014;6(224):224ra25). Factors include fevers, hypoxia, hypotension, neurologic changes, elevated serum levels of inflammatory cytokines, such as a set of seven cytokines (IFNγ, IL-5, IL-6, IL-10, Flt-3L, fractalkine, and GM-CSF) whose treatment-induced elevation can correlate well with both pretreatment tumor burden and sCRS symptoms. Other guidelines on the diagnosis and management of CRS are known (see e.g., Lee et al, Blood. 2014;124(2):188-95). In some instances disclosed herein, the criteria reflective of CRS grade are those detailed in Table 3 below.

| **Table 3:** | **Exemplary Grading Criteria for CRS** |
|---|---|
| **Grade** | **Description of Symptoms** |
| 1 Mild | Not life-threatening, require only symptomatic treatment such as antipyretics and anti-emetics (e.g., fever, nausea, fatigue, headache, myalgias, malaise) |
| 2 Moderate | Require and respond to moderate intervention: |
| | • Oxygen requirement < 40%, or |
| | • Hypotension responsive to fluids or low dose of a single vasopressor, or |
| | • Grade 2 organ toxicity (by CTCAE v4.0) |
| 3 Severe | Require and respond to aggressive intervention: |
| | • Oxygen requirement ≥ 40%, or |
| | • Hypotension requiring high dose of a single vasopressor (e.g., norepinephrine ≥ 20 µg/kg/min, dopamine ≥ 10 µg/kg/min, phenylephrine ≥ 200 µg/kg/min, or epinephrine ≥ 10 µg/kg/min), or |
| | • Hypotension requiring multiple vasopressors (e.g., vasopressin + one of the above agents, or combination vasopressors equivalent to ≥ 20 µg/kg/min norepinephrine), or |
| | • Grade 3 organ toxicity or Grade 4 transaminitis (by CTCAE v4.0) |
| 4 | Life-threatening: |
| Life-threatening | • Requirement for ventilator support, or |
| | • Grade 4 organ toxicity (excluding transaminitis) |
| 5 Fatal | Death |

In some instances disclosed herein, outcomes associated with severe CRS or grade 3 CRS or greater, such as grade 4 or greater, include one or more of: persistent fever, e.g., fever of a specified temperature, e.g., greater than at or about 38 degrees Celsius, for two or more, e.g., three or more, e.g., four or more days or for at least three consecutive days; fever greater than at or about 38 degrees Celsius; elevation of cytokines, such as a max fold change, e.g., of at least at or about 75, compared to pre-treatment levels of at least two cytokines (e.g., at least two of the group consisting of interferon gamma (IFNγ), GM-CSF, IL-6, IL-10, Flt-3L, fracktalkine, and IL-5, and/or tumor necrosis factor alpha (TNFα)), or a max fold change, e.g., of at least at or about 250 of at least one of such cytokines; and/or at least one clinical sign of toxicity, such as hypotension (e.g., as measured by at least one intravenous vasoactive pressor); hypoxia (e.g., plasma oxygen (PO2) levels of less than at or about 90%); and/or one or more neurologic disorders (including mental status changes, obtundation, and seizures). In some instances disclosed herein, severe CRS includes CRS that requires management or care in the intensive care unit (ICU).

In some instances disclosed herein, the CRS, such as severe CRS, encompasses a combination of (1) persistent fever (fever of at least 38 degrees Celsius for at least three days) and (2) a serum level of CRP of at least at or about 20 mg/dL. In some instances disclosed herein, the CRS encompasses hypotension requiring the use of two or more vasopressors or respiratory failure requiring mechanical ventilation. In some instances disclosed herein, the dosage of vasopressors is increased in a second or subsequent administration.

In some instances disclosed herein, severe CRS or grade 3 CRS encompasses an increase in alanine aminotransferase, an increase in aspartate aminotransferase, chills, febrile neutropenia, headache, left ventricular dysfunction, encephalopathy, hydrocephalus, and/or tremor.

The method of measuring or detecting the various outcomes may be specified.

In some disclosures, the toxic outcome is or is associated with neurotoxicity. In some instances disclosed herein, symptoms associated with a clinical risk of neurotoxicity include confusion, delirium, aphasia, expressive aphasia, obtundation, myoclonus, lethargy, altered mental status, convulsions, seizure-like activity, seizures (optionally as confirmed by electroencephalogram [EEG]), elevated levels of beta amyloid (Aβ), elevated levels of glutamate, and elevated levels of oxygen radicals. In some instances disclosed herein, neurotoxicity is graded based on severity (e.g., using a Grade 1-5 scale (see, e.g., Guido Cavaletti & Paola Marmiroli Nature Reviews Neurology 6, 657-666 (December 2010); National Cancer Institute-Common Toxicity Criteria version 4.03 (NCI-CTCAE v4.03).

In some instances, neurologic symptoms may be the earliest symptoms of sCRS. In some instances disclosed herein, neurologic symptoms are seen to begin 5 to 7 days after cell therapy infusion. In some instances disclosed herein, duration of neurologic changes may range from 3 to 19 days. In some instances disclosed herein, recovery of neurologic changes occurs after other symptoms of sCRS have resolved. In some instances disclosed herein, time or degree of resolution of neurologic changes is not hastened by treatment with anti-IL-6 and/or steroid(s).

In some instances disclosed herein, a subject is deemed to develop "severe neurotoxicity" in response to or secondary to administration of a cell therapy or dose of cells thereof, if, following administration, the subject displays symptoms that limit self-care (e.g. bathing, dressing and undressing, feeding, using the toilet, taking medications) from among: 1) symptoms of peripheral motor neuropathy, including inflammation or degeneration of the peripheral motor nerves; 2) symptoms of peripheral sensory neuropathy, including inflammation or degeneration of the peripheral sensory nerves, dysesthesia, such as distortion of sensory perception, resulting in an abnormal and unpleasant sensation, neuralgia, such as intense painful sensation along a nerve or a group of nerves, and/or paresthesia, such as functional disturbances of sensory neurons resulting in abnormal cutaneous sensations of tingling, numbness, pressure, cold and warmth in the absence of stimulus. In some instances disclosed herein, severe neurotoxicity includes neurotoxicity with a grade of 3 or greater, such as set forth in Table 4.

| **Table 4:** | **Exemplary Grading Criteria for neurotoxicity** | |
|---|---|---|
| **Grade** | | **Description of Symptoms** |
| 1 Asymptomatic or Mild | | Mild or asymptomatic symptoms |
| 2 Moderate | | Presence of symptoms that limit instrumental activities of daily living (ADL), such as preparing meals, shopping for groceries or clothes, using the telephone, managing money |
| 3 Severe | | Presence of symptoms that limit self-care ADL, such as bathing, dressing and undressing, feeding self, using the toilet, taking medications |
| 4 Life-threatening | | Symptoms that are life-threatening, requiring urgent intervention |
| 5 Fatal | | Death |

In some instances disclosed herein, the methods reduce symptoms associated with CRS or neurotoxicity compared to other methods. In some disclosures, the disclosed methods reduce symptoms, outcomes or factors associated with CRS, including symptoms, outcomes or factors associated with severe CRS or grade 3 or higher CRS, compared to other methods. For example, subjects treated according to the present methods may lack detectable and/or have reduced symptoms, outcomes or factors of CRS, e.g. severe CRS or grade 3 or higher CRS, such as any described, e.g. set forth in Table 3. In some instances disclosed herein, subjects treated according to the present methods may have reduced symptoms of neurotoxicity, such as limb weakness or numbness, loss of memory, vision, and/or intellect, uncontrollable obsessive and/or compulsive behaviors, delusions, headache, cognitive and behavioral problems including loss of motor control, cognitive deterioration, and autonomic nervous system dysfunction, and sexual dysfunction, compared to subjects treated by other methods. In some instances disclosed herein, subjects treated according to the present methods may have reduced symptoms associated with peripheral motor neuropathy, peripheral sensory neuropathy, dysethesia, neuralgia or paresthesia.

In some instances disclosed herein, the methods reduce outcomes associated with neurotoxicity including damages to the nervous system and/or brain, such as the death of neurons. In some disclosures, the methods reduce the level of factors associated with neurotoxicity such as beta amyloid (Aβ), glutamate, and oxygen radicals.

In some instances disclosed herein, the toxicity outcome is a dose-limiting toxicity (DLT). In some instances disclosed herein, the toxic outcome is a dose-limiting toxicity. In some instances disclosed herein, the toxic outcome is the absence of a dose-limiting toxicity. In some instances disclosed herein, a dose-limiting toxicity (DLT) is defined as any grade 3 or higher toxicity as assessed by any known or published guidelines for assessing the particular toxicity, such as any described above and including the National Cancer Institute (NCI) Common Terminology Criteria for Adverse Events (CTCAE) version 4.0.

In some disclosures, a DLT can be described as any treatment-emergent grade 4 or 5 AEs, except those listed in the exceptions below; any treatment-emergent grade 3 AEs that do not resolve to grade ≤2 within 7 days, except those listed in the exceptions below; any treatment-emergent grade 3 seizures that do not resolve to grade ≤2 within 3 days; and any treatment-emergent autoimmune toxicity grade ≥3, with the exception of B-cell aplasia (which is an expected risk associated with administration of engineered cell); exceptions listed below are not considered DLTs: any treatment-emergent AE that is clearly unrelated to the administration of the engineered cells (e.g., motor vehicle accident); grade 4 infusional toxicities that are reversible to grade ≤2 in 8 hours; grade 3 or 4 fever or febrile neutropenia for ≤2 weeks; grade 4 transaminitis that is considered a symptom of CRS; grade 3 transaminitis for ≤2 weeks; grade 3 bone pain due to T-cell expansion in marrow compartments for ≤2 weeks; grade 3 or 4 TLS for ≤7 days; grade 3 or 4 hypotension (without other CRS symptoms) requiring a single vasopressor for support that resolves to grade <3 in ≤72 hours; grade 3 or 4 CRS with hypotension alone requiring a single vasopressor for support (not requiring intubation) that resolves to grade <3 in ≤72 hours, or grade 3 CRS with severity based on grade 4 transaminitis; grade 3 or 4 encephalopathy for ≤7 days that resolves to baseline in ≤14 days from the beginning of the grade ≥3 event; grade 3 chills; grade 3 or 4 lymphopenia; grade 3 or 4 leukopenia; grade 3 or 4 asymptomatic electrolyte abnormalities that resolve with replacement; grade 3 or 4 thrombocytopenia; grade 3 or 4 anemia; and grade 3 or 4 B-cell aplasia and hypogammaglobulinemia.

In some instances disclosed herein, the low rate, risk or likelihood of developing a toxicity, e.g. CRS or neurotoxicity or severe CRS or neurotoxicity, e.g. grade 3 or higher CRS or neurotoxicity, observed with administering a dose of T cells in accord with the disclosed methods, and/or with the disclosed articles of manufacture or compositions, permits administration of the cell therapy on an outpatient basis. In some instances disclosed herein, the administration of the cell therapy, e.g. dose of T cells (e.g. CAR+ T cells) in accord with the disclosed methods, and/or with the disclosed articles of manufacture or compositions, is performed on an outpatient basis or does not require admission to the subject to the hospital, such as admission to the hospital requiring an overnight stay.

In some disclosures, subjects administered the cell therapy, e.g. dose of T cells (e.g. CAR+ T cells) in accord with the disclosed methods, and/or with the disclosed articles of manufacture or compositions, including subjects treated on an outpatient basis, are not administered an intervention for treating any toxicity prior to or with administration of the cell dose, unless or until the subject exhibits a sign or symptom of a toxicity, such as of a neurotoxicity or CRS. Exemplary agents for treating, delaying, attenuating or ameliorating a toxicity are described in Section II.

In some instances disclosed herein, if a subject administered the cell therapy, e.g. dose of T cells (e.g. CAR+ T cells), including subjects treated on an outpatient basis, exhibits a fever the subject is given or is instructed to receive or administer a treatment to reduce the fever. In some instances disclosed herein, the fever in the subject is characterized as a body temperature of the subject that is (or is measured at) at or above a certain threshold temperature or level. In some disclosures, the threshold temperature is that associated with at least a low-grade fever, with at least a moderate fever, and/or with at least a high-grade fever. In some instances disclosed herein, the threshold temperature is a particular temperature or range. For example, the threshold temperature may be at or about or at least at or about 38, 39, 40, 41, or 42 degrees Celsius, and/or may be a range of at or about 38 degrees Celsius to at or about 39 degrees Celsius, a range of at or about 39 degrees Celsius to at or about 40 degrees Celsius, a range of at or about 40 degrees Celsius to at or about 41 degrees, or a range of at or about 41 degrees Celsius to at or about 42 degrees Celsius.

In some instances disclosed herein, the treatment designed to reduce fever includes treatment with an antipyretic. An antipyretic may include any agent, e.g., compound, composition, or ingredient, that reduces fever, such as one of any number of agents known to have antipyretic effects, such as NSAIDs (such as ibuprofen, naproxen, ketoprofen, and nimesulide), salicylates, such as aspirin, choline salicylate, magnesium salicylate, and sodium salicylate, paracetamol, acetaminophen, Metamizole, Nabumetone, Phenaxone, antipyrine, febrifuges. In some instances disclosed herein, the antipyretic is acetaminophen. In some instances disclosed herein, acetaminophen can be administered at a dose of 12.5 mg/kg orally or intravenously up to every four hours. In some instances disclosed herein, it is or comprises ibuprofen or aspirin.

In some instances disclosed herein, if the fever is a sustained fever, the subject is administered an alternative treatment for treating the toxicity, such as any described in Section II below. For subjects treated on an outpatient basis, the subject is instructed to return to the hospital if the subject has and/or is determined to or to have a sustained fever. In some instances disclosed herein, the subject has, and/or is determined to or considered to have, a sustained fever if he or she exhibits a fever at or above the relevant threshold temperature, and where the fever or body temperature of the subject is not reduced, or is not reduced by or by more than a specified amount (e.g., by more than 1 °C, and generally does not fluctuate by about, or by more than about, 0.5 °C, 0.4 °C, 0.3 °C, or 0.2 °C), following a specified treatment, such as a treatment designed to reduce fever such as treatment with an antipyretic, e.g. NSAID or salicylates, e.g. ibuprofen, acetaminophen or aspirin. For example, a subject is considered to have a sustained fever if he or she exhibits or is determined to exhibit a fever of at least at or about 38 or 39 degrees Celsius, which is not reduced by or is not reduced by more than at or about 0.5 °C, 0.4 °C, 0.3 °C, or 0.2 °C, or by at or about 1%, 2%, 3%, 4%, or 5%, over a period of 6 hours, over a period of 8 hours, or over a period of 12 hours, or over a period of 24 hours, even following treatment with the antipyretic such as acetaminophen. In some instances disclosed herein, the dosage of the antipyretic is a dosage ordinarily effective in such as subject to reduce fever or fever of a particular type such as fever associated with a bacterial or viral infection, e.g., a localized or systemic infection.

In some instances disclosed herein, the subject has, and/or is determined to or considered to have, a sustained fever if he or she exhibits a fever at or above the relevant threshold temperature, and where the fever or body temperature of the subject does not fluctuate by about, or by more than about, 1 °C, and generally does not fluctuate by about, or by more than about, 0.5 °C, 0.4 °C, 0.3 °C, or 0.2 °C. Such absence of fluctuation above or at a certain amount generally is measured over a given period of time (such as over a 24-hour, 12-hour, 8-hour, 6-hour, 3-hour, or 1-hour period of time, which may be measured from the first sign of fever or the first temperature above the indicated threshold). For example, in some instances disclosed herein, a subject is considered to or is determined to exhibit sustained fever if he or she exhibits a fever of at least at or about or at least at or about 38 or 39 degrees Celsius, which does not fluctuate in temperature by more than at or about 0.5°C, 0.4 °C, 0.3 °C, or 0.2 °C, over a period of 6 hours, over a period of 8 hours, or over a period of 12 hours, or over a period of 24 hours.

In some instances disclosed herein, the fever is a sustained fever; in some disclosures, the subject is treated at a time at which a subject has been determined to have a sustained fever, such as within one, two, three, four, five six, or fewer hours of such determination or of the first such determination following the initial therapy having the potential to induce the toxicity, such as the cell therapy, such as dose of T cells, e.g. CAR+ T cells.

In some instances disclosed herein, one or more interventions or agents for treating the toxicity, such as a toxicity-targeting therapies, is administered at a time at which or immediately after which the subject is determined to or confirmed to (such as is first determined or confirmed to) exhibit sustained fever, for example, as measured according to any of the aforementioned instances disclosed herein. In some instances disclosed herein, the one or more toxicity-targeting therapies is administered within a certain period of time of such confirmation or determination, such as within 30 minutes, 1 hour, 2 hours, 3 hours, 4 hours, 6 hours, or 8 hours thereof.

### E. Biomarkers

Among the disclosed methods are methods of treatment that involves assessing a risk for developing toxicity associated with cell therapy in a subject that involves assessing or detecting biomarkers (*e.g.,* analytes) or parameters that are associated with the toxicity, *e.g.,* neurotoxicity, such as severe neurotoxicity, and/or CRS, such as severe CRS. In some disclosures, the subject is administered a therapy, e.g., engineered T cell therapy, based on the assessment. In some instances disclosed herein, the biomarkers include TNF-alpha (TNFα) and interleukin-16 (IL-16).

In some instances disclosed herein, the methods involve assessing or detecting the presence or absence of biomarkers (e.g. TNFα or IL-16) and/or parameters (e.g. concentration, amount, level or activity) associated with biomarkers (e.g. TNFα or IL-16). In some instances disclosed herein, the methods can include comparing the one or more parameters to a particular reference value, such as a threshold level (also called "threshold value" herein), *e.g*., those associated with a risk for developing toxicity, such as CRS or NT, and/or severe toxicity, e.g., severe CRS or NT. In some instances disclosed herein, the methods also involve selecting subjects for treatment with a cell therapy based on the assessment of the presence or absence of the biomarker and/or comparison of the biomarkers to a reference value or threshold level of the biomarker. In some instances disclosed herein, the methods also involve administering an agent or a therapy that can treat, prevent, delay and/or attenuate development of the toxicity, *e.g.*, based on the assessment of the presence or absence of the biomarker and/or comparison of the biomarkers to a reference value or threshold level of the biomarker.

In some instances disclosed herein, the methods involve assessing the risk of development of a toxicity, before and/or after administration of a cell therapy. In some instances disclosed herein, the methods involve assessing the level, amount or concentration of the biomarkers (e.g. TNFα or IL-16) in a biological sample, wherein the biological sample is from a subject that is a candidate for treatment with the cell therapy, said cell therapy optionally comprising a dose or composition of genetically engineered cells expressing a recombinant receptor; and the biological sample is obtained from the subject prior to administering the cell therapy and/or said biological sample does not comprise the recombinant receptor and/or said engineered cells. In some disclosures, the methods involve comparing, individually, the level, amount or concentration of the analyte in the sample to a threshold level, thereby determining a risk of developing a toxicity after administration of the cell therapy. In some disclosures, the comparisons can be used to determine the risk of development of a toxicity, after administration of a cell therapy.

In some instances disclosed herein, the methods involve assaying a biological sample for the level, amount or concentration of TNF-alpha, wherein the biological sample is from a subject that is a candidate for treatment, optionally with a cell therapy, said cell therapy comprising a dose of engineered cells comprising T cells expressing a CAR for treating a disease or condition, wherein the biological sample is obtained from the subject prior to administering the cell therapy and/or said biological sample does not comprise the CAR and/or said engineered cells; and comparing the level, amount or concentration of TNF-alpha to a threshold level, wherein: (1) if the level, amount or concentration of TNF-alpha is at or above a threshold level, identifying the subject as at risk for developing a grade 3 or higher neurotoxicity following administration of the cell therapy; and (2) if the level, amount or concentration of TNF-alpha is below the threshold level, identifying the subject as not at risk for developing a grade 3 or higher neurotoxicity following administration of the cell therapy.

In some instances disclosed herein, if the subject is identified as at risk for developing a grade 3 or higher neurotoxicity, the method further involves: (i) administering to the subject the cell therapy, optionally at a reduced dose, optionally wherein (a) the method further comprises administering to the subject an agent or other treatment capable of treating, preventing, delaying, reducing or attenuating the development or risk of development of the neurotoxicity; and/or (b) the administering the cell therapy to the subject is carried out or is specified to be carried out in an in-patient setting and/or with admission to the hospital for one or more days; or (ii) administering to the subject an alternative treatment other than the cell therapy for treating the disease or condition.

In some instances disclosed herein, if the subject is identified as not at risk for developing a grade 3 or higher neurotoxicity following administration of the cell therapy: (i) the subject is not administered an agent or other treatment capable of treating, preventing, delaying, reducing or attenuating the development or risk of development of a toxicity unless or until the subjects exhibits a sign or symptom of a toxicity, optionally at or after the subject exhibits a sustained fever or a fever that is or has not been reduced or not reduced by more than 1°C after treatment with an antipyretic; and/or (ii) the administration and any follow-up is carried out on an outpatient basis and/or without admitting the subject to a hospital and/or without an overnight stay at a hospital and/or without requiring admission to or an overnight stay at a hospital, optionally unless or until the subject exhibits a sustained fever or a fever that is or has not been reduced or not reduced by more than 1°C after treatment with an antipyretic.

In some instances disclosed herein, the the assaying method involves: (a) contacting a biological sample with one or more reagent capable of detecting or that is specific for TNF-alpha, optionally wherein the one or more reagent comprises an antibody that specifically recognizes TNF-alpha; and (b) detecting the presence or absence of a complex comprising the one or more reagent and TNF-alpha.

In some instances disclosed herein, the method involves administering to a subject a cell therapy for treating a disease or condition, said cell therapy comprising a dose of engineered cells comprising T cells expressing a CAR, wherein: (1) if the subject has a level, amount or concentration of TNF-alpha in a biological sample from the subject that is at or above a threshold level, the subject is identified as at risk of developing grade 3 or higher neurotoxicity following administration of the cell therapy: (i) administering to the subject the cell therapy at a reduced dose, (ii) further administering to the subject an agent or other treatment capable of treating, preventing, delaying, reducing or attenuating the development or risk of development of a toxicity; and/or (iii) the administering the cell therapy to the subject is carried out or is specified to be carried out in an in-patient setting and/or with admission to the hospital for one or more days; or (2) if the subject is selected or identified as having a level, amount or concentration of TNF-alpha in a biological sample from the subject that is below a threshold level, the subject is identified as not at risk of developing grade 3 or higher neurotoxicity following administration of the cell therapy: (i) not administering to the subject an agent or other treatment capable of treating, preventing, delaying, reducing or attenuating the development or risk of development of a toxicity unless or until the subjects exhibits a sign or symptom of a toxicity, optionally at or after the subject exhibits a sustained fever or a fever that is or has not been reduced or not reduced by more than 1°C after treatment with an antipyretic; and/or (ii) the administering and any follow-up is carried out on an outpatient basis and/or without admitting the subject to a hospital and/or without an overnight stay at a hospital and/or without requiring admission to or an overnight stay at a hospital, optionally unless or until the subject exhibits a sustained fever or a fever that is or has not been reduced or not reduced by more than 1°C after treatment with an antipyretic, wherein the subject is a candidate for treatment with the cell therapy, said biological sample obtained from the subject prior to administering the cell therapy and/or said biological sample does not comprise the CAR and/or said engineered cells.

In some instances disclosed herein, if the subject is identified as at risk of developing grade 3 or higher neurotoxicity following administration of the cell therapy, the method involves administering the agent or other treatment capable of treating, preventing, delaying, reducing or attenuating the development or risk of development of a toxicity, wherein the agent is administered to the subject concurrently with the cell therapy or within three days of administering the cell therapy to the subject.

In some instances disclosed herein, the method includes (a) assaying a biological sample from a subject for the level, amount or concentration of IL-16, said subject having received administration of a cell therapy comprising a dose of engineered cells comprising T cells expressing a CAR for treating a disease or condition, wherein the biological sample is obtained from the subject within one, two, or three days after the initiation of administration of the cell therapy; and (b) comparing the level, amount or concentration of IL-16 to a threshold level, wherein: (1) if the level, amount or concentration of IL-16 is at or above a threshold level, identifying the subject as at risk for developing a grade 3 or higher neurotoxicity; and (2) if the level, amount or concentration of IL-16 is below the threshold level, identifying the subject as not at risk for developing a grade 3 or higher neurotoxicity.

In some instances disclosed herein, if the subject is identified at risk of developing a grade 3 or higher neurotoxicity, the method involves administering an agent or other treatment capable of treating, preventing, delaying, reducing or attenuating the development or risk of development of a toxicity. In some instances disclosed herein, the assaying involves (a) contacting a biological sample with one or more reagent capable of detecting or that is specific for IL-16, optionally wherein the one or more reagent comprises an antibody that specifically recognizes IL-16; and (b) detecting the presence or absence of a complex comprising the one or more reagent and IL-16. In some instances disclosed herein, the method involves prior to the assaying, administering to the subject the cell therapy.

In some instances disclosed herein, the method involves administering to a subject, identified as at risk of developing a grade 3 or higher neurotoxicity, an agent or other treatment capable of treating, preventing, delaying, reducing or attenuating the development or risk of development of a toxicity, said subject having previously received administration of a cell therapy for treating a disease or condition, wherein, at or immediately prior to administering the agent, the subject is selected or identified as being at risk of developing a grade 3 or higher neurotoxicity if the level or amount or concentration of IL-16 in a biological sample, obtained from the subject within one, two, or three days of the initiation of administration of the cell therapy, is above a threshold level. In some embodiemtns, the administering the agent is carried out at a time when the subject exhibits a sustained fever or a fever that is or has not been reduced or not reduced by more than 1°C after treatment with an antipyretic. In some embodiemnents, the administering to the subject the cell therapy was carried out on an outpatient basis and, if the level, amount or concentration of IL-16 is above a threshold level the method comprises admitting the patient to the hospital for one or more days.

In some instances disclosed herein, the biological sample is or is obtained from a blood, plasma or serum sample. In some instances disclosed herein, the assaying comprises an immunoassay.In some instances disclosed herein is a cell therapy for use in a method of treatment, wherein the method comprises: assaying a biological sample for the level, amount or concentration of TNF-alpha, wherein the biological sample is from a subject that is a candidate for treatment, optionally with a cell therapy, said cell therapy comprising a dose of engineered cells comprising T cells expressing a CAR for treating a disease or condition, wherein the biological sample is obtained from the subject prior to administering the cell therapy and/or said biological sample does not comprise the CAR and/or said engineered cells; comparing the level, amount or concentration of TNF-alpha to a threshold level, wherein: (1) if the level, amount or concentration of TNF-alpha is at or above a threshold level, identifying the subject as at risk for developing a grade 3 or higher neurotoxicity following administration of the cell therapy; and (2) if the level, amount or concentration of TNF-alpha is below the threshold level, identifying the subject as not at risk for developing a grade 3 or higher neurotoxicity following administration of the cell therapy; and administering to the subject the cell therapy, optionally at a reduced dose, optionally wherein (a) the method further comprises administering to the subject an agent or other treatment capable of treating, preventing, delaying, reducing or attenuating the development or risk of development of the neurotoxicity; and/or (b) the administering to the subject of the cell therapy is carried out or is specified to be carried out in an in-patient setting and/or with admission to the hospital for one or more days.

In some of any instances disclosed herein is an agent or other treatment capable of treating, preventing, delaying, reducing or attenuating the development or risk of development of a neurotoxicity for use in a method of reducing neurotoxicity after administration of a cell therapy, wherein the method comprises: assaying a biological sample for the level, amount or concentration of TNF-alpha, wherein the biological sample is from a subject that is a candidate for treatment, optionally with a cell therapy, said cell therapy comprising a dose of engineered cells comprising T cells expressing a CAR for treating a disease or condition, wherein the biological sample is obtained from the subject prior to administering the cell therapy and/or said biological sample does not comprise the CAR and/or said engineered cells; comparing the level, amount or concentration of TNF-alpha to a threshold level, wherein: (1) if the level, amount or concentration of TNF-alpha is at or above a threshold level, identifying the subject as at risk for developing a grade 3 or higher neurotoxicity following administration of the cell therapy; and (2) if the level, amount or concentration of TNF-alpha is below the threshold level, identifying the subject as not at risk for developing a grade 3 or higher neurotoxicity following administration of the cell therapy; and administering to the subject the agent or other treatment and cell therapy, optionally at a reduced dose and/or (b) the administering to the subject of the cell therapy is carried out or is specified to be carried out in an in-patient setting and/or with admission to the hospital for one or more days.

In some instances disclosed herein is a cell therapy for use in a method of treatment, wherein the method comprises administering to a subject the cell therapy, optionally at a reduced dose, optionally wherein (a) the method further comprises administering to the subject an agent or other treatment capable of treating, preventing, delaying, reducing or attenuating the development or risk of development of the neurotoxicity; and/or (b) the administering to the subject of the cell therapy is carried out or is specified to be carried out in an in-patient setting and/or with admission to the hospital for one or more days, wherein said patient has been identified as at risk for developing a grade 3 or higher neurotoxicity following administration of the cell therapy on the basis of a method comprising: assaying a biological sample from the subject that is a candidate for treatment with a cell therapy, said cell therapy comprising a dose of engineered cells comprising T cells expressing a CAR for treating a disease or condition, wherein the biological sample is obtained from the subject prior to administering the cell therapy and/or said biological sample does not comprise the CAR and/or said engineered cells for the level, amount or concentration of TNF-alpha, and comparing the level, amount or concentration of TNF-alpha to a threshold level, wherein: (1) if the level, amount or concentration of TNF-alpha is at or above a threshold level, identifying the subject as at risk for developing a grade 3 or higher neurotoxicity following administration of the cell therapy; and (2) if the level, amount or concentration of TNF-alpha is below the threshold level, identifying the subject as not at risk for developing a grade 3 or higher neurotoxicity following administration of the cell therapy.

In some instances disclosed herein is an agent or other treatment capable of treating, preventing, delaying, reducing or attenuating the development or risk of development of a neurotoxicity for use in a method of reducing neurotoxicity after administration of a cell therapy, wherein the method comprises: administering to a subject the cell therapy, optionally at a reduced dose, and the agent or other treatment, optionally wherein the administering to the subject of the cell therapy and the agent or other treatment is carried out or is specified to be carried out in an in-patient setting and/or with admission to the hospital for one or more days, wherein said patient has been identified as at risk for developing a grade 3 or higher neurotoxicity following administration of the cell therapy on the basis of a method comprising: assaying a biological sample from the subject that is a candidate for treatment with a cell therapy, said cell therapy comprising a dose of engineered cells comprising T cells expressing a CAR for treating a disease or condition, wherein the biological sample is obtained from the subject prior to administering the cell therapy and/or said biological sample does not comprise the CAR and/or said engineered cells for the level, amount or concentration of TNF-alpha, and comparing the level, amount or concentration of TNF-alpha to a threshold level, wherein: (1) if the level, amount or concentration of TNF-alpha is at or above a threshold level, identifying the subject as at risk for developing a grade 3 or higher neurotoxicity following administration of the cell therapy; and (2) if the level, amount or concentration of TNF-alpha is below the threshold level, identifying the subject as not at risk for developing a grade 3 or higher neurotoxicity following administration of the cell therapy.

### II. INTERVENTIONS OR AGENTS THAT TREAT OR AMELIORATE SYMPTOMS OF TOXICITY

In some instances disclosed herein, the disclosed methods and articles of manufacture can be used in connection with, or involve or include, one or more agents or treatments for treating, preventing, delaying, or attenuating the development of a toxicity. In some instances disclosed herein, the agent or other treatment capable of treating, preventing, delaying, or attenuating the development of a toxicity is administered prior to and/or concurrently with administration of a therapeutic cell composition comprising the genetically engineered cells.

In some instances disclosed herein, the agent, e.g., a toxicity-targeting agent, or treatment capable of treating, preventing, delaying, or attenuating the development of a toxicity is a steroid, is an antagonist or inhibitor of a cytokine receptor, such as IL-6 receptor, CD 122 receptor (IL-2Rbeta receptor), or CCR2, or is an inhibitor of a cytokine, such as IL-6, MCP-1, IL-10, IFN-γ, IL-8, or IL-18. In some instances disclosed herein, the agent is an agonist of a cytokine receptor and/or cytokine, such as TGF-β. In some instances disclosed herein, the agent, e.g., agonist, antagonist or inhibitor, is an antibody or antigen-binding fragment, a small molecule, a protein or peptide, or a nucleic acid.

In some instances disclosed herein, a fluid bolus can be employed as an intervention, such as to treat hypotension associated with CRS. In some instances disclosed herein, the target hematocrit levels are >24%. In some instances disclosed herein, the intervention includes the use of absorbent resin technology with blood or plasma filtration. In some instances disclosed herein, the intervention includes dialysis, plasmapheresis, or similar technologies. In some instances disclosed herein, vasopressors or acetaminophen can be employed.

In some instances disclosed herein, the agent can be administered sequentially, intermittently, or at the same time as or in the same composition as the therapy, such as cells for adoptive cell therapy. For example, the agent can be administered before, during, simultaneously with, or after administration of the immunotherapy and/or cell therapy.

In some instances disclosed herein, the agent is administered at a time as described herein and in accord with the disclosed methods, and/or with the disclosed articles of manufacture or compositions. In some instances disclosed herein, the toxicity-targeting agent is administered at a time that is within, such as less than or no more than, 3, 4, 5, 6, 7, 8, 9 or 10 days after initiation of the immunotherapy and/or cell therapy. In some instances disclosed herein, the toxicity-targeting agent is administered within or within about 1 day, 2 days or 3 days after initiation of administration of the immunotherapy and/or cell therapy.

In some instances disclosed herein, the agent, e.g., toxicity-targeting agent, is administered to a subject after initiation of administration of the immunotherapy and/or cell therapy at a time at which the subject does not exhibit grade 2 or higher CRS or grade 2 or higher neurotoxicity. In some disclosures, the toxicity-targeting agent is administered after initiation of administration of the immunotherapy and/or cell therapy at a time at which the subject does not exhibit severe CRS or severe neurotoxicity. Thus, between initiation of administration of the immunotherapy and/or cell therapy and the toxicity-targeting agent, the subject is one that does not exhibit grade 2 or higher CRS, such as severe CRS, and/or does not exhibit grade 2 or higher neurotoxicity, such as severe neurotoxicity.

Non-limiting examples of interventions for treating or ameliorating a toxicity, such as severe CRS (sCRS) or severe neurotoxicity, are described in **Table 5.** In some instances disclosed herein, the intervention includes tocilizumab or other toxicity-targeting agent as described, which can be at a time in which there is a sustained or persistent fever of greater than or about 38 °C or greater than or greater than about 39 °C in the subject. In some instances disclosed herein, the fever is sustained in the subject for more than 10 hours, more than 12 hours, more than 16 hours, or more than 24 hours before intervention.

| **Table 5. Exemplary Interventions.** | |
|---|---|
| **Symptoms related to CRS** | **Suggested Intervention** |
| Fever of ≥ 38.3°C | Acetaminophen (12.5 mg/kg) PO/IV up to every four hours |
| Persistent fever of ≥ 39°C for 10 hours that is unresponsive to acetaminophen | Tocilizumab (8-12 mg/kg) IV |
| Persistent fever of ≥ 39°C after tocilizumab | Dexamethasone 5-10 mg IV/PO up to every 6-12 hours with continued fevers |
| Recurrence of symptoms 48 hours after initial dose of tocilizumab | Tocilizumab (8-12 mg/kg) IV |
| Hypotension | Fluid bolus, target hematocrit >24% |
| Persistent/recurrent hypotension after initial fluid bolus (within 6 hours) | Tocilizumab (8-12 mg/kg) IV |
| Use of low dose pressors for hypotension for longer than 12 hours | Dexamethasone 5-10 mg IV/PO up to every 6 hours with continued use of pressors |
| Initiation of higher dose pressors or addition of a second pressor for hypotension | Dexamethasone 5-10 mg IV/PO up to every 6 hours with continued use of pressors |
| Initiation of oxygen supplementation | Tocilizumab (8-12 mg/kg) IV |
| Increasing respiratory support with concern for impending intubation | Dexamethasone 5-10 mg IV/PO up to every 6 hours with continued use of pressors |
| Recurrence/Persistence of symptoms for which tocilizumab was given ≥ 48 hours after initial dose was administered | Tocilizumab (8-12 mg/kg) IV |

In some instances disclosed herein, the agent or therapy or intervention, e.g., toxicity-targeting agent, is administered alone or is administered as part of a composition or formulation, such as a pharmaceutical composition or formulation, as described herein. Thus, the agent alone or as part of a pharmaceutical composition can be administered intravenously or orally, or by any other acceptable known route of administration or as described herein.

In some instances disclosed herein, the dosage of agent or the frequency of administration of the agent in a dosage regimen is reduced compared to the dosage of the agent or its frequency in a method in which a subject is treated with the agent after grade 2 or higher CRS or neurotoxicity, such as after severe, e.g., grade 3 or higher, CRS or after severe, e.g., grade 3 or higher neurotoxicity, has developed or been diagnosed (e.g. after physical signs or symptoms of grade 3 or higher CRS or neurotoxicity has manifested). In some instances disclosed herein, the dosage of agent or the frequency of administration of the agent in a dosage regimen is reduced compared to the dosage of the agent or its frequency in a method in which a subject is treated for CRS or neurotoxicity greater than 3 days, 4 days, 5 days, 6 days, 1 week, 2 weeks, three weeks, or more after administration of the immunotherapy and/or cell therapy. In some instances disclosed herein, the dosage is reduced by greater than or greater than about 1.2-fold, 1.5-fold, 2-fold, 3-fold, 4-fold, 5-fold, 6-fold, 7-fold, 8-fold, 9-fold, 10-fold or more. In some instances disclosed herein, the dosage is reduced by greater than or about 10%, 20%, 30%, 40%, 50%, 60%, 70%, 80%, 90%, or more. In some instances disclosed herein, the frequency of dosing is reduced, such as the number of daily doses is reduced or the number of days of dosing is reduced.

### A. Steroid

In some instances disclosed herein, the agent, e.g., toxicity-targeting agent, that treats and/or that prevents, delays, or attenuates the development of or risk for developing a toxicity to an immunotherapy and/or a cell therapy, is a steroid, e.g., corticosteroid. Corticosteroids typically include glucocorticoids and mineralocorticoids.

Any corticosteroid, e.g., glucocorticoid, can be used in the methods disclosed herein. In some instances disclosed herein, glucocorticoids include synthetic and non-synthetic glucocorticoids. Exemplary glucocorticoids include, but are not limited to: alclomethasones, algestones, beclomethasones (e.g. beclomethasone dipropionate), betamethasones (e.g. betamethasone 17-valerate, betamethasone sodium acetate, betamethasone sodium phosphate, betamethasone valerate), budesonides, clobetasols (e.g. clobetasol propionate), clobetasones, clocortolones (e.g. clocortolone pivalate), cloprednols, corticosterones, cortisones and hydrocortisones (e.g. hydrocortisone acetate), cortivazols, deflazacorts, desonides, desoximethasones, dexamethasones (e.g. dexamethasone 21-phosphate, dexamethasone acetate, dexamethasone sodium phosphate), diflorasones (e.g. diflorasone diacetate), diflucortolones, difluprednates, enoxolones, fluazacorts, flucloronides, fludrocortisones (e.g., fludrocortisone acetate), flumethasones (e.g. flumethasone pivalate), flunisolides, fluocinolones (e.g. fluocinolone acetonide), fluocinonides, fluocortins, fluocortolones, fluorometholones (e.g. fluorometholone acetate), fluperolones (e.g., fluperolone acetate), fluprednidenes, fluprednisolones, flurandrenolides, fluticasones (e.g. fluticasone propionate), formocortals, halcinonides, halobetasols, halometasones, halopredones, hydrocortamates, hydrocortisones (e.g. hydrocortisone 21-butyrate, hydrocortisone aceponate, hydrocortisone acetate, hydrocortisone buteprate, hydrocortisone butyrate, hydrocortisone cypionate, hydrocortisone hemisuccinate, hydrocortisone probutate, hydrocortisone sodium phosphate, hydrocortisone sodium succinate, hydrocortisone valerate), loteprednol etabonate, mazipredones, medrysones, meprednisones, methylprednisolones (methylprednisolone aceponate, methylprednisolone acetate, methylprednisolone hemisuccinate, methylprednisolone sodium succinate), mometasones (e.g., mometasone furoate), paramethasones (e.g., paramethasone acetate), prednicarbates, prednisolones (e.g. prednisolone 25-diethylaminoacetate, prednisolone sodium phosphate, prednisolone 21-hemisuccinate, prednisolone acetate; prednisolone famesylate, prednisolone hemisuccinate, prednisolone-21 (beta-D-glucuronide), prednisolone metasulphobenzoate, prednisolone steaglate, prednisolone tebutate, prednisolone tetrahydrophthalate), prednisones, prednivals, prednylidenes, rimexolones, tixocortols, triamcinolones (e.g. triamcinolone acetonide, triamcinolone benetonide, triamcinolone hexacetonide, triamcinolone acetonide 21-palmitate, triamcinolone diacetate). These glucocorticoids and the salts thereof are discussed in detail, for example, in Remington's Pharmaceutical Sciences, A. Osol, ed., Mack Pub. Co., Easton, Pa. (16th ed. 1980).

In some instances disclosed herein, the glucocorticoid is selected from among cortisones, dexamethasones, hydrocortisones, methylprednisolones, prednisolones and prednisones. In a particular example, the glucocorticoid is dexamethasone.

In some instances disclosed herein, the agent is a corticosteroid and is administered in an amount that is therapeutically effective to treat, ameliorate or reduce one or more symptoms of a toxicity to an immunotherapy and/or a cell therapy, such as CRS or neurotoxicity. In some instances disclosed herein, indicators of improvement or successful treatment include determination of the failure to manifest a relevant score on toxicity grading scale (e.g. CRS or neurotoxicity grading scale), such as a score of less than 3, or a change in grading or severity on the grading scale as discussed herein, such as a change from a score of 4 to a score of 3, or a change from a score of 4 to a score of 2, 1 or 0.

In some disclosures, the corticosteroid is provided in a therapeutically effective dose. Therapeutically effective concentration can be determined empirically by testing in known in vitro or in vivo (e.g. animal model) systems. For example, the amount of a selected corticosteroid to be administered to ameliorate symptoms or adverse effects of a toxicity to an immunotherapy and/or a cell therapy, such as CRS or neurotoxicity, can be determined by standard clinical techniques. In addition, animal models can be employed to help identify optimal dosage ranges. The precise dosage, which can be determined empirically, can depend on the particular therapeutic preparation, the regime and dosing schedule, the route of administration and the seriousness of the disease.

The corticosteroid can be administered in any amount that is effective to ameliorate one or more symptoms associated with the toxicity, such as with the CRS or neurotoxicity. The corticosteroid, e.g., glucocorticoid, can be administered, for example, at an amount between at or about 0.1 and 100 mg, per dose, 0.1 to 80 mg, 0.1 to 60 mg, 0.1 to 40 mg, 0.1 to 30 mg, 0.1 to 20 mg, 0.1 to 15 mg, 0.1 to 10 mg, 0.1 to 5 mg, 0.2 to 40 mg, 0.2 to 30 mg, 0.2 to 20 mg, 0.2 to 15 mg, 0.2 to 10 mg, 0.2 to 5 mg, 0.4 to 40 mg, 0.4 to 30 mg, 0.4 to 20 mg, 0.4 to 15 mg, 0.4 to 10 mg, 0.4 to 5 mg, 0.4 to 4 mg, 1 to 20 mg, 1 to 15 mg or 1 to 10 mg, to a 70 kg adult human subject. Typically, the corticosteroid, such as a glucocorticoid is administered at an amount between at or about 0.4 and 20 mg, for example, at or about 0.4 mg, 0.5 mg, 0.6 mg, 0.7 mg, 0.75 mg, 0.8 mg, 0.9 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg or 20 mg per dose, to an average adult human subject.

In some instances disclosed herein, the corticosteroid can be administered, for example, at a dosage of at or about 0.001 mg/kg (of the subject), 0.002 mg/kg, 0.003 mg/kg, 0.004 mg/kg, 0.005 mg/kg, 0.006 mg/kg, 0.007 mg/kg, 0.008 mg/kg, 0.009 mg/kg, 0.01 mg/kg, 0.015 mg/kg, 0.02 mg/kg, 0.025 mg/kg, 0.03 mg/kg, 0.035 mg/kg, 0.04 mg/kg, 0.045 mg/kg, 0.05 mg/kg, 0.055 mg/kg, 0.06 mg/kg, 0.065 mg/kg, 0.07 mg/kg, 0.075 mg/kg, 0.08 mg/kg, 0.085 mg/kg, 0.09 mg/kg, 0.095 mg/kg, 0.1 mg/kg, 0.15 mg/kg, 0.2 mg/kg, 0.25 mg/kg, 0.30 mg/kg, 0.35 mg/kg, 0.40 mg/kg, 0.45 mg/kg, 0.50 mg/kg, 0.55 mg/kg, 0.60 mg/kg, 0.65 mg/kg, 0.70 mg/kg, 0.75 mg/kg, 0.80 mg/kg, 0.85 mg/kg, 0.90 mg/kg, 0.95 mg/kg, 1 mg/kg, 1.05 mg/kg, 1.1 mg/kg, 1.15 mg/kg, 1.20 mg/kg, 1.25 mg/kg, 1.3 mg/kg, 1.35 mg/kg or 1.4 mg/kg, to an average adult human subject, typically weighing about 70 kg to 75 kg.

The corticosteroid, or glucocorticoid, for example dexamethasone, can be administered orally (tablets, liquid or liquid concentrate), PO, intravenously (IV), intramuscularly or by any other known route or route described herein (e.g., with respect to pharmaceutical formulations). In some disclosures, the corticosteroid is administered as a bolus, and in other disclosures it may be administered over a period of time.

In some disclosures, the glucocorticoid can be administered over a period of more than one day, such as over two days, over 3 days, or over 4 or more days. In some instances disclosed herein, the corticosteroid can be administered one per day, twice per day, or three times or more per day. For example, the corticosteroid, e.g., dexamethasone, may in some instances disclosed herein be administered at 10 mg (or equivalent) IV twice a day for three days.

In some instances disclosed herein, the dosage of corticosteroid, e.g., glucocorticoid, is administered in successively lower dosages per treatment. Hence, in some such treatment regimes, the dose of corticosteroid is tapered. For example, the corticosteroid may be administered at an initial dose (or equivalent dose, such as with reference to dexamethasone) of 4 mg, and upon each successive administration the dose may be lowered, such that the dose is 3 mg for the next administration, 2 mg for the next administration, and 1 mg for the next administration

Generally, the dose of corticosteroid administered is dependent upon the specific corticosteroid, as a difference in potency exists between different corticosteroids. It is typically understood that drugs vary in potency, and that doses can therefore vary, in order to obtain equivalent effects. **Table** 6 shows equivalence in terms of potency for various glucocorticoids and routes of administration. Equivalent potency in clinical dosing is well known. Information relating to equivalent steroid dosing (in a non-chronotherapeutic manner) may be found in the British National Formulary (BNF) 37, March 1999.

| **Table 6: Glucocorticoid administration** | |
|---|---|
| **Glucocorticoid (Route)** | **Equivalency Potency** |
| Hydrocortisone (IV or PO) | 20 |
| Prednisone | 5 |
| Prednisolone (IV or PO) | 5 |
| Methylprednisolone sodium succinate (IV) | 4 |
| Dexamethasone (IV or PO) | 0.5-0.75 |

Thus, in some instances disclosed herein, the steroid is administered in an equivalent dosage amount of from or from about 1.0 mg to 20 mg dexamethasone per day, such as 1.0 mg to 15 mg dexamethasone per day, 1.0 mg to 10 mg dexamethasone per day, 2.0 mg to 8 mg dexamethasone per day, or 2.0 mg to 6.0 mg dexamethasone per day, each inclusive. In some instances disclosed herein, the steroid is administered in an equivalent dose of at or about 4 mg or at or about 8 mg dexamethasone per day.

In some instances disclosed herein, the steroid is administered if fever persists after treatment with tocilizumab. For example, in some instances disclosed herein, dexamethasone is administered orally or intravenously at a dosage of 5-10 mg up to every 6-12 hours with continued fevers. In some instances disclosed herein, tocilizumab is administered concurrently with or subsequent to oxygen supplementation.

### B. Microglial Cell Inhibitor

In some instances disclosed herein, the inhibitor in the combination therapy is an inhibitor of a microglial cell activity. In some instances disclosed herein, the administration of the inhibitor modulates the activity of microglia. In some instances disclosed herein, the inhibitor is an antagonist that inhibits the activity of a signaling pathway in microglia. In some instances disclosed herein, the microglia inhibitor affects microglial homeostasis, survival, and/or proliferation. In some instances disclosed herein, the inhibitor targets the CSF1R signaling pathway. In some instances disclosed herein, the inhibitor is an inhibitor of CSF1R. In some instances disclosed herein, the inhibitor is a small molecule. In some instances disclosed herein, the inhibitor is an antibody.

In some disclosures, administration of the inhibitor results in one or more effects selected from an alteration in microglial homeostasis and viability, a decrease or blockade of microglial cell proliferation, a reduction or elimination of microglial cells, a reduction in microglial activation, a reduction in nitric oxide production from microglia, a reduction in nitric oxide synthase activity in microglia, or protection of motor neurons affected by microglial activation. In some instances disclosed herein, the agent alters the level of a serum or blood biomarker of CSF1R inhibition, or a decrease in the level of urinary collagen type 1 cross-linked N-telopeptide (NTX) compared to at a time just prior to initiation of the administration of the inhibitor. In some instances disclosed herein, the administration of the agent transiently inhibits the activity of microglia activity and/or wherein the inhibition of microglia activity is not permanent. In some instances disclosed herein, the administration of the agent transiently inhibits the activity of CSF1R and/or wherein the inhibition of CSF1R activity is not permanent.

In some instances disclosed herein, the agent that reduces microglial cell activity is a small molecule, peptide, protein, antibody or antigen-binding fragment thereof, an antibody mimetic, an aptamer, or a nucleic acid molecule. In some instances disclosed herein, the method involves administration of an inhibitor of microglia activity. In some instances disclosed herein, the agent is an antagonist that inhibits the activity of a signaling pathway in microglia. In some instances disclosed herein, the agent that reduces microglial cell activity affects microglial homeostasis, survival, and/or proliferation.

In some instances disclosed herein, the agent that reduces microglial cell activation is selected from an anti-inflammatory agent, an inhibitor of NADPH oxidase (NOX2), a calcium channel blocker, a sodium channel blocker, inhibits GM-CSF, inhibits CSF1R, specifically binds CSF-1, specifically binds IL-34, inhibits the activation of nuclear factor kappa B (NF-κB), activates a CB₂ receptor and/or is a CB₂ agonist, a phosphodiesterase inhibitor, inhibits microRNA-155 (miR-155), upregulates microRNA-124 (miR-124), inhibits nitric oxide production in microglia, inhibits nitric oxide synthase, or activates the transcription factor NRF2 (also called nuclear factor (erythroid-derived 2)-like 2, or NFE2L2).

In some instances disclosed herein, the agent that reduces microglial cell activity targets CSF1 (also called macrophage colony-stimulating factor MCSF). In some instances disclosed herein, the agent that reduces microglial cell activity affects MCSF-stimulated phosphorylation of the M-CSF receptor (Pryer et al. Proc Am Assoc Cancer Res, AACR Abstract nr DDT02-2 (2009)). In some instances disclosed herein, the agent that reduces microglial cell activity is MCS110 (international patent application publication number WO2014001802; Clinical Trial Study Record Nos.:A1 NCT00757757; NCT02807844; NCT02435680; NCT01643850).

In some instances disclosed herein, the agent that reduces microglial cell activity is a small molecule that targets the CSF1 pathway. In some instances disclosed herein, the agent is a small molecule that binds CSF1R. In some instances disclosed herein, the agent is a small molecule which inhibits CSF1R kinase activity by competing with ATP binding to CSF1R kinase. In some instances disclosed herein, the agent is a small molecule which inhibits the activation of the CFS1R receptor. In some instances disclosed herein, the binding of the CSF-1 ligand to the CSF1R is inhibited. In some instances disclosed herein, the agent that reduces microglial cell activity is any of the inhibitors described in US Patent Application Publication Number US20160032248.

In some instances disclosed herein, the agent is a small molecule inhibitor selected from PLX-3397, PLX7486, JNJ-40346527, JNJ28312141, ARRY-382, PLX73086 (AC-708), DCC-3014, AZD6495, GW2580, Ki20227, BLZ945, PLX647, PLX5622. In some instances disclosed herein, the agent is any of the inhibitors described in Conway et al., Proc Natl Acad Sci U S A, 102(44):16078-83 (2005); Dagher et al., Journal of Neuroinflammation, 12:139 (2015); Ohno et al., Mol Cancer Ther. 5(11):2634-43 (2006); von Tresckow et al. Clin Cancer Res.,21(8) (2015); Manthey et al. Mol Cancer Ther. (8(11):3151-61 (2009); Pyonteck et al., Nat Med. 19(10): 1264-1272 (2013); Haegel et al., Cancer Res AACR Abstract nr 288 (2015); Smith et al., Cancer Res AACR Abstract nr 4889 (2016); Clinical Trial Study Record Nos.: NCT01525602; NCT02734433; NCT02777710; NCT01804530; NCT01597739; NCT01572519; NCT01054014; NCT01316822; NCT02880371; NCT02673736; international patent application publication numbers WO2008063888A2, WO2006009755A2, US patent application publication numbers US20110044998, US 2014/0065141, and US 2015/0119267.

In some instances disclosed herein, the agent that reduces microglial cell activity is 4-((2-(((1R,2R)-2-hydroxycyclohexyl)amino)benzo[d]thiazol-6-yl)oxy)-N-methylpicolinamide (BLZ945) or a pharmaceutically acceptable salt thereof or derivatives thereof. In some instances disclosed herein, the agent is the following compound: 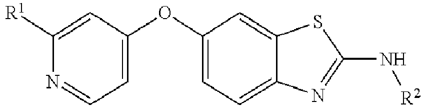 wherein R1 is an alkyl pyrazole or an alkyl carboxamide, and R2 is a hydroxycycloalkyl or a pharmaceutically acceptable salt thereof.

In some instances disclosed herein, the agent that reduces microglial cell activity is 5-((5-chloro-1H-pyrrolo [2,3-b]pyridin-3-yl)methyl)-N-((6-(trifluoromethyl)pyridin-3-yl)methyl)pyridin-2-amine, N-[5-[(5-Chloro-1H-pyrrolo[2,3-b]pyridin-3-yl)methyl]-2-pyridinyl]-6-(trifluoromethyl)-3-pyridinemethanamine) (PLX 3397) or a pharmaceutically acceptable salt thereof or derivatives thereof. In some instances disclosed herein, the agent is 5-(1H-Pyrrolo[2,3-b]pyridin-3-ylmethyl)-N-[[4-(trifluoromethyl)phenyl]methyl]-2-pyridinamine dihydrochloride (PLX647) or a pharmaceutically acceptable salt thereof or derivatives thereof. In some instances disclosed herein, the agent that reduces microglial cell activity is the following compound: 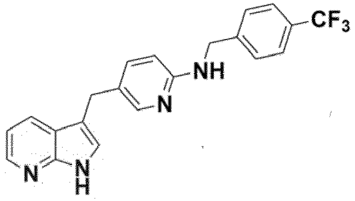 or a pharmaceutically acceptable salt thereof. In some instances disclosed herein, the agent that reduces microglial cell activity is the following compound: 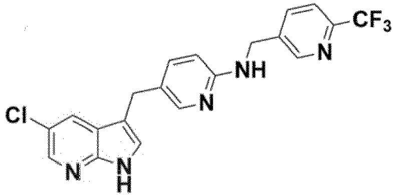 or a pharmaceutically acceptable salt thereof. In some instances disclosed herein, the agent is any of the inhibitors described in US patent number US7893075.

In some instances disclosed herein, the agent that reduces microglial cell activity is 4-cyano-N-[2-(1-cyclohexen-1-yl)-4-[1-[(dimethylamino)acetyl]-4-piperidinyl]phenyl]-1H-imidazole-2-carboxamide monohydrochloride (JNJ28312141) or a pharmaceutically acceptable salt thereof or derivatives thereof. In some instances disclosed herein, the agent is the following compound: 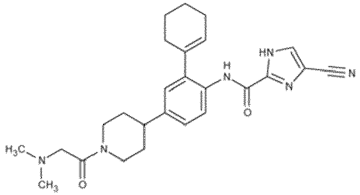 or a pharmaceutically acceptable salt thereof. In some instances disclosed herein, the agent is any of the inhibitors described in US patent number US7645755.

In some instances disclosed herein, the agent that reduces microglial cell activity is 1H-Imidazole-2-carboxamide, 5-cyano-N-(2-(4,4-dimethyl-1-cyclohexen-1-yl)-6-(tetrahydro-2,2,6,6-tetramethyl-2H-pyran-4-yl)-3-pyridinyl)-, 4-Cyano-1H-imidazole-2-carboxylic acid N-(2-(4,4-dimethylcyclohex-1-enyl)-6-(2,2,6,6-tetramethyltetrahydropyran-4-yl)pyridin-3-yl)amide, 4-Cyano-N-(2-(4,4-dimethylcyclohex-1-en-1-yl)-6-(2,2,6,6-tetramethyl-tetrahydro-2H-pyran-4-yl)pyridin-3-yl)-1H-imidazole-2-carboxamide (JNJ-40346527) or a pharmaceutically acceptable salt thereof or derivatives thereof. In some instances disclosed herein, the agent is the following compound: 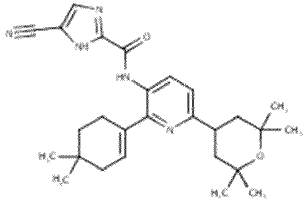 or a pharmaceutically acceptable salt thereof.

In another instance disclosed herein, the agent that reduces microglial cell activity is 5-(3-Methoxy-4-((4-methoxybenzyl)oxy)benzyl)pyrimidine-2,4-diamine (GW2580) or a pharmaceutically acceptable salt thereof or derivatives thereof. In some instances disclosed herein, the agent is the following compound: 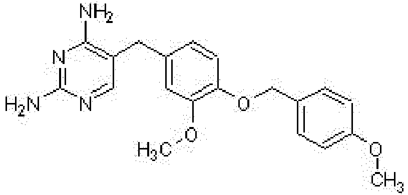 or a pharmaceutically acceptable salt thereof (international patent application publication number WO2009099553).

In some instances disclosed herein, the agent that reduces microglial cell activity is 4-(2,4-difluoroanilino)-7-ethoxy-6-(4-methylpiperazin-1-yl)quinoline-3-carboxamide (AZD6495) or a pharmaceutically acceptable salt thereof or derivatives thereof. In some instances disclosed herein, the agent is the following compound: 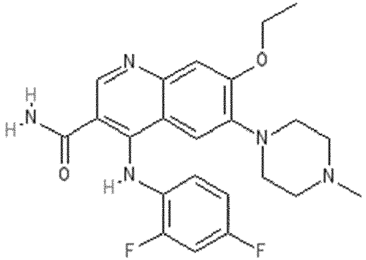 or a pharmaceutically acceptable salt thereof.

In some instances disclosed herein, the agent that reduces microglial cell activity is N-{4-[(6,7-dimethoxy-4-quinolyl)oxy]-2-methoxyphenyl}-N0-[1-(1,3-thiazole-2-yl)ethyl]urea (Ki20227) or a pharmaceutically acceptable salt thereof or derivatives thereof. In some instances disclosed herein, the agent is the following compound: 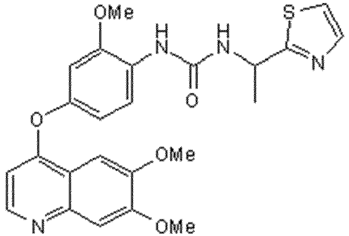 or a pharmaceutically acceptable salt thereof.

In some instances disclosed herein, the agent that reduces microglial cell activation is an antibody that targets the CSF1 pathway. In some instances disclosed herein, the agent is an antibody that binds CSF1R. In some instances disclosed herein, the anti-CSF1R antibody blocks CSF1R dimerization. In some instances disclosed herein, the anti-CSF1R antibody blocks the CSF1R dimerization interface that is formed by domains D4 and D5 (Ries et al. Cancer Cell 25(6):846-59 (2014)). In some instances disclosed herein, the agent is selected from emactuzumab (RG7155; RO5509554), Cabiralizumab (FPA-008), LY-3022855 (IMC-CS4), AMG-820, TG-3003, MCS110, H27K15, 12-2D6, 2-4A5 (Rovida and Sbarba, J Clin Cell Immunol.6:6 (2015); Clinical Trial Study Record Nos.: NCT02760797; NCT01494688; NCT02323191; NCT01962337; NCT02471716; NCT02526017; NCT01346358; NCT02265536; NCT01444404; NCT02713529, NCT00757757; NCT02807844; NCT02435680; NCT01643850).

In some instances disclosed herein, the agent that reduces microglial cell activation is a tetracycline antibiotic. For example, the agent affects IL-1b, IL-6, TNF-α, or iNOS concentration in microglia cells (Yrjänheikki et al. PNAS 95(26): 15769-15774 (1998); Clinical Trial Study Record No: NCT01120899). In some instances disclosed herein, the agent is an opioid antagonist (Younger et al. Pain Med. 10(4):663-672 (2009.) In some instances disclosed herein, the agent reduces glutamatergic neurotransmission (US Patent Number 5,527,814). In some instances disclosed herein, the agent modulates NFkB signaling (Valera et al J. Neuroinflammation 12:93 (2015); Clinical Trial Study Record No: NCT00231140). In some instances disclosed herein, the agent targets cannabinoid receptors (Ramírez et al. J. Neurosci 25(8):1904-13(2005)). In some instances disclosed herein, the agent is selected from minocycline, naloxone, riluzole, lenalidomide, and a cannabinoid (optionally WIN55 or 212-2).

Nitric oxide production from microglia is believed, in some instances disclosed herein, to result in or increase neurotoxicity. In some instances disclosed herein, the agent modulates or inhibits nitric oxide production from microglia. In some instances disclosed herein, the agent inhibits nitric oxide synthase (NOS). In some instances disclosed herein, the NOS inhibitor is Ronopterin (VAS-203), also known as 4-amino-tetrahydrobiopterin (4-ABH4). In some instances disclosed herein, the NOS inhibitor is cindunistat, A-84643, ONO-1714, L-NOARG, NCX-456, VAS-2381, GW-273629, NXN-462, CKD-712, KD-7040, or guanidinoethyldisulfide. In some instances disclosed herein, the agent is any of the inhibitors described in Höing et al., Cell Stem Cell. 2012 Nov 2;11(5):620-32.

In some instances disclosed herein, the agent blocks T cell trafficking, such as to the central nervous system. In some instances disclosed herein, blocking T cell trafficking can reduce or prevent immune cells from crossing blood vessel walls into the central nervous system, including crossing the blood-brain barrier. In some instances disclosed herein, activated antigen-specific T cells produce pro-inflammatory cytokines, including IFN-γ and TNF, upon reactivation in the CNS, leading to activation of resident cells such as microglia and astrocytes. See Kivisäkk et al., Neurology. 2009 Jun 2; 72(22): 1922-1930. Thus, in some instances disclosed herein, sequestering activated T cells from microglial cells, such as by blocking trafficking and/or inhibiting the ability of such cells to cross the blood-brain barrier, can reduce or eliminate microglial activation. In some instances disclosed herein, the agent inhibits adhesion molecules on immune cells, including T cells. In some instances disclosed herein, the agent inhibits an integrin. In some instances disclosed herein, the integrin is alpha-4 integrin. In some instances disclosed herein, the agent is natalizumab (Tysabri^{®}). In some instances disclosed herein, the agent modulates a cell surface receptor. In some instances disclosed herein, the agent modulates the sphingosine-1-phosphate (S1P) receptor, such as S1PR1 or S1PR5. In some instances disclosed herein, the agent causes the internalization of a cellular receptor, such as a sphingosine-1-phosphate (S1P) receptor, such as S1PR1 or S1PR5. In some instances disclosed herein, the agent is fingolimod (Gilenya^{®}) or ozanimod (RPC-1063).

The transcription factor NRF2 is believed to regulate the anti-oxidant response, for example, by turning on genes that contain a cis-acting element in their promoter region. An example of such an element includes an antioxidant response element (ARE). In some instances disclosed herein, the agent activates NRF2. In some instances disclosed herein, activating NRF2 in microglial cells reduces the microglial cells' responsiveness to IFN and LPS. In some instances disclosed herein, activating NRF2 inhibits, slows, or reduces demyelination, axonal loss, neuronal death, and/or oligodendrocyte death. In some instances disclosed herein, the agent upregulates the cellular cytoprotective pathway regulated by NRF2. In some instances disclosed herein, the agent that activates NRF2 is dimethyl fumarate (Tecfidera^{®}). In some instances disclosed herein, the agent is any of the inhibitors described in US patent number 8,399,514. In some instances disclosed herein, the agent is any of the inhibitors described in Höing et al., Cell Stem Cell. 2012 Nov 2;11(5):620-32.

In some instances disclosed herein, the agent that reduces microglial cell activation is (4S,4aS,5aR,112aS)-4,7-bis(dimethylamino)-3,10,12,12a-tetrahydroxy-1,11-dioxo-1,4,4a,5,5a,6,11,12a-octahydrotetracene-2-carboxamide (Minocycline) or a pharmaceutically acceptable salt thereof or derivatives thereof. In some instances disclosed herein, the agent is any of the compounds described in US patent application publication number US20100190755. In some instances disclosed herein, the agent is the following compound: 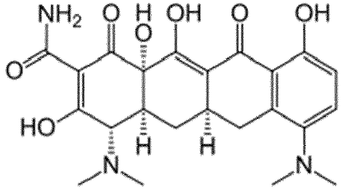 or a pharmaceutically acceptable salt thereof.

In some instances disclosed herein, the agent that reduces microglial cell activation is 3-(7-amino-3-oxo-1H-isoindol-2-yl)piperidine-2,6-dione (lenalidomide) or a pharmaceutically acceptable salt thereof or derivatives thereof. In some instances disclosed herein, the agent is the following compound: 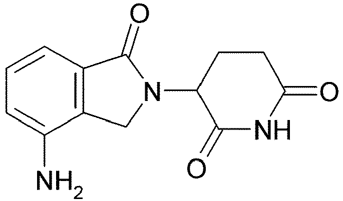 or a pharmaceutically acceptable salt thereof.

In some instances disclosed herein, the agent that reduces microglial cell activation is 4R,4aS,7aR,12bS)-4a,9-dihydroxy-3-prop-2-enyl-2,4,5,6,7a,13-hexahydro-1H-4,12-methanobenzofuro[3,2-e]isoquinoline-7-one (naloxone) or a pharmaceutically acceptable salt thereof or derivatives thereof. In some instances disclosed herein, the agent is any of the compounds described in US patent number US8247425. In some instances disclosed herein, the agent is the following compound: 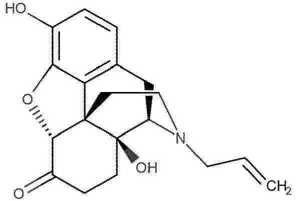 or a pharmaceutically acceptable salt thereof.

In some instances disclosed herein, the agent that reduces microglial cell activation is 2-amino-6-(trifluoromethoxy)benzothiazole, 6-(trifluoromethoxy)benzo[d]thiazol-2-amine, or 6-(trifluoromethoxy)-1,3-benzothiazol-2-amine (riluzole) or a pharmaceutically acceptable salt thereof or derivatives thereof as described in US patent number US5527814. In some instances disclosed herein, the agent is the following compound: 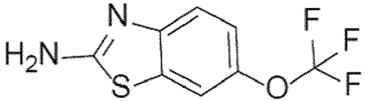 or a pharmaceutically acceptable salt thereof.

In some instances disclosed herein, the agent that reduces microglial cell activation is a modulator of a signaling pathway in microglia. In some instances disclosed herein, the agent reduces microglia singling. In some instances disclosed herein, the agent is a GM-CSF (CSF2) inhibitor. In other instances disclosed herein, the agent that reduces microglial cell activation is an ion channel blocker. In some specific instances disclosed herein, the agent is a calcium channel blocker. For example, in some specific instances disclosed herein, the agent is a dihydropyridine calcium channel blocker. In some instances disclosed herein, the agent is a microRNA inhibitor. For example, the agent targets miR-155. In some instances disclosed herein, the agent that reduces microglial cell activation is selected from MOR103, Nimodipine, IVIg, and LNA-anti-miR-155 (Butoxsky et al. Ann Neurol., 77(1):75-99 (2015) and Sanz et al., Br J Pharmacol. 167(8): 1702-1711 (2012); Winter et al., Ann Clin and Transl Neurol. 2328-9503 (2016); Clinical Trial Study Record Nos.: NCT01517282, NCT00750867).

In some instances disclosed herein, the agent that reduces microglial cell activation is 3-(2-methoxyethyl) 5-propan-2-yl 2,6-dimethyl-4-(3-nitrophenyl)-1,4-dihydropyridine-3,5-dicarboxylate (nimodipine) or a pharmaceutically acceptable salt thereof or derivatives thereof. In some instances disclosed herein, the agent is any of the inhibitors described in US patent number US3799934. In some instances disclosed herein, the agent is the following compound: 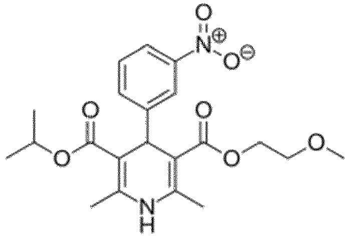 or a pharmaceutically acceptable salt thereof.

In some instances disclosed herein, the agent that reduces microglial cell activation is administered in a form that only affects to central nervous system and/or does not affect tumor-associated macrophages. In some instances disclosed herein, the agent promotes microglia quiescence but does not eliminate or reduce the number of microglia. In some instances disclosed herein, the method involves inhibiting microglia activity specifically in the brain such as described in Ponomarev et al., Nature Medicine, (1):64-70 (2011)

Exemplary agents that reduce microglial cell activation, and exemplary dosing regimens for administering such agents, are set forth in **Table 7** below.

| **Table 7. Exemplary microglia inhibitors and dosage regimens** | | | |
|---|---|---|---|
| **Exemplary Inhibitor** | **Type of Molecule** | **Molecular Target(s)** | **Exemplary Dosing Regimen(s)** |
| Pexidartinib (PLX3397) | small molecule | CSF1R; c-Kit; FLT3 | 200 mg tablets, twice daily for 28 days; Administer daily as split dose regimen, five dose-levels possible in dose escalation part: 400mg 5 days on 2 days off (intermittent schedule), 400 mg, 600 mg, 800 mg or 1000 mg; 1000 mg/day for 2 weeks then 800 mg/day for 22 weeks |
| Emactuzumab (RG1755; RO5509554) | monoclonal antibody | CSF1R | 100-3000 mg once every 2 weeks |
| Cabiralizumab (FPA-008) | antibody | CSF1R | Intravenous infusion over 30 minutes every 2 weeks |
| LY-3022855 (IMC-CS4) | monoclonal antibody | CSF1R | 1.25 mg/kg intravenous delivery every 2 weeks for 6 weeks |
| JNJ-40346527 | small molecule | CSF1R | 100 mg twice daily for 12 weeks; 100-1000 mg capsule daily |
| MCS110 | antibody | MCSF (CSF1) | Up to 4 doses of 10 mg/kg MCS110 administered intravenously once every 4 weeks starting at Day 1 |
| MOR103 | antibody | GM-CSF | 6 doses of 0.5-2.0 mg/kg over 70 days |
| IVIg | immunoglob ulin | Unknown | Intravenous infusion of 0.4g/kg each month for 6 months |
| Minocyline | small molecule | broad spectrum antibiotic: IL-1b; IL-6, TNF-a; iNOS | Oral dose of 100 mg of minocycline twice daily for 24 months |
| Naloxone | small molecule | Opioid receptors | 4.5 mg naltrexone hydrochloride capsules once/day for 8 weeks |
| Lenalidomide/th alidomide | small molecule | NFkB signaling | 100-400 mg daily |
| Riluzole | small molecule | Glutamate release by microglia | 50 mg twice daily |
| Cannabinoids/ca nnabidiol (*e.g*. WIN55,212-2) | small molecule | cannabinoid receptors | Orally 10 mg/kg/day for 6 weeks (average of 700 mg/day) |
| Dimethyl fumarate (Tecfidera^{®}). | small molecule | Nrf2 signaling | Starting dose of 120 mg taken orally twice/day for 7 days. Dose increased to 240 mg taken orally twice/day thereafter |
| natalizumab (Tysabri^{®}) | antibody | alpha-4 integrin | 300 mg infused intravenously over one hour, every four weeks |
| fingolimod (Gilenya^{®}) | small molecule | S1P receptors, including S1PR1 | 0.5 mg orally once-daily |
| ozanimod (RPC-1063) | small molecule | S1PR1 and S1PR5 | 0.25 mg, 0.5 mg, or 1 mg once daily |

### C. Other Agents (e.g., cytokine targeting agents)

In some instances disclosed herein, the agent, e.g. toxicity-targeting agent, that treats or ameliorates symptoms of a toxicity of immunotherapy and/or a cell therapy, such as CRS or neurotoxicity, is one that targets a cytokine, e.g., is an antagonist or inhibitor of a cytokine, such as transforming growth factor beta (TGF-beta), interleukin 6 (IL-6), interleukin 10 (IL-10), IL-2, MIP1β (CCL4), TNF alpha, IL-1, interferon gamma (IFN-gamma), or monocyte chemoattractant protein-1 (MCP-1). In some instances disclosed herein, the agent that treats or ameliorates symptoms of a toxicity of an immunotherapy and/or a cell therapy, such as CRS or neurotoxicity, is one that targets (e.g. inhibits or is an antagonist of) a cytokine receptor, such as IL-6 receptor (IL-6R), IL-2 receptor (IL-2R/CD25), MCP-1 (CCL2) receptor (CCR2 or CCR4), a TGF-beta receptor (TGF-beta I, II, or III), IFN-gamma receptor (IFNGR), MIP1β receptor (e.g., CCR5), TNF alpha receptor (e.g., TNFR1), IL-1 receptor (IL1-Rα/IL-1Rβ), or IL-10 receptor (IL-10R).

The amount of a selected agent that treats or ameliorates symptoms of a toxicity of an immunotherapy and/or a cell therapy, such as CRS or neurotoxicity to be administered to ameliorate symptoms or adverse effects of a toxicity to an immunotherapy and/or a cell therapy, such as CRS or neurotoxicity, can be determined by standard clinical techniques. Exemplary adverse events include, but are not limited to, an increase in alanine aminotransferase, an increase in aspartate aminotransferase, chills, febrile neutropenia, headache, hypotension, left ventricular dysfunction, encephalopathy, hydrocephalus, seizure, and/or tremor.

In some instances disclosed herein, the agent is administered in a dosage amount of from or from about 30 mg to 5000 mg, such as 50 mg to 1000 mg, 50 mg to 500 mg, 50 mg to 200 mg, 50 mg to 100 mg, 100 mg to 1000 mg, 100 mg to 500 mg, 100 mg to 200 mg, 200 mg to 1000 mg, 200 mg to 500 mg or 500 mg to 1000 mg.

In some instances disclosed herein, the agent is administered from or from about 0.5 mg/kg to 100 mg/kg, such as from or from about 1 mg/kg to 50 mg/kg, 1 mg/kg to 25 mg/kg, 1 mg/kg to 10 mg/kg, 1 mg/kg to 5 mg/kg, 5 mg/kg to 100 mg/kg, 5 mg/kg to 50 mg/kg, 5 mg/kg to 25 mg/kg, 5 mg/kg to 10 mg/kg, 10 mg/kg to 100 mg/kg, 10 mg/kg to 50 mg/kg, 10 mg/kg to 25 mg/kg, 25 mg/kg to 100 mg/kg, 25 mg/kg to 50 mg/kg to 50 mg/kg to 100 mg/kg. In some instances disclosed herein, the agent is administered in a dosage amount of from or from about 1 mg/kg to 10 mg/kg, 2 mg/kg to 8 mg/kg, 2 mg/kg to 6 mg/kg, 2 mg/kg to 4 mg/kg or 6 mg/kg to 8 mg/kg, each inclusive. In some disclosures, the agent is administered in a dosage amount of at least or at least about or about 1 mg/kg, 2 mg/kg, 4 mg/kg, 6 mg/kg, 8 mg/kg, 10 mg/kg or more. In some instances disclosed herein, the agent is administered at a dose of 4 mg/kg or 8 mg/kg.

In some instances disclosed herein, the agent is administered by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some instances disclosed herein, they are administered by parenteral, intrapulmonary, and intranasal, and, if desired for local treatment, intralesional administration. Parenteral infusions include intramuscular, intravenous, intraarterial, intraperitoneal, or subcutaneous administration.

In some instances disclosed herein, the amount of the agent is administered about or approximately twice daily, daily, every other day, three times a week, weekly, every other week or once a month.

In some instances disclosed herein, the agent is administered as part of a composition or formulation, such as a pharmaceutical composition or formulation as described below. Thus, in some instances disclosed herein, the composition comprising the agent is administered as described below. In other disclosures, the agent is administered alone and may be administered by any known acceptable route of administration or by one described herein, such as with respect to compositions and pharmaceutical formulations.

In some instances disclosed herein, the agent that treats or ameliorates symptoms of a toxicity of the immunotherapy and/or cell therapy, such as CRS or neurotoxicity, is an antibody or antigen binding fragment. In some instances disclosed herein, the agent is tocilizumab, siltuximab, sarilumab, olokizumab (CDP6038), elsilimomab, ALD518/BMS-945429, sirukumab (CNTO 136), CPSI-2634, ARGX-109, FE301, or FM101.

In some instances disclosed herein, the agent is an antagonist or inhibitor of IL-6 or the IL-6 receptor (IL-6R). In some disclosures, the agent is an antibody that neutralizes IL-6 activity, such as an antibody or antigen-binding fragment that binds to IL-6 or IL-6R. For example, in some instances disclosed herein, the agent is or comprises tocilizumab (atlizumab) or sarilumab, anti-IL-6R antibodies. In some instances disclosed herein, the agent is an anti-IL-6R antibody described in U.S. Patent No: 8,562,991. In some instances disclosed herein, the agent that targets IL-6 is an anti-IL-6 antibody, such as siltuximab, elsilimomab, ALD518/BMS-945429, sirukumab (CNTO 136), CPSI-2634, ARGX-109, FE301, FM101, or olokizumab (CDP6038). In some disclosures, the agent may neutralize IL-6 activity by inhibiting the ligand-receptor interactions. The feasibility of this general type of approach has been demonstrated with a natural occurring receptor antagonist for interleukin-1. *See* Harmum, C. H. et al., Nature (1990) 343:336-340. In some disclosures, the IL-6/IL-6R antagonist or inhibitor is an IL-6 mutein, such as one described in U.S. Patent No. 5591827. In some instances disclosed herein, the agent that is an antagonist or inhibitor of IL-6/IL-6R is a small molecule, a protein or peptide, or a nucleic acid.

In some instances disclosed herein, the agent is tocilizumab. In some instances disclosed herein, tocilizumab is administered as an early intervention in accord with the disclosed methods, and/or with the disclosed articles of manufacture or compositions, at a dosage of from or from about 1 mg/kg to 12 mg/kg, such as at or about 4 mg/kg, 8 mg/kg, or 10 mg/kg. In some instances disclosed herein, tocilizumab is administered by intravenous infusion. In some instances disclosed herein, tocilizumab is administered for a persistent fever of greater than 39°C lasting 10 hours that is unresponsive to acetaminophen. In some instances disclosed herein, a second administration of tocilizumab is provided if symptoms recur after 48 hours of the initial dose.

In some instances disclosed herein, the agent is an agonist or stimulator of TGF-β or a TGF-β receptor (e.g., TGF-β receptor I, II, or III). In some disclosures, the agent is an antibody that increases TGF-β activity, such as an antibody or antigen-binding fragment that binds to TGF-β or one of its receptors. In some instances disclosed herein, the agent that is an agonist or stimulator of TGF-β and/or its receptor is a small molecule, a protein or peptide, or a nucleic acid.

In some instances disclosed herein, the agent is an antagonist or inhibitor of MCP-1 (CCL2) or a MCP-1 receptor (e.g., MCP-1 receptor CCR2 or CCR4). In some disclosures, the agent is an antibody that neutralizes MCP-1 activity, such as an antibody or antigen-binding fragment that binds to MCP-1 or one of its receptors (CCR2 or CCR4). In some instances disclosed herein, the MCP-1 antagonist or inhibitor is any described in Gong et al. J Exp Med. 1997 Jul 7; 186(1): 131-137 or Shahrara et al. J Immunol 2008; 180:3447-3456. In some instances disclosed herein, the agent that is an antagonist or inhibitor of MCP-1 and/or its receptor (CCR2 or CCR4) is a small molecule, a protein or peptide, or a nucleic acid.

In some instances disclosed herein, the agent is an antagonist or inhibitor of IFN-γ or an IFN-γ receptor (IFNGR). In some disclosures, the agent is an antibody that neutralizes IFN-γ activity, such as an antibody or antigen-binding fragment that binds to IFN-γ or its receptor (IFNGR). In some disclosures, the IFN-gamma neutralizing antibody is any described in Dobber et al. Cell Immunol. 1995 Feb;160(2): 185-92 or Ozmen et al. J Immunol. 1993 Apr 1;150(7):2698-705. In some instances disclosed herein, the agent that is an antagonist or inhibitor of IFN-y/IFNGR is a small molecule, a protein or peptide, or a nucleic acid.

In some instances disclosed herein, the agent is an antagonist or inhibitor of IL-10 or the IL-10 receptor (IL-10R). In some disclosures, the agent is an antibody that neutralizes IL-10 activity, such as an antibody or antigen-binding fragment that binds to IL-10 or IL-10R. In some disclosures, the IL-10 neutralizing antibody is any described in Dobber et al. Cell Immunol. 1995 Feb;160(2):185-92 or Hunter et al. J Immunol. 2005 Jun 1;174(11):7368-75. In some instances disclosed herein, the agent that is an antagonist or inhibitor of IL-10/IL-10R is a small molecule, a protein or peptide, or a nucleic acid.

In some instances disclosed herein, the agent is an antagonist or inhibitor of IL-1 or the IL-1 receptor (IL-1R). In some disclosures, the agent is an IL-1 receptor antagonist, which is a modified form of IL-1R, such as anakinra (see, e.g., Fleischmann et al., (2006) Annals of the rheumatic diseases. 65(8): 1006-12). In some disclosures, the agent is an antibody that neutralizes IL-1 activity, such as an antibody or antigen-binding fragment that binds to IL-1 or IL-1R, such as canakinumab (see also EP 2277543). In some instances disclosed herein, the agent that is an antagonist or inhibitor of IL-1/IL-1R is a small molecule, a protein or peptide, or a nucleic acid.

In some instances disclosed herein, the agent is an antagonist or inhibitor of a tumor necrosis factor (TNF) or a tumor necrosis factor receptor (TNFR). In some disclosures, the agent is an antibody that blocks TNF activity, such as an antibody or antigen-binding fragment that binds to a TNF, such as TNFα, or its receptor (TNFR, e.g., TNFRp55 or TNFRp75). In some disclosures, the agent is selected from among infliximab, adalimumab, certolizumab pegol, golimumab and etanercept. In some instances disclosed herein, the agent that is an antagonist or inhibitor of TNF/TNFR is a small molecule, a protein or peptide, or a nucleic acid. In some instances disclosed herein, the agent is a small molecule that affects TNF, such as lenalidomide (see, e.g., Muller et al. (1999) Bioorganic & Medicinal Chemistry Letters. 9 (11): 1625).

In some instances disclosed herein, the agent is an antagonist or inhibitor of signaling through the Janus kinase (JAK) and two Signal Transducer and Activator of Transcription (STAT) signaling cascade. JAK/STAT proteins are common components of cytokine and cytokine receptor signaling. In some instances disclosed herein, the agent that is an antagonist or inhibitor of JAK/STAT, such as ruxolitinib (see, e.g., Mesa et al. (2012) Nature Reviews Drug Discovery. 11(2):103-104), tofacitinib (also known as Xeljanz, Jakvinus tasocitinib and CP-690550), Baricitinib (also known as LY-3009104, INCB-28050), Filgotinib (G-146034, GLPG-0634), Gandotinib (LY-2784544), Lestaurtinib (CEP-701), Momelotinib (GS-0387, CYT-387), Pacritinib (SB1518), and Upadacitinib (ABT-494). In some instances disclosed herein, the agent is a small molecule, a protein or peptide, or a nucleic acid.

In some instances disclosed herein, the agent is a kinase inhibitor. In some instances disclosed herein, the agent is an inhibitor of Bruton's tyrosine kinase (BTK). In some instances disclosed herein, the inhibitor is or comprises ibrutinib or acalabrutinib (see, e.g., Barrett et al., ASH 58th Annual Meeting San Diego, CA December 3-6, 2016, Abstract 654; Ruella et al., ASH 58th Annual Meeting San Diego, CA December 3-6, 2016, Abstract 2159). In some instances disclosed herein, the agent is an inhibitor as described in U.S. Patent No. 7,514,444; 8,008,309; 8,476,284; 8,497,277; 8,697,711; 8,703,780; 8,735,403; 8,754,090; 8,754,091; 8.957,079; 8,999,999; 9,125,889; 9,181,257; or 9,296,753.

In some instances disclosed herein, a device, such as absorbent resin technology with blood or plasma filtration, can be used to reduce cytokine levels. In some instances disclosed herein, the device used to reduce cytokine levels is a physical cytokine absorber, such as an extracorporeal cytokine absorber. In some instances disclosed herein, a physical cytokine absorber can be used to eliminate cytokines from the bloodstream in an *ex vivo,* extracorporeal manner. In some instances disclosed herein, the agent is a porous polymer. In some instances disclosed herein, the agent is CytoSorb (see, e.g., Basu et al. Indian J Crit Care Med. (2014) 18(12): 822-824).

### III. RECOMBINANT ANTIGEN RECEPTORS EXPRESSED BY THE CELLS

In some instances disclosed herein, the cells for use in or administered in connection with the disclosed methods contain or are engineered to contain an engineered receptor, e.g., an engineered antigen receptor, such as a chimeric antigen receptor (CAR), or a T cell receptor (TCR). Also disclosed are populations of such cells, compositions containing such cells and/or enriched for such cells, such as in which cells of a certain type such as T cells or CD8⁺ or CD4⁺ cells are enriched or selected. Among the compositions are pharmaceutical compositions and formulations for administration, such as for adoptive cell therapy. Also disclosed are therapeutic methods for administering the cells and compositions to subjects, e.g., patients, in accord with the disclosed methods, and/or with the disclosed articles of manufacture or compositions.

In some instances disclosed herein, the cells include one or more nucleic acids introduced via genetic engineering, and thereby express recombinant or genetically engineered products of such nucleic acids. In some instances disclosed herein, gene transfer is accomplished by first stimulating the cells, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications.

The cells generally express recombinant receptors, such as antigen receptors including functional non-TCR antigen receptors, e.g., chimeric antigen receptors (CARs), and other antigen-binding receptors such as transgenic T cell receptors (TCRs). Also among the receptors are other chimeric receptors.

### A. Chimeric Antigen Receptors (CARs)

In some instances disclosed herein, chimeric receptors, such as a chimeric antigen receptors, contain one or more domains that combine a ligand-binding domain (e.g. antibody or antibody fragment) that provides specificity for a desired antigen (e.g., tumor antigen) with intracellular signaling domains. In some instances disclosed herein, the intracellular signaling domain is a stimulating or an activating intracellular domain portion, such as a T cell stimulating or activating domain, providing a primary activation signal or a primary signal. In some instances disclosed herein, the intracellular signaling domain contains or additionally contains a costimulatory signaling domain to facilitate effector functions. In some instances disclosed herein, chimeric receptors when genetically engineered into immune cells can modulate T cell activity, and, in some instances disclosed herein, can modulate T cell differentiation or homeostasis, thereby resulting in genetically engineered cells with improved longevity, survival and/or persistence *in vivo,* such as for use in adoptive cell therapy methods.

Exemplary antigen receptors, including CARs, and methods for engineering and introducing such receptors into cells, include those described, for example, in international patent application publication numbers WO200014257, WO2013126726, WO2012/129514, WO2014031687, WO2013/166321, WO2013/071154, WO2013/123061 U.S. patent application publication numbers US2002131960, US2013287748, US20130149337, U.S. Patent Nos.: 6,451,995, 7,446,190, 8,252,592, 8,339,645, 8,398,282, 7,446,179, 6,410,319, 7,070,995, 7,265,209, 7,354,762, 7,446,191, 8,324,353, and 8,479,118, and European patent application number EP2537416,and/or those described by Sadelain et al., Cancer Discov. 2013 April; 3(4): 388-398; Davila et al. (2013) PLoS ONE 8(4): e61338; Turtle et al., Curr. Opin. Immunol., 2012 October; 24(5): 633-39; Wu et al., Cancer, 2012 March 18(2): 160-75. In some disclosures, the antigen receptors include a CAR as described in U.S. Patent No.: 7,446,190, and those described in International Patent Application Publication No.: WO/2014055668 A1. Examples of the CARs include CARs as disclosed in any of the aforementioned publications, such as WO2014031687, US 8,339,645, US 7,446,179, US 2013/0149337, U.S. Patent No.: 7,446,190, US Patent No.: 8,389,282, Kochenderfer et al., 2013, Nature Reviews Clinical Oncology, 10, 267-276 (2013); Wang et al. (2012) J. Immunother. 35(9): 689-701; and Brentjens et al., Sci Transl Med. 2013 5(177). See also WO2014031687, US 8,339,645, US 7,446,179, US 2013/0149337, U.S. Patent No.: 7,446,190, and US Patent No.: 8,389,282.

The chimeric receptors, such as CARs, generally include an extracellular antigen binding domain, such as a portion of an antibody molecule, generally a variable heavy (V_{H}) chain region and/or variable light (V_{L}) chain region of the antibody, e.g., an scFv antibody fragment.

In some instances disclosed herein, the antigen targeted by the receptor is a polypeptide. In some instances disclosed herein, it is a carbohydrate or other molecule. In some instances disclosed herein, the antigen is selectively expressed or overexpressed on cells of the disease or condition, e.g., the tumor or pathogenic cells, as compared to normal or non-targeted cells or tissues. In other instances disclosed herein, the antigen is expressed on normal cells and/or is expressed on the engineered cells.

In some instances disclosed herein, the antigen targeted by the receptor is or comprises selected from among αvβ6 integrin (avb6 integrin), B cell maturation antigen (BCMA), B7-H3, B7-H6, carbonic anhydrase 9 (CA9, also known as CAIX or G250), a cancer-testis antigen, cancer/testis antigen 1B (CTAG, also known as NY-ESO-1 and LAGE-2), carcinoembryonic antigen (CEA), a cyclin, cyclin A2, C-C Motif Chemokine Ligand 1 (CCL-1), CD19, CD20, CD22, CD23, CD24, CD30, CD33, CD38, CD44, CD44v6, CD44v7/8, CD 123, CD133, CD138, CD171, chondroitin sulfate proteoglycan 4 (CSPG4), epidermal growth factor protein (EGFR), type III epidermal growth factor receptor mutation (EGFR vIII), epithelial glycoprotein 2 (EPG-2), epithelial glycoprotein 40 (EPG-40), ephrinB2, ephrin receptor A2 (EPHa2), estrogen receptor, Fc receptor like 5 (FCRL5; also known as Fc receptor homolog 5 or FCRH5), fetal acetylcholine receptor (fetal AchR), a folate binding protein (FBP), folate receptor alpha, ganglioside GD2, O-acetylated GD2 (OGD2), ganglioside GD3, glycoprotein 100 (gp100), glypican-3 (GPC3), G protein-coupled receptor class C group 5 member D (GPRC5D), Her2/neu (receptor tyrosine kinase erb-B2), Her3 (erb-B3), Her4 (erb-B4), erbB dimers, Human high molecular weight-melanoma-associated antigen (HMW-MAA), hepatitis B surface antigen, Human leukocyte antigen A1 (HLA-A1), Human leukocyte antigen A2 (HLA-A2), IL-22 receptor alpha (IL-22Rα), IL-13 receptor alpha 2 (IL-13Rα2), kinase insert domain receptor (kdr), kappa light chain, L1 cell adhesion molecule (L1-CAM), CE7 epitope of L1-CAM, Leucine Rich Repeat Containing 8 Family Member A (LRRC8A), Lewis Y, Melanoma-associated antigen (MAGE)-A1, MAGE-A3, MAGE-A6, MAGE-A10, mesothelin (MSLN), c-Met, murine cytomegalovirus (CMV), mucin 1 (MUC1), MUC16, natural killer group 2 member D (NKG2D) ligands, melan A (MART-1), neural cell adhesion molecule (NCAM), oncofetal antigen, Preferentially expressed antigen of melanoma (PRAME), progesterone receptor, a prostate specific antigen, prostate stem cell antigen (PSCA), prostate specific membrane antigen (PSMA), Receptor Tyrosine Kinase Like Orphan Receptor 1 (ROR1), survivin, Trophoblast glycoprotein (TPBG also known as 5T4), tumor-associated glycoprotein 72 (TAG72), Tyrosinase related protein 1 (TRP1, also known as TYRP1 or gp75), Tyrosinase related protein 2 (TRP2, also known as dopachrome tautomerase, dopachrome delta-isomerase or DCT), vascular endothelial growth factor receptor (VEGFR), vascular endothelial growth factor receptor 2 (VEGFR2), Wilms Tumor 1 (WT-1), a pathogen-specific or pathogen-expressed antigen, or an antigen associated with a universal tag, and/or biotinylated molecules, and/or molecules expressed by HIV, HCV, HBV or other pathogens. Antigens targeted by the receptors in some instances disclosed herein include antigens associated with a B cell malignancy, such as any of a number of known B cell marker. In some instances disclosed herein, the antigen targeted by the receptor is CD20, CD19, CD22, ROR1, CD45, CD21, CD5, CD33, Igkappa, Iglambda, CD79a, CD79b or CD30. In some instances disclosed herein, the antigen is or includes a pathogen-specific or pathogen-expressed antigen. In some instances disclosed herein, the antigen is a viral antigen (such as a viral antigen from HIV, HCV, HBV, etc.), bacterial antigens, and/or parasitic antigens.

In some instances disclosed herein, the antigen is CD19. In some instances disclosed herein, the scFv contains a V_{H} and a V_{L} derived from an antibody or an antibody fragment specific to CD19. In some instances disclosed herein, the antibody or antibody fragment that binds CD19 is a mouse derived antibody such as FMC63 and SJ25C1. In some instances disclosed herein, the antibody or antibody fragment is a human antibody, e.g., as described in U.S. Patent Publication No. US 2016/0152723.

In some instances disclosed herein the scFv and/or V_{H} domains is derived from FMC63. FMC63 generally refers to a mouse monoclonal IgG1 antibody raised against Nalm-1 and -16 cells expressing CD19 of human origin (Ling, N. R., et al. (1987). Leucocyte typing III. 302). In some instances disclosed herein, the FMC63 antibody comprises a CDR-H1 and a CDR-H2 set forth in SEQ ID NOS: 38 and 39, respectively, and a CDR-H3 set forth in SEQ ID NO: 40 or 54; and a CDR-L1 set forth in SEQ ID NO: 35 and a CDR-L2 set forth in SEQ ID NO: 36 or 55 and a CDR-L3 set forth in SEQ ID NO: 37 or 56. In some instances disclosed herein, the FMC63 antibody comprises a heavy chain variable region (V_{H}) comprising the amino acid sequence of SEQ ID NO: 41 and a light chain variable region (V_{L}) comprising the amino acid sequence of SEQ ID NO: 42.

In some instances disclosed herein, the scFv comprises a variable light chain containing a CDR-L1 sequence of SEQ ID NO:35, a CDR-L2 sequence of SEQ ID NO:36, and a CDR-L3 sequence of SEQ ID NO:37 and/or a variable heavy chain containing a CDR-H1 sequence of SEQ ID NO:38, a CDR-H2 sequence of SEQ ID NO:39, and a CDR-H3 sequence of SEQ ID NO:40. In some instances disclosed herein, the scFv comprises a variable heavy chain region set forth in SEQ ID NO:41 and a variable light chain region set forth in SEQ ID NO:42. In some instances disclosed herein, the variable heavy and variable light chains are connected by a linker. In some instances disclosed herein, the linker is set forth in SEQ ID NO:24. In some instances disclosed herein, the scFv comprises, in order, a V_{H}, a linker, and a V_{L}. In some instances disclosed herein, the scFv comprises, in order, a V_{L}, a linker, and a V_{H}. In some instances disclosed herein, the scFv is encoded by a sequence of nucleotides set forth in SEQ ID NO:25 or a sequence that exhibits at least 85%, 86%, 87%, 88%, 89%, 90%, 91%, 92%, 93%, 94%, 95%, 96%, 97%, 98%, or 99% sequence identity to SEQ ID NO:25. In some instances disclosed herein, the scFv comprises the sequence of amino acids set forth in SEQ ID NO:43 or a sequence that exhibits at least 85%, 86%, 87%, 88%, 89%, 90%, 91%, 92%, 93%, 94%, 95%, 96%, 97%, 98%, or 99% sequence identity to SEQ ID NO:43.

In some instances disclosed herein the scFv is derived from SJ25C1. SJ25C1 is a mouse monoclonal IgG1 antibody raised against Nalm-1 and -16 cells expressing CD19 of human origin (Ling, N. R., et al. (1987). Leucocyte typing III. 302). In some instances disclosed herein, the SJ25C1 antibody comprises a CDR-H1, a CDR-H2 and a CDR-H3 sequence set forth in SEQ ID NOS: 47-49, respectively, and a CDR-L1, a CDR-L2 and a CDR-L3 sequence set forth in SEQ ID NOS: 44-46, respectively. In some instances disclosed herein, the SJ25C1 antibody comprises a heavy chain variable region (V_{H}) comprising the amino acid sequence of SEQ ID NO: 50 and a light chain variable region (V_{L}) comprising the amino acid sequence of SEQ ID NO: 51.

In some instances disclosed herein, the scFv comprises a variable light chain containing a CDR-L1 sequence of SEQ ID NO:44, a CDR-L2 sequence of SEQ ID NO: 45, and a CDR-L3 sequence of SEQ ID NO:46 and/or a variable heavy chain containing a CDR-H1 sequence of SEQ ID NO:47, a CDR-H2 sequence of SEQ ID NO:48, and a CDR-H3 sequence of SEQ ID NO:49. In some instances disclosed herein, the scFv comprises a variable heavy chain region set forth in SEQ ID NO:50 and a variable light chain region set forth in SEQ ID NO:51. In some instances disclosed herein, the variable heavy and variable light chain are connected by a linker. In some instances disclosed herein, the linker is set forth in SEQ ID NO:52. In some instances disclosed herein, the scFv comprises, in order, a V_{H}, a linker, and a V_{L}. In some instances disclosed herein, the scFv comprises, in order, a V_{L}, a linker, and a V_{H}. In some instances disclosed herein, the scFv comprises the sequence of amino acids set forth in SEQ ID NO:53 or a sequence that exhibits at least 85%, 86%, 87%, 88%, 89%, 90%, 91%, 92%, 93%, 94%, 95%, 96%, 97%, 98%, or 99% sequence identity to SEQ ID NO:53.

In some instances disclosed herein, the chimeric antigen receptor includes an extracellular portion containing an antibody or antibody fragment. In some disclosures, the chimeric antigen receptor includes an extracellular portion containing the antibody or fragment and an intracellular signaling domain. In some instances disclosed herein, the antibody or fragment includes an scFv.

The term "antibody" herein is used in the broadest sense and includes polyclonal and monoclonal antibodies, including intact antibodies and functional (antigen-binding) antibody fragments, including fragment antigen binding (Fab) fragments, F(ab')₂ fragments, Fab' fragments, Fv fragments, recombinant IgG (rIgG) fragments, heavy chain variable (V_{H}) regions capable of specifically binding the antigen, single chain antibody fragments, including single chain variable fragments (scFv), and single domain antibodies (e.g., sdAb, sdFv, nanobody) fragments. The term encompasses genetically engineered and/or otherwise modified forms of immunoglobulins, such as intrabodies, peptibodies, chimeric antibodies, fully human antibodies, humanized antibodies, and heteroconjugate antibodies, multispecific, e.g., bispecific or trispecific, antibodies, diabodies, triabodies, and tetrabodies, tandem di-scFv, tandem tri-scFv. Unless otherwise stated, the term "antibody" should be understood to encompass functional antibody fragments thereof also referred to herein as "antigen-binding fragments." The term also encompasses intact or full-length antibodies, including antibodies of any class or sub-class, including IgG and sub-classes thereof, IgM, IgE, IgA, and IgD.

The terms "complementarity determining region," and "CDR," synonymous with "hypervariable region" or "HVR," are known, in some cases, to refer to non-contiguous sequences of amino acids within antibody variable regions, which confer antigen specificity and/or binding affinity. In general, there are three CDRs in each heavy chain variable region (CDR-H1, CDR-H2, CDR-H3) and three CDRs in each light chain variable region (CDR-L1, CDR-L2, CDR-L3). "Framework regions" and "FR" are known, in some cases,to refer to the non-CDR portions of the variable regions of the heavy and light chains. In general, there are four FRs in each full-length heavy chain variable region (FR-H1, FR-H2, FR-H3, and FR-H4), and four FRs in each full-length light chain variable region (FR-L1, FR-L2, FR-L3, and FR-L4).

The precise amino acid sequence boundaries of a given CDR or FR can be readily determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme); Al-Lazikani et al., (1997) JMB 273,927-948 ("Chothia" numbering scheme); MacCallum et al., J. Mol. Biol. 262:732-745 (1996), "Antibody-antigen interactions: Contact analysis and binding site topography," J. Mol. Biol. 262, 732-745." ("Contact" numbering scheme); Lefranc MP et al., "IMGT unique numbering for immunoglobulin and T cell receptor variable domains and Ig superfamily V-like domains," Dev Comp Immunol, 2003 Jan;27(1):55-77 ("IMGT" numbering scheme); Honegger A and Plückthun A, "Yet another numbering scheme for immunoglobulin variable domains: an automatic modeling and analysis tool," J Mol Biol, 2001 Jun 8;309(3):657-70, ("Aho" numbering scheme); and Martin et al., "Modeling antibody hypervariable loops: a combined algorithm," PNAS, 1989, 86(23):9268-9272, ("AbM" numbering scheme).

The boundaries of a given CDR or FR may vary depending on the scheme used for identification. For example, the Kabat scheme is based on structural alignments, while the Chothia scheme is based on structural information. Numbering for both the Kabat and Chothia schemes is based upon the most common antibody region sequence lengths, with insertions accommodated by insertion letters, for example, "30a," and deletions appearing in some antibodies. The two schemes place certain insertions and deletions ("indels") at different positions, resulting in differential numbering. The Contact scheme is based on analysis of complex crystal structures and is similar in many respects to the Chothia numbering scheme. The AbM scheme is a compromise between Kabat and Chothia definitions based on that used by Oxford Molecular's AbM antibody modeling software.

**Table 8,** below, lists exemplary position boundaries of CDR-L1, CDR-L2, CDR-L3 and CDR-H1, CDR-H2, CDR-H3 as identified by Kabat, Chothia, AbM, and Contact schemes, respectively. For CDR-H1, residue numbering is listed using both the Kabat and Chothia numbering schemes. FRs are located between CDRs, for example, with FR-L1 located before CDR-L1, FR-L2 located between CDR-L1 and CDR-L2, FR-L3 located between CDR-L2 and CDR-L3 and so forth. It is noted that because the shown Kabat numbering scheme places insertions at H35A and H35B, the end of the Chothia CDR-H1 loop when numbered using the shown Kabat numbering convention varies between H32 and H34, depending on the length of the loop.

**Table 8. Boundaries of CDRs according to various numbering schemes.**

| **CDR** | **Kabat** | **Chothia** | **AbM** | **Contact** |
|---|---|---|---|---|
| CDR-L1 | L24--L34 | L24--L34 | L24--L34 | L30--L36 |
| CDR-L2 | L50--L56 | L50--L56 | L50--L56 | L46--L55 |
| CDR-L3 | L89--L97 | L89--L97 | L89--L97 | L89--L96 |
| CDR-H1 (Kabat Numbering¹) | H31--H35B | H26--H32.34 | H26--H35B | H30--H35B |
| CDR-H1 (Chothia Numbering²) | H31--H35 | H26--H32 | H26--H35 | H30--H35 |
| CDR-H2 | H50--H65 | H52--H56 | H50--H58 | H47--H58 |
| CDR-H3 | H95--H102 | H95--H102 | H95--H102 | H93--H101 |

| | | | | |
|---|---|---|---|---|
| 1 - Kabat et al. (1991), "Sequences of Proteins of Immunological Interest," 5th Ed. Public Health Service, National Institutes of Health, Bethesda, MD 2 - Al-Lazikani et al., (1997) JMB 273,927-948 | | | | |

Thus, unless otherwise specified, a "CDR" or "complementary determining region," or individual specified CDRs (*e.g*., CDR-H1, CDR-H2, CDR-H3), of a given antibody or region thereof, such as a variable region thereof, should be understood to encompass a (or the specific) complementary determining region as defined by any of the aforementioned schemes, or other known schemes. For example, where it is stated that a particular CDR (*e.g.,* a CDR-H3) contains the amino acid sequence of a corresponding CDR in a given V_{H} or V_{L} region amino acid sequence, it is understood that such a CDR has a sequence of the corresponding CDR (*e.g.,* CDR-H3) within the variable region, as defined by any of the aforementioned schemes, or other known schemes. In some instances disclosed herein, specific CDR sequences are specified. Exemplary CDR sequences of disclosed antibodies are described using various numbering schemes, although it is understood that a disclosed antibody can include CDRs as described according to any of the other aforementioned numbering schemes or other numbering schemes known to a skilled artisan.

Likewise, unless otherwise specified, a FR or individual specified FR(s) (*e.g.,* FR-H1, FR-H2, FR-H3, FR-H4), of a given antibody or region thereof, such as a variable region thereof, should be understood to encompass a (or the specific) framework region as defined by any of the known schemes. In some instances, the scheme for identification of a particular CDR, FR, or FRs or CDRs is specified, such as the CDR as defined by the Kabat, Chothia, AbM or Contact method, or other known schemes. In other instances disclosed herein, the particular amino acid sequence of a CDR or FR is given.

The term "variable region" or "variable domain" refers to the domain of an antibody heavy or light chain that is involved in binding the antibody to antigen. The variable regions of the heavy chain and light chain (V_{H} and V_{L}, respectively) of a native antibody generally have similar structures, with each domain comprising four conserved framework regions (FRs) and three CDRs. (See, *e.g.,* Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, *e.g.,* Portolano et al., J. Immunol. 150:880-887 (1993); Clarkson et al., Nature 352:624-628 (1991).

Among the antibodies included in the disclosed CARs are antibody fragments. An "antibody fragment" or "antigen-binding fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; heavy chain variable (V_{H}) regions, single-chain antibody molecules such as scFvs and single-domain antibodies comprising only the V_{H} region; and multispecific antibodies formed from antibody fragments. In some instances disclosed herein, the antigen-binding domain in the disclosed CARs is or comprises an antibody fragment comprising a variable heavy chain (V_{H}) and a variable light chain (V_{L}) region. In particular instances disclosed herein, the antibodies are single-chain antibody fragments comprising a heavy chain variable (V_{H}) region and/or a light chain variable (V_{L}) region, such as scFvs.

The term "variable region" or "variable domain" refers to the domain of an antibody heavy or light chain that is involved in binding the antibody to antigen. The variable domains of the heavy chain and light chain (V_{H} and V_{L}, respectively) of a native antibody generally have similar structures, with each domain comprising four conserved framework regions (FRs) and three CDRs. (See, *e.g*., Kindt et al. Kuby Immunology, 6th ed., W.H. Freeman and Co., page 91 (2007). A single V_{H} or V_{L} domain may be sufficient to confer antigen-binding specificity. Furthermore, antibodies that bind a particular antigen may be isolated using a V_{H} or V_{L} domain from an antibody that binds the antigen to screen a library of complementary V_{L} or V_{H} domains, respectively. See, *e.g.*, Portolano et al., J. Immunol. 150:880-887 (1993); Clarkson et al., Nature 352:624-628 (1991).

Single-domain antibodies are antibody fragments comprising all or a portion of the heavy chain variable domain or all or a portion of the light chain variable domain of an antibody. In certain instances disclosed herein, a single-domain antibody is a human single-domain antibody. In some instances disclosed herein, the CAR comprises an antibody heavy chain domain that specifically binds the antigen, such as a cancer marker or cell surface antigen of a cell or disease to be targeted, such as a tumor cell or a cancer cell, such as any of the target antigens described herein or known.

Antibody fragments can be made by various techniques, including but not limited to proteolytic digestion of an intact antibody as well as production by recombinant host cells. In some instances disclosed herein, the antibodies are recombinantly-produced fragments, such as fragments comprising arrangements that do not occur naturally, such as those with two or more antibody regions or chains joined by synthetic linkers, *e.g*., peptide linkers, and/or that are may not be produced by enzyme digestion of a naturally-occurring intact antibody. In some instances disclosed herein, the antibody fragments are scFvs.

A "humanized" antibody is an antibody in which all or substantially all CDR amino acid residues are derived from non-human CDRs and all or substantially all FR amino acid residues are derived from human FRs. A humanized antibody optionally may include at least a portion of an antibody constant region derived from a human antibody. A "humanized form" of a non-human antibody, refers to a variant of the non-human antibody that has undergone humanization, typically to reduce immunogenicity to humans, while retaining the specificity and affinity of the parental non-human antibody. In some instances disclosed herein, some FR residues in a humanized antibody are substituted with corresponding residues from a non-human antibody (*e.g*., the antibody from which the CDR residues are derived), *e.g*., to restore or improve antibody specificity or affinity.

In some instances disclosed herein, the antibody portion of the recombinant receptor, e.g., CAR, further includes at least a portion of an immunoglobulin constant region, such as a hinge region, e.g., an IgG4 hinge region, and/or a C_{H}1/C_{L} and/or Fc region. In some instances disclosed herein, the constant region or portion is of a human IgG, such as IgG4 or IgG 1. In some disclosures, the portion of the constant region serves as a spacer region between the antigen-recognition component, e.g., scFv, and transmembrane domain. The spacer can be of a length that provides for increased responsiveness of the cell following antigen binding, as compared to in the absence of the spacer. Exemplary spacers include, but are not limited to, those described in Hudecek et al. (2013) Clin. Cancer Res., 19:3153, international patent application publication number WO2014031687, U.S. Patent No. 8,822,647 or published app. No. US2014/0271635.

In some instances disclosed herein, the constant region or portion is of a human IgG, such as IgG4 or IgG1. In some instances disclosed herein, the spacer has the sequence ESKYGPPCPPCP (set forth in SEQ ID NO: 1), and is encoded by the sequence set forth in SEQ ID NO: 2. In some instances disclosed herein, the spacer has the sequence set forth in SEQ ID NO: 3. In some instances disclosed herein, the spacer has the sequence set forth in SEQ ID NO: 4. In some instances disclosed herein, the constant region or portion is of IgD. In some instances disclosed herein, the spacer has the sequence set forth in SEQ ID NO: 5. In some instances disclosed herein, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 1, 3, 4 or 5. In some instances disclosed herein, the spacer has the sequence set forth in SEQ ID NOS: 26-34. In some instances disclosed herein, the spacer has a sequence of amino acids that exhibits at least 85%, 86%, 87%, 88%, 89%, 90%, 91%, 92%, 93%, 94%, 95%, 96%, 97%, 98%, 99% or more sequence identity to any of SEQ ID NOS: 26-34.

In some instances disclosed herein, the antigen receptor comprises an intracellular domain linked directly or indirectly to the extracellular domain. In some instances disclosed herein, the chimeric antigen receptor includes a transmembrane domain linking the extracellular domain and the intracellular signaling domain. In some instances disclosed herein, the intracellular signaling domain comprises an ITAM. For example, in some disclosures, the antigen recognition domain (e.g. extracellular domain) generally is linked to one or more intracellular signaling components, such as signaling components that mimic activation through an antigen receptor complex, such as a TCR complex, in the case of a CAR, and/or signal via another cell surface receptor. In some instances disclosed herein, the chimeric receptor comprises a transmembrane domain linked or fused between the extracellular domain (e.g. scFv) and intracellular signaling domain. Thus, in some instances disclosed herein, the antigen-binding component (e.g., antibody) is linked to one or more transmembrane and intracellular signaling domains.

In one instance disclosed herein, a transmembrane domain that naturally is associated with one of the domains in the receptor, e.g., CAR, is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some instances disclosed herein is derived either from a natural or from a synthetic source. Where the source is natural, the domain in some disclosures is derived from any membrane-bound or transmembrane protein. Transmembrane regions include those derived from (i.e. comprise at least the transmembrane region(s) of) the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154. Alternatively the transmembrane domain in some instances disclosed herein is synthetic. In some disclosures, the synthetic transmembrane domain comprises predominantly hydrophobic residues such as leucine and valine. In some disclosures, a triplet of phenylalanine, tryptophan and valine will be found at each end of a synthetic transmembrane domain. In some instances disclosed herein, the linkage is by linkers, spacers, and/or transmembrane domain(s). In some disclosures, the transmembrane domain contains a transmembrane portion of CD28.

In some instances disclosed herein, the extracellular domain and transmembrane domain can be linked directly or indirectly. In some instances disclosed herein, the extracellular domain and transmembrane are linked by a spacer, such as any described herein. In some instances disclosed herein, the receptor contains extracellular portion of the molecule from which the transmembrane domain is derived, such as a CD28 extracellular portion.

Among the intracellular signaling domains are those that mimic or approximate a signal through a natural antigen receptor, a signal through such a receptor in combination with a costimulatory receptor, and/or a signal through a costimulatory receptor alone. In some instances disclosed herein, a short oligo- or polypeptide linker, for example, a linker of between 2 and 10 amino acids in length, such as one containing glycines and serines, e.g., glycine-serine doublet, is present and forms a linkage between the transmembrane domain and the cytoplasmic signaling domain of the CAR.

T cell activation is in some disclosures described as being mediated by two classes of cytoplasmic signaling sequences: those that initiate antigen-dependent primary activation through the TCR (primary cytoplasmic signaling sequences), and those that act in an antigen-independent manner to provide a secondary or co-stimulatory signal (secondary cytoplasmic signaling sequences). In some disclosures, the CAR includes one or both of such signaling components.

The receptor, e.g., the CAR, generally includes at least one intracellular signaling component or components. In some disclosures, the CAR includes a primary cytoplasmic signaling sequence that regulates primary activation of the TCR complex. Primary cytoplasmic signaling sequences that act in a stimulatory manner may contain signaling motifs which are known as immunoreceptor tyrosine-based activation motifs or ITAMs. Examples of ITAM containing primary cytoplasmic signaling sequences include those derived from CD3 zeta chain, FcR gamma, CD3 gamma, CD3 delta and CD3 epsilon. In some instances disclosed herein, cytoplasmic signaling molecule(s) in the CAR contain(s) a cytoplasmic signaling domain, portion thereof, or sequence derived from CD3 zeta.

In some instances disclosed herein, the receptor includes an intracellular component of a TCR complex, such as a TCR CD3 chain that mediates T-cell activation and cytotoxicity, e.g., CD3 zeta chain. Thus, in some disclosures, the antigen-binding portion is linked to one or more cell signaling modules. In some instances disclosed herein, cell signaling modules include CD3 transmembrane domain, CD3 intracellular signaling domains, and/or other CD transmembrane domains. In some instances disclosed herein, the receptor, e.g., CAR, further includes a portion of one or more additional molecules such as Fc receptor γ, CD8, CD4, CD25, or CD 16. For example, in some disclosures, the CAR or other chimeric receptor includes a chimeric molecule between CD3-zeta (CD3-ζ) or Fc receptor γ and CD8, CD4, CD25 or CD16.

In some instances disclosed herein, upon ligation of the CAR or other chimeric receptor, the cytoplasmic domain or intracellular signaling domain of the receptor activates at least one of the normal effector functions or responses of the immune cell, e.g., T cell engineered to express the CAR. For example, in some contexts, the CAR induces a function of a T cell such as cytolytic activity or T-helper activity, such as secretion of cytokines or other factors. In some instances disclosed herein, a truncated portion of an intracellular signaling domain of an antigen receptor component or costimulatory molecule is used in place of an intact immunostimulatory chain, for example, if it transduces the effector function signal. In some instances disclosed herein, the intracellular signaling domain or domains include the cytoplasmic sequences of the T cell receptor (TCR), and in some disclosures also those of co-receptors that in the natural context act in concert with such receptors to initiate signal transduction following antigen receptor engagement.

In the context of a natural TCR, full activation generally requires not only signaling through the TCR, but also a costimulatory signal. Thus, in some instances disclosed herein, to promote full activation, a component for generating secondary or co-stimulatory signal is also included in the CAR. In other instances disclosed herein, the CAR does not include a component for generating a costimulatory signal. In some disclosures, an additional CAR is expressed in the same cell and provides the component for generating the secondary or costimulatory signal.

In some instances disclosed herein, the chimeric antigen receptor contains an intracellular domain of a T cell costimulatory molecule. In some instances disclosed herein, the CAR includes a signaling domain and/or transmembrane portion of a costimulatory receptor, such as CD28, 4-1BB, OX40, DAP10, and ICOS. In some disclosures, the same CAR includes both the activating and costimulatory components. In some instances disclosed herein, the chimeric antigen receptor contains an intracellular domain derived from a T cell costimulatory molecule or a functional variant thereof, such as between the transmembrane domain and intracellular signaling domain. In some disclosures, the T cell costimulatory molecule is CD28 or 41BB.

In some instances disclosed herein, the activating domain is included within one CAR, whereas the costimulatory component is provided by another CAR recognizing another antigen. In some instances disclosed herein, the CARs include activating or stimulatory CARs, costimulatory CARs, both expressed on the same cell (see WO2014/055668). In some disclosures, the cells include one or more stimulatory or activating CAR and/or a costimulatory CAR. In some instances disclosed herein, the cells further include inhibitory CARs (iCARs, see Fedorov et al., Sci. Transl. Medicine, 5(215) (December, 2013), such as a CAR recognizing an antigen other than the one associated with and/or specific for the disease or condition whereby an activating signal delivered through the disease-targeting CAR is diminished or inhibited by binding of the inhibitory CAR to its ligand, e.g., to reduce off-target effects.

In certain instances disclosed herein, the intracellular signaling domain comprises a CD28 transmembrane and signaling domain linked to a CD3 (e.g., CD3-zeta) intracellular domain. In some instances disclosed herein, the intracellular signaling domain comprises a chimeric CD28 and CD137 (4-1BB, TNFRSF9) co-stimulatory domains, linked to a CD3 zeta intracellular domain.

In some instances disclosed herein, the CAR encompasses one or more, e.g., two or more, costimulatory domains and an activation domain, e.g., primary activation domain, in the cytoplasmic portion. Exemplary CARs include intracellular components of CD3-zeta, CD28, and 4-1BB.

In some instances disclosed herein, the vector encoding the antigen receptor, and/or the cells expressing the CAR or other antigen receptor further includes a nucleic acid sequence encoding one or more marker(s). In some instances disclosed herein, the one or more marker(s) is a transduction marker, surrogate marker and/or a selection marker. In some embodiemnts, the marker is a surrogate marker, such as a cell surface marker, which may be used to confirm transduction or engineering of the cell to express the receptor

In some instances disclosed herein, the marker is a transduction marker or a surrogate marker. A transduction marker or a surrogate marker can be used to detect cells that have been introduced with the polynucleotide, e.g., a polynucleotide encoding a recombinant receptor. In some instances disclosed herein, the transduction marker can indicate or confirm modification of a cell. In some instances disclosed herein, the surrogate marker is a protein that is made to be co-expressed on the cell surface with the recombinant receptor, e.g. CAR. In particular instances disclosed herein, such a surrogate marker is a surface protein that has been modified to have little or no activity. In certain instances disclosed herein, the surrogate marker is encoded on the same polynucleotide that encodes the recombinant receptor. In some instances disclosed herein, the nucleic acid sequence encoding the recombinant receptor is operably linked to a nucleic acid sequence encoding a marker, optionally separated by an internal ribosome entry site (IRES), or a nucleic acid encoding a self-cleaving peptide or a peptide that causes ribosome skipping, such as a 2A sequence, such as a T2A, a P2A, an E2A or an F2A. Extrinsic marker genes may in some instances disclosed herein be utilized in connection with engineered cell to permit detection or selection of cells and, in some instances disclosed herein, also to promote cell suicide.

Exemplary surrogate markers can include truncated forms of cell surface polypeptides, such as truncated forms that are non-functional and to not transduce or are not capable of transducing a signal or a signal ordinarily transduced by the full-length form of the cell surface polypeptide, and/or do not or are not capable of internalizing. Exemplary truncated cell surface polypeptides including truncated forms of growth factors or other receptors such as a truncated human epidermal growth factor receptor 2 (tHER2), a truncated epidermal growth factor receptor (tEGFR, exemplary tEGFR sequence set forth in SEQ ID NO: 11 or 76) or a prostate-specific membrane antigen (PSMA) or modified form thereof. tEGFR may contain an epitope recognized by the antibody cetuximab (Erbitux^{®}) or other therapeutic anti-EGFR antibody or binding molecule, which can be used to identify or select cells that have been engineered with the tEGFR construct and an encoded exogenous protein, and/or to eliminate or separate cells expressing the encoded exogenous protein. See U.S. Patent No. 8,802,374 and Liu et al., Nature Biotech. 2016 April; 34(4): 430-434). In some disclosures, the marker, e.g. surrogate marker, includes all or part (e.g., truncated form) of CD34, a NGFR, a CD19 or a truncated CD19, e.g., a truncated non-human CD19, or epidermal growth factor receptor (e.g., tEGFR). In some instances disclosed herein, the marker is or comprises a fluorescent protein, such as green fluorescent protein (GFP), enhanced green fluorescent protein (EGFP), such as super-fold GFP (sfGFP), red fluorescent protein (RFP), such as tdTomato, mCherry, mStrawberry, AsRed2, DsRed or DsRed2, cyan fluorescent protein (CFP), blue green fluorescent protein (BFP), enhanced blue fluorescent protein (EBFP), and yellow fluorescent protein (YFP), and variants thereof, including species variants, monomeric variants, and codon-optimized and/or enhanced variants of the fluorescent proteins. In some instances disclosed herein, the marker is or comprises an enzyme, such as a luciferase, the lacZ gene from *E*. *coli,* alkaline phosphatase, secreted embryonic alkaline phosphatase (SEAP), chloramphenicol acetyl transferase (CAT). Exemplary light-emitting reporter genes include luciferase (luc), β-galactosidase, chloramphenicol acetyltransferase (CAT), β-glucuronidase (GUS) or variants thereof.

In some instances disclosed herein, the marker is a selection marker. In some instances disclosed herein, the selection marker is or comprises a polypeptide that confers resistance to exogenous agents or drugs. In some instances disclosed herein, the selection marker is an antibiotic resistance gene. In some instances disclosed herein, the selection marker is an antibiotic resistance gene confers antibiotic resistance to a mammalian cell. In some instances disclosed herein, the selection marker is or comprises a Puromycin resistance gene, a Hygromycin resistance gene, a Blasticidin resistance gene, a Neomycin resistance gene, a Geneticin resistance gene or a Zeocin resistance gene or a modified form thereof.

In some instances disclosed herein, the molecule is a non-self molecule, e.g., non-self protein, i.e., one that is not recognized as "self' by the immune system of the host into which the cells will be adoptively transferred.

In some instances disclosed herein, the marker serves no therapeutic function and/or produces no effect other than to be used as a marker for genetic engineering, e.g., for selecting cells successfully engineered. In other instances disclosed herein, the marker may be a therapeutic molecule or molecule otherwise exerting some desired effect, such as a ligand for a cell to be encountered in vivo, such as a costimulatory or immune checkpoint molecule to enhance and/or dampen responses of the cells upon adoptive transfer and encounter with ligand.

In some instances disclosed herein, the nucleic acid encoding the marker is operably linked to a polynucleotide encoding for a linker sequence, such as a cleavable linker sequence, *e.g*., a T2A. For example, a marker, and optionally a linker sequence, can be any as disclosed in PCT Pub. No. WO2014031687. For example, the marker can be a truncated EGFR (tEGFR) that is, optionally, linked to a linker sequence, such as a T2A cleavable linker sequence.

An exemplary polypeptide for a truncated EGFR (e.g. tEGFR) comprises the sequence of amino acids set forth in SEQ ID NO: 7 or 16 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 16. An exemplary T2A linker sequence comprises the sequence of amino acids set forth in SEQ ID NO: 6 or 17 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 6 or 17.

In some instances disclosed herein, the marker is a molecule, e.g., cell surface protein, not naturally found on T cells or not naturally found on the surface of T cells, or a portion thereof.

In some instances disclosed herein, CARs are referred to as first, second, and/or third generation CARs. In some disclosures, a first generation CAR is one that solely provides a CD3-chain induced signal upon antigen binding; in some disclosures, a second-generation CARs is one that provides such a signal and costimulatory signal, such as one including an intracellular signaling domain from a costimulatory receptor such as CD28 or CD137; in some disclosures, a third generation CAR is one that includes multiple costimulatory domains of different costimulatory receptors.

For example, in some instances disclosed herein, the CAR contains an antibody, e.g., an antibody fragment, a transmembrane domain that is or contains a transmembrane portion of CD28 or a functional variant thereof, and an intracellular signaling domain containing a signaling portion of CD28 or functional variant thereof and a signaling portion of CD3 zeta or functional variant thereof. In some instances disclosed herein, the CAR contains an antibody, e.g., antibody fragment, a transmembrane domain that is or contains a transmembrane portion of CD28 or a functional variant thereof, and an intracellular signaling domain containing a signaling portion of a 4-1BB or functional variant thereof and a signaling portion of CD3 zeta or functional variant thereof. In some such instances disclosed herein, the receptor further includes a spacer containing a portion of an Ig molecule, such as a human Ig molecule, such as an Ig hinge, e.g. an IgG4 hinge, such as a hinge-only spacer.

In some instances disclosed herein, the transmembrane domain of the recombinant receptor, e.g., the CAR, is or includes a transmembrane domain of human CD28 (e.g. Accession No. P01747.1) or variant thereof, such as a transmembrane domain that comprises the sequence of amino acids set forth in SEQ ID NO: 8 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 8; in some instances disclosed herein, the transmembrane-domain containing portion of the recombinant receptor comprises the sequence of amino acids set forth in SEQ ID NO: 9 or a sequence of amino acids having at least at or about 85%, 86%, 87%, 88%, 89%, 90%, 91%, 92%, 93%, 94%, 95%, 96%, 97%, 98%, 99% or more sequence identity thereto.

In some instances disclosed herein, the intracellular signaling component(s) of the recombinant receptor, e.g. the CAR, contains an intracellular costimulatory signaling domain of human CD28 or a functional variant or portion thereof, such as a domain with an LL to GG substitution at positions 186-187 of a native CD28 protein. For example, the intracellular signaling domain can comprise the sequence of amino acids set forth in SEQ ID NO: 10 or 11 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 10 or 11. In some instances disclosed herein, the intracellular domain comprises an intracellular costimulatory signaling domain of 4-1BB (e.g. (Accession No. Q07011.1) or functional variant or portion thereof, such as the sequence of amino acids set forth in SEQ ID NO: 12 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 12.

In some instances disclosed herein, the intracellular signaling domain of the recombinant receptor, e.g. the CAR, comprises a human CD3 zeta stimulatory signaling domain or functional variant thereof, such as an 112 AA cytoplasmic domain of isoform 3 of human CD3ζ (Accession No.: P20963.2) or a CD3 zeta signaling domain as described in U.S. Patent No.: 7,446,190 or U.S. Patent No. 8,911,993. For example, in some instances disclosed herein, the intracellular signaling domain comprises the sequence of amino acids as set forth in SEQ ID NO: 13, 14 or 15 or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 13, 14 or 15.

In some disclosures, the spacer contains only a hinge region of an IgG, such as only a hinge of IgG4 or IgG1, such as the hinge only spacer set forth in SEQ ID NO: 1. In other instances disclosed herein, the spacer is or contains an Ig hinge, e.g., an IgG4-derived hinge, optionally linked to a C_{H}2 and/or C_{H}3 domains. In some instances disclosed herein, the spacer is an Ig hinge, e.g., an IgG4 hinge, linked to C_{H}2 and C_{H}3 domains, such as set forth in SEQ ID NO: 4. In some instances disclosed herein, the spacer is an Ig hinge, e.g., an IgG4 hinge, linked to a C_{H}3 domain only, such as set forth in SEQ ID NO: 3. In some instances disclosed herein, the spacer is or comprises a glycine-serine rich sequence or other flexible linker such as known flexible linkers.

For example, in some instances disclosed herein, the CAR includes an antibody such as an antibody fragment, including scFvs, a spacer, such as a spacer containing a portion of an immunoglobulin molecule, such as a hinge region and/or one or more constant regions of a heavy chain molecule, such as an Ig-hinge containing spacer, a transmembrane domain containing all or a portion of a CD28-derived transmembrane domain, a CD28-derived intracellular signaling domain, and a CD3 zeta signaling domain. In some instances disclosed herein, the CAR includes an antibody or fragment, such as scFv, a spacer such as any of the Ig-hinge containing spacers, a CD28-derived transmembrane domain, a 4-1BB-derived intracellular signaling domain, and a CD3 zeta-derived signaling domain.

In particular instances disclosed herein, the CAR is a CD19-directed CAR containing an scFv antigen-binding domain from FMC63; a immunoglobulin hinge spacer, a transmembrane domain, and an intracellular signaling domain containing a costimulatory signaling region that is a signaling domain of 4-1BB and a signaling domain of a CD3-zeta (CD3ζ) chain. In some instances disclosed herein, the scFv contains the sequence set forth in SEQ ID NO::43. In some instances disclosed herein, the scFv ha a VL having CDRs having an amino acid sequences RASQDISKYLN (SEQ ID NO: 35), an amino acid sequence of SRLHSGV (SEQ ID NO: 36), and an amino acid sequence of GNTLPYTFG (SEQ ID NO: 37); and a VH with CDRs having an amino acid sequence of DYGVS (SEQ ID NO: 38), an amino acid sequence of VIWGSETTYYNSALKS (SEQ ID NO: 39) and YAMDYWG (SEQ ID NO: 40)). In some instances disclosed herein, the transmembrane domain has the sequence set forth in SEQ ID NO:8. In some instances disclosed herein, the transmembrane domain has a sequence that has at least 85%, 86%, 87%, 88%, 89%, 90%, 91%, 92%, 93%, 94%, 95%, 96%, 97%, 98%, 99% or more sequence identity to SEQ ID NO:8. In som instances disclosed herein, the 4-1BB costimulatory signaling domain has the sequence set forth in SEQ ID NO: 12. In some instances disclosed herein, the 4-1BB costimulatory signaling domain has a sequence at least 85%, 86%, 87%, 88%, 89%, 90%, 91%, 92%, 93%, 94%, 95%, 96%, 97%, 98%, 99% or more sequence identity to SEQ ID NO: 12. In some instances disclosed herein, the CD3-zeta domain has the sequence set forth in SEQ ID NO: 13. In some instances disclosed herein, the CD3zeta signaling domain has a sequence having at least 85%, 86%, 87%, 88%, 89%, 90%, 91%, 92%, 93%, 94%, 95%, 96%, 97%, 98%, 99% or more sequence identity thereto. In some instances disclosed herein, the CD19-directed CAR binds to CD 19 and mediates cytokine production and/or cytotoxic activity against CD19+ target cells when expressed in a T cell and stimulated via the CAR, such as by binding to CD 19.

In some instances disclosed herein, nucleic acid molecules encoding such CAR constructs further includes a sequence encoding a T2A ribosomal skip element and/or a tEGFR sequence, e.g., downstream of the sequence encoding the CAR. In some instances disclosed herein, the sequence encodes a T2A ribosomal skip element set forth in SEQ ID NO: 6 or 17, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 6 or 17. In some instances disclosed herein, T cells expressing an antigen receptor (e.g. CAR) can also be generated to express a truncated EGFR (EGFRt) as a non-immunogenic selection epitope (e.g. by introduction of a construct encoding the CAR and EGFRt separated by a T2A ribosome switch to express two proteins from the same construct), which then can be used as a marker to detect such cells (see e.g. U.S. Patent No. 8,802,374). In some instances disclosed herein, the sequence encodes an tEGFR sequence set forth in SEQ ID NO: 7 or 16, or a sequence of amino acids that exhibits at least 85%, 86%, 87%, 88%, 89%, 90%, 91%, 92%, 93%, 94%, 95%, 96%, 97%, 98%, 99% or more sequence identity to SEQ ID NO: 7 or 16.

In some instances disclosed herein, a single promoter may direct expression of an RNA that contains, in a single open reading frame (ORF), two or three genes (*e.g.* encoding the molecule involved in modulating a metabolic pathway and encoding the recombinant receptor) separated from one another by sequences encoding a self-cleavage peptide *(e.g.,* 2A sequences) or a protease recognition site (*e.g.,* furin). The ORF thus encodes a single polypeptide, which, either during (in the case of 2A) or after translation, is processed into the individual proteins. In some instances disclosed herein, the peptide, such as T2A, can cause the ribosome to skip (ribosome skipping) synthesis of a peptide bond at the C-terminus of a 2A element, leading to separation between the end of the 2A sequence and the next peptide downstream (*see,* for example, de Felipe. Genetic Vaccines and Ther. 2:13 (2004) and de Felipe et al. Traffic 5:616-626 (2004)). Many 2A elements are known. Examples of 2A sequences that can be used in the methods and nucleic acids disclosed herein, without limitation, 2A sequences from the foot-and-mouth disease virus (F2A, *e.g.,* SEQ ID NO: 21), equine rhinitis A virus (E2A, *e.g.,* SEQ ID NO: 20), Thosea asigna virus (T2A, *e.g.,* SEQ ID NO: 6 or 17), and porcine teschovirus-1 (P2A, *e.g.,* SEQ ID NO: 18 or 19) as described in U.S. Patent Publication No. 20070116690.

The recombinant receptors, such as CARs, expressed by the cells administered to the subject generally recognize or specifically bind to a molecule that is expressed in, associated with, and/or specific for the disease or condition or cells thereof being treated. Upon specific binding to the molecule, e.g., antigen, the receptor generally delivers an immunostimulatory signal, such as an ITAM-transduced signal, into the cell, thereby promoting an immune response targeted to the disease or condition. For example, in some instances disclosed herein, the cells express a CAR that specifically binds to an antigen expressed by a cell or tissue of the disease or condition or associated with the disease or condition.

### B. T Cell Receptors (TCRs)

In some instances disclosed herein, engineered cells, such as T cells, used in connection with the disclosed methods, uses, articles of manufacture or compositions are cells that express a T cell receptor (TCR) or antigen-binding portion thereof that recognizes an peptide epitope or T cell epitope of a target polypeptide, such as an antigen of a tumor, viral or autoimmune protein.

In some instances disclosed herein, a "T cell receptor" or "TCR" is a molecule that contains a variable α and β chains (also known as TCRα and TCRβ, respectively) or a variable γ and δ chains (also known as TCRα and TCRβ, respectively), or antigen-binding portions thereof, and which is capable of specifically binding to a peptide bound to an MHC molecule. In some instances disclosed herein, the TCR is in the αβ form. Typically, TCRs that exist in αβ and γδ forms are generally structurally similar, but T cells expressing them may have distinct anatomical locations or functions. A TCR can be found on the surface of a cell or in soluble form. Generally, a TCR is found on the surface of T cells (or T lymphocytes) where it is generally responsible for recognizing antigens bound to major histocompatibility complex (MHC) molecules.

Unless otherwise stated, the term "TCR" should be understood to encompass full TCRs as well as antigen-binding portions or antigen-binding fragments thereof. In some instances disclosed herein, the TCR is an intact or full-length TCR, including TCRs in the αβ form or γδ form. In some instances disclosed herein, the TCR is an antigen-binding portion that is less than a full-length TCR but that binds to a specific peptide bound in an MHC molecule, such as binds to an MHC-peptide complex. In some instances disclosed herein, an antigen-binding portion or fragment of a TCR can contain only a portion of the structural domains of a full-length or intact TCR, but yet is able to bind the peptide epitope, such as MHC-peptide complex, to which the full TCR binds. In some instances disclosed herein, an antigen-binding portion contains the variable domains of a TCR, such as variable α chain and variable β chain of a TCR, sufficient to form a binding site for binding to a specific MHC-peptide complex. Generally, the variable chains of a TCR contain complementarity determining regions involved in recognition of the peptide, MHC and/or MHC-peptide complex.

In some instances disclosed herein, the variable domains of the TCR contain hypervariable loops, or complementarity determining regions (CDRs), which generally are the primary contributors to antigen recognition and binding capabilities and specificity. In some instances disclosed herein, a CDR of a TCR or combination thereof forms all or substantially all of the antigen-binding site of a given TCR molecule. The various CDRs within a variable region of a TCR chain generally are separated by framework regions (FRs), which generally display less variability among TCR molecules as compared to the CDRs (see, e.g., Jores et al., Proc. Nat'l Acad. Sci. U.S.A. 87:9138, 1990; Chothia et al., EMBO J. 7:3745, 1988; see also Lefranc et al., Dev. Comp. Immunol. 27:55, 2003). In some instances disclosed herein, CDR3 is the main CDR responsible for antigen binding or specificity, or is the most important among the three CDRs on a given TCR variable region for antigen recognition, and/or for interaction with the processed peptide portion of the peptide-MHC complex. In some contexts, the CDR1 of the alpha chain can interact with the N-terminal part of certain antigenic peptides. In some contexts, CDR1 of the beta chain can interact with the C-terminal part of the peptide. In some contexts, CDR2 contributes most strongly to or is the primary CDR responsible for the interaction with or recognition of the MHC portion of the MHC-peptide complex. In some instances disclosed herein, the variable region of the β-chain can contain a further hypervariable region (CDR4 or HVR4), which generally is involved in superantigen binding and not antigen recognition (Kotb (1995) Clinical Microbiology Reviews, 8:411-426).

In some instances disclosed herein, a TCR also can contain a constant domain, a transmembrane domain and/or a short cytoplasmic tail (see, e.g., Janeway et al., Immunobiology: The Immune System in Health and Disease, 3rd Ed., Current Biology Publications, p. 4:33, 1997). In some disclosures, each chain of the TCR can possess one N-terminal immunoglobulin variable domain, one immunoglobulin constant domain, a transmembrane region, and a short cytoplasmic tail at the C-terminal end. In some instances disclosed herein, a TCR is associated with invariant proteins of the CD3 complex involved in mediating signal transduction.

In some instances disclosed herein, a TCR chain contains one or more constant domain. For example, the extracellular portion of a given TCR chain (e.g., α-chain or β-chain) can contain two immunoglobulin-like domains, such as a variable domain (e.g., Vα or Vβ; typically amino acids 1 to 116 based on Kabat numbering Kabat et al., "Sequences of Proteins of Immunological Interest, US Dept. Health and Human Services, Public Health Service National Institutes of Health, 1991, 5th ed.) and a constant domain (e.g., α-chain constant domain or Cα, typically positions 117 to 259 of the chain based on Kabat numbering or β chain constant domain or C_{β}, typically positions 117 to 295 of the chain based on Kabat) adjacent to the cell membrane. For example, in some instances disclosed herein, the extracellular portion of the TCR formed by the two chains contains two membrane-proximal constant domains, and two membrane-distal variable domains, which variable domains each contain CDRs. The constant domain of the TCR may contain short connecting sequences in which a cysteine residue forms a disulfide bond, thereby linking the two chains of the TCR. In some instances disclosed herein, a TCR may have an additional cysteine residue in each of the α and β chains, such that the TCR contains two disulfide bonds in the constant domains.

In some instances disclosed herein, the TCR chains contain a transmembrane domain. In some instances disclosed herein, the transmembrane domain is positively charged. In some instances disclosed herein, the TCR chain contains a cytoplasmic tail. In some instances disclosed herein, the structure allows the TCRto associate with other molecules like CD3 and subunits thereof. For example, a TCR containing constant domains with a transmembrane region may anchor the protein in the cell membrane and associate with invariant subunits of the CD3 signaling apparatus or complex. The intracellular tails of CD3 signaling subunits (e.g. CD3γ, CD3δ, CD3ε and CD3ζ chains) contain one or more immunoreceptor tyrosine-based activation motif or ITAM that are involved in the signaling capacity of the TCR complex.

In some instances disclosed herein, the TCR may be a heterodimer of two chains α and β (or optionally γ and δ) or it may be a single chain TCR construct. In some instances disclosed herein, the TCR is a heterodimer containing two separate chains (α and β chains or γ and δ chains) that are linked, such as by a disulfide bond or disulfide bonds.

In some instances disclosed herein, the TCR can be generated from a known TCR sequence(s), such as sequences of Vα,β chains, for which a substantially full-length coding sequence is readily available. Methods for obtaining full-length TCR sequences, including V chain sequences, from cell sources are well known. In some instances disclosed herein, nucleic acids encoding the TCR can be obtained from a variety of sources, such as by polymerase chain reaction (PCR) amplification of TCR-encoding nucleic acids within or isolated from a given cell or cells, or synthesis of publicly available TCR DNA sequences.

In some instances disclosed herein, the TCR is obtained from a biological source, such as from cells such as from a T cell (e.g. cytotoxic T cell), T-cell hybridomas or other publicly available source. In some instances disclosed herein, the T-cells can be obtained from *in vivo* isolated cells. In some instances disclosed herein, the TCR is a thymically selected TCR. In some instances disclosed herein, the TCR is a neoepitope-restricted TCR. In some instances disclosed herein, the T- cells can be a cultured T-cell hybridoma or clone. In some instances disclosed herein, the TCR or antigen-binding portion thereof or antigen-binding fragment thereof can be synthetically generated from knowledge of the sequence of the TCR.

In some instances disclosed herein, the TCR is generated from a TCR identified or selected from screening a library of candidate TCRs against a target polypeptide antigen, or target T cell epitope thereof. TCR libraries can be generated by amplification of the repertoire of Vα and Vβ from T cells isolated from a subject, including cells present in PBMCs, spleen or other lymphoid organ. In some instances disclosed herein, T cells can be amplified from tumor-infiltrating lymphocytes (TILs). In some instances disclosed herein, TCR libraries can be generated from CD4⁺ or CD8⁺ cells. In some instances disclosed herein, the TCRs can be amplified from a T cell source of a normal of healthy subject, i.e. normal TCR libraries. In some instances disclosed herein, the TCRs can be amplified from a T cell source of a diseased subject, i.e. diseased TCR libraries. In some instances disclosed herein, degenerate primers are used to amplify the gene repertoire of Vα and Vβ, such as by RT-PCR in samples, such as T cells, obtained from humans. In some instances disclosed herein, scTv libraries can be assembled from naive Vα and Vβ libraries in which the amplified products are cloned or assembled to be separated by a linker. Depending on the source of the subject and cells, the libraries can be HLA allele-specific. Alternatively, in some instances disclosed herein, TCR libraries can be generated by mutagenesis or diversification of a parent or scaffold TCR molecule. In some disclosures, the TCRs are subjected to directed evolution, such as by mutagenesis, e.g., of the α or β chain. In some disclosures, particular residues within CDRs of the TCR are altered. In some instances disclosed herein, selected TCRs can be modified by affinity maturation. In some instances disclosed herein, antigen-specific T cells may be selected, such as by screening to assess CTL activity against the peptide. In some disclosures, TCRs, e.g. present on the antigen-specific T cells, may be selected, such as by binding activity, e.g., particular affinity or avidity for the antigen.

In some instances disclosed herein, the TCR or antigen-binding portion thereof is one that has been modified or engineered. In some instances disclosed herein, directed evolution methods are used to generate TCRs with altered properties, such as with higher affinity for a specific MHC-peptide complex. In some instances disclosed herein, directed evolution is achieved by display methods including, but not limited to, yeast display (Holler et al. (2003) Nat Immunol, 4, 55-62; Holler et al. (2000) Proc Natl Acad Sci USA, 97, 5387-92), phage display (Li et al. (2005) Nat Biotechnol, 23, 349-54), or T cell display (Chervin et al. (2008) J Immunol Methods, 339, 175-84). In some instances disclosed herein, display approaches involve engineering, or modifying, a known, parent or reference TCR. For example, in some instances disclosed herein, a wild-type TCR can be used as a template for producing mutagenized TCRs in which in one or more residues of the CDRs are mutated, and mutants with an desired altered property, such as higher affinity for a desired target antigen, are selected.

In some instances disclosed herein, peptides of a target polypeptide for use in producing or generating a TCR of interest are known or can be readily identified. In some instances disclosed herein, peptides suitable for use in generating TCRs or antigen-binding portions can be determined based on the presence of an HLA-restricted motif in a target polypeptide of interest, such as a target polypeptide described below. In some instances disclosed herein, peptides are identified using available computer prediction models. In some instances disclosed herein, for predicting MHC class I binding sites, such models include, but are not limited to, ProPred1 (Singh and Raghava (2001) Bioinformatics 17(12): 1236-1237, and SYFPEITHI (see Schuler et al. (2007) Immunoinformatics Methods in Molecular Biology, 409(1): 75-93 2007). In some instances disclosed herein, the MHC-restricted epitope is HLA-A0201, which is expressed in approximately 39-46% of all Caucasians and therefore, represents a suitable choice of MHC antigen for use preparing a TCR or other MHC-peptide binding molecule.

HLA-A0201-binding motifs and the cleavage sites for proteasomes and immune-proteasomes using computer prediction models are known. For predicting MHC class I binding sites, such models include, but are not limited to, ProPred1 (described in more detail in Singh and Raghava, ProPred: prediction of HLA-DR binding sites. BIOINFORMATICS 17(12): 1236-1237 2001), and SYFPEITHI (see Schuler et al. SYFPEITHI, Database for Searching and T-Cell Epitope Prediction. in Immunoinformatics Methods in Molecular Biology, vol 409(1): 75-93 2007)

In some instances disclosed herein, the TCR or antigen binding portion thereof may be a recombinantly produced natural protein or mutated form thereof in which one or more property, such as binding characteristic, has been altered. In some instances disclosed herein, a TCR may be derived from one of various animal species, such as human, mouse, rat, or other mammal. A TCR may be cell-bound or in soluble form. In some instances disclosed herein, for purposes of the disclosed methods, the TCR is in cell-bound form expressed on the surface of a cell.

In some instances disclosed herein, the TCR is a full-length TCR. In some instances disclosed herein, the TCR is an antigen-binding portion. In some instances disclosed herein, the TCR is a dimeric TCR (dTCR). In some instances disclosed herein, the TCR is a single-chain TCR (sc-TCR). In some instances disclosed herein, a dTCR or scTCR have the structures as described in WO 03/020763, WO 04/033685, WO2011/044186.

In some instances disclosed herein, the TCR contains a sequence corresponding to the transmembrane sequence. In some instances disclosed herein, the TCR does contain a sequence corresponding to cytoplasmic sequences. In some instances disclosed herein, the TCR is capable of forming a TCR complex with CD3. In some instances disclosed herein, any of the TCRs, including a dTCR or scTCR, can be linked to signaling domains that yield an active TCR on the surface of a T cell. In some instances disclosed herein, the TCR is expressed on the surface of cells.

In some instances disclosed herein a dTCR contains a first polypeptide wherein a sequence corresponding to a TCR α chain variable region sequence is fused to the N terminus of a sequence corresponding to a TCR α chain constant region extracellular sequence, and a second polypeptide wherein a sequence corresponding to a TCR β chain variable region sequence is fused to the N terminus a sequence corresponding to a TCR β chain constant region extracellular sequence, the first and second polypeptides being linked by a disulfide bond. In some instances disclosed herein, the bond can correspond to the native inter-chain disulfide bond present in native dimeric αβ TCRs. In some instances disclosed herein, the interchain disulfide bonds are not present in a native TCR. For example, in some instances disclosed herein, one or more cysteines can be incorporated into the constant region extracellular sequences of dTCR polypeptide pair. In some instances disclosed herein, both a native and a non-native disulfide bond may be desirable. In some instances disclosed herein, the TCR contains a transmembrane sequence to anchor to the membrane.

In some instances disclosed herein, a dTCR contains a TCR α chain containing a variable α domain, a constant α domain and a first dimerization motif attached to the C-terminus of the constant α domain, and a TCR β chain comprising a variable β domain, a constant β domain and a first dimerization motif attached to the C-terminus of the constant β domain, wherein the first and second dimerization motifs easily interact to form a covalent bond between an amino acid in the first dimerization motif and an amino acid in the second dimerization motif linking the TCR α chain and TCR β chain together.

In some instances disclosed herein, the TCR is a scTCR. Typically, a scTCR can be generated using methods known, See e.g., Soo Hoo, W. F. et al. PNAS (USA) 89, 4759 (1992); Wülfing, C. and Plückthun, A., J. Mol. Biol. 242, 655 (1994); Kurucz, I. et al. PNAS (USA) 90 3830 (1993); International published PCT Nos. WO 96/13593, WO 96/18105, WO99/60120, WO99/18129, WO 03/020763, WO2011/044186; and Schlueter, C. J. et al. J. Mol. Biol. 256, 859 (1996). In some instances disclosed herein, a scTCR contains an introduced non-native disulfide interchain bond to facilitate the association of the TCR chains (see e.g. International published PCT No. WO 03/020763). In some instances disclosed herein, a scTCR is a non-disulfide linked truncated TCR in which heterologous leucine zippers fused to the C-termini thereof facilitate chain association (see e.g. International published PCT No. WO99/60120). In some instances disclosed herein, a scTCR contain a TCRα variable domain covalently linked to a TCRβ variable domain via a peptide linker (see e.g., International published PCT No. WO99/18129).

In some instances disclosed herein, a scTCR contains a first segment constituted by an amino acid sequence corresponding to a TCR α chain variable region, a second segment constituted by an amino acid sequence corresponding to a TCR β chain variable region sequence fused to the N terminus of an amino acid sequence corresponding to a TCR β chain constant domain extracellular sequence, and a linker sequence linking the C terminus of the first segment to the N terminus of the second segment.

In some instances disclosed herein, a scTCR contains a first segment constituted by an α chain variable region sequence fused to the N terminus of an α chain extracellular constant domain sequence, and a second segment constituted by a β chain variable region sequence fused to the N terminus of a sequence β chain extracellular constant and transmembrane sequence, and, optionally, a linker sequence linking the C terminus of the first segment to the N terminus of the second segment.

In some instances disclosed herein, a scTCR contains a first segment constituted by a TCR β chain variable region sequence fused to the N terminus of a β chain extracellular constant domain sequence, and a second segment constituted by an α chain variable region sequence fused to the N terminus of a sequence α chain extracellular constant and transmembrane sequence, and, optionally, a linker sequence linking the C terminus of the first segment to the N terminus of the second segment.

In some instances disclosed herein, the linker of a scTCRs that links the first and second TCR segments can be any linker capable of forming a single polypeptide strand, while retaining TCR binding specificity. In some instances disclosed herein, the linker sequence may, for example, have the formula -P-AA-P- wherein P is proline and AA represents an amino acid sequence wherein the amino acids are glycine and serine. In some instances disclosed herein, the first and second segments are paired so that the variable region sequences thereof are orientated for such binding. Hence, in some instances disclosed herein, the linker has a sufficient length to span the distance between the C terminus of the first segment and the N terminus of the second segment, or vice versa, but is not too long to block or reduces bonding of the scTCR to the target ligand. In some instances disclosed herein, the linker can contain from or from about 10 to 45 amino acids, such as 10 to 30 amino acids or 26 to 41 amino acids residues, for example 29, 30, 31 or 32 amino acids. In some instances disclosed herein, the linker has the formula - PGGG-(SGGGG)₅-P- wherein P is proline, G is glycine and S is serine (SEQ ID NO:28). In some instances disclosed herein, the linker has the sequence GSADDAKKDAAKKDGKS (SEQ ID NO:29)

In some instances disclosed herein, the scTCR contains a covalent disulfide bond linking a residue of the immunoglobulin region of the constant domain of the α chain to a residue of the immunoglobulin region of the constant domain of the β chain. In some instances disclosed herein, the interchain disulfide bond in a native TCR is not present. For example, in some instances disclosed herein, one or more cysteines can be incorporated into the constant region extracellular sequences of the first and second segments of the scTCR polypeptide. In some instances disclosed herein, both a native and a non-native disulfide bond may be desirable.

In some instances disclosed herein of a dTCR or scTCR containing introduced interchain disulfide bonds, the native disulfide bonds are not present. In some instances disclosed herein, the one or more of the native cysteines forming a native interchain disulfide bonds are substituted to another residue, such as to a serine or alanine. In some instances disclosed herein, an introduced disulfide bond can be formed by mutating non-cysteine residues on the first and second segments to cysteine. Exemplary non-native disulfide bonds of a TCR are described in published International PCT No. WO2006/000830.

In some instances disclosed herein, the TCR or antigen-binding fragment thereof exhibits an affinity with an equilibrium binding constant for a target antigen of between or between about 10-5 and 10-12 M and all individual values and ranges therein. In some instances disclosed herein, the target antigen is an MHC-peptide complex or ligand.

In some instances disclosed herein, nucleic acid or nucleic acids encoding a TCR, such as α and β chains, can be amplified by PCR, cloning or other suitable means and cloned into a suitable expression vector or vectors. The expression vector can be any suitable recombinant expression vector, and can be used to transform or transfect any suitable host. Suitable vectors include those designed for propagation and expansion or for expression or both, such as plasmids and viruses.

In some instances disclosed herein, the vector can a vector of the pUC series (Fermentas Life Sciences), the pBluescript series (Stratagene, LaJolla, Calif.), the pET series (Novagen, Madison, Wis.), the pGEX series (Pharmacia Biotech, Uppsala, Sweden), or the pEX series (Clontech, Palo Alto, Calif.). In some instances disclosed herein, bacteriophage vectors, such as λG10, λGT11, λZapII (Stratagene), λEMBL4, and λNM1149, also can be used. In some instances disclosed herein, plant expression vectors can be used and include pBI01, pBI101.2, pBI101.3, pBI121 and pBIN19 (Clontech). In some instances disclosed herein, animal expression vectors include pEUK-Cl, pMAM and pMAMneo (Clontech). In some instances disclosed herein, a viral vector is used, such as a retroviral vector.

In some instances disclosed herein, the recombinant expression vectors can be prepared using standard recombinant DNA techniques. In some instances disclosed herein, vectors can contain regulatory sequences, such as transcription and translation initiation and termination codons, which are specific to the type of host (e.g., bacterium, fungus, plant, or animal) into which the vector is to be introduced, as appropriate and taking into consideration whether the vector is DNA- or RNA-based. In some instances disclosed herein, the vector can contain a nonnative promoter operably linked to the nucleotide sequence encoding the TCR or antigen-binding portion (or other MHC-peptide binding molecule). In some instances disclosed herein, the promoter can be a non-viral promoter or a viral promoter, such as a cytomegalovirus (CMV) promoter, an SV40 promoter, an RSV promoter, and a promoter found in the long-terminal repeat of the murine stem cell virus. Other known promoters also are contemplated.

In some instances disclosed herein, to generate a vector encoding a TCR, the α and β chains are PCR amplified from total cDNA isolated from a T cell clone expressing the TCR of interest and cloned into an expression vector. In some instances disclosed herein, the α and β chains are cloned into the same vector. In some instances disclosed herein, the α and β chains are cloned into different vectors. In some instances disclosed herein, the generated α and β chains are incorporated into a retroviral, e.g. lentiviral, vector. Genetically Engineered Cells and Methods of Producing Cells

In some instances disclosed herein, the disclosed methods involve administering to a subject having a disease or condition cells expressing a recombinant antigen receptor. Various methods for the introduction of genetically engineered components, e.g., recombinant receptors, e.g., CARs or TCRs, are well known and may be used with the disclosed methods and compositions. Exemplary methods include those for transfer of nucleic acids encoding the receptors, including via viral, e.g., retroviral or lentiviral, transduction, transposons, and electroporation.

Among the cells expressing the receptors and administered by the disclosed methods are engineered cells. The genetic engineering generally involves introduction of a nucleic acid encoding the recombinant or engineered component into a composition containing the cells, such as by retroviral transduction, transfection, or transformation.

### C. Chimeric Auto-Antibody Receptors (CAARs)

In some instances disclosed herein, among the recombinant receptor expressed by the engineered cells used in connection with the disclosed methods, uses, articles of manufacture and compositions is a chimeric autoantibody receptor (CAAR). In some instances disclosed herein, the CAAR is specific for an autoantibody. In some instances disclosed herein, a cell expressing the CAAR, such as a T cell engineered to express a CAAR, can be used to specifically bind to and kill autoantibody-expressing cells, but not normal antibody expressing cells. In some instances disclosed herein, CAAR-expressing cells can be used to treat an autoimmune disease associated with expression of self-antigens, such as autoimmune diseases. In some instances disclosed herein, CAAR-expressing cells can target B cells that ultimately produce the autoantibodies and display the autoantibodies on their cell surfaces, mark these B cells as disease-specific targets for therapeutic intervention. In some instances disclosed herein, CAAR-expressing cells can be used to efficiently targeting and killing the pathogenic B cells in autoimmune diseases by targeting the disease-causing B cells using an antigen-specific chimeric autoantibody receptor. In some instances disclosed herein, the recombinant receptor is a CAAR, such as any described in U.S. Patent Application Pub. No. US 2017/0051035.

In some instances disclosed herein, the CAAR comprises an autoantibody binding domain, a transmembrane domain, and an intracellular signaling region. In some instances disclosed herein, the intracellular signaling region comprises an intracellular signaling domain. In some instances disclosed herein, the intracellular signaling domain is or comprises a primary signaling domain, a signaling domain that is capable of inducing a primary activation signal in a T cell, a signaling domain of a T cell receptor (TCR) component, and/or a signaling domain comprising an immunoreceptor tyrosine-based activation motif (ITAM). In some instances disclosed herein, the intracellular signaling region comprises a secondary or costimulatory signaling region (secondary intracellular signaling regions).

In some instances disclosed herein, the autoantibody binding domain comprises an autoantigen or a fragment thereof. The choice of autoantigen can depend upon the type of autoantibody being targeted. For example, the autoantigen may be chosen because it recognizes an autoantibody on a target cell, such as a B cell, associated with a particular disease state, e.g. an autoimmune disease, such as an autoantibody-mediated autoimmune disease. In some instances disclosed herein, the autoimmune disease includes pemphigus vulgaris (PV). Exemplary autoantigens include desmoglein 1 (Dsg1) and Dsg3.

### D. Multi-targeting

In some instances disclosed herein, the cells used in connection with the disclosed methods, uses, articles of manufacture and compositions include cells employing multi-targeting strategies, such as expression of two or more genetically engineered receptors on the cell, each recognizing the same of a different antigen and typically each including a different intracellular signaling component. Such multi-targeting strategies are described, for example, in International Patent Application, Publication No.: WO 2014055668 A1 (describing combinations of activating and costimulatory CARs, e.g., targeting two different antigens present individually on off-target, e.g., normal cells, but present together only on cells of the disease or condition to be treated) and Fedorov et al., Sci. Transl. Medicine, 5(215) (2013) (describing cells expressing an activating and an inhibitory CAR, such as those in which the activating CAR binds to one antigen expressed on both normal or non-diseased cells and cells of the disease or condition to be treated, and the inhibitory CAR binds to another antigen expressed only on the normal cells or cells which it is not desired to treat).

For example, in some instances disclosed herein, the cells include a receptor expressing a first genetically engineered antigen receptor (e.g., CAR or TCR) which is capable of inducing an activating or stimulatory signal to the cell, generally upon specific binding to the antigen recognized by the first receptor, e.g., the first antigen. In some instances disclosed herein, the cell further includes a second genetically engineered antigen receptor (e.g., CAR or TCR), e.g., a chimeric costimulatory receptor, which is capable of inducing a costimulatory signal to the immune cell, generally upon specific binding to a second antigen recognized by the second receptor. In some instances disclosed herein, the first antigen and second antigen are the same. In some instances disclosed herein, the first antigen and second antigen are different.

In some instances disclosed herein, the first and/or second genetically engineered antigen receptor (e.g. CAR or TCR) is capable of inducing an activating signal to the cell. In some instances disclosed herein, the receptor includes an intracellular signaling component containing ITAM or ITAM-like motifs. In some instances disclosed herein, the activation induced by the first receptor involves a signal transduction or change in protein expression in the cell resulting in initiation of an immune response, such as ITAM phosphorylation and/or initiation of ITAM-mediated signal transduction cascade, formation of an immunological synapse and/or clustering of molecules near the bound receptor (e.g. CD4 or CD8, etc.), activation of one or more transcription factors, such as NF-κB and/or AP-1, and/or induction of gene expression of factors such as cytokines, proliferation, and/or survival.

In some instances disclosed herein, the first and/or second receptor includes intracellular signaling domains or regions of costimulatory receptors such as CD28, CD137 (4-1BB), OX40, and/or ICOS. In some instances disclosed herein, the first and second receptor include an intracellular signaling domain of a costimulatory receptor that are different. In one instance disclosed herein, the first receptor contains a CD28 costimulatory signaling region and the second receptor contain a 4-1BB co-stimulatory signaling region or vice versa.

In some instances disclosed herein, the first and/or second receptor includes both an intracellular signaling domain containing ITAM or ITAM-like motifs and an intracellular signaling domain of a costimulatory receptor.

In some instances disclosed herein, the first receptor contains an intracellular signaling domain containing ITAM or ITAM-like motifs and the second receptor contains an intracellular signaling domain of a costimulatory receptor. The costimulatory signal in combination with the activating signal induced in the same cell is one that results in an immune response, such as a robust and sustained immune response, such as increased gene expression, secretion of cytokines and other factors, and T cell mediated effector functions such as cell killing.

In some instances disclosed herein, neither ligation of the first receptor alone nor ligation of the second receptor alone induces a robust immune response. In some disclosures, if only one receptor is ligated, the cell becomes tolerized or unresponsive to antigen, or inhibited, and/or is not induced to proliferate or secrete factors or carry out effector functions. In some such instances disclosed herein, however, when the plurality of receptors are ligated, such as upon encounter of a cell expressing the first and second antigens, a desired response is achieved, such as full immune activation or stimulation, e.g., as indicated by secretion of one or more cytokine, proliferation, persistence, and/or carrying out an immune effector function such as cytotoxic killing of a target cell.

In some instances disclosed herein, the two receptors induce, respectively, an activating and an inhibitory signal to the cell, such that binding by one of the receptor to its antigen activates the cell or induces a response, but binding by the second inhibitory receptor to its antigen induces a signal that suppresses or dampens that response. Examples are combinations of activating CARs and inhibitory CARs or iCARs. Such a strategy may be used, for example, in which the activating CAR binds an antigen expressed in a disease or condition but which is also expressed on normal cells, and the inhibitory receptor binds to a separate antigen which is expressed on the normal cells but not cells of the disease or condition.

In some instances disclosed herein, the multi-targeting strategy is employed in a case where an antigen associated with a particular disease or condition is expressed on a non-diseased cell and/or is expressed on the engineered cell itself, either transiently (e.g., upon stimulation in association with genetic engineering) or permanently. In such instances disclosed herein, by requiring ligation of two separate and individually specific antigen receptors, specificity, selectivity, and/or efficacy may be improved.

In some instances disclosed herein, the plurality of antigens, e.g., the first and second antigens, are expressed on the cell, tissue, or disease or condition being targeted, such as on the cancer cell. In some disclosures, the cell, tissue, disease or condition is multiple myeloma or a multiple myeloma cell. In some instances disclosed herein, one or more of the plurality of antigens generally also is expressed on a cell which it is not desired to target with the cell therapy, such as a normal or non-diseased cell or tissue, and/or the engineered cells themselves. In such instances disclosed herein, by requiring ligation of multiple receptors to achieve a response of the cell, specificity and/or efficacy is achieved.

### IV. METHODS OF ENGINEERING CELLS

In particular instances disclosed herein, the engineered cells are produced by a process that generates an output composition of enriched T cells from one or more input compositions and/or from a single biological sample. In certain instances disclosed herein, the output composition contains cells that express a recombinant receptor, e.g., a CAR, such as an anti-CD 19 CAR. In particular instances disclosed herein, the cells of the output compositions are suitable for administration to a subject as a therapy, e.g., an autologous cell therapy. In some instances disclosed herein, the output composition is a composition of enriched CD4+ or CD8+ T cells.

In some instances disclosed herein, the process for generating or producing engineered cells is by a process that includes some or all of the steps of: collecting or obtaining a biological sample; isolating, selecting, or enriching input cells from the biological sample; cryopreserving and storing the input cells; thawing and/or incubating the input cells under stimulating conditions; engineering the stimulated cells to express or contain a recombinant polynucleotide, e.g., a polynucleotide encoding a recombinant receptor such as a CAR; cultivating the engineered cells, e.g. to a threshold amount, density, or expansion; formulating the cultivated cells in an output composition; and/or cryopreserved and storing the formulated output cells until the cells are released for infusion and/or are suitable to be administered to a subject. In certain instances disclosed herein, the process is performed with two or more input compositions of enriched T cells, such as a separate CD4+ composition and a separate CD8+ composition, that are separately processed and engineered from the same starting or initial biological sample and re-infused back into the subject at a defined ratio, e.g. 1:1 ratio of CD4+ to CD8+ T cells. In some instances disclosed herein, the enriched T cells are or include engineered T cells, e.g., T cells transduced to express a recombinant receptor.

In particular instances disclosed herein, an output composition of engineered cells expressing a recombinant receptor (e.g. anti-CD 19 CAR) is produced from an initial and/or input composition of cells. In some instances disclosed herein, the input composition is a composition of enriched T cells, enriched CD4+ T cells, and/or enriched CD8+ T cells (herein after also referred to as compositions of enriched T cells, compositions of enriched CD4+ T cells, and compositions of enriched CD8+ T cells, respectively). In some instances disclosed herein, the dose of engineered T cells employed in the instances disclosed herein, for administration to a subject, is enriched for CD4+ or CD8+ T cells. In some disclosures, the enrichment is compared to the amount or percentage of CD4+ or CD8+ cells that are present in the input composition and/or a single biological sample, such as a sample obtained from the subject. In some instances disclosed herein, a composition enriched in CD4+ T cells contains at least 60%, 65%, 70%, 75%, 80%, 85%, 90%, 95%, 98%, 99%, or 99.9% CD4+ T cells. In particular instances disclosed herein, the composition of enriched CD4+ T cells contains 100% CD4+ T cells contains about 100% CD4+ T cells. In certain instances disclosed herein, the composition of enriched T cells includes or contains less than 20%, less than 10%, less than 5%, less than 1%, less than 0.1%, or less than 0.01% CD8+ T cells, and/or contains no CD8+ T cells, and/or is free or substantially free of CD8+ T cells. In some instances disclosed herein, the populations of cells consist essentially of CD4+ T cells. In some instances disclosed herein, a composition enriched in CD8+ T cells contains at least 75%, 80%, 85%, 90%, 95%, 98%, 99%, or 99.9% CD8+ T cells, or contains or contains about 100% CD8+ T cells. In certain instances disclosed herein, the composition of enriched CD8+ T cells includes or contains less than 20%, less than 10%, less than 5%, less than 1%, less than 0.1%, or less than 0.01% CD4+ T cells, and/or contains no CD4+ T cells, and/or is free or substantially free of CD4+ T cells. In some instances disclosed herein, the populations of cells consist essentially of CD8+ T cells.

In certain instances disclosed herein, the process for producing engineered cells further can include one or more of: activating and/or stimulating a cells, e.g., cells of an input composition; genetically engineering the activated and/or stimulated cells, e.g., to introduce a polynucleotide encoding a recombinant protein by transduction or transfection; and/or cultivating the engineered cells, e.g., under conditions that promote proliferation and/or expansion. In particular instances disclosed herein, the disclosed methods may be used in connection with harvesting, collecting, and/or formulating output compositions produced after the cells have been incubated, activated, stimulated, engineered, transduced, transfected, and/or cultivated.

In some instances disclosed herein, engineered cells, such as those that express an anti-CD19 CAR as described, used in accord with the disclosed methods are produced or generated by a process for selecting, isolating, activating, stimulating, expanding, cultivating, and/or formulating cells. In some instances disclosed herein, such methods include any as described.

In some instances disclosed herein, engineered cells, such as those that express an anti-CD 19 CAR as described, used in accord with the disclosed methods and uses are produced or generated by a process for selecting, isolating, activating, stimulating, expanding, cultivating, and/or formulating cells. In some instances disclosed herein, such methods include any as described.

In some instances disclosed herein, engineered cells, such as those that express an anti-CD 19 CAR as described, used in accord with the disclosed methods and uses are produced or generated by exemplary processes as described in, for example, WO 2019/089855 and WO 2015/164675.

In some of any instances disclosed herein, exemplary processes for generating, producing or manufacturing the engineered cells, such as those that express an anti-CD 19 CAR as described, or a composition comprising such cells, such as a composition comprising engineered CD4+ T cells and engineered CD8+ T cells each expressing the same anti-CD19 chimeric antigen receptor (CAR), involve subjecting enriched CD4+ and enriched CD8+ cell populations, separately, to process steps. In some disclosures of the exemplary process for generating or manufacturing engineered cells, CD4+ and CD8+ cells are separately selected from human peripheral blood mononuclear cells (PBMCs), for example, that are obtained by leukapheresis, generating separate enriched CD4+ and enriched CD8+ cell compositions. In some disclosures, such cells can be cryopreserved. In some disclosures, the CD4+ and CD8+ compositions can be subsequently thawed and separately subject to steps for stimulation, transduction, and expansion.

In some disclosures of the exemplary process for generating or manufacturing engineered cells, thawed CD4+ and CD8+ cells are separately stimulated, for example, in the presence of paramagnetic polystyrene-coated beads coupled to anti-CD3 and anti-CD28 antibodies (such as at a 1:1 bead to cell ratio). In some disclosures, the stimulation is carried out in media containing human recombinant IL-2, human recombinant IL-15, and N-Acetyl Cysteine (NAC). In some disclosures, the cell culture media for CD4+ cells also can include human recombinant IL-7.

In some disclosures of the exemplary process for generating or manufacturing engineered cells, following the introduction of the beads, CD4+ and CD8+ cells are separately transduced with a lentiviral vector encoding the same CAR, such as the same anti-CD 19 CAR. In some disclosures, the CAR can contain an anti-CD 19 scFv derived from a murine antibody, an immunoglobulin spacer, a transmembrane domain derived from CD28, a costimulatory region derived from 4-1BB, and a CD3-zeta intracellular signaling domain. In some disclosures, the vector can encode a truncated receptor that serves as a surrogate marker for CAR expression that is connected to the CAR construct by a T2A sequence. In some disclosures of the exemplary process, the cells are transduced in the presence of 10 µg/ml protamine sulfate.

In some disclosures of the exemplary process for generating or manufacturing engineered cells, following transduction, the beads are removed from the cell compositions by exposure to a magnetic field. In some disclosures, the CD4+ and CD8+ cell compositions are separately cultivated for expansion with continual mixing and oxygen transfer by a bioreactor (for example, a Xuri W25 Bioreactor). In some instances disclosed herein, poloxamer is added to the media. In some disclosures, both the CD4+ and the CD8+ cell compositions are cultivated in the presence of IL-2 and IL-15. In some disclosures, the CD4+ cell media also included IL-7. In some instances disclosed herein, the CD4+ and CD8+ cells are each cultivated, prior to harvest, to 4-fold expansion. In some disclosures, one day after reaching the threshold, cells from each composition can be separately harvested, formulated, and cryopreserved. In some disclosures, the exemplary processes for generating, producing or manufacturing the engineered cells, such as those that express an anti-CD 19 CAR as described, or a composition comprising such cells, such as a composition comprising engineered CD4+ T cells and engineered CD8+ T cells each expressing the same anti-CD19 chimeric antigen receptor (CAR), include those described in Table 11 below.

**Table 11: Exemplary process for generating CD4+ and CD8+ CAR-T cells**

| **Stage** | **CD4+ cells** | | **CD8+ cells** | |
|---|---|---|---|---|
| **Stimulation** (day 1-2) | | • anti-CD3/CD28 antibody conjugated beads | | • anti-CD3/CD28 antibody conjugated beads |
| | | • 1:1 bead to cell ratio | | • 1:1 bead to cell ratio |
| | | • media: IL-2, IL-7, IL-15, and NAC | | • media: IL-2, IL-15, and NAC |
| **Transduction** (day 2-5) | | • transduction adjuvant (e.g. 10 µg/ml protamine sulfate) | | • transduction adjuvant (e.g. 10 µg/ml protamine sulfate) |
| **Bead removal** (day 5*) | | • magnetic bead removal | | • magnetic bead removal |
| **Expansion (day 5* - Harvest)** | | • rocking motion bioreactor and/or continuous mixing | | • rocking motion bioreactor and/or continuous mixing |
| | | • media: IL-2, IL-7, IL15, and poloxamer | | • media: IL-2, IL15, and poloxamer |

| | | | | |
|---|---|---|---|---|
| *Approximate | | | | |

In other disclosures, a different exemplary process for generating, producing or manufacturing the engineered cells or a composition comprising such cells include a process that differs from the exemplary process above in that: NAC is not added to the media during stimulation; CD4+ cell media does not contain IL-2; cells are stimulated at a bead to cell ratio of 3: 1; cells are transduced with a higher concentration of protamine sulfate; bead removal occurs at about day 7; and expansion is performed at a static setting, i.e., without continual mixing or perfusion (e.g., semi-continuous and/or stepwise perfusion), and without poloxamer.

In some instances disclosed herein, at least one separate composition of enriched CD4+ T cells and at least one separate composition of enriched CD8+ T cells are isolated, selected, enriched, or obtained from a single biological sample, e.g., a sample of PBMCs or other white blood cells from the same donor such as a patient or healthy individual. In some instances disclosed herein, a separate composition of enriched CD4+ T cells and a separate composition of enriched CD8+ T cells originated, e.g., were initially isolated, selected, and/or enriched, from the same biological sample, such as a single biological sample obtained, collected, and/or taken from a single subject. In some instances disclosed herein, a biological sample is first subjected to selection of CD4+ T cells, where both the negative and positive fractions are retained, and the negative fraction is further subjected to selection of CD8+ T cells. In other instances disclosed herein, a biological sample is first subjected to selection of CD8+ T cells, where both the negative and positive fractions are retained, and the negative fraction is further subjected to selection of CD4+ T cells. In some instances disclosed herein, methods of selection are carried out as described in International PCT publication No. WO2015/164675. In some instances disclosed herein, methods of selection are carried out as described in International PCT publication No. WO 2019/089855. In some disclosures, a biological sample is first positively selected for CD8+ T cells to generate at least one composition of enriched CD8+ T cells, and the negative fraction is then positively selected for CD4+ T cells to generate at least one composition of enriched CD4+ T cells, such that the at least one composition of enriched CD8+ T cells and the at least one composition of enriched CD4+ T cells are separate compositions from the same biological sample, e.g., from the same donor patient or healthy individual. In some disclosures, two or more separate compositions of enriched T cells, e.g., at least one being a composition of enriched CD4+ T cells and at least one being a separate composition of enriched CD8+ T cells from the same donor, are separately frozen, e.g., cryoprotectedor cryopreserved in a cryopreservation media.

In some disclosures, two or more separate compositions of enriched T cells, e.g., at least one being a composition of enriched CD4+ T cells and at least one being a separate composition of enriched CD8+ T cells from the same biological sample, are activated and/or stimulated by contacting with a stimulatory reagent (e.g., by incubation with anti-CD3/anti-CD28 conjugated magnetic beads for T cell activation). In some disclosures, each of the activated/stimulated cell composition is engineered, transduced, and/or transfected, e.g., using a viral vector encoding a recombinant protein (e.g. CAR), to express the same recombinant protein in the CD4+ T cells and CD8+ T cells of each cell composition. In some disclosures, the method comprises removing the stimulatory reagent, e.g., magnetic beads, from the cell composition. In some disclosures, a cell composition containing engineered CD4+ T cells and a cell composition containing engineered CD8+ T cells are separately cultivated, e.g., for separate expansion of the CD4+ T cell and CD8+ T cell populations therein. In certain instances disclosed herein, a cell composition from the cultivation is harvested and/or collected and/or formulated, e.g., by washing the cell composition in a formulation buffer. In certain instances disclosed herein, a formulated cell composition comprising CD4+ T cells and a formulated cell composition comprising CD8+ T cells is frozen, e.g., cryoprotected or cryopreserved in a cryopreservation media. In some disclosures, engineered CD4+ T cells and CD8+ T cells in each formulation originate from the same donor or biological sample and express the same recombination protein (e.g., CAR, such as anti-CD19 CAR). In some disclosures, a separate engineered CD4+ formulation and a separate engineered CD8+ formulation are administered at a defined ratio, e.g. 1:1, to a subject in need thereof such as the same donor.

### A. Cells and Preparation of Cells for Genetic Engineering

In some instances disclosed herein, cells, such as T cells, used in connection with the disclosed methods, uses, articles of manufacture or compositions are cells have been genetically engineered to express a recombinant receptor, e.g., a CAR or a TCR described herein. In some instances disclosed herein, the engineered cells are used in the context of cell therapy, e.g., adoptive cell therapy. In some instances disclosed herein, the engineered cells are immune cells. In some instances disclosed herein, the engineered cells are T cells, such as CD4+ or CD8+ T cells.

In some instances disclosed herein, the nucleic acids, such as nucleic acids encoding a recombinant receptor, are heterologous, i.e., normally not present in a cell or sample obtained from the cell, such as one obtained from another organism or cell, which for example, is not ordinarily found in the cell being engineered and/or an organism from which such cell is derived. In some instances disclosed herein, the nucleic acids are not naturally occurring, such as a nucleic acid not found in nature, including one comprising chimeric combinations of nucleic acids encoding various domains from multiple different cell types.

The cells generally are eukaryotic cells, such as mammalian cells, and typically are human cells. In some instances disclosed herein, the cells are derived from the blood, bone marrow, lymph, or lymphoid organs, are cells of the immune system, such as cells of the innate or adaptive immunity, e.g., myeloid or lymphoid cells, including lymphocytes, typically T cells and/or NK cells. Other exemplary cells include stem cells, such as multipotent and pluripotent stem cells, including induced pluripotent stem cells (iPSCs). The cells typically are primary cells, such as those isolated directly from a subject and/or isolated from a subject and frozen. In some instances disclosed herein, the cells include one or more subsets of T cells or other cell types, such as whole T cell populations, CD4⁺ cells, CD8⁺ cells, and subpopulations thereof, such as those defined by function, activation state, maturity, potential for differentiation, expansion, recirculation, localization, and/or persistence capacities, antigen-specificity, type of antigen receptor, presence in a particular organ or compartment, marker or cytokine secretion profile, and/or degree of differentiation. With reference to the subject to be treated, the cells may be allogeneic and/or autologous. Among the methods include off-the-shelf methods. In some disclosures, such as for off-the-shelf technologies, the cells are pluripotent and/or multipotent, such as stem cells, such as induced pluripotent stem cells (iPSCs). In some instances disclosed herein, the methods include isolating cells from the subject, preparing, processing, culturing, and/or engineering them, and reintroducing them into the same subject, before or after cryopreservation.

Among the sub-types and subpopulations of T cells and/or of CD4⁺ and/or of CD8⁺ T cells are naive T (T_{N}) cells, effector T cells (T_{EFF}), memory T cells and sub-types thereof, such as stem cell memory T (T_{SCM}), central memory T (T_{CM}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells.

In some instances disclosed herein, the cells are natural killer (NK) cells. In some instances disclosed herein, the cells are monocytes or granulocytes, e.g., myeloid cells, macrophages, neutrophils, dendritic cells, mast cells, eosinophils, and/or basophils.

In some instances disclosed herein, the cells include one or more nucleic acids introduced via genetic engineering, and thereby express recombinant or genetically engineered products of such nucleic acids. In some instances disclosed herein, the nucleic acids are heterologous, i.e., normally not present in a cell or sample obtained from the cell, such as one obtained from another organism or cell, which for example, is not ordinarily found in the cell being engineered and/or an organism from which such cell is derived. In some instances disclosed herein, the nucleic acids are not naturally occurring, such as a nucleic acid not found in nature, including one comprising chimeric combinations of nucleic acids encoding various domains from multiple different cell types.

In some instances disclosed herein, preparation of the engineered cells includes one or more culture and/or preparation steps. The cells for introduction of the nucleic acid encoding the transgenic receptor such as the CAR, may be isolated from a sample, such as a biological sample, e.g., one obtained from or derived from a subject. In some instances disclosed herein, the subject from which the cell is isolated is one having the disease or condition or in need of a cell therapy or to which cell therapy will be administered. The subject in some instances disclosed herein is a human in need of a particular therapeutic intervention, such as the adoptive cell therapy for which cells are being isolated, processed, and/or engineered.

Accordingly, the cells in some instances disclosed herein are primary cells, e.g., primary human cells. The samples include tissue, fluid, and other samples taken directly from the subject, as well as samples resulting from one or more processing steps, such as separation, centrifugation, genetic engineering (e.g. transduction with viral vector), washing, and/or incubation. The biological sample can be a sample obtained directly from a biological source or a sample that is processed. Biological samples include, but are not limited to, body fluids, such as blood, plasma, serum, cerebrospinal fluid, synovial fluid, urine and sweat, tissue and organ samples, including processed samples derived therefrom.

In some disclosures, the sample from which the cells are derived or isolated is blood or a blood-derived sample, or is or is derived from an apheresis or leukapheresis product. Exemplary samples include whole blood, peripheral blood mononuclear cells (PBMCs), leukocytes, bone marrow, thymus, tissue biopsy, tumor, leukemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom. Samples include, in the context of cell therapy, e.g., adoptive cell therapy, samples from autologous and allogeneic sources.

In some instances disclosed herein, the cells are derived from cell lines, e.g., T cell lines. The cells in some instances disclosed herein are obtained from a xenogeneic source, for example, from mouse, rat, non-human primate, and pig.

In some instances disclosed herein, isolation of the cells includes one or more preparation and/or non-affinity based cell separation steps. In some instances disclosed herein, cells are washed, centrifuged, and/or incubated in the presence of one or more reagents, for example, to remove unwanted components, enrich for desired components, lyse or remove cells sensitive to particular reagents. In some instances disclosed herein, cells are separated based on one or more property, such as density, adherent properties, size, sensitivity and/or resistance to particular components.

In some instances disclosed herein, cells from the circulating blood of a subject are obtained, e.g., by apheresis or leukapheresis. The samples, in some disclosures, contain lymphocytes, including T cells, monocytes, granulocytes, B cells, other nucleated white blood cells, red blood cells, and/or platelets, and in some disclosures contains cells other than red blood cells and platelets.

In some instances disclosed herein, the blood cells collected from the subject are washed, e.g., to remove the plasma fraction and to place the cells in an appropriate buffer or media for subsequent processing steps. In some instances disclosed herein, the cells are washed with phosphate buffered saline (PBS). In some instances disclosed herein, the wash solution lacks calcium and/or magnesium and/or many or all divalent cations. In some disclosures, a washing step is accomplished a semi-automated "flow-through" centrifuge (for example, the Cobe 2991 cell processor, Baxter) according to the manufacturer's instructions. In some disclosures, a washing step is accomplished by tangential flow filtration (TFF) according to the manufacturer's instructions. In some instances disclosed herein, the cells are resuspended in a variety of biocompatible buffers after washing, such as, for example, Ca⁺⁺/Mg⁺⁺ free PBS. In certain instances disclosed herein, components of a blood cell sample are removed and the cells directly resuspended in culture media.

In some instances disclosed herein, the methods include density-based cell separation methods, such as the preparation of white blood cells from peripheral blood by lysing the red blood cells and centrifugation through a Percoll or Ficoll gradient.

In some instances disclosed herein, at least a portion of the selection step includes incubation of cells with a selection reagent. The incubation with a selection reagent or reagents, e.g., as part of selection methods which may be performed using one or more selection reagents for selection of one or more different cell types based on the expression or presence in or on the cell of one or more specific molecules, such as surface markers, e.g., surface proteins, intracellular markers, or nucleic acid. In some instances disclosed herein, any known method using a selection reagent or reagents for separation based on such markers may be used. In some instances disclosed herein, the selection reagent or reagents result in a separation that is affinity- or immunoaffinity-based separation. For example, the selection in some disclosures includes incubation with a reagent or reagents for separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

In some disclosures of such processes, a volume of cells is mixed with an amount of a desired affinity-based selection reagent. The immunoaffinity-based selection can be carried out using any system or method that results in a favorable energetic interaction between the cells being separated and the molecule specifically binding to the marker on the cell, e.g., the antibody or other binding partner on the solid surface, e.g., particle. In some instances disclosed herein, methods are carried out using particles such as beads, e.g. magnetic beads, that are coated with a selection agent (e.g. antibody) specific to the marker of the cells. The particles (e.g. beads) can be incubated or mixed with cells in a container, such as a tube or bag, while shaking or mixing, with a constant cell density-to-particle (e.g., bead) ratio to aid in promoting energetically favored interactions. In other instances disclosed herein, the methods include selection of cells in which all or a portion of the selection is carried out in the internal cavity of a centrifugal chamber, for example, under centrifugal rotation. In some instances disclosed herein, incubation of cells with selection reagents, such as immunoaffinity-based selection reagents, is performed in a centrifugal chamber. In certain instances disclosed herein, the isolation or separation is carried out using a system, device, or apparatus described in International Patent Application, Publication Number WO2009/072003, or US 20110003380 A1. In one instance disclosed herein, the system is a system as described in International Publication Number WO2016/073602.

In some instances disclosed herein, by conducting such selection steps or portions thereof (e.g., incubation with antibody-coated particles, e.g., magnetic beads) in the cavity of a centrifugal chamber, the user is able to control certain parameters, such as volume of various solutions, addition of solution during processing and timing thereof, which can provide advantages compared to other available methods. For example, the ability to decrease the liquid volume in the cavity during the incubation can increase the concentration of the particles (e.g. bead reagent) used in the selection, and thus the chemical potential of the solution, without affecting the total number of cells in the cavity. This in turn can enhance the pairwise interactions between the cells being processed and the particles used for selection. In some instances disclosed herein, carrying out the incubation step in the chamber, e.g., when associated with the systems, circuitry, and control as described herein, permits the user to effect agitation of the solution at desired time(s) during the incubation, which also can improve the interaction.

In some instances disclosed herein, at least a portion of the selection step is performed in a centrifugal chamber, which includes incubation of cells with a selection reagent. In some disclosures of such processes, a volume of cells is mixed with an amount of a desired affinity-based selection reagent that is far less than is normally employed when performing similar selections in a tube or container for selection of the same number of cells and/or volume of cells according to manufacturer's instructions. In some instances disclosed herein, an amount of selection reagent or reagents that is/are no more than 5%, no more than 10%, no more than 15%, no more than 20%, no more than 25%, no more than 50%, no more than 60%, no more than 70% or no more than 80% of the amount of the same selection reagent(s) employed for selection of cells in a tube or container-based incubation for the same number of cells and/or the same volume of cells according to manufacturer's instructions is employed.

In some instances disclosed herein, for selection, e.g., immunoaffinity-based selection of the cells, the cells are incubated in the cavity of the chamber in a composition that also contains the selection buffer with a selection reagent, such as a molecule that specifically binds to a surface marker on a cell that it desired to enrich and/or deplete, but not on other cells in the composition, such as an antibody, which optionally is coupled to a scaffold such as a polymer or surface, e.g., bead, e.g., magnetic bead, such as magnetic beads coupled to monoclonal antibodies specific for CD4 and CD8. In some instances disclosed herein, as described, the selection reagent is added to cells in the cavity of the chamber in an amount that is substantially less than (e.g. is no more than 5%, 10%, 20%, 30%, 40%, 50%, 60%, 70% or 80% of the amount) as compared to the amount of the selection reagent that is typically used or would be necessary to achieve about the same or similar efficiency of selection of the same number of cells or the same volume of cells when selection is performed in a tube with shaking or rotation. In some instances disclosed herein, the incubation is performed with the addition of a selection buffer to the cells and selection reagent to achieve a target volume with incubation of the reagent of, for example, 10 mL to 200 mL, such as at least or about at least 10 mL, 20 mL, 30 mL, 40 mL, 50 mL, 60 mL, 70 mL, 80 mL, 90 mL, 100 mL, 150 mL or 200 mL. In some instances disclosed herein, the selection buffer and selection reagent are pre-mixed before addition to the cells. In some instances disclosed herein, the selection buffer and selection reagent are separately added to the cells. In some instances disclosed herein, the selection incubation is carried out with periodic gentle mixing condition, which can aid in promoting energetically favored interactions and thereby permit the use of less overall selection reagent while achieving a high selection efficiency.

In some instances disclosed herein, the total duration of the incubation with the selection reagent is from or from about 5 minutes to 6 hours, such as 30 minutes to 3 hours, for example, at least or about at least 30 minutes, 60 minutes, 120 minutes or 180 minutes.

In some instances disclosed herein, the incubation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some instances disclosed herein, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In some instances disclosed herein, such process is carried out within the entirely closed system to which the chamber is integral. In some instances disclosed herein, this process (and in some disclosures also one or more additional step, such as a previous wash step washing a sample containing the cells, such as an apheresis sample) is carried out in an automated fashion, such that the cells, reagent, and other components are drawn into and pushed out of the chamber at appropriate times and centrifugation effected, so as to complete the wash and binding step in a single closed system using an automated program.

In some instances disclosed herein, after the incubation and/or mixing of the cells and selection reagent and/or reagents, the incubated cells are subjected to a separation to select for cells based on the presence or absence of the particular reagent or reagents. In some instances disclosed herein, the separation is performed in the same closed system in which the incubation of cells with the selection reagent was performed. In some instances disclosed herein, after incubation with the selection reagents, incubated cells, including cells in which the selection reagent has bound are transferred into a system for immunoaffinity-based separation of the cells. In some instances disclosed herein, the system for immunoaffinity-based separation is or contains a magnetic separation column.

In some instances disclosed herein, the isolation methods include the separation of different cell types based on the expression or presence in the cell of one or more specific molecules, such as surface markers, e.g., surface proteins, intracellular markers, or nucleic acid. In some instances disclosed herein, any known method for separation based on such markers may be used. In some instances disclosed herein, the separation is affinity- or immunoaffinity-based separation. For example, the isolation in some disclosures includes separation of cells and cell populations based on the cells' expression or expression level of one or more markers, typically cell surface markers, for example, by incubation with an antibody or binding partner that specifically binds to such markers, followed generally by washing steps and separation of cells having bound the antibody or binding partner, from those cells having not bound to the antibody or binding partner.

Such separation steps can be based on positive selection, in which the cells having bound the reagents are retained for further use, and/or negative selection, in which the cells having not bound to the antibody or binding partner are retained. In some instances disclosed herein, both fractions are retained for further use. In some disclosures, negative selection can be particularly useful where no antibody is available that specifically identifies a cell type in a heterogeneous population, such that separation is best carried out based on markers expressed by cells other than the desired population.

The separation need not result in 100% enrichment or removal of a particular cell population or cells expressing a particular marker. For example, positive selection of or enrichment for cells of a particular type, such as those expressing a marker, refers to increasing the number or percentage of such cells, but need not result in a complete absence of cells not expressing the marker. Likewise, negative selection, removal, or depletion of cells of a particular type, such as those expressing a marker, refers to decreasing the number or percentage of such cells, but need not result in a complete removal of all such cells.

In some instances disclosed herein, multiple rounds of separation steps are carried out, where the positively or negatively selected fraction from one step is subjected to another separation step, such as a subsequent positive or negative selection. In some instances disclosed herein, a single separation step can deplete cells expressing multiple markers simultaneously, such as by incubating cells with a plurality of antibodies or binding partners, each specific for a marker targeted for negative selection. Likewise, multiple cell types can simultaneously be positively selected by incubating cells with a plurality of antibodies or binding partners expressed on the various cell types.

For example, in some disclosures, specific subpopulations of T cells, such as cells positive or expressing high levels of one or more surface markers, e.g., CD28⁺, CD62L⁺, CCR7⁺, CD27⁺, CD127⁺, CD4⁺, CD8⁺, CD45RA⁺, and/or CD45RO⁺ T cells, are isolated by positive or negative selection techniques.

For example, CD3⁺, CD28⁺ T cells can be positively selected using anti-CD3/anti-CD28 conjugated magnetic beads (e.g., DYNABEADS^{®} M-450 CD3/CD28 T Cell Expander).

In some instances disclosed herein, isolation is carried out by enrichment for a particular cell population by positive selection, or depletion of a particular cell population, by negative selection. In some instances disclosed herein, positive or negative selection is accomplished by incubating cells with one or more antibodies or other binding agent that specifically bind to one or more surface markers expressed or expressed (marker⁺) at a relatively higher level (marker^{high}) on the positively or negatively selected cells, respectively.

In particular instances disclosed herein, a biological sample, e.g., a sample of PBMCs or other white blood cells, are subjected to selection of CD4+ T cells, where both the negative and positive fractions are retained. In certain instances disclosed herein, CD8+ T cells are selected from the negative fraction. In some instances disclosed herein, a biological sample is subjected to selection of CD8+ T cells, where both the negative and positive fractions are retained. In certain instances disclosed herein, CD4+ T cells are selected from the negative fraction.

In some instances disclosed herein, T cells are separated from a PBMC sample by negative selection of markers expressed on non-T cells, such as B cells, monocytes, or other white blood cells, such as CD14. In some disclosures, a CD4⁺ or CD8⁺ selection step is used to separate CD4⁺ helper and CD8⁺ cytotoxic T cells. Such CD4⁺ and CD8⁺ populations can be further sorted into sub-populations by positive or negative selection for markers expressed or expressed to a relatively higher degree on one or more naive, memory, and/or effector T cell subpopulations.

In some instances disclosed herein, CD8⁺ cells are further enriched for or depleted of naive, central memory, effector memory, and/or central memory stem cells, such as by positive or negative selection based on surface antigens associated with the respective subpopulation. In some instances disclosed herein, enrichment for central memory T (T_{CM}) cells is carried out to increase efficacy, such as to improve long-term survival, expansion, and/or engraftment following administration, which in some disclosures is particularly robust in such sub-populations. *See* Terakura et al. (2012) Blood. 1:72-82; Wang et al. (2012) J Immunother. 35(9):689-701. In some instances disclosed herein, combining T_{CM}-enriched CD8⁺ T cells and CD4⁺T cells further enhances efficacy.

In instances disclosed herein, memory T cells are present in both CD62L⁺ and CD62L⁻ subsets of CD8⁺ peripheral blood lymphocytes. PBMC can be enriched for or depleted of CD62L⁻CD8⁺ and/or CD62L⁺CD8⁺ fractions, such as using anti-CD8 and anti-CD62L antibodies.

In some instances disclosed herein, the enrichment for central memory T (T_{CM}) cells is based on positive or high surface expression of CD45RO, CD62L, CCR7, CD28, CD3, and/or CD127; in some disclosures, it is based on negative selection for cells expressing or highly expressing CD45RA and/or granzyme B. In some disclosures, isolation of a CD8⁺ population enriched for T_{CM} cells is carried out by depletion of cells expressing CD4, CD 14, CD45RA, and positive selection or enrichment for cells expressing CD62L. In one disclosure, enrichment for central memory T (T_{CM}) cells is carried out starting with a negative fraction of cells selected based on CD4 expression, which is subjected to a negative selection based on expression of CD 14 and CD45RA, and a positive selection based on CD62L. Such selections in some disclosures are carried out simultaneously and in other disclosures are carried out sequentially, in either order. In some disclosures, the same CD4 expression-based selection step used in preparing the CD8⁺ cell population or subpopulation, also is used to generate the CD4⁺ cell population or sub-population, such that both the positive and negative fractions from the CD4-based separation are retained and used in subsequent steps of the methods, optionally following one or more further positive or negative selection steps.

In a particular instance disclosed herein, a sample of PBMCs or other white blood cell sample is subjected to selection of CD4⁺ cells, where both the negative and positive fractions are retained. The negative fraction then is subjected to negative selection based on expression of CD 14 and CD45RA or CD 19, and positive selection based on a marker characteristic of central memory T cells, such as CD62L or CCR7, where the positive and negative selections are carried out in either order.

CD4⁺ T helper cells are sorted into naive, central memory, and effector cells by identifying cell populations that have cell surface antigens. CD4⁺ lymphocytes can be obtained by standard methods. In some instances disclosed herein, naive CD4⁺ T lymphocytes are CD45RO⁻, CD45RA⁺, CD62L⁺, CD4⁺ T cells. In some instances disclosed herein, central memory CD4⁺ cells are CD62L⁺ and CD45RO⁺. In some instances disclosed herein, effector CD4⁺ cells are CD62L⁻ and CD45RO⁻.

In one instance disclosed herein, to enrich for CD4⁺ cells by negative selection, a monoclonal antibody cocktail typically includes antibodies to CD14, CD20, CD11b, CD16, HLA-DR, and CD8. In some instances disclosed herein, the antibody or binding partner is bound to a solid support or matrix, such as a magnetic bead or paramagnetic bead, to allow for separation of cells for positive and/or negative selection. For example, in some instances disclosed herein, the cells and cell populations are separated or isolated using immunomagnetic (or affinitymagnetic) separation techniques (reviewed in Methods in Molecular Medicine, vol. 58: Metastasis Research Protocols, Vol. 2: Cell Behavior In Vitro and In Vivo, p 17-25 Edited by: S. A. Brooks and U. Schumacher © Humana Press Inc., Totowa, NJ).

In some disclosures, the sample or composition of cells to be separated is incubated with small, magnetizable or magnetically responsive material, such as magnetically responsive particles or microparticles, such as paramagnetic beads (e.g., such as Dynalbeads or MACS beads). The magnetically responsive material, e.g., particle, generally is directly or indirectly attached to a binding partner, e.g., an antibody, that specifically binds to a molecule, e.g., surface marker, present on the cell, cells, or population of cells that it is desired to separate, e.g., that it is desired to negatively or positively select.

In some instances disclosed herein, the magnetic particle or bead comprises a magnetically responsive material bound to a specific binding member, such as an antibody or other binding partner. There are many well-known magnetically responsive materials used in magnetic separation methods. Suitable magnetic particles include those described in Molday, U.S. Pat. No. 4,452,773, and in European Patent Specification EP 452342 B. Colloidal sized particles, such as those described in Owen U.S. Pat. No. 4,795,698, and Liberti et al., U.S. Pat. No. 5,200,084 are other examples.

The incubation generally is carried out under conditions whereby the antibodies or binding partners, or molecules, such as secondary antibodies or other reagents, which specifically bind to such antibodies or binding partners, which are attached to the magnetic particle or bead, specifically bind to cell surface molecules if present on cells within the sample.

In some disclosures, the sample is placed in a magnetic field, and those cells having magnetically responsive or magnetizable particles attached thereto will be attracted to the magnet and separated from the unlabeled cells. For positive selection, cells that are attracted to the magnet are retained; for negative selection, cells that are not attracted (unlabeled cells) are retained. In some disclosures, a combination of positive and negative selection is performed during the same selection step, where the positive and negative fractions are retained and further processed or subject to further separation steps.

In certain instances disclosed herein, the magnetically responsive particles are coated in primary antibodies or other binding partners, secondary antibodies, lectins, enzymes, or streptavidin. In certain instances disclosed herein, the magnetic particles are attached to cells via a coating of primary antibodies specific for one or more markers. In certain instances disclosed herein, the cells, rather than the beads, are labeled with a primary antibody or binding partner, and then cell-type specific secondary antibody- or other binding partner (e.g., streptavidin)-coated magnetic particles, are added. In certain instances disclosed herein, streptavidin-coated magnetic particles are used in conjunction with biotinylated primary or secondary antibodies.

In some instances disclosed herein, the magnetically responsive particles are left attached to the cells that are to be subsequently incubated, cultured and/or engineered; in some disclosures, the particles are left attached to the cells for administration to a patient. In some instances disclosed herein, the magnetizable or magnetically responsive particles are removed from the cells. Methods for removing magnetizable particles from cells are known and include, e.g., the use of competing non-labeled antibodies, and magnetizable particles or antibodies conjugated to cleavable linkers. In some instances disclosed herein, the magnetizable particles are biodegradable.

In some instances disclosed herein, the affinity-based selection is via magnetic-activated cell sorting (MACS) (Miltenyi Biotec, Auburn, CA). Magnetic Activated Cell Sorting (MACS) systems are capable of high-purity selection of cells having magnetized particles attached thereto. In certain instances disclosed herein, MACS operates in a mode wherein the non-target and target species are sequentially eluted after the application of the external magnetic field. That is, the cells attached to magnetized particles are held in place while the unattached species are eluted. Then, after this first elution step is completed, the species that were trapped in the magnetic field and were prevented from being eluted are freed in some manner such that they can be eluted and recovered. In certain instances disclosed herein, the non-target cells are labelled and depleted from the heterogeneous population of cells.

In certain instances disclosed herein, the isolation or separation is carried out using a system, device, or apparatus that carries out one or more of the isolation, cell preparation, separation, processing, incubation, culture, and/or formulation steps of the methods. In some disclosures, the system is used to carry out each of these steps in a closed or sterile environment, for example, to minimize error, user handling and/or contamination. In one instance disclosed herein, the system is a system as described in International Patent Application, Publication Number WO2009/072003, or US 20110003380 A1.

In some instances disclosed herein, the system or apparatus carries out one or more, e.g., all, of the isolation, processing, engineering, and formulation steps in an integrated or self-contained system, and/or in an automated or programmable fashion. In some disclosures, the system or apparatus includes a computer and/or computer program in communication with the system or apparatus, which allows a user to program, control, assess the outcome of, and/or adjust various aspects of the processing, isolation, engineering, and formulation steps.

In some disclosures, the separation and/or other steps is carried out using CliniMACS system (Miltenyi Biotec), for example, for automated separation of cells on a clinical-scale level in a closed and sterile system. Components can include an integrated microcomputer, magnetic separation unit, peristaltic pump, and various pinch valves. The integrated computer in some disclosures controls all components of the instrument and directs the system to perform repeated procedures in a standardized sequence. The magnetic separation unit in some disclosures includes a movable permanent magnet and a holder for the selection column. The peristaltic pump controls the flow rate throughout the tubing set and, together with the pinch valves, ensures the controlled flow of buffer through the system and continual suspension of cells.

The CliniMACS system in some disclosures uses antibody-coupled magnetizable particles that are supplied in a sterile, non-pyrogenic solution. In some instances disclosed herein, after labelling of cells with magnetic particles the cells are washed to remove excess particles. A cell preparation bag is then connected to the tubing set, which in turn is connected to a bag containing buffer and a cell collection bag. The tubing set consists of pre-assembled sterile tubing, including a pre-column and a separation column, and are for single use only. After initiation of the separation program, the system automatically applies the cell sample onto the separation column. Labelled cells are retained within the column, while unlabeled cells are removed by a series of washing steps. In some instances disclosed herein, the cell populations for use with the methods described herein are unlabeled and are not retained in the column. In some instances disclosed herein, the cell populations for use with the methods described herein are labeled and are retained in the column. In some instances disclosed herein, the cell populations for use with the methods described herein are eluted from the column after removal of the magnetic field, and are collected within the cell collection bag.

In certain instances disclosed herein, separation and/or other steps are carried out using the CliniMACS Prodigy system (Miltenyi Biotec). The CliniMACS Prodigy system in some disclosures is equipped with a cell processing unity that permits automated washing and fractionation of cells by centrifugation. The CliniMACS Prodigy system can also include an onboard camera and image recognition software that determines the optimal cell fractionation endpoint by discerning the macroscopic layers of the source cell product. For example, peripheral blood is automatically separated into erythrocytes, white blood cells and plasma layers. The CliniMACS Prodigy system can also include an integrated cell cultivation chamber which accomplishes cell culture protocols such as, e.g., cell differentiation and expansion, antigen loading, and long-term cell culture. Input ports can allow for the sterile removal and replenishment of media and cells can be monitored using an integrated microscope. See, e.g., Klebanoff et al. (2012) J Immunother. 35(9): 651-660, Terakura et al. (2012) Blood.1:72-82, and Wang et al. (2012) J Immunother. 35(9):689-701.

In some instances disclosed herein, a cell population described herein is collected and enriched (or depleted) via flow cytometry, in which cells stained for multiple cell surface markers are carried in a fluidic stream. In some instances disclosed herein, a cell population described herein is collected and enriched (or depleted) via preparative scale (FACS)-sorting. In certain instances disclosed herein, a cell population described herein is collected and enriched (or depleted) by use of microelectromechanical systems (MEMS) chips in combination with a FACS-based detection system (see, e.g., WO 2010/033140, Cho et al. (2010) Lab Chip 10, 1567-1573; and Godin et al. (2008) J Biophoton. 1(5):355-376. In both instances disclosed herein, cells can be labeled with multiple markers, allowing for the isolation of well-defined T cell subsets at high purity.

In some instances disclosed herein, the antibodies or binding partners are labeled with one or more detectable marker, to facilitate separation for positive and/or negative selection. For example, separation may be based on binding to fluorescently labeled antibodies. In some instances disclosed herein, separation of cells based on binding of antibodies or other binding partners specific for one or more cell surface markers are carried in a fluidic stream, such as by fluorescence-activated cell sorting (FACS), including preparative scale (FACS) and/or microelectromechanical systems (MEMS) chips, e.g., in combination with a flow-cytometric detection system. Such methods allow for positive and negative selection based on multiple markers simultaneously.

In some instances disclosed herein, the preparation methods include steps for freezing, e.g., cryopreserving, the cells, either before or after isolation, incubation, and/or engineering. In some instances disclosed herein, the freeze and subsequent thaw step removes granulocytes and, to some extent, monocytes in the cell population. In some instances disclosed herein, the cells are suspended in a freezing solution, e.g., following a washing step to remove plasma and platelets. Any of a variety of known freezing solutions and parameters in some disclosures may be used. One instance disclosed herein involves using PBS containing 20% DMSO and 8% human serum albumin (HSA), or other suitable cell freezing media. This is then diluted 1:1 with media so that the final concentration of DMSO and HSA are 10% and 4%, respectively. The cells are generally then frozen to -80° C. at a rate of 1° per minute and stored in the vapor phase of a liquid nitrogen storage tank.

In some instances disclosed herein, the isolation and/or selection results in one or more input compositions of enriched T cells, e.g., CD3+ T cells, CD4+ T cells, and/or CD8+ T cells. In some instances disclosed herein, two or more separate input composition are isolated, selected, enriched, or obtained from a single biological sample. In some instances disclosed herein, separate input compositions are isolated, selected, enriched, and/or obtained from separate biological samples collected, taken, and/or obtained from the same subject.

In certain instances disclosed herein, the one or more input compositions is or includes a composition of enriched T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD3+ T cells. In particular instance disclosed herein, the input composition of enriched T cells consists essentially of CD3+ T cells.

In certain instances disclosed herein, the one or more input compositions is or includes a composition of enriched CD4+ T cells that includes at least 60%, at least 65%, at least 70%, at least 75%, at least 80%, at least 85%, at least 90%, at least 95%, at least 98%, at least 99%, at least 99.5%, at least 99.9%, or at or at about 100% CD4+ T cells. In certain instances disclosed herein, the input composition of CD4+ T cells includes less than 40%, less than 35%, less than 30%, less than 25%, less than 20%, less than 15%, less than 10%, less than 5%, less than 1%, less than 0.1%, or less than 0.01% CD8+ T cells, and/or contains no CD8+ T cells, and/or is free or substantially free of CD8+ T cells. In some instances disclosed herein, the composition of enriched T cells consists essentially of CD4+ T cells.

In certain instances disclosed herein, the one or more compositions is or includes a composition of CD8+ T cells that is or includes at least 60%, at least 65%, at least 70%, at least 75%, at least 80%, at least 85%, at least 90%, at least 95%, at least 98%, at least 99%, at least 99.5%, at least 99.9%, or at or at about 100% CD8+ T cells. In certain instances disclosed herein, the composition of CD8+ T cells contains less than 40%, less than 35%, less than 30%, less than 25%, less than 20%, less than 15%, less than 10%, less than 5%, less than 1%, less than 0.1%, or less than 0.01% CD4+ T cells, and/or contains no CD4+ T cells, and/or is free of or substantially free of CD4+ T cells. In some instances disclosed herein, the composition of enriched T cells consists essentially of CD8+ T cells.

### B. Activation and Stimulation

In some instances disclosed herein, the cells are incubated and/or cultured prior to or in connection with genetic engineering. The incubation steps can include culture, cultivation, stimulation, activation, and/or propagation. The incubation and/or engineering may be carried out in a culture vessel, such as a unit, chamber, well, column, tube, tubing set, valve, vial, culture dish, bag, or other container for culture or cultivating cells. In some instances disclosed herein, the compositions or cells are incubated in the presence of stimulating conditions or a stimulatory agent. Such conditions include those designed to induce proliferation, expansion, activation, and/or survival of cells in the population, to mimic antigen exposure, and/or to prime the cells for genetic engineering, such as for the introduction of a recombinant antigen receptor.

The conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to activate the cells.

In some instances disclosed herein, the stimulating conditions or agents include one or more agent, e.g., ligand, which is capable of stimulating or activating an intracellular signaling domain of a TCR complex. In some disclosures, the agent turns on or initiates TCR/CD3 intracellular signaling cascade in a T cell. Such agents can include antibodies, such as those specific for a TCR, e.g. anti-CD3. In some instances disclosed herein, the stimulating conditions include one or more agent, e.g. ligand, which is capable of stimulating a costimulatory receptor, e.g., anti-CD28. In some instances disclosed herein, such agents and/or ligands may be, bound to solid support such as a bead, and/or one or more cytokines. Optionally, the expansion method may further comprise the step of adding anti-CD3 and/or anti CD28 antibody to the culture medium (e.g., at a concentration of at least about 0.5 ng/mL). In some instances disclosed herein, the stimulating agents include IL-2, IL-15 and/or IL-7. In some disclosures, the IL-2 concentration is at least about 10 units/mL.

In some disclosures, incubation is carried out in accordance with techniques such as those described in US Patent No. 6,040,177 to Riddell et al., Klebanoff et al.(2012) J Immunother. 35(9): 651-660, Terakura et al. (2012) Blood.1:72-82, and/or Wang et al. (2012) J Immunother. 35(9):689-701.

In some instances disclosed herein, the T cells are expanded by adding to a culture-initiating composition feeder cells, such as non-dividing peripheral blood mononuclear cells (PBMC), (e.g., such that the resulting population of cells contains at least about 5, 10, 20, or 40 or more PBMC feeder cells for each T lymphocyte in the initial population to be expanded); and incubating the culture (e.g. for a time sufficient to expand the numbers of T cells). In some disclosures, the non-dividing feeder cells can comprise gamma-irradiated PBMC feeder cells. In some instances disclosed herein, the PBMC are irradiated with gamma rays in the range of about 3000 to 3600 rads to prevent cell division. In some disclosures, the feeder cells are added to culture medium prior to the addition of the populations of T cells.

In some instances disclosed herein, the stimulating conditions include temperature suitable for the growth of human T lymphocytes, for example, at least about 25 degrees Celsius, generally at least about 30 degrees, and generally at or about 37 degrees Celsius. Optionally, the incubation may further comprise adding non-dividing EBV-transformed lymphoblastoid cells (LCL) as feeder cells. LCL can be irradiated with gamma rays in the range of about 6000 to 10,000 rads. The LCL feeder cells in some disclosures is provided in any suitable amount, such as a ratio of LCL feeder cells to initial T lymphocytes of at least about 10:1.

In instances disclosed herein, antigen-specific T cells, such as antigen-specific CD4⁺ and/or CD8⁺ T cells, are obtained by stimulating naive or antigen specific T lymphocytes with antigen. For example, antigen-specific T cell lines or clones can be generated to cytomegalovirus antigens by isolating T cells from infected subjects and stimulating the cells in vitro with the same antigen.

In some instances disclosed herein, at least a portion of the incubation in the presence of one or more stimulating conditions or a stimulatory agents is carried out in the internal cavity of a centrifugal chamber, for example, under centrifugal rotation, such as described in International Publication Number WO2016/073602. In some instances disclosed herein, at least a portion of the incubation performed in a centrifugal chamber includes mixing with a reagent or reagents to induce stimulation and/or activation. In some instances disclosed herein, cells, such as selected cells, are mixed with a stimulating condition or stimulatory agent in the centrifugal chamber. In some disclosures of such processes, a volume of cells is mixed with an amount of one or more stimulating conditions or agents that is far less than is normally employed when performing similar stimulations in a cell culture plate or other system.

In some instances disclosed herein, the stimulating agent is added to cells in the cavity of the chamber in an amount that is substantially less than (e.g. is no more than 5%, 10%, 20%, 30%, 40%, 50%, 60%, 70% or 80% of the amount) as compared to the amount of the stimulating agent that is typically used or would be necessary to achieve about the same or similar efficiency of selection of the same number of cells or the same volume of cells when selection is performed without mixing in a centrifugal chamber, e.g. in a tube or bag with periodic shaking or rotation. In some instances disclosed herein, the incubation is performed with the addition of an incubation buffer to the cells and stimulating agent to achieve a target volume with incubation of the reagent of, for example, 10 mL to 200 mL, such as at least or about at least or about or 10 mL, 20 mL, 30 mL, 40 mL, 50 mL, 60 mL, 70 mL, 80 mL, 90 mL, 100 mL, 150 mL or 200 mL. In some instances disclosed herein, the incubation buffer and stimulating agent are pre-mixed before addition to the cells. In some instances disclosed herein, the incubation buffer and stimulating agent are separately added to the cells. In some instances disclosed herein, the stimulating incubation is carried out with periodic gentle mixing condition, which can aid in promoting energetically favored interactions and thereby permit the use of less overall stimulating agent while achieving stimulating and activation of cells.

In some instances disclosed herein, the incubation generally is carried out under mixing conditions, such as in the presence of spinning, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm), such as at an RCF at the sample or wall of the chamber or other container of from or from about 80g to 100g (e.g. at or about or at least 80 g, 85 g, 90 g, 95 g, or 100 g). In some instances disclosed herein, the spin is carried out using repeated intervals of a spin at such low speed followed by a rest period, such as a spin and/or rest for 1, 2, 3, 4, 5, 6, 7, 8, 9, or 10 seconds, such as a spin at approximately 1 or 2 seconds followed by a rest for approximately 5, 6, 7, or 8 seconds.

In some instances disclosed herein, the total duration of the incubation, e.g. with the stimulating agent, is between or between about 1 hour and 96 hours, 1 hour and 72 hours, 1 hour and 48 hours, 4 hours and 36 hours, 8 hours and 30 hours or 12 hours and 24 hours, such as at least or about at least 6 hours, 12 hours, 18 hours, 24 hours, 36 hours or 72 hours. In some instances disclosed herein, the further incubation is for a time between or about between 1 hour and 48 hours, 4 hours and 36 hours, 8 hours and 30 hours or 12 hours and 24 hours, inclusive.

In particular instances disclosed herein, the stimulating conditions include incubating, culturing, and/or cultivating a composition of enriched T cells with and/or in the presence of one or more cytokines. In particular instances disclosed herein, the one or more cytokines are recombinant cytokines. In some instances disclosed herein, the one or more cytokines are human recombinant cytokines. In certain instances disclosed herein, the one or more cytokines bind to and/or are capable of binding to receptors that are expressed by and/or are endogenous to T cells. In particular instances disclosed herein, the one or more cytokines is or includes a member of the 4-alpha-helix bundle family of cytokines. In some instances disclosed herein, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF).

In some instances disclosed herein, the stimulation results in activation and/or proliferation of the cells, for example, prior to transduction.

### C. Vectors and Methods for Genetic Engineering

In some instances disclosed herein, engineered cells, such as T cells, used in connection with the disclosed methods, uses, articles of manufacture or compositions are cells have been genetically engineered to express a recombinant receptor, e.g., a CAR or a TCR described herein. In some instances disclosed herein, the cells are engineered by introduction, delivery or transfer of nucleic acid sequences that encode the recombinant receptor and/or other molecules.

In some instances disclosed herein, methods for producing engineered cells includes the introduction of a polynucleotide encoding a recombinant receptor (e.g. anti-CD 19 CAR) into a cell, e.g., such as a stimulated or activated cell. In particular instances disclosed herein, the recombinant proteins are recombinant receptors, such as any described. Introduction of the nucleic acid molecules encoding the recombinant protein, such as recombinant receptor, in the cell may be carried out using any of a number of known vectors. Such vectors include viral and non-viral systems, including lentiviral and gammaretroviral systems, as well as transposon-based systems such as PiggyBac or Sleeping Beauty-based gene transfer systems. Exemplary methods include those for transfer of nucleic acids encoding the receptors, including via viral, e.g., retroviral or lentiviral, transduction, transposons, and electroporation. In some instances disclosed herein, the engineering produces one or more engineered compositions of enriched T cells.

In certain instances disclosed herein, the one or more compositions of stimulated T cells are or include two separate stimulated compositions of enriched T cells. In particular instances disclosed herein, two separate compositions of enriched T cells, e.g., two separate compositions of enriched T cells that have been selected, isolated, and/or enriched from the same biological sample, are separately engineered. In certain instances disclosed herein, the two separate compositions include a composition of enriched CD4+ T cells. In particular instances disclosed herein, the two separate compositions include a composition of enriched CD8+ T cells. In some instances disclosed herein, two separate compositions of enriched CD4+ T cells and enriched CD8+ T cells are genetically engineered separately.

In some instances disclosed herein, gene transfer is accomplished by first stimulating the cell, such as by combining it with a stimulus that induces a response such as proliferation, survival, and/or activation, e.g., as measured by expression of a cytokine or activation marker, followed by transduction of the activated cells, and expansion in culture to numbers sufficient for clinical applications. In certain instances disclosed herein, the gene transfer is accomplished by first incubating the cells under stimulating conditions, such as by any of the methods described.

In some instances disclosed herein, methods for genetic engineering are carried out by contacting one or more cells of a composition with a nucleic acid molecule encoding the recombinant protein, e.g. recombinant receptor. In some instances disclosed herein, the contacting can be effected with centrifugation, such as spinoculation (e.g. centrifugal inoculation). Such methods include any of those as described in International Publication Number WO2016/073602. Exemplary centrifugal chambers include those produced and sold by Biosafe SA, including those for use with the Sepax^{®} and Sepax^{®} 2 system, including an A-200/F and A-200 centrifugal chambers and various kits for use with such systems. Exemplary chambers, systems, and processing instrumentation and cabinets are described, for example, in US Patent No. 6,123,655, US Patent No. 6,733,433 and Published U.S. Patent Application, Publication No.: US 2008/0171951, and published international patent application, publication no. WO 00/38762. Exemplary kits for use with such systems include, but are not limited to, single-use kits sold by BioSafe SA under product names CS-430.1, CS-490.1, CS-600.1 or CS-900.2.

In some instances disclosed herein, the contacting can be effected with centrifugation, such as spinoculation (e.g., centrifugal inoculation). In some instances disclosed herein, the composition containing cells, viral particles and reagent can be rotated, generally at relatively low force or speed, such as speed lower than that used to pellet the cells, such as from or from about 600 rpm to 1700 rpm (e.g., at or about or at least 600 rpm, 1000 rpm, or 1500 rpm or 1700 rpm). In some instances disclosed herein, the rotation is carried at a force, e.g., a relative centrifugal force, of from or from about 100 g to 3200 g (e.g., at or about or at least at or about 100 g, 200 g, 300 g, 400 g, 500 g, 1000 g, 1500 g, 2000 g, 2500 g, 3000 g or 3200 g), as measured for example at an internal or external wall of the chamber or cavity. The term "relative centrifugal force" or RCF is generally understood to be the effective force imparted on an object or substance (such as a cell, sample, or pellet and/or a point in the chamber or other container being rotated), relative to the earth's gravitational force, at a particular point in space as compared to the axis of rotation. The value may be determined using well-known formulas, taking into account the gravitational force, rotation speed and the radius of rotation (distance from the axis of rotation and the object, substance, or particle at which RCF is being measured).

In some instances disclosed herein, the introducing is carried out by contacting one or more cells of a composition with a nucleic acid molecule encoding the recombinant protein, e.g. recombinant receptor. In some instances disclosed herein, the contacting can be effected with centrifugation, such as spinoculation (e.g. centrifugal inoculation). Such methods include any of those as described in International Publication Number WO2016/073602. Exemplary centrifugal chambers include those produced and sold by Biosafe SA, including those for use with the Sepax^{®} and Sepax^{®} 2 system, including an A-200/F and A-200 centrifugal chambers and various kits for use with such systems. Exemplary chambers, systems, and processing instrumentation and cabinets are described, for example, in US Patent No. 6,123,655, US Patent No. 6,733,433 and Published U.S. Patent Application, Publication No.: US 2008/0171951, and published international patent application, publication no. WO 00/38762. Exemplary kits for use with such systems include, but are not limited to, single-use kits sold by BioSafe SA under product names CS-430.1, CS-490.1, CS-600.1 or CS-900.2.

In some instances disclosed herein, the system is included with and/or placed into association with other instrumentation, including instrumentation to operate, automate, control and/or monitor aspects of the transduction step and one or more various other processing steps performed in the system, e.g. one or more processing steps that can be carried out with or in connection with the centrifugal chamber system as described herein or in International Publication Number WO2016/073602. This instrumentation in some instances disclosed herein is contained within a cabinet. In some instances disclosed herein, the instrumentation includes a cabinet, which includes a housing containing control circuitry, a centrifuge, a cover, motors, pumps, sensors, displays, and a user interface. An exemplary device is described in US Patent No. 6,123,655, US Patent No. 6,733,433 and US 2008/0171951.

In some instances disclosed herein, the system comprises a series of containers, e.g., bags, tubing, stopcocks, clamps, connectors, and a centrifuge chamber. In some instances disclosed herein, the containers, such as bags, include one or more containers, such as bags, containing the cells to be transduced and the viral vector particles, in the same container or separate containers, such as the same bag or separate bags. In some instances disclosed herein, the system further includes one or more containers, such as bags, containing medium, such as diluent and/or wash solution, which is pulled into the chamber and/or other components to dilute, resuspend, and/or wash components and/or compositions during the methods. The containers can be connected at one or more positions in the system, such as at a position corresponding to an input line, diluent line, wash line, waste line and/or output line.

In some instances disclosed herein, the chamber is associated with a centrifuge, which is capable of effecting rotation of the chamber, such as around its axis of rotation. Rotation may occur before, during, and/or after the incubation in connection with transduction of the cells and/or in one or more of the other processing steps. Thus, in some instances disclosed herein, one or more of the various processing steps is carried out under rotation, e.g., at a particular force. The chamber is typically capable of vertical or generally vertical rotation, such that the chamber sits vertically during centrifugation and the side wall and axis are vertical or generally vertical, with the end wall(s) horizontal or generally horizontal.

In some instances disclosed herein, the composition containing cells, the vector, e.g., viral particles, and reagent can be rotated, generally at relatively low force or speed, such as speed lower than that used to pellet the cells, such as from or from about 600 rpm to 1700 rpm (e.g. at or about or at least 600 rpm, 1000 rpm, or 1500 rpm or 1700 rpm). In some instances disclosed herein, the rotation is carried at a force, e.g., a relative centrifugal force, of from or from about 100 g to 3200 g (e.g. at or about or at least at or about 100 g, 200 g, 300 g, 400 g, 500 g, 1000 g, 1500 g, 2000 g, 2500 g, 3000 g or 3200 g), as measured for example at an internal or external wall of the chamber or cavity. The term "relative centrifugal force" or RCF is generally understood to be the effective force imparted on an object or substance (such as a cell, sample, or pellet and/or a point in the chamber or other container being rotated), relative to the earth's gravitational force, at a particular point in space as compared to the axis of rotation. The value may be determined using well-known formulas, taking into account the gravitational force, rotation speed and the radius of rotation (distance from the axis of rotation and the object, substance, or particle at which RCF is being measured).

In some instances disclosed herein, during at least a part of the genetic engineering, e.g. transduction, and/or subsequent to the genetic engineering the cells are transferred to a bioreactor bag assembly for culture of the genetically engineered cells, such as for cultivation or expansion of the cells.

In some instances disclosed herein, recombinant nucleic acids are transferred into cells using recombinant infectious virus particles, such as, e.g., vectors derived from simian virus 40 (SV40), adenoviruses, adeno-associated virus (AAV). In some instances disclosed herein, recombinant nucleic acids are transferred into T cells using recombinant lentiviral vectors or retroviral vectors, such as gamma-retroviral vectors (see, e.g., Koste et al. (2014) Gene Therapy 2014 Apr 3. doi: 10.1038/gt.2014.25; Carlens et al. (2000) Exp Hematol 28(10): 1137-46; Alonso-Camino et al. (2013) Mol Ther Nucl Acids 2, e93; Park et al., Trends Biotechnol. 2011 November 29(11): 550-557.

In some instances disclosed herein, the retroviral vector has a long terminal repeat sequence (LTR), e.g., a retroviral vector derived from the Moloney murine leukemia virus (MoMLV), myeloproliferative sarcoma virus (MPSV), murine embryonic stem cell virus (MESV), murine stem cell virus (MSCV) or spleen focus forming virus (SFFV). Most retroviral vectors are derived from murine retroviruses. In some instances disclosed herein, the retroviruses include those derived from any avian or mammalian cell source. The retroviruses typically are amphotropic, meaning that they are capable of infecting host cells of several species, including humans. In one instance disclosed herein, the gene to be expressed replaces the retroviral gag, pol and/or env sequences. A number of illustrative retroviral systems have been described (e.g., U.S. Pat. Nos. 5,219,740; 6,207,453; 5,219,740; Miller and Rosman (1989) BioTechniques 7:980-990; Miller, A. D. (1990) Human Gene Therapy 1:5-14; Scarpa et al. (1991) Virology 180:849-852; Burns et al. (1993) Proc. Natl. Acad. Sci. USA 90:8033-8037; and Boris-Lawrie and Temin (1993) Cur. Opin. Genet. Develop. 3:102-109.

Methods of lentiviral transduction are known. Exemplary methods are described in, e.g., Wang et al. (2012) J. Immunother. 35(9): 689-701; Cooper et al. (2003) Blood. 101:1637-1644; Verhoeyen et al. (2009) Methods Mol Biol. 506: 97-114; and Cavalieri et al. (2003) Blood. 102(2): 497-505.

In some instances disclosed herein, the viral vector particles contain a genome derived from a retroviral genome based vector, such as derived from a lentiviral genome based vector. In some disclosures of the disclosed viral vectors, the heterologous nucleic acid encoding a recombinant receptor, such as an antigen receptor, such as a CAR, is contained and/or located between the 5' LTR and 3' LTR sequences of the vector genome.

In some instances disclosed herein, the viral vector genome is a lentivirus genome, such as an HIV-1 genome or an SIV genome. For example, lentiviral vectors have been generated by multiply attenuating virulence genes, for example, the genes env, vif, vpu and nef can be deleted, making the vector safer for therapeutic purposes. Lentiviral vectors are known. See Naldini et al., (1996 and 1998); Zufferey et al., (1997); Dull et al., 1998, U.S. Pat. Nos. 6,013,516; and 5,994,136). In some instances disclosed herein, these viral vectors are plasmid-based or virus-based, and are configured to carry the essential sequences for incorporating foreign nucleic acid, for selection, and for transfer of the nucleic acid into a host cell. Known lentiviruses can be readily obtained from depositories or collections such as the American Type Culture Collection ("ATCC"; 10801 University Blvd., Manassas, Va. 20110-2209), or isolated from known sources using commonly available techniques.

Non-limiting examples of lentiviral vectors include those derived from a lentivirus, such as Human Immunodeficiency Virus 1 (HIV-1), HIV-2, an Simian Immunodeficiency Virus (SIV), Human T-lymphotropic virus 1 (HTLV-1), HTLV-2 or equine infection anemia virus (E1AV). For example, lentiviral vectors have been generated by multiply attenuating the HIV virulence genes, for example, the genes env, vif, vpr, vpu and nef are deleted, making the vector safer for therapeutic purposes. Lentiviral vectors are known in the art, see Naldini et al., (1996 and 1998); Zufferey et al., (1997); Dull et al., 1998, U.S. Pat. Nos. 6,013,516; and 5,994,136). In some instances disclosed herein, these viral vectors are plasmid-based or virus-based, and are configured to carry the essential sequences for incorporating foreign nucleic acid, for selection, and for transfer of the nucleic acid into a host cell. Known lentiviruses can be readily obtained from depositories or collections such as the American Type Culture Collection ("ATCC"; 10801 University Blvd., Manassas, Va. 20110-2209), or isolated from known sources using commonly available techniques.

In some instances disclosed herein, the viral genome vector can contain sequences of the 5' and 3' LTRs of a retrovirus, such as a lentivirus. In some disclosures, the viral genome construct may contain sequences from the 5' and 3' LTRs of a lentivirus, and in particular can contain the R and U5 sequences from the 5' LTR of a lentivirus and an inactivated or self-inactivating 3' LTR from a lentivirus. The LTR sequences can be LTR sequences from any lentivirus from any species. For example, they may be LTR sequences from HIV, SIV, FIV or BIV. Typically, the LTR sequences are HIV LTR sequences.

In some instances disclosed herein, the nucleic acid of a viral vector, such as an HIV viral vector, lacks additional transcriptional units. The vector genome can contain an inactivated or self-inactivating 3' LTR (Zufferey et al. J Virol 72: 9873, 1998; Miyoshi et al., J Virol 72:8150, 1998). For example, deletion in the U3 region of the 3' LTR of the nucleic acid used to produce the viral vector RNA can be used to generate self-inactivating (SIN) vectors. This deletion can then be transferred to the 5' LTR of the proviral DNA during reverse transcription. A self-inactivating vector generally has a deletion of the enhancer and promoter sequences from the 3' long terminal repeat (LTR), which is copied over into the 5' LTR during vector integration. In some instances disclosed herein enough sequence can be eliminated, including the removal of a TATA box, to abolish the transcriptional activity of the LTR. This can prevent production of full-length vector RNA in transduced cells. In some disclosures, the U3 element of the 3' LTR contains a deletion of its enhancer sequence, the TATA box, Sp1, and NF-kappa B sites. As a result of the self-inactivating 3' LTR, the provirus that is generated following entry and reverse transcription contains an inactivated 5' LTR. This can improve safety by reducing the risk of mobilization of the vector genome and the influence of the LTR on nearby cellular promoters. The self-inactivating 3' LTR can be constructed by any method known in the art. In some instances disclosed herein, this does not affect vector titers or the in vitro or in vivo properties of the vector.

Optionally, the U3 sequence from the lentiviral 5' LTR can be replaced with a promoter sequence in the viral construct, such as a heterologous promoter sequence. This can increase the titer of virus recovered from the packaging cell line. An enhancer sequence can also be included. Any enhancer/promoter combination that increases expression of the viral RNA genome in the packaging cell line may be used. In one one instance disclosed herein, the CMV enhancer/promoter sequence is used (U.S. Pat. No. 5,385,839 and U.S. Pat. No. 5,168,062).

In certain instances disclosed herein, the risk of insertional mutagenesis can be minimized by constructing the retroviral vector genome, such as lentiviral vector genome, to be integration defective. A variety of approaches can be pursued to produce a non-integrating vector genome. In some instances disclosed herein, a mutation(s) can be engineered into the integrase enzyme component of the pol gene, such that it encodes a protein with an inactive integrase. In some instances disclosed herein, the vector genome itself can be modified to prevent integration by, for example, mutating or deleting one or both attachment sites, or making the 3' LTR-proximal polypurine tract (PPT) non-functional through deletion or modification. In some instances disclosed herein, non-genetic approaches are available; these include pharmacological agents that inhibit one or more functions of integrase. The approaches are not mutually exclusive; that is, more than one of them can be used at a time. For example, both the integrase and attachment sites can be non-functional, or the integrase and PPT site can be non-functional, or the attachment sites and PPT site can be non-functional, or all of them can be non-functional. Such methods and viral vector genomes are known and available (see Philpott and Thrasher, Human Gene Therapy 18:483, 2007; Engelman et al. J Virol 69:2729, 1995; Brown et al J Virol 73:9011 (1999); WO 2009/076524; McWilliams et al., J Virol 77:11150, 2003; Powell and Levin J Virol 70:5288, 1996).

In some instances disclosed herein, the vector contains sequences for propagation in a host cell, such as a prokaryotic host cell. In some instances disclosed herein, the nucleic acid of the viral vector contains one or more origins of replication for propagation in a prokaryotic cell, such as a bacterial cell. In some instances disclosed herein, vectors that include a prokaryotic origin of replication also may contain a gene whose expression confers a detectable or selectable marker such as drug resistance.

The viral vector genome is typically constructed in a plasmid form that can be transfected into a packaging or producer cell line. Any of a variety of known methods can be used to produce retroviral particles whose genome contains an RNA copy of the viral vector genome. In some instances disclosed herein, at least two components are involved in making a virus-based gene delivery system: first, packaging plasmids, encompassing the structural proteins as well as the enzymes necessary to generate a viral vector particle, and second, the viral vector itself, i.e., the genetic material to be transferred. Biosafety safeguards can be introduced in the design of one or both of these components.

In some instances disclosed herein, the packaging plasmid can contain all retroviral, such as HIV-1, proteins other than envelope proteins (Naldini et al., 1998). In other instances disclosed herein, viral vectors can lack additional viral genes, such as those that are associated with virulence, e.g., vpr, vif, vpu and nef, and/or Tat, a primary transactivator of HIV. In some instances disclosed herein, lentiviral vectors, such as HIV-based lentiviral vectors, comprise only three genes of the parental virus: gag, pol and rev, which reduces or eliminates the possibility of reconstitution of a wild-type virus through recombination.

In some instances disclosed herein, the viral vector genome is introduced into a packaging cell line that contains all the components necessary to package viral genomic RNA, transcribed from the viral vector genome, into viral particles. Alternatively, the viral vector genome may comprise one or more genes encoding viral components in addition to the one or more sequences, e.g., recombinant nucleic acids, of interest. In some disclosures, in order to prevent replication of the genome in the target cell, however, endogenous viral genes required for replication are removed and provided separately in the packaging cell line.

In some instances disclosed herein, a packaging cell line is transfected with one or more plasmid vectors containing the components necessary to generate the particles. In some instances disclosed herein, a packaging cell line is transfected with a plasmid containing the viral vector genome, including the LTRs, the cis-acting packaging sequence and the sequence of interest, i.e. a nucleic acid encoding an antigen receptor, such as a CAR; and one or more helper plasmids encoding the virus enzymatic and/or structural components, such as Gag, pol and/or rev. In some instances disclosed herein, multiple vectors are utilized to separate the various genetic components that generate the retroviral vector particles. In some such instances disclosed herein, providing separate vectors to the packaging cell reduces the chance of recombination events that might otherwise generate replication competent viruses. In some instances disclosed herein, a single plasmid vector having all of the retroviral components can be used.

In some instances disclosed herein, the retroviral vector particle, such as lentiviral vector particle, is pseudotyped to increase the transduction efficiency of host cells. For example, a retroviral vector particle, such as a lentiviral vector particle, in some instances disclosed herein is pseudotyped with a VSV-G glycoprotein, which provides a broad cell host range extending the cell types that can be transduced. In some instances disclosed herein, a packaging cell line is transfected with a plasmid or polynucleotide encoding a non-native envelope glycoprotein, such as to include xenotropic, polytropic or amphotropic envelopes, such as Sindbis virus envelope, GALV or VSV-G.

In some instances disclosed herein, the packaging cell line provides the components, including viral regulatory and structural proteins, that are required in trans for the packaging of the viral genomic RNA into lentiviral vector particles. In some instances disclosed herein, the packaging cell line may be any cell line that is capable of expressing lentiviral proteins and producing functional lentiviral vector particles. In some disclosures, suitable packaging cell lines include 293 (ATCC CCL X), 293T, HeLA (ATCC CCL 2), D17 (ATCC CCL 183), MDCK (ATCC CCL 34), BHK (ATCC CCL-10) and Cf2Th (ATCC CRL 1430) cells.

In some instances disclosed herein, the packaging cell line stably expresses the viral protein(s). For example, in some disclosures, a packaging cell line containing the gag, pol, rev and/or other structural genes but without the LTR and packaging components can be constructed. In some instances disclosed herein, a packaging cell line can be transiently transfected with nucleic acid molecules encoding one or more viral proteins along with the viral vector genome containing a nucleic acid molecule encoding a heterologous protein, and/or a nucleic acid encoding an envelope glycoprotein.

In some instances disclosed herein, the viral vectors and the packaging and/or helper plasmids are introduced via transfection or infection into the packaging cell line. The packaging cell line produces viral vector particles that contain the viral vector genome. Methods for transfection or infection are well known. Non-limiting examples include calcium phosphate, DEAE-dextran and lipofection methods, electroporation and microinjection.

When a recombinant plasmid and the retroviral LTR and packaging sequences are introduced into a special cell line (e.g., by calcium phosphate precipitation for example), the packaging sequences may permit the RNA transcript of the recombinant plasmid to be packaged into viral particles, which then may be secreted into the culture media. The media containing the recombinant retroviruses in some instances disclosed herein is then collected, optionally concentrated, and used for gene transfer. For example, in some disclosures, after cotransfection of the packaging plasmids and the transfer vector to the packaging cell line, the viral vector particles are recovered from the culture media and titered by standard methods used by those of skill in the art.

In some instances disclosed herein, a retroviral vector, such as a lentiviral vector, can be produced in a packaging cell line, such as an exemplary HEK 293T cell line, by introduction of plasmids to allow generation of lentiviral particles. In some instances disclosed herein, a packaging cell is transfected and/or contains a polynucleotide encoding gag and pol, and a polynucleotide encoding a recombinant receptor, such as an antigen receptor, for example, a CAR. In some instances disclosed herein, the packaging cell line is optionally and/or additionally transfected with and/or contains a polynucleotide encoding a rev protein. In some instances disclosed herein, the packaging cell line is optionally and/or additionally transfected with and/or contains a polynucleotide encoding a non-native envelope glycoprotein, such as VSV-G. In some such instances disclosed herein, approximately two days after transfection of cells, e.g., HEK 293T cells, the cell supernatant contains recombinant lentiviral vectors, which can be recovered and titered.

Recovered and/or produced retroviral vector particles can be used to transduce target cells using the methods as described. Once in the target cells, the viral RNA is reverse-transcribed, imported into the nucleus and stably integrated into the host genome. One or two days after the integration of the viral RNA, the expression of the recombinant protein, e.g., antigen receptor, such as CAR, can be detected.

In some instances disclosed herein, the disclosed methods involve methods of transducing cells by contacting, e.g., incubating, a cell composition comprising a plurality of cells with a viral particle. In some instances disclosed herein, the cells to be transfected or transduced are or comprise primary cells obtained from a subject, such as cells enriched and/or selected from a subject.

In some instances disclosed herein, the concentration of cells to be transduced of the composition is from or from about 1.0 × 10⁵ cells/mL to 1.0 × 10⁸ cells/mL, such as at least or about at least or about 1.0 × 10⁵ cells/mL, 5 × 10⁵ cells/mL, 1 × 10⁶ cells/mL, 5 × 10⁶ cells/mL, 1 × 10⁷ cells/mL, 5 × 10⁷ cells/mL or 1 × 10⁸ cells/mL.

In some instances disclosed herein, the viral particles are provided at a certain ratio of copies of the viral vector particles or infectious units (IU) thereof, per total number of cells to be transduced (lU/cell). For example, in some instances disclosed herein, the viral particles are present during the contacting at or about or at least at or about 0.5, 1, 2, 3, 4, 5, 10, 15, 20, 30, 40, 50, or 60 IU of the viral vector particles per one of the cells.

In some instances disclosed herein, the titer of viral vector particles is between or between about 1x 10⁶ IU/mL and 1 × 10⁸ IU/mL, such as between or between about 5 × 10⁶ IU/mL and 5 × 10⁷ IU/mL, such as at least 6 × 10⁶ IU/mL, 7 × 10⁶ IU/mL, 8 × 10⁶ IU/mL, 9 × 10⁶ IU/mL, 1 × 10⁷ IU/mL, 2 × 10⁷ IU/mL, 3 × 10⁷ IU/mL, 4 × 10⁷ IU/mL, or 5 ×10⁷ IU/mL.

In some instances disclosed herein, transduction can be achieved at a multiplicity of infection (MOI) of less than 100, such as generally less than 60, 50, 40, 30, 20, 10, 5 or less.

In some instances disclosed herein, the method involves contacting or incubating, the cells with the viral particles. In some instances disclosed herein, the contacting is for 30 minutes to 72 hours, such as 30 minute to 48 hours, 30 minutes to 24 hours or 1 hour to 24 hours, such as at least or about at least 30 minutes, 1 hour, 2 hours, 6 hours, 12 hours, 24 hours, 36 hours or more.

In some instances disclosed herein, contacting is performed in solution. In some instances disclosed herein, the cells and viral particles are contacted in a volume of from or from about 0.5 mL to 500 mL, such as from or from about 0.5 mL to 200 mL, 0.5 mL to 100 mL, 0.5 mL to 50 mL, 0.5 mL to 10 mL, 0.5 mL to 5 mL, 5 mL to 500 mL, 5 mL to 200 mL, 5 mL to 100 mL, 5 mL to 50 mL, 5 mL to 10 mL, 10 mL to 500 mL, 10 mL to 200 mL, 10 mL to 100 mL, 10 mL to 50 mL, 50 mL to 500 mL, 50 mL to 200 mL, 50 mL to 100 mL, 100 mL to 500 mL, 100 mL to 200 mL or 200 mL to 500 mL.

In certain instances disclosed herein, the input cells are treated, incubated, or contacted with particles that comprise binding molecules that bind to or recognize the recombinant receptor that is encoded by the viral DNA.

In some instances disclosed herein, the incubation of the cells with the viral vector particles results in or produces an output composition comprising cells transduced with the viral vector particles.

In some instances disclosed herein, recombinant nucleic acids are transferred into T cells via electroporation (see, e.g., Chicaybam et al, (2013) PLoS ONE 8(3): e60298 and Van Tedeloo et al. (2000) Gene Therapy 7(16): 1431-1437). In some instances disclosed herein, recombinant nucleic acids are transferred into T cells via transposition (see, e.g., Manuri et al. (2010) Hum Gene Ther 21(4): 427-437; Sharma et al. (2013) Molec Ther Nucl Acids 2, e74; and Huang et al. (2009) Methods Mol Biol 506: 115-126). Other methods of introducing and expressing genetic material in immune cells include calcium phosphate transfection (e.g., as described in Current Protocols in Molecular Biology, John Wiley & Sons, New York. N.Y.), protoplast fusion, cationic liposome-mediated transfection; tungsten particle-facilitated microparticle bombardment (Johnston, Nature, 346: 776-777 (1990)); and strontium phosphate DNA coprecipitation (Brash et al., Mol. Cell Biol., 7: 2031-2034 (1987)).

Other approaches and vectors for transfer of the nucleic acids encoding the recombinant products are those described, e.g., in international patent application, Publication No.: WO2014055668, and U.S. Patent No. 7,446,190.

In some instances disclosed herein, the cells, e.g., T cells, may be transfected either during or after expansion e.g. with a T cell receptor (TCR) or a chimeric antigen receptor (CAR). This transfection for the introduction of the gene of the desired receptor can be carried out with any suitable retroviral vector, for example. The genetically modified cell population can then be liberated from the initial stimulus (the anti-CD3/anti-CD28 stimulus, for example) and subsequently be stimulated with a second type of stimulus e.g. via a de novo introduced receptor). This second type of stimulus may include an antigenic stimulus in form of a peptide/MHC molecule, the cognate (cross-linking) ligand of the genetically introduced receptor (e.g. natural ligand of a CAR) or any ligand (such as an antibody) that directly binds within the framework of the new receptor (e.g. by recognizing constant regions within the receptor). See, for example, Cheadle et al, "Chimeric antigen receptors for T-cell based therapy" Methods Mol Biol. 2012; 907:645-66 or Barrett et al., Chimeric Antigen Receptor Therapy for Cancer Annual Review of Medicine Vol. 65: 333-347 (2014).

In some instances disclosed herein, a vector may be used that does not require that the cells, e.g., T cells, are activated. In some such instances, the cells may be selected and/or transduced prior to activation. Thus, the cells may be engineered prior to, or subsequent to culturing of the cells, and in some instances disclosed herein at the same time as or during at least a portion of the culturing.

Among additional nucleic acids, e.g., genes for introduction are those to improve the efficacy of therapy, such as by promoting viability and/or function of transferred cells; genes to provide a genetic marker for selection and/or evaluation of the cells, such as to assess *in vivo* survival or localization; genes to improve safety, for example, by making the cell susceptible to negative selection in vivo as described by Lupton S. D. et al., Mol. and Cell Biol., 11:6 (1991); and Riddell et al., Human Gene Therapy 3:319-338 (1992); see also the publications of PCT/US91/08442 and PCT/US94/05601 by Lupton et al. describing the use of bifunctional selectable fusion genes derived from fusing a dominant positive selectable marker with a negative selectable marker. *See,* e.g., Riddell et al., US Patent No. 6,040,177, at columns 14-17.

### D. Cultivation, Expansion and Formulation of Engineered Cells

In some instances disclosed herein, the methods for generating the engineered cells, e.g., for cell therapy in accord with any of disclosed methods, uses, articles of manufacture or compositions, include one or more steps for cultivating cells, e.g., cultivating cells under conditions that promote proliferation and/or expansion. In some instances disclosed herein, cells are cultivated under conditions that promote proliferation and/or expansion subsequent to a step of genetically engineering, e.g., introducing a recombinant polypeptide to the cells by transduction or transfection. In particular instances disclosed herein, the cells are cultivated after the cells have been incubated under stimulating conditions and transduced or transfected with a recombinant polynucleotide, e.g., a polynucleotide encoding a recombinant receptor. Thus, in some instances disclosed herein, a composition of CAR-positive T cells that has been engineered by transduction or transfection with a recombinant polynucleotide encoding the CAR, is cultivated under conditions that promote proliferation and/or expansion.

In certain instances disclosed herein, the one or more compositions of engineered T cells are or include two separate compositions of enriched T cells, such as two separate compositions of enriched T cells that have been engineered with a polynucleotide encoding a recombinant receptor, e.g. a CAR. In particular instances disclosed herein, two separate compositions of enriched T cells, e.g., two separate compositions of enriched T cells selected, isolated, and/or enriched from the same biological sample, are separately cultivated under stimulating conditions, such as subsequent to a step of genetically engineering, e.g., introducing a recombinant polypeptide to the cells by transduction or transfection. In certain instances disclosed herein, the two separate compositions include a composition of enriched CD4+ T cells, such as a composition of enriched CD4+ T cells that have been engineered with a polynucleotide encoding a recombinant receptor, e.g. a CAR. In particular instances disclosed herein, the two separate compositions include a composition of enriched CD8+ T cells, such as a composition of enriched CD4+ T cells that have been engineered with a polynucleotide encoding a recombinant receptor, e.g. a CAR. In some instances disclosed herein, two separate compositions of enriched CD4+ T cells and enriched CD8+ T cells, such as a composition of enriched CD4+ T cells and a composition of enriched CD8+ T cells that have each been separately engineered with a polynucleotide encoding a recombinant receptor, e.g. a CAR, are separately cultivated, e.g., under conditions that promote proliferation and/or expansion.

In some instances disclosed herein, cultivation is carried out under conditions that promote proliferation and/or expansion. In some instances disclosed herein, such conditions may be designed to induce proliferation, expansion, activation, and/or survival of cells in the population. In particular instances disclosed herein, the stimulating conditions can include one or more of particular media, temperature, oxygen content, carbon dioxide content, time, agents, e.g., nutrients, amino acids, antibiotics, ions, and/or stimulatory factors, such as cytokines, chemokines, antigens, binding partners, fusion proteins, recombinant soluble receptors, and any other agents designed to promote growth, division, and/or expansion of the cells.

In particular instances disclosed herein, the cells are cultivated in the presence of one or more cytokines. In particular instances disclosed herein, the one or more cytokines are recombinant cytokines. In some instances disclosed herein, the one or more cytokines are human recombinant cytokines. In certain instances disclosed herein, the one or more cytokines bind to and/or are capable of binding to receptors that are expressed by and/or are endogenous to T cells. In particular instances disclosed herein, the one or more cytokines, e.g. a recombinant cytokine, is or includes a member of the 4-alpha-helix bundle family of cytokines. In some instances disclosed herein, members of the 4-alpha-helix bundle family of cytokines include, but are not limited to, interleukin-2 (IL-2), interleukin-4 (IL-4), interleukin-7 (IL-7), interleukin-9 (IL-9), interleukin 12 (IL-12), interleukin 15 (IL-15), granulocyte colony-stimulating factor (G-CSF), and granulocyte-macrophage colony-stimulating factor (GM-CSF). In some instances disclosed herein, the one or more recombinant cytokine includes IL-2, IL-7 and/or IL-15. In some instances disclosed herein, the cells, e.g., engineered cells, are cultivated in the presence of a cytokine, e.g., a recombinant human cytokine, at a concentration of between 1 IU/mL and 2,000 IU/mL, between 10 IU/mL and 100 IU/mL, between 50 IU/mL and 200 IU/mL, between 100 IU/mL and 500 IU/mL, between 100 IU/mL and 1,000 IU/mL, between 500 IU/mL and 2,000 IU/mL, or between 100 IU/mL and 1,500 IU/mL.

In some instances disclosed herein, the cultivation is performed under conditions that generally include a temperature suitable for the growth of primary immune cells, such as human T lymphocytes, for example, at least about 25 degrees Celsius, generally at least about 30 degrees, and generally at or about 37 degrees Celsius. In some instances disclosed herein, the composition of enriched T cells is incubated at a temperature of 25 to 38°C, such as 30 to 37°C, for example at or about 37 °C ± 2 °C. In some instances disclosed herein, the incubation is carried out for a time period until the culture, e.g. cultivation or expansion, results in a desired or threshold density, number or dose of cells. In some instances disclosed herein, the incubation is greater than or greater than about or is for about or 24 hours, 48 hours, 72 hours, 96 hours, 5 days, 6 days, 7 days, 8 days, 9 days or more.

In particular instances disclosed herein, the cultivation is performed in a closed system. In certain instances disclosed herein, the cultivation is performed in a closed system under sterile conditions. In particular instances disclosed herein, the cultivation is performed in the same closed system as one or more steps of the disclosed systems. In some instances disclosed herein the composition of enriched T cells is removed from a closed system and placed in and/or connected to a bioreactor for the cultivation. Examples of suitable bioreactors for the cultivation include, but are not limited to, GE Xuri W25, GE Xuri W5, Sartorius BioSTAT RM 20 | 50, Finesse SmartRocker Bioreactor Systems, and Pall XRS Bioreactor Systems. In some instances disclosed herein, the bioreactor is used to perfuse and/or mix the cells during at least a portion of the cultivation step.

In some instances disclosed herein, the mixing is or includes rocking and/or motioning. In some instances disclosed herein, the bioreactor can be subject to motioning or rocking, which, in some disclosures, can increase oxygen transfer. Motioning the bioreactor may include, but is not limited to rotating along a horizontal axis, rotating along a vertical axis, a rocking motion along a tilted or inclined horizontal axis of the bioreactor or any combination thereof. In some instances disclosed herein, at least a portion of the incubation is carried out with rocking. The rocking speed and rocking angle may be adjusted to achieve a desired agitation. In some instances disclosed herein the rock angle is 20°, 19°, 18°, 17°, 16°, 15°, 14°, 13°, 12°, 11°, 10°, 9°, 8°, 7°, 6°, 5°, 4°, 3°, 2° or 1°. In certain instances disclosed herein, the rock angle is between 6-16°. In other instances disclosed herein, the rock angle is between 7-16°. In other instances disclosed herein, the rock angle is between 8-12°. In some instances disclosed herein, the rock rate is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 rpm. In some instances disclosed herein, the rock rate is between 4 and 12 rpm, such as between 4 and 6 rpm, inclusive.

In some instances disclosed herein, the bioreactor maintains the temperature at or near 37°C and CO2 levels at or near 5% with a steady air flow at, at about, or at least 0.01 L/min, 0.05 L/min, 0.1 L/min, 0.2 L/min, 0.3 L/min, 0.4 L/min, 0.5 L/min, 1.0 L/min, 1.5 L/min, or 2.0 L/min or greater than 2.0 L/min. In certain instances disclosed herein, at least a portion of the cultivation is performed with perfusion, such as with a rate of 290 ml/day, 580 ml/day, and/or 1160 ml/day, e.g., depending on the timing in relation to the start of the cultivation and/or density of the cultivated cells. In some instances disclosed herein, at least a portion of the cell culture expansion is performed with a rocking motion, such as at an angle of between 5° and 10°, such as 6°, at a constant rocking speed, such as a speed of between 5 and 15 RPM, such as 6 RMP or 10 RPM.

In some instances disclosed herein, the methods for manufacturing, generating or producing a cell therapy and/or engineered cells, in accord with the disclosed methods, uses or articles of manufacture, may include formulation of cells, such as formulation of genetically engineered cells resulting from the processing steps prior to or after the incubating, engineering, and cultivating, and/or one or more other processing steps as described. In some instances disclosed herein, one or more of the processing steps, including formulation of cells, can be carried out in a closed system. In some instances disclosed herein, the cells are processed in one or more steps (e.g. carried out in the centrifugal chamber and/or closed system) for manufacturing, generating or producing a cell therapy and/or engineered cells may include formulation of cells, such as formulation of genetically engineered cells resulting from the transduction processing steps prior to or after the culturing, e.g. cultivation and expansion, and/or one or more other processing steps as described. In some instances disclosed herein, the genetically engineered cells are formulated as unit dose form compositions including the number of cells for administration in a given dose or fraction thereof.

In some instances disclosed herein, the dose of cells comprising cells engineered with a recombinant antigen receptor, e.g. CAR or TCR, is provided as a composition or formulation, such as a pharmaceutical composition or formulation. Such compositions can be used in accord with the disclosed methods, such as in the treatment of diseases, conditions, and disorders, or in detection, diagnostic, and prognostic methods, and uses and articles of manufacture. In some instances disclosed herein, the cells can be formulated in an amount for dosage administration, such as for a single unit dosage administration or multiple dosage administration.

In some instances disclosed herein, the cells can be formulated into a container, such as a bag or vial. In some instances disclosed herein, the vial may be an infusion vial. In some instances disclosed herein, the vial is formulated with a single unit dose of the engineered cells, such as including the number of cells for administration in a given dose or fraction thereof.

In some instances disclosed herein, the cells are formulated in a pharmaceutically acceptable buffer, which may, in some disclosures, include a pharmaceutically acceptable carrier or excipient. In some instances disclosed herein, the processing includes exchange of a medium into a medium or formulation buffer that is pharmaceutically acceptable or desired for administration to a subject. In some instances disclosed herein, the processing steps can involve washing the transduced and/or expanded cells to replace the cells in a pharmaceutically acceptable buffer that can include one or more optional pharmaceutically acceptable carriers or excipients. Exemplary of such pharmaceutical forms, including pharmaceutically acceptable carriers or excipients, can be any described below in conjunction with forms acceptable for administering the cells and compositions to a subject. The pharmaceutical composition in some instances disclosed herein contains the cells in amounts effective to treat or prevent the disease or condition, such as a therapeutically effective or prophylactically effective amount.

In some instances disclosed herein, the formulation buffer contains a cryopreservative. In some instances disclosed herein, the cell are formulated with a cyropreservative solution that contains 1.0% to 30% DMSO solution, such as a 5% to 20% DMSO solution or a 5% to 10% DMSO solution. In some instances disclosed herein, the cryopreservation solution is or contains, for example, PBS containing 20% DMSO and 8% human serum albumin (HSA), or other suitable cell freezing media. In some instances disclosed herein, the cryopreservative solution is or contains, for example, at least or about 7.5% DMSO. In some instances disclosed herein, the processing steps can involve washing the transduced and/or expanded cells to replace the cells in a cryopreservative solution. In some instances disclosed herein, the cells are frozen, e.g., cryoprotected or cryopreserved, in media and/or solution with a final concentration of or of about 12.5%, 12.0%, 11.5%, 11.0%, 10.5%, 10.0%, 9.5%, 9.0%, 8.5%, 8.0%, 7.5%, 7.0%, 6.5%, 6.0%, 5.5%, or 5.0% DMSO, or between 1% and 15%, between 6% and 12%, between 5% and 10%, or between 6% and 8% DMSO. In particular instances disclosed herein, the cells are frozen, e.g., cryoprotected or cryopreserved, in media and/or solution with a final concentration of or of about 5.0%, 4.5%, 4.0%, 3.5%, 3.0%, 2.5%, 2.0%, 1.5%, 1.25%, 1.0%, 0.75%, 0.5%, or 0.25% HSA, or between 0.1% and 5%, between 0.25% and 4%, between 0.5% and 2%, or between 1% and 2% HSA.

In some instances disclosed herein, the formulation is carried out using one or more processing step including washing, diluting or concentrating the cells, such as the cultured or expanded cells. In some instances disclosed herein, the processing can include dilution or concentration of the cells to a desired concentration or number, such as unit dose form compositions including the number of cells for administration in a given dose or fraction thereof. In some instances disclosed herein, the processing steps can include a volume-reduction to thereby increase the concentration of cells as desired. In some instances disclosed herein, the processing steps can include a volume-addition to thereby decrease the concentration of cells as desired. In some instances disclosed herein, the processing includes adding a volume of a formulation buffer to transduced and/or expanded cells. In some instances disclosed herein, the volume of formulation buffer is from or from about 10 mL to 1000 mL, such as at least or at least about or about or 50 mL, 100 mL, 200 mL, 300 mL, 400 mL, 500 mL, 600 mL, 700 mL, 800 mL, 900 mL or 1000 mL.

In some instances disclosed herein, such processing steps for formulating a cell composition is carried out in a closed system. Exemplary of such processing steps can be performed using a centrifugal chamber in conjunction with one or more systems or kits associated with a cell processing system, such as a centrifugal chamber produced and sold by Biosafe SA, including those for use with the Sepax^{®} or Sepax 2^{®} cell processing systems. An exemplary system and process is described in International Publication Number WO2016/073602. In some instances disclosed herein, the method includes effecting expression from the internal cavity of the centrifugal chamber a formulated composition, which is the resulting composition of cells formulated in a formulation buffer, such as pharmaceutically acceptable buffer, in any of the above instances disclosed herein as described. In some instances disclosed herein, the expression of the formulated composition is to a container, such as the vials of the biomedical material vessels described herein, that is operably linked as part of a closed system with the centrifugal chamber. In some instances disclosed herein, the biomedical material vessels are configured for integration and or operable connection and/or is integrated or operably connected, to a closed system or device that carries out one or more processing steps. In some instances disclosed herein, the biomedical material vessel is connected to a system at an output line or output position. In some instances disclosed herein, the closed system is connected to the vial of the biomedical material vessel at the inlet tube. Exemplary close systems for use with the biomedical material vessels described herein include the Sepax^{®} and Sepax^{®} 2 system.

In some instances disclosed herein, the closed system, such as associated with a centrifugal chamber or cell processing system, includes a multi-port output kit containing a multi-way tubing manifold associated at each end of a tubing line with a port to which one or a plurality of containers can be connected for expression of the formulated composition. In some disclosures, a desired number or plurality of vials, can be sterilely connected to one or more, generally two or more, such as at least 3, 4, 5, 6, 7, 8 or more of the ports of the multi-port output. For example, in some instances disclosed herein, one or more containers, e.g., biomedical material vessels, can be attached to the ports, or to fewer than all of the ports. Thus, in some instances disclosed herein, the system can effect expression of the output composition into a plurality of vials of the biomedical material vessels.

In some disclosures, cells can be expressed to the one or more of the plurality of output containers, e.g., vials, in an amount for dosage administration, such as for a single unit dosage administration or multiple dosage administration. For example, in some instances disclosed herein, the vials, may each contain the number of cells for administration in a given dose or fraction thereof. Thus, each vial, in some disclosures, may contain a single unit dose for administration or may contain a fraction of a desired dose such that more than one of the plurality of vials, such as two of the vials, or 3 of the vials, together constitute a dose for administration. In some instances disclosed herein, 4 vials together constitute a dose for administration.

Thus, the containers, e.g. bags or vials, generally contain the cells to be administered, e.g., one or more unit doses thereof. The unit dose may be an amount or number of the cells to be administered to the subject or twice the number (or more) of the cells to be administered. It may be the lowest dose or lowest possible dose of the cells that would be administered to the subject. In some disclosures, the disclosed articles of manufacture includes one or more of the plurality of output containers.

In some instances disclosed herein, each of the containers, e.g. bags or vials, individually comprises a unit dose of the cells. Thus in some instances disclosed herein, each of the containers comprises the same or approximately or substantially the same number of cells. In some instances disclosed herein, each unit dose contains at or about or at least or at least about 1 × 10⁶, 2 × 10⁶, 5 × 10⁶, 1 × 10⁷, 5 × 10⁷, or 1 × 10⁸ engineered cells, total cells, T cells, or PBMCs. In some instances disclosed herein, each unit dose contains at or about or at least or at least about 1 × 10⁶, 2 × 10⁶, 5 × 10⁶, 1 × 10⁷, 5 × 10⁷, or 1 × 10⁸ CAR+ T cells that are CD3+, such as CD4+ or CD8+, or a viable subset thereof. In some instances disclosed herein, the volume of the formulated cell composition in each container, e.g. bag or vial, is between at or about 10 mL and at or about 100 mL, such as at or about or at least or at least about 20 mL, 30 mL, 40 mL, 50 mL, 60 mL, 70 mL, 80 mL, 90 mL or 100 mL. In some instances disclosed herein, the volume of the formulated cell composition in each container, e.g. bag or vial, is between at or about 1 mL and at or about 10 mL, such as between at or about 1 mL and at or about 5 mL. In some instances disclosed herein, the volume of the formulated cell composition in each container, e.g. bag or vial, is between at or about 4 mL and at or about 5 mL. In some instances disclosed herein, the volume of the formulated cell composition in each container, e.g. bag or vial, is or is about 4.4 mL. In some instances disclosed herein, the volume of the formulated cell composition in each container, e.g. bag or vial, is or is about 4.5 mL. In some instances disclosed herein, the volume of the formulated cell composition in each container, e.g. bag or vial, is or is about 4.6 mL. In some instances disclosed herein, the volume of the formulated cell composition in each container, e.g. bag or vial, is or is about 4.7 mL. In some instances disclosed herein, the volume of the formulated cell composition in each container, e.g. bag or vial, is or is about 4.8 mL. In some instances disclosed herein, the volume of the formulated cell composition in each container, e.g. bag or vial, is or is about 4.9 mL. In some instances disclosed herein, the volume of the formulated cell composition in each container, e.g. bag or vial, is or is about 5.0 mL.

In some instances disclosed herein, the formulated cell composition has a concentration of greater than at or about 0.5 × 10⁶ recombinant receptor-expressing (e.g. CAR⁺)/CD3+ cells or such viable cells per mL, greater than at or about 1.0 × 10⁶ recombinant receptor-expressing (e.g. CAR⁺)/CD3+ cells or such viable cells per mL, greater than at or about 1.5 × 10⁶ recombinant receptor-expressing (e.g. CAR⁺)/CD3+ cells or such viable cells per mL, greater than at or about 2.0 × 10⁶ recombinant receptor-expressing (e.g. CAR⁺)/CD3+ cells or such viable cells per mL. greater than at or about 2.5 × 10⁶ recombinant receptor-expressing (e.g. CAR⁺)/CD3+ cells or such viable cells per mL, greater than at or about 2.6 × 10⁶ recombinant receptor-expressing (e.g. CAR⁺)/CD3+ cells or such viable cells per mL, greater than at or about 2.7 × 10⁶ recombinant receptor-expressing (e.g. CAR⁺)/CD3+ cells or such viable cells per mL, greater than at or about 2.8 × 10⁶ recombinant receptor-expressing (e.g. CAR⁺)/CD3+ cells or such viable cells per mL, greater than at or about 2.9 × 10⁶ recombinant receptor-expressing (e.g. CAR⁺)/CD3+ cells or such viable cells per mL greater than at or about 3.0 × 10⁶ recombinant receptor-expressing (e.g. CAR⁺)/CD3+ cells or such viable cells per mL, greater than at or about 3.5 × 10⁶ recombinant receptor-expressing (e.g. CAR⁺)/CD3+ cells or such viable cells per mL, greater than at or about 4.0 × 10⁶ recombinant receptor-expressing (e.g. CAR⁺)/CD3+ cells or such viable cells per mL, greater than at or about 4.5 × 10⁶ recombinant receptor-expressing (e.g. CAR⁺)/CD3+ cells or such viable cells per mL or greater than at or about 5 × 10⁶ recombinant receptor-expressing (e.g. CAR⁺)/CD3+ cells or such viable cells per mL. In some instances disclosed herein, the CD3+ cells are CD4+ T cells. In some instances disclosed herein, the CD3+ cells are CD8+ T cells. In some instances disclosed herein, the CD3+ T cells are CD4+ and CD8+ T cells.

In some instances disclosed herein, the cells in the container, e.g. bag or vials, can be cryopreserved. In some instances disclosed herein, the container, e.g. vials, can be stored in liquid nitrogen until further use.

In some instances disclosed herein, such cells produced by the method, or a composition comprising such cells, are administered to a subject for treating a disease or condition, for example, in accord with the methods, uses and articles of manufacture described herein.

### V. COMPOSITIONS AND FORMULATIONS

In some instances disclosed herein, the dose of cells comprising cells engineered with a recombinant antigen receptor, e.g. CAR or TCR, is provided as a composition or formulation, such as a pharmaceutical composition or formulation. Exemplary compositions and formulations are described above, including those produced in connection with methods of engineering the cells. Such compositions can be used in accord with the disclosed methods or uses, and/or with the disclosed articles of manufacture or compositions, such as in the prevention or treatment of diseases, conditions, and disorders, or in detection, diagnostic, and prognostic methods.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

In some disclosures, the choice of carrier is determined in part by the particular cell or agent and/or by the method of administration. Accordingly, there are a variety of suitable formulations. For example, the pharmaceutical composition can contain preservatives. Suitable preservatives may include, for example, methylparaben, propylparaben, sodium benzoate, and benzalkonium chloride. In some disclosures, a mixture of two or more preservatives is used. The preservative or mixtures thereof are typically present in an amount of about 0.0001% to about 2% by weight of the total composition. Carriers are described, e.g., by Remington's Pharmaceutical Sciences 16th edition, Osol, A. Ed. (1980). Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG).

Buffering agents in some disclosures are included in the compositions. Suitable buffering agents include, for example, citric acid, sodium citrate, phosphoric acid, potassium phosphate, and various other acids and salts. In some disclosures, a mixture of two or more buffering agents is used. The buffering agent or mixtures thereof are typically present in an amount of about 0.001% to about 4% by weight of the total composition. Methods for preparing administrable pharmaceutical compositions are known. Exemplary methods are described in more detail in, for example, Remington: The Science and Practice of Pharmacy, Lippincott Williams & Wilkins; 21st ed. (May 1, 2005).

The formulation or composition may also contain more than one active ingredient useful for the particular indication, disease, or condition being prevented or treated with the cells or agents, where the respective activities do not adversely affect one another. Such active ingredients are suitably present in combination in amounts that are effective for the purpose intended. Thus, in some instances disclosed herein, the pharmaceutical composition further includes other pharmaceutically active agents or drugs, such as chemotherapeutic agents, e.g., asparaginase, busulfan, carboplatin, cisplatin, daunorubicin, doxorubicin, fluorouracil, gemcitabine, hydroxyurea, methotrexate, paclitaxel, rituximab, vinblastine, vincristine, etc. In some instances disclosed herein, the agents or cells are administered in the form of a salt, e.g., a pharmaceutically acceptable salt. Suitable pharmaceutically acceptable acid addition salts include those derived from mineral acids, such as hydrochloric, hydrobromic, phosphoric, metaphosphoric, nitric, and sulphuric acids, and organic acids, such as tartaric, acetic, citric, malic, lactic, fumaric, benzoic, glycolic, gluconic, succinic, and arylsulphonic acids, for example, p-toluenesulphonic acid.

The pharmaceutical composition in some instances disclosed herein contains agents or cells in amounts effective to treat or prevent the disease or condition, such as a therapeutically effective or prophylactically effective amount. Therapeutic or prophylactic efficacy in some instances disclosed herein is monitored by periodic assessment of treated subjects. For repeated administrations over several days or longer, depending on the condition, the treatment is repeated until a desired suppression of disease symptoms occurs. However, other dosage regimens may be useful and can be determined. The desired dosage can be delivered by a single bolus administration of the composition, by multiple bolus administrations of the composition, or by continuous infusion administration of the composition.

The agents or cells can be administered by any suitable means, for example, by bolus infusion, by injection, e.g., intravenous or subcutaneous injections, intraocular injection, periocular injection, subretinal injection, intravitreal injection, trans-septal injection, subscleral injection, intrachoroidal injection, intracameral injection, subconjectval injection, subconjuntival injection, sub-Tenon's injection, retrobulbar injection, peribulbar injection, or posterior juxtascleral delivery. In some instances disclosed herein, they are administered by parenteral, intrapulmonary, and intranasal, and, if desired for local treatment, intralesional administration. Parenteral infusions include intramuscular, intravenous, intraarterial, intraperitoneal, or subcutaneous administration. In some instances disclosed herein, a given dose is administered by a single bolus administration of the cells or agent. In some instances disclosed herein, it is administered by multiple bolus administrations of the cells or agent, for example, over a period of no more than 3 days, or by continuous infusion administration of the cells or agent.

For the prevention or treatment of disease, the appropriate dosage may depend on the type of disease to be treated, the type of agent or agents, the type of cells or recombinant receptors, the severity and course of the disease, whether the agent or cells are administered for preventive or therapeutic purposes, previous therapy, the subject's clinical history and response to the agent or the cells, and the discretion of the attending physician. The compositions are in some instances disclosed herein suitably administered to the subject at one time or over a series of treatments.

The cells or agents may be administered using standard administration techniques, formulations, and/or devices. Disclosed are formulations and devices, such as syringes and vials, for storage and administration of the compositions. With respect to cells, administration can be autologous or heterologous. For example, immunoresponsive cells or progenitors can be obtained from one subject, and administered to the same subject or a different, compatible subject. Peripheral blood derived immunoresponsive cells or their progeny (e.g., in vivo, ex vivo or in vitro derived) can be administered via localized injection, including catheter administration, systemic injection, localized injection, intravenous injection, or parenteral administration. When administering a therapeutic composition (e.g., a pharmaceutical composition containing a genetically modified immunoresponsive cell or an agent that treats or ameliorates symptoms of neurotoxicity), it will generally be formulated in a unit dosage injectable form (solution, suspension, emulsion).

Formulations include those for oral, intravenous, intraperitoneal, subcutaneous, pulmonary, transdermal, intramuscular, intranasal, buccal, sublingual, or suppository administration. In some instances disclosed herein, the agent or cell populations are administered parenterally. The term "parenteral," as used herein, includes intravenous, intramuscular, subcutaneous, rectal, vaginal, and intraperitoneal administration. In some instances disclosed herein, the agent or cell populations are administered to a subject using peripheral systemic delivery by intravenous, intraperitoneal, or subcutaneous injection.

Compositions in some instances disclosed herein are provided as sterile liquid preparations, e.g., isotonic aqueous solutions, suspensions, emulsions, dispersions, or viscous compositions, which may in some disclosures be buffered to a selected pH. Liquid preparations are normally easier to prepare than gels, other viscous compositions, and solid compositions. Additionally, liquid compositions are somewhat more convenient to administer, especially by injection. Viscous compositions, on the other hand, can be formulated within the appropriate viscosity range to provide longer contact periods with specific tissues. Liquid or viscous compositions can comprise carriers, which can be a solvent or dispersing medium containing, for example, water, saline, phosphate buffered saline, polyol (for example, glycerol, propylene glycol, liquid polyethylene glycol) and suitable mixtures thereof.

Sterile injectable solutions can be prepared by incorporating the agent or cells in a solvent, such as in admixture with a suitable carrier, diluent, or excipient such as sterile water, physiological saline, glucose, dextrose, or the like.

The formulations to be used for in vivo administration are generally sterile. Sterility may be readily accomplished, e.g., by filtration through sterile filtration membranes.

### VI. ARTICLES OF MANUFACTURE AND KITS

Also disclosed are articles of manufacture and kits containing engineered cells expressing a recombinant receptor or compositions thereof, and optionally instructions for use, for example, instructions for administering, according to the disclosed methods.

In some instances disclosed herein are articles of manufacture and/or kits that include a composition comprising a therapeutically effective amount of any of the engineered cells described herein, and instructions for administering, to a subject for treating a disease or condition. In some instances disclosed herein, the instructions can specify some or all of the elements of the methods disclosed herein. In some instances disclosed herein, the instructions specify particular instructions for administration of the cells for cell therapy, e.g., doses, timing, selection and/or identification of subjects for administration and conditions for administration. In some instances disclosed herein, the articles of manufacture and/or kits further include one or more additional agents for therapy, e.g., lymphodepleting therapy and/or combination therapy, such as any described herein and optionally further includes instructions for administering the additional agent for therapy. In some instances disclosed herein, the articles of manufacture and/or kits further comprise an agent for lymphodepleting therapy, and optionally further includes instructions for administering the lymphodepleting therapy. In some instances disclosed herein, the instructions can be included as a label or package insert accompanying the compositions for administration.

In some instances disclosed herein, such criteria include subjects having relapsed/refractory CLL and/or high-risk CLL, or SLL. In some disclosures, the population to be treated includes, e.g., subjects having an Eastern Cooperative Oncology Group Performance Status (ECOG) that is anywhere from 0-1. In some instances disclosed herein, of any of the instances disclosed herein, the subjects to be treated have failed two or more prior therapies.

In some instances disclosed herein, the instructions specify the dose of cells to be administered. For example, in some instances disclosed herein, the dose specified in the instructions include a total recombinant receptor (e.g., CAR)-expressing cells, such as 2.5 × 10⁷, 5 × 10⁷, or 1 × 10⁸ total such cells.

In some instances disclosed herein, the article of manufacture or kit comprises a container, optionally a vial comprising a plurality of CD4⁺ T cells expressing a recombinant receptor (e.g. CAR), and a container, optionally a vial comprising a plurality of CD8⁺ T cells expressing a recombinant receptor (e.g. CAR). In some instances disclosed herein, the article of manufacture or kit comprises a container, optionally a vial comprising a plurality of CD4⁺ T cells expressing a recombinant receptor, and further comprises, in the same container, a plurality of CD8⁺ T cells expressing a recombinant receptor (e.g. CAR). In some instances disclosed herein, a cryoprotectant is included with the cells. In some disclosures the container is a bag. n some disclosures, the container is a vial.

In some instances disclosed herein, the container such as the vial comprises greater than at or about 0.5 × 10⁶ recombinant receptor-expressing (e.g. CAR⁺)/CD3+ cells or such viable cells per mL, greater than at or about 1.0 × 10⁶ recombinant receptor-expressing (e.g. CAR⁺)/CD3+ cells or such viable cells per mL, greater than at or about 1.5 × 10⁶ recombinant receptor-expressing (e.g. CAR⁺)/CD3+ cells or such viable cells per mL, greater than at or about 2.0 × 10⁶ recombinant receptor-expressing (e.g. CAR⁺)/CD3+ cells or such viable cells per mL. greater than at or about 2.5 × 10⁶ recombinant receptor-expressing (e.g. CAR⁺)/CD3+ cells or such viable cells per mL, greater than at or about 2.6 × 10⁶ recombinant receptor-expressing (e.g. CAR⁺)/CD3+ cells or such viable cells per mL, greater than at or about 2.7 × 10⁶ recombinant receptor-expressing (e.g. CAR⁺)/CD3+ cells or such viable cells per mL, greater than at or about 2.8 × 10⁶ recombinant receptor-expressing (e.g. CAR⁺)/CD3+ cells or such viable cells per mL, greater than at or about 2.9 × 10⁶ recombinant receptor-expressing (e.g. CAR⁺)/CD3+ cells or such viable cells per mL greater than at or about 3.0 × 10⁶ recombinant receptor-expressing (e.g. CAR⁺)/CD3+ cells or such viable cells per mL, greater than at or about 3.5 × 10⁶ recombinant receptor-expressing (e.g. CAR⁺)/CD3+ cells or such viable cells per mL, greater than at or about 4.0 × 10⁶ recombinant receptor-expressing (e.g. CAR⁺)/CD3+ cells or such viable cells per mL, greater than at or about 4.5 x 10⁶ recombinant receptor-expressing (e.g. CAR⁺)/CD3+ cells or such viable cells per mL or greater than at or about 5 × 10⁶ recombinant receptor-expressing (e.g. CAR⁺)/CD3+ cells or such viable cells per mL. In some instances disclosed herein, the CD3+ cells are CD4+ T cells. In some instances disclosed herein, the CD3+ cells are CD8+ T cells. In some instances disclosed herein, the CD3+ T cells are CD4+ and CD8+ T cells.

In some instances disclosed herein, the plurality of vials or plurality of cells or unit dose of cells specified for administration, collectively, comprises a dose of cells comprising from or from about 2.5 × 10⁷ to 5 × 10⁷ total recombinant receptor-expressing T cells or total T cells, or 5 × 10⁷ to 1 × 10⁸ total recombinant receptor-expressing T cells or total T cells. In some instances disclosed herein, the T cells are CD3+ cells. In some instances disclosed herein, the CD3+ cells are CD8+ T cells. In some instances disclosed herein, the CD3+ T cells are CD4+ and CD8+ T cells. In some instances disclosed herein, the plurality of vials or plurality of cells or unit dose of cells specified for administration include one or more unit doses of recombinant receptor (e.g. CAR)-expressing CD3+ CD4+ T cells and one or more unit doses of recombinant receptor (e.g. CAR)-expressing CD3+ CD8+ T cells. In some instances disclosed herein, the number of cells of each unit dose are viable cells.

In some disclosures, the article comprises one or more unit dose of the CD4⁺ and CD8⁺ cells or of the CD4⁺receptor⁺ (e.g. CAR+) cells and CD8⁺receptor⁺ (e.g. CAR+) cells, wherein the unit dose comprises between at or about 1 × 10⁷ and at or about 2 × 10⁸ recombinant receptor (e.g. CAR)-expressing T cells, between at or about 5 × 10⁷ and at or about 1.5 × 10⁸ recombinant receptor (e.g. CAR)-expressing T cells, at or about 5 × 10⁷ recombinant receptor (e.g. CAR)-expressing T cells, at or about 1× 10⁸ recombinant receptor (e.g. CAR)-expressing T cells, or at or about 1.5 × 10⁸ recombinant receptor (e.g. CAR)-expressing T cells, optionally wherein the information in the article specifies administration of one or of a plurality of unit doses and/or a volume corresponding to such one or plurality of unit doses. In some instances disclosed herein, the article comprises one or more unit doses of the CD8⁺ cells, wherein the dose comprises between at or about 5 × 10⁶ and at or about 1 × 10⁸ recombinant receptor (e.g. CAR)-expressing CD8⁺ T cells, the dose comprises between at or about 1 × 10⁷ and at or about 0.75 × 10⁸ recombinant receptor (e.g. CAR)-expressing CD8⁺ T cells, the dose comprises at or about 2.5 × 10⁷ recombinant receptor (e.g. CAR)-expressing CD8⁺ T cells, or the dose comprises at or about 5 × 10⁷ recombinant receptor (e.g.)-expressing CD8⁺ T cells, or the dose comprises at or about 0.75 × 10⁸ recombinant receptor (e.g. CAR)-expressing CD8⁺ T cells, optionally wherein the information in the article specifies administration of one or of a plurality of unit doses and/or a volume corresponding to such one or plurality of unit doses. In some instances disclosed herein, the article comprises one or more unit doses of the CD4⁺ cells, wherein the dose comprises between at or about 5 × 10⁶ and at or about 1 × 10⁸ recombinant receptor (e.g. CAR)-expressing CD4⁺ T cells, the dose comprises between at or about 1 × 10⁷ and at or about 0.75 × 10⁸ recombinant receptor (e.g. CAR)-expressing CD4⁺ T cells, the dose comprises at or about 2.5 × 10⁷ recombinant receptor (e.g. CAR)-expressing CD4⁺ T cells, or the dose comprises at or about 5 × 10⁷ recombinant receptor (e.g.)-expressing CD4⁺ T cells, or the dose comprises at or about 0.75 × 10⁸ recombinant receptor (e.g. CAR)-expressing CD4⁺ T cells, optionally wherein the information in the article specifies administration of one or of a plurality of unit doses and/or a volume corresponding to such one or plurality of unit doses. n some instances disclosed herein, the cells in the article, collectively, comprise a dose of cells comprising no more than at or about 1 × 10⁸ total recombinant receptor (e.g. CAR)-expressing T cells or total T cells or CD3+ cells, no more than at or about 1 × 10⁷ total recombinant receptor (e.g. CAR)-expressing T cells or total T cells or CD3+ cells, no more than at or about 0.5 × 10⁷ total recombinant receptor (e.g. CAR)-expressing T cells or total T cells or CD3+ cells, no more than at or about 1 × 10⁶ total recombinant receptor (e.g. CAR)-expressing T cells or total T cells or CD3+, no more than at or about 0.5 × 10⁶ total recombinant receptor (e.g. CAR)-expressing T cells or total T cells or CD3+ cells. In some instances disclosed herein, the number of cells of each unit dose are viable cells.

In some instances disclosed herein, each vial or the plurality of vials or plurality of cells or unit dose of cells specified for administration, collectively, comprises a flat dose of cells or fixed dose of cells such that the dose of cells is not tied to or based on the body surface area or weight of a subject.

In some instances disclosed herein, a unit dose of a cell is or comprises the number or amount of cells, such as engineered T cells, that can be administered to a subject or a patient in a single dose. In some instances disclosed herein, as unit dose is a fraction of the number of cells for administration in a given dose.

In some instances disclosed herein, the instructions for administration of a dose specify administering a number of cells comprising at least or at least about 2.5 × 10⁷ CD3+/CAR⁺, CD8⁺/CAR⁺, or CD4⁺/CD8⁺/CAR⁺ T cells, at least or at least about 5 × 10⁷ CD3+/CAR⁺, CD8⁺/CAR⁺, or CD4⁺/CD8⁺/CAR⁺ T cells, or at least or at least about 1 × 10⁸ CD3+/CAR⁺, CD8⁺/CAR⁺, or CD4⁺/CD8⁺/CAR⁺ T cells. In some instances disclosed herein, instructions for administration of a dose specify administering a number of cells comprising at or about 2.5 × 10⁷ CD3+/CAR⁺, CD8⁺/CAR⁺, or CD4⁺/CD8⁺/CAR⁺ T cells, at or about 5 × 10⁷ CD3+/CAR⁺, CD8⁺/CAR⁺, or CD4⁺/CD8⁺/CAR⁺ T cells, or at or about 1 × 10⁸ CD3+/CAR⁺, CD8⁺/CAR⁺, or CD4⁺/CD8⁺/CAR⁺ T cells. In some instances disclosed herein, the number of cells is the number of such cells that are viable cells.

In some instances disclosed herein, the article of manufacture or kit comprises a plurality of CD4⁺ T cells expressing a recombinant receptor, and instructions for administering, to a subject having a disease or condition, all or a portion of the plurality of CD4⁺ T cells and further administering CD8⁺ T cells expressing a recombinant receptor. In some instances disclosed herein, the instructions specify administering the CD4⁺ T cells prior to administering the CD8⁺ cells. In some instances disclosed herein, the instructions specify administering the CD8⁺ T cells prior to administering the CD4⁺ cells. In some instances disclosed herein, the article of manufacture or kit comprises a plurality of CD8⁺ T cells expressing a recombinant receptor, and instructions for administering, to a subject having a disease or condition, all or a portion of the plurality of CD8⁺ T cells and CD4⁺ T cells expressing a recombinant receptor. In some instances disclosed herein, the instructions specify dosage regimen and timing of the administration of the cells.

In some instances disclosed herein, instructions for administration of a dose specify administering a number of cells that is or is about 5 × 10⁷ CD3+ CAR+ viable cells, that includes a separate dose of at or about 2.5 × 10⁷ CD4+ CAR+ viable cells and at or about 2.5 × 10⁷ CD8+CAR+ viable cells. In some instances disclosed herein, instructions for administration of a dose specify administering a number of cells that is or is about 1 × 10⁸ CD3+CAR+ viable cells, that includes a separate dose of at or about 5 × 10⁷ CD4+CAR+ viable cells and at or about 5 ×10⁷ CD8+CAR+ viable cells. In some instances disclosed herein, instructions for administration of a dose specify administering a number of cells that is or is about 1.5 × 10⁸ CD3+CAR+ viable cells, that includes a separate dose of at or about 0.75 × 10⁸ CD4+CAR+ viable cells and at or about 0.75 ×10⁸ CD8+CAR+ viable cells.

In some disclosures, the instructions specify administering all or a portion of the CD4⁺ T cells and the all or a portion of the CD8⁺ T cells with 48 hours apart, such as no more than 36 hours apart, no more than 24 hours apart, no more than 12 hours, apart, such as 0 to 12 hours apart, 0 to 6 hours apart or 0 to 2 hours apart. In some instances disclosed herein, the instructions specify administering the CD4⁺ T cells and the CD8⁺ T cells no more than 2 hours, no more than 1 hour, no more than 30 minutes, no more than 15 minutes, no more than 10 minutes or no more than 5 minutes apart. In some instances disclosed herein, the instructions specify administering the CD8+ T cells prior to the CD4+ T cells.

In some instances disclosed herein, the articles of manufacture and/or kits further include one or more additional agents for therapy, e.g., lymphodepleting therapy, as described herein, and optionally instructions for administering the additional agents.

In some instances disclosed herein, the articles of manufacture and/or kits further include one or more agents or treatments for treating, preventing, delaying, reducing or attenuating the development or risk of development of a toxicity and/or instructions for the administration of one or more agents or treatments for treating, preventing, delaying, reducing or attenuating the development or risk of development of a toxicity in the subject. In some instances disclosed herein, the agent is or comprises an anti-IL-6 antibody or anti-IL-6 receptor antibody. For example, in some instances disclosed herein, the agent or treatment is or comprises an agent selected from among tocilizumab, siltuximab, clazakizumab, sarilumab, olokizumab (CDP6038), elsilimomab, ALD518/BMS-945429, sirukumab (CNTO 136), CPSI-2634, ARGX-109, FE301 and FM101. For example, in some instances disclosed herein, the agent or treatment is or comprises one or more of a steroid; an antagonist or inhibitor of a cytokine receptor or cytokine selected from among IL-10, IL-10R, IL-6, IL-6 receptor, IFNγ, IFNGR, IL-2, IL-2R/CD25, MCP-1, CCR2, CCR4, MIP1β, CCR5, TNFalpha, TNFR1, IL-1, and IL-1Ralpha/IL-1beta; or an agent capable of preventing, blocking or reducing microglial cell activity or function.

In some instances disclosed herein, the agent capable of preventing, blocking or reducing microglial cell activity or function is selected from an anti-inflammatory agent, an inhibitor of NADPH oxidase (NOX2), a calcium channel blocker, a sodium channel blocker, inhibits GM-CSF, inhibits CSF1R, specifically binds CSF-1, specifically binds IL-34, inhibits the activation of nuclear factor kappa B (NF-κB), activates a CB₂ receptor and/or is a CB₂ agonist, a phosphodiesterase inhibitor, inhibits microRNA-155 (miR-155) or upregulates microRNA-124 (miR-124). In some instances disclosed herein, the agent is selected from minocycline, naloxone, nimodipine, Riluzole, MOR103, lenalidomide, a cannabinoid (optionally WIN55 or 212-2), intravenous immunoglobulin (IVIg), ibudilast, anti-miR-155 locked nucleic acid (LNA), MCS110, PLX-3397, PLX647, PLX108-D1, PLX7486, JNJ-40346527, JNJ28312141, ARRY-382, AC-708, DCC-3014, 5-(3-methoxy-4-((4-methoxybenzyl)oxy)benzyl) pyrimidine-2,4-diamine (GW2580), AZD6495, Ki20227, BLZ945, emactuzumab, IMC-CS4, FPA008, LY-3022855, AMG-820 and TG-3003. In some instances disclosed herein, the agent is an inhibitor of colony stimulating factor 1 receptor (CSF1R). For example, the agent PLX-3397, PLX647, PLX108-D1, PLX7486, JNJ-40346527, JNJ28312141, ARRY-382, AC-708, DCC-3014, 5-(3-methoxy-4-((4-methoxybenzyl)oxy)benzyl)pyrimidine-2,4-diamine (GW2580), AZD6495, Ki20227, BLZ945 or a pharmaceutical salt or prodrug thereof; emactuzumab, IMC-CS4, FPA008, LY-3022855, AMG-820 and TG-3003 or is an antigen-binding fragment thereof, or a combination of any of the foregoing.

In some instances disclosed herein, the articles of manufacture and/or kits further include one or more reagents for assaying biological samples, e.g., biological samples from subjects who are candidates for administration or who have been administered the therapy, and optionally instructions for use of the reagents or assays. In some instances disclosed herein, the biological sample is or is obtained from a blood, plasma or serum sample In some instances disclosed herein, the reagents can be used prior to the administration of the cell therapy or after the administration of cell therapy, for diagnostic purposes, to identify subjects and/or to assess treatment outcomes and/or toxicities. For example, in some instances disclosed herein, the article of manufacture and/or kits further contain reagents for measuring the level of particular biomarkers, e.g., cytokines or analytes, that are associated with toxicity, and instructions for measuring. In some instances disclosed herein, the reagents include components for performing an *in vitro* assay to measure the biomarkers (e.g. analytes), such as an immunoassay, an aptamer-based assay, a histological or cytological assay, or an mRNA expression level assay. In some instances disclosed herein, the *in vitro* assay is selected from among an enzyme linked immunosorbent assay (ELISA), immunoblotting, immunoprecipitation, radioimmunoassay (RIA), immunostaining, flow cytometry assay, surface plasmon resonance (SPR), chemiluminescence assay, lateral flow immunoassay, inhibition assay and avidity assay. In some disclosures, the reagent is a binding reagent that specifically binds the biomarkers (e.g. analytes). In some instances disclosed herein, the binding reagent is an antibody or antigen-binding fragment thereof, an aptamer or a nucleic acid probe.

In some instances disclosed herein, the articles of manufacture and/or kits comprise one or more reagent capable of detecting one or more analytes, and instructions for using the reagent to assay a biological sample from a subject that is a candidate for treatment, wherein the one or more analytes can be TNF-alpha or IL-16. In some instances disclosed herein, instructions for assaying presence or absence, level, amount, or concentration of an analyte in the subject compared to a threshold level of the analyte is also included. In some instances disclosed herein, the instructions specify methods for carrying out assessment or monitoring of such analytes, e.g. TNF-alpha or IL-16, according to any of the disclosed methods.

In some instances disclosed herein, the instructions are included which specify, if the level, amount or concentration of the analyte in the sample is at or above a threshold level for the analyte, administering to the subject an agent or other treatment capable of treating, preventing, delaying, reducing or attenuating the development or risk of development of a toxicity (i) prior to, (ii) within one, two, or three days of, (iii) concurrently with and/or (iv) at first fever following, the initiation of administration of the cell therapy to the subject. In some instances disclosed herein, the instructions specify that if the level, amount or concentration of the analyte in the sample is at or above a threshold level for the analyte, the cell therapy is administered to the subject at a reduced dose or at a dose that is not associated with risk of developing toxicity or severe toxicity, or is not associated with a risk of developing a toxicity or severe toxicity in a majority of subjects, and/or a majority of subjects having a disease or condition that the subject has or is suspected of having, following administration of the cell therapy. In some instances disclosed herein, the instructions specify that if the level, amount or concentration of the analyte in the sample is at or above a threshold level for the analyte, the cell therapy is administered in an in-patient setting and/or with admission to the hospital for one or more days, optionally wherein the cell therapy is otherwise to be administered to subjects on an outpatient basis or without admission to the hospital for one or more days.

In some instances disclosed herein, the instructions for administering the cell therapy specify, if the level, amount or concentration of the analyte in the sample, is below a threshold level, administering to the subject the cell therapy, optionally at a non-reduced dose, optionally on an outpatient basis or without admission to the hospital for one or more days. In some instances disclosed herein, the instructions for administering the cell therapy specify, if the level, amount or concentration of the analyte in the sample, is below a threshold level, prior to or concurrently with administering the cell therapy and/or prior to the development of a sign or symptom of a toxicity other than fever, an agent or treatment capable of treating, preventing, delaying, or attenuating the development of the toxicity is not administered to the subject. In some disclosures, the instructions for administering the cell therapy specify that if the level, amount or concentration of the analyte in the sample, is below a threshold level, the administration of the cell therapy is to be or may be administered to the subject on an outpatient setting and/or without admission of the subject to the hospital overnight or for one or more consecutive days and/or is without admission of the subject to the hospital for one or more days.

The articles of manufacture and/or kits may further include a cell therapy and/or further include instructions for use with, prior to and/or in connection with treatment with the cell therapy. In some instances disclosed herein, the instructions are included for administering the agent and the instructions specify if the level, amount or concentration of the analyte in the sample, is at or above a threshold level administering to the subject the agent. In some disclosures, the instructions further specify administering a cell therapy to the subject, wherein administration of the agent is to be carried out (i) prior to, (ii) within one, two, or three days of, (iii) concurrently with and/or (iv) at first fever following, the initiation of administration of the cell therapy to the subject.

The articles of manufacture and/or kits may include a container and a label or package insert on or associated with the container. Suitable containers include, for example, bottles, vials, syringes, IV solution bags, etc. The containers may be formed from a variety of materials such as glass or plastic. The container in some instances disclosed herein holds a composition which is by itself or combined with another composition effective for treating, preventing and/or diagnosing the condition. In some instances disclosed herein, the container has a sterile access port. Exemplary containers include an intravenous solution bags, vials, including those with stoppers pierceable by a needle for injection, or bottles or vials for orally administered agents. The label or package insert may indicate that the composition is used for treating a disease or condition. The article of manufacture may include (a) a first container with a composition contained therein, wherein the composition includes engineered cells expressing a recombinant receptor; and (b) a second container with a composition contained therein, wherein the composition includes the second agent. In some instances disclosed herein, the article of manufacture may include (a) a first container with a first composition contained therein, wherein the composition includes a subtype of engineered cells expressing a recombinant receptor; and (b) a second container with a composition contained therein, wherein the composition includes a different subtype of engineered cells expressing a recombinant receptor. The article of manufacture may further include a package insert indicating that the compositions can be used to treat a particular condition. Alternatively, or additionally, the article of manufacture may further include another or the same container comprising a pharmaceutically-acceptable buffer. It may further include other materials such as other buffers, diluents, filters, needles, and/or syringes.

### VIII. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The terms "polypeptide" and "protein" are used interchangeably to refer to a polymer of amino acid residues, and are not limited to a minimum length. Polypeptides, including the provided receptors and other polypeptides, e.g., linkers or peptides, may include amino acid residues including natural and/or non-natural amino acid residues. The terms also include post-expression modifications of the polypeptide, for example, glycosylation, sialylation, acetylation, and phosphorylation. In some aspects, the polypeptides may contain modifications with respect to a native or natural sequence, as long as the protein maintains the desired activity. These modifications may be deliberate, as through site-directed mutagenesis, or may be accidental, such as through mutations of hosts which produce the proteins or errors due to PCR amplification.

As used herein, a "subject" is a mammal, such as a human or other animal, and typically is human. In some embodiments, the subject, e.g., patient, to whom the agent or agents, cells, cell populations, or compositions are administered, is a mammal, typically a primate, such as a human. In some embodiments, the primate is a monkey or an ape. The subject can be male or female and can be any suitable age, including infant, juvenile, adolescent, adult, and geriatric subjects. In some embodiments, the subject is a non-primate mammal, such as a rodent.

As used herein, "treatment" (and grammatical variations thereof such as "treat" or "treating") refers to complete or partial amelioration or reduction of a disease or condition or disorder, or a symptom, adverse effect or outcome, or phenotype associated therewith. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The terms do not imply complete curing of a disease or complete elimination of any symptom or effect(s) on all symptoms or outcomes.

As used herein, "delaying development of a disease" means to defer, hinder, slow, retard, stabilize, suppress and/or postpone development of the disease (such as cancer). This delay can be of varying lengths of time, depending on the history of the disease and/or individual being treated. In some embodiments, sufficient or significant delay can, in effect, encompass prevention, in that the individual does not develop the disease. For example, a late stage cancer, such as development of metastasis, may be delayed.

"Preventing," as used herein, includes providing prophylaxis with respect to the occurrence or recurrence of a disease in a subject that may be predisposed to the disease but has not yet been diagnosed with the disease. In some embodiments, the provided cells and compositions are used to delay development of a disease or to slow the progression of a disease.

As used herein, to "suppress" a function or activity is to reduce the function or activity when compared to otherwise same conditions except for a condition or parameter of interest, or alternatively, as compared to another condition. For example, cells that suppress tumor growth reduce the rate of growth of the tumor compared to the rate of growth of the tumor in the absence of the cells.

An "effective amount" of an agent, e.g., a pharmaceutical formulation, cells, or composition, in the context of administration, refers to an amount effective, at dosages/amounts and for periods of time necessary, to achieve a desired result, such as a therapeutic or prophylactic result.

A "therapeutically effective amount" of an agent, e.g., a pharmaceutical formulation or cells, refers to an amount effective, at dosages and for periods of time necessary, to achieve a desired therapeutic result, such as for treatment of a disease, condition, or disorder, and/or pharmacokinetic or pharmacodynamic effect of the treatment. The therapeutically effective amount may vary according to factors such as the disease state, age, sex, and weight of the subject, and the populations of cells administered. In some embodiments, the provided methods involve administering the cells and/or compositions at effective amounts, e.g., therapeutically effective amounts.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount. In the context of lower tumor burden, the prophylactically effective amount in some aspects will be higher than the therapeutically effective amount.

The term "about" as used herein refers to the usual error range for the respective value readily known to the skilled person in this technical field. Reference to "about" a value or parameter herein includes (and describes) embodiments that are directed to that value or parameter per se.

As used herein, the singular forms "a," "an," and "the" include plural referents unless the context clearly dictates otherwise. For example, "a" or "an" means "at least one" or "one or more."

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

As used herein, a composition refers to any mixture of two or more products, substances, or compounds, including cells. It may be a solution, a suspension, liquid, powder, a paste, aqueous, nonaqueous or any combination thereof.

As used herein, "enriching" when referring to one or more particular cell type or cell population, refers to increasing the number or percentage of the cell type or population, e.g., compared to the total number of cells in or volume of the composition, or relative to other cell types, such as by positive selection based on markers expressed by the population or cell, or by negative selection based on a marker not present on the cell population or cell to be depleted. The term does not require complete removal of other cells, cell type, or populations from the composition and does not require that the cells so enriched be present at or even near 100% in the enriched composition.

As used herein, a statement that a cell or population of cells is "positive" for a particular marker refers to the detectable presence on or in the cell of a particular marker, typically a surface marker. When referring to a surface marker, the term refers to the presence of surface expression as detected by flow cytometry, for example, by staining with an antibody that specifically binds to the marker and detecting said antibody, wherein the staining is detectable by flow cytometry at a level substantially above the staining detected carrying out the same procedure with an isotype-matched control or fluorescence minus one (FMO) gating control under otherwise identical conditions and/or at a level substantially similar to that for cell known to be positive for the marker, and/or at a level substantially higher than that for a cell known to be negative for the marker.

As used herein, a statement that a cell or population of cells is "negative" for a particular marker refers to the absence of substantial detectable presence on or in the cell of a particular marker, typically a surface marker. When referring to a surface marker, the term refers to the absence of surface expression as detected by flow cytometry, for example, by staining with an antibody that specifically binds to the marker and detecting said antibody, wherein the staining is not detected by flow cytometry at a level substantially above the staining detected carrying out the same procedure with an isotype-matched control or fluorescence minus one (FMO) gating control under otherwise identical conditions, and/or at a level substantially lower than that for cell known to be positive for the marker, and/or at a level substantially similar as compared to that for a cell known to be negative for the marker.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

Unless defined otherwise, all terms of art, notations and other technical and scientific terms or terminology used herein are intended to have the same meaning as is commonly understood to which the claimed subject matter pertains. In some cases, terms with commonly understood meanings are defined herein for clarity and/or for ready reference, and the inclusion of such definitions herein should not necessarily be construed to represent a substantial difference over what is generally understood.

The section headings used herein are for organizational purposes only and are not to be construed as limiting the subject matter described.

### IX. EXAMPLES

The following examples are included for illustrative purposes only and are not intended to limit the scope of the invention.

### Example 1: Administration of Anti-CD19 CAR-Expressing Cells to Subjects with Relapsed and Refractory Chronic Lymphocytic Leukemia (CLL) or small lymphocytic lymphoma (SLL)

Therapeutic CAR+ T cell compositions containing autologous T cells expressing a chimeric antigen-receptor (CAR) specific for CD19 were administered to human subjects with chronic lymphocytic leukemia (CLL) or small lymphocytic lymphoma (SLL).

Subjects were greater than or at least eighteen years old and had a diagnosis of CLL or SLL. CLL diagnosis was with indication of treatment based on the International Workshop on Chronic Lymphocytic Leukemia (iwCLL) guidelines and clinical measurable disease (bone marrow involvement by > 30% lymphocytes, peripheral blood lymphocytosis > 5×10⁹/L, and/or measurable lymph nodes and/or hepatic or splenomegaly. SLL diagnosis was based on lymphadenopathy and/or splenomegaly and < 5×10⁹ CD19+ CD5+ clonal B lymphocytes/L [< 5000/µL] in the peripheral blood at diagnosis with measurable disease defined as at least one lesion > 1.5 cm in the greatest transverse diameter, and that is biopsy-proven SLL.

The subjects were either ineligible for treatment with Bruton's tyrosine kinase inhibitor (BTKi, e.g., ibrutinib) due to a requirement for full-dose anticoagulation or history or arrhythmia, or had failed treatment after having been previously administered BTKi as determined by stable disease (SD) or progressive disease (PD) as best response, PD after previous response, or discontinuation due to intolerance (e.g. unmanageable toxicity). More specifically, subjects were eligible if they had high risk disease (as determined by complex cytogenetic abnormalities (e.g., complex karyotype), del(17p), TP53 mutation, unmutated IGVH) and had failed greater than or equal to (e.g., at least) 2 prior therapies; or if they had standard-risk disease and had failed greater than or equal to (e.g., at least) 3 prior therapies. Subjects with active untreated CNS disease, ECOG >1, or Richter's transformation were excluded.

### A. Treatment

Eligible subjects were administered the anti-CD19 therapeutic T cell compositions. The therapeutic T cell compositions administered had been generated by a process including immunoaffinity-based (e.g., immunomagnetic selection) enrichment of CD4⁺ and CD8+ cells from leukapheresis samples from the individual subjects to be treated. Isolated CD4+ and CD8+ T cells were separately activated and independently transduced with a viral vector (e.g., lentiviral vector) encoding an anti-CD 19 CAR, followed by separate expansion and cryopreservation of the engineered cell populations in a low-volume. The CAR contained an anti-CD 19 scFv derived from a murine antibody (variable region derived from FMC63, VL-linker-VH orientation), an immunoglobulin-derived spacer, a transmembrane domain derived from CD28, a costimulatory region derived from 4-1BB, and a CD3-zeta intracellular signaling domain. The viral vector further contained sequences encoding a truncated receptor, which served as a surrogate marker for CAR expression; separated from the CAR sequence by a T2A ribosome skip sequence.

The cryopreserved cell compositions were thawed prior to intravenous administration. The therapeutic T cell dose was administered as a defined cell composition by administering a formulated CD4+ CAR+ cell population and a formulated CD8+ CAR+ population administered at a target ratio of approximately 1:1.

Subjects were administered a single dose of CAR-expressing T cells (each single dose via separate infusions of CD4⁺ CAR-expressing T cells and CD8+ CAR-expressing T cells, respectively) as follows: a single dose of dose level 1 (DL-1) containing 5 × 10⁷ total CAR-expressing T cells or a single dose of dose level 2 (DL-2) containing 1 × 10⁸ total CAR-expressing T cells. If determined necessary, the dose level is decreased to 2.5×10⁷ CAR+ T cells. Dose escalation followed a modified toxicity probability-interval-2 (mTIPI-2) algorithm. Dose-limiting toxicities (DLTs) were evaluated for twenty-eight days post administration.

Prior to CAR+ T cell infusion, subjects received a lymphodepleting chemotherapy with fludarabine (flu, 30 mg/m²) and cyclophosphamide (Cy, 300mg/m²) for three (3) days. The subjects received CAR-expressing T cells after lymphodepletion.

### B. Assessment of Response

Subjects were monitored for response at one month (thirty days) and three months after administration of the CAR+ T cells.

Responses were assessed by iwCLL criteria (2008 criteria: Hallek et al. Blood 2008; 111(12):5446-5456; 2018 criteria Hallek et al., Blood 2018 131 (25): 2745-2760), except that the initial efficacy evaluation was carried out at Day 30. Response was assessed as complete remission (CR), CR with incomplete marrow recovery (CRi), nodular partial remission PR (nPR), partial remission (PR), stable disease, or PD based on the iwCLL guidelines. nPR and PR were confirmed with a repeat assessment at least eight weeks later. Disease was also assessed with PET at baseline and after treatment with CAR-expressing T cells on an exploratory basis as indicated in the schedule of events.

A response assessment was conducted at day thirty post-treatment; months two (hematology only), three, six, nine, twelve, eighteen, and twenty-four (+ fourteen days) or until PD, evidence of clinical progression, withdrawal from study, or a requirement for alternative therapy. Efficacy assessments included, as appropriate: Hematology (CBC with differential), CT (diagnostic-quality), PET, bone marrow aspirate (BMA)/bone marrow biopsy (BMB) (at baseline and day thirty evaluation, and thereafter only if other results were newly consistent with CR, and in subjects who had no evidence of CLL in peripheral blood or marrow but had PR based on residual lymphadenopathy or splenomegaly), assessment of minimal residual disease (MRD; in subjects who have no evidence of CLL in peripheral blood or marrow, i.e., CR or PR based on residual lymphadenopathy or splenomegaly).

Minimal residual disease (MRD) was assessed at 1×10⁻⁴ sensitivity by six-color flow cytometry using peripheral blood and, for those who were undetectable by flow, in bone marrow at 1×10⁻⁶ sensitivity using next generation sequencing (NGS) by clonoSEQ^{®} (Adaptive) deep sequencing of bone marrow (BM) aspirates.

### Example 1.B.1

Results are described in this Example 1.B.1 for evaluation in an ongoing clinical trial. At this analysis time point, a cohort often (10) adult human subjects were assessed. The demographics and baseline characteristics of the cohort subjects are set forth in **Table E1.**

| **Table E1: Baseline Characteristics** | | |
|---|---|---|
| **Characteristic** | | **N=10** |
| Median Age, years (range) | | 64.5 (51-76) |

| **Stage, n (%)** | | |
|---|---|---|
| Rai Stage III/IV | | 6 (60) |
| Binet Stage C | | 6 (60) |
| **High-risk cytogenetics, n (%)** | | **7 (70)** |
| Del(17p), n (%) | | 4 (40) |
| TP53 mutation, n (%) | | 5 (50) |
| IGHV unmutated, n (%) | | 1 (10) |
| Complex karyotype, n (%) | | 4 (40) |

| **Patient Characteristics** | | |
|---|---|---|
| Prior lines of therapy, median (range) | | 4 (3-8) |
| Prior ibrutinib, n (%) | | 9 (90) |
| | Ibrutinib progression and prior venetoclax,^{a} n (%) | 6 (60) |

Thirty days post-dose, six of eight subjects (75%) who were evaluable for response had an objective response including four CRs (50%). Six of seven subjects (85.7%) evaluable for minimal residual disease (MRD) had undetectable disease by flow at the day thirty assessment. Four of five subjects evaluable for response at three months post-dose had ongoing response and one progressed with Richter's transformation. All four subjects with ongoing response continued to have undetectable MRD by flow cytometry at three months post-dose. Responses, including CRs and undetectable MRD responses, occurred in subjects with high-risk and with standard-risk disease. Available bone marrow analyses from clonoSEQ^{®} supported these observations. The results are summarized in **Table E2.**

| **Table E2. Response** | |
|---|---|
| **Response, n (%)** | **Total (n=8)** |
| Overall Response | 6 (75) |
| CR | 4 (50) |
| PR | 2 (25) |
| SD | 1 (12.5) |
| PD | 1 (12.5) |
| **Undetectable MRD (10⁻⁴ blood), n (%)** | **(n = 7)** |
| Overall undetectable MRD | 6 (85.7) |
| CR | 3 (42.9) |
| PR | 2 (28.6) |
| SD | 1 (14.3) |

### Example 1.B.2

At a subsequent point in time in the clinical study described in Example 1.B.1 above, results were analyzed. At this analysis time point, a cohort of sixteen (16) adult human subjects with relapsed or refractory (R/R) chronic lymphocytic leukemia (CLL) who have failed or are ineligible of BTKi therapy were assessed.

The demographics and baseline characteristics of the cohort subjects are set forth in **Table E3.**

| **Table E3. Baseline Characteristics** | | |
|---|---|---|
| **Characteristic** | | **N=16** |
| Median Age, years (range) | | 64.5 (51-76) |
| Male, n (%) | | 8 (50) |

| **Stage, n (%)** | | |
|---|---|---|
| Rai Stage III/IV | | 10 (62.5) |
| Binet Stage C | | 10 (62.5) |
| **High-risk cytogenetics (any), n (%)** | | **12 (75)** |
| Del (17p) | | 7 (43.8) |
| TP53 mutation | | 10 (62.5) |
| IGHV unmutated | | 2 (12.5) |
| Complex karyotype | | 8 (50) |

| **Patient Characteristics** | | |
|---|---|---|
| Prior lines of therapy, median (range) | | 4.5 (2-11) |
| Prior ibrutinib, n (%) | | 16 (100) |
| Ibrutinib relapse/refractory, n (%) | | 13 (81.3) |
| | Ibrutinib progression and prior venetoclax,^{a} n (%) | 8 (50) |

Seven patients progressed on venetoclax; 1 patient had best response of SD alter 3 months of treatment.

Response rates are summarized in **Table E4.** For subjects across all doses, the observed best overall response was an ORR of 81.3% with a CR/CRi rate of 43.8%. At this time point in the study, CR continued in 5 of 6 patients with at least 3 months of follow-up.

| **Table E4. Response** | | | |
|---|---|---|---|
| | **Total** | **DL1** | **DL2** |
| **Best Overall Response, n (%)** | **(N = 16)** | **(N** = **6)** | **(N = 10)** |
| ORR | 13 (81.3) | 6 (100) | 7 (70) |
| CR/CRi | 7 (43.8) | 5 (83.3) | 2 (20) |
| PR/nPR | 6 (37.5) | 1 (16.7) | 5 (50) |
| SD | 2 (12.5) | 0 | 2 (20) |
| PD | 1 (6.3) | 0 | 1 (10) |

| **Response at 30 Days Post-Dose, n (%)** | **(N = 16)** | **(N = 6)** | **(N = 10)** |
|---|---|---|---|
| ORR | 12 (75) | 6 (100) | 6 (60) |
| CR/CRi | 5 (31.3) | 3 (50.0) | 2 (20.0) |
| PR/nPR | 7 (43.8) | 3 (50.0) | 4 (40.0) |

| **Response at 3 Months Post-Dose, n (%)** | **(N = 10)** | **(N = 6)** | **(N = 4)** |
|---|---|---|---|
| ORR | 8 (80.0) | 5 (83.3) | 3 (75.0) |
| CR/CRi | 5 (50.0) | 3 (50.0) | 2 (50.0) |
| PR/nPR | 3 (30.0) | 2 (33.3) | 1 (25.0) |

Eleven of fifteen subjects (73.3%) had undetectable MRD (uMRD) in blood by flow cytometry at day thirty. All subjects continue to remain undetectable at the latest follow-up at the last time-point assessed in this study. Additionally, all five patients who had month six follow-up continued to maintain uMRD response (CR, n = 4 and PR, n = 1 by iwCLL criteria). These results are summarized in **Table E5.**

| **Table E5. Minimal Residual Disease: Undetectable MRD at Any Timepoint** | | | |
|---|---|---|---|
| MRD Negativity, n (%) | **Total** | **DL1** | **DL2** |
| | (N = 15) | (n = 6) | (n = 9) |
| Peripheral blood, flow (10⁻⁴) | 11 (73.3) | 6 (100) | 5 (55.6) |
| | (N = 8) | (n = 5) | (n = 3) |
| Bone marrow, NGS (10⁻⁴) | 7 (87.5) | 4 (80.0) | 3 (100) |

### C. Assessment of Safety

Adverse events (AEs), serious adverse events (SAEs), and laboratory abnormalities (type, frequency, and severity) were collected. Adverse events of special interest (AESIs) included infusion reactions, cytokine release syndrome (CRS), neurological toxicity, macrophage activation syndrome (MAS), and tumor lysis syndrome (TLS).

The presence or absence of treatment-emergent adverse events (TEAE) following administration of the CAR-T cell therapy was assessed. Subjects were assessed and monitored for neurotoxicity ( neurological complications including symptoms of confusion, aphasia, encephalopathy, myoclonus seizures, convulsions, lethargy, and/or altered mental status), graded on a 1-5 scale, according to the National Cancer Institute-Common Toxicity Criteria (CTCAE) scale, version 4.03 (NCI-CTCAE v4.03). See Common Terminology for Adverse Events (CTCAE) Version 4, U.S. Department of Health and Human Services, Published: May 28, 2009 (v4.03: June 14, 2010); and Guido Cavaletti & Paola Marmiroli Nature Reviews Neurology 6, 657-666 (December 2010). Cytokine release syndrome (CRS) was determined and monitored, graded based on severity. See Lee et al, Blood. 2014;124(2): 188-95. Dose-limiting toxicities (DLT) were determined within 28 days following infusion of CAR-expressing T cells.

### Example 1.C.1

Example 1.C.1 describes AEs based on the analysis time-point in Example 1.B.1. No DLTs were identified. Cytokine release syndrome (CRS), anemia, thrombocytopenia, and leukopenia were the most common adverse events. Neurologic events (NE) were reported in three often subjects: Grade 1 impaired concentration and aphasia, Grade 3 encephalopathy, and Grade 3 aphasia. The median time to onset of CRS and NE was 4.5 with a range of 1-9 days, and 11 with a range of 11-21 days, respectively. The median duration of CRS and NE was 5.5 with a range of 3-30 days, and 6 with a range of 2-20 days, respectively. Six subjects received tocilizumab and/or steroids for the management of CRS and/or NE. Serious AEs of Grades 3-4, were reported in five often subjects. **Table E6** depicts the percentage of subjects who were observed to have experienced TEAEs.

| **Table E6. Assessment of Presence or Absence of TEAEs for Example 1.B.1** | |
|---|---|
| **Adverse Event, n (%)** | **Total** |
| All Grade 3-4 Events | 9 (90) |
| Anemia | 5 (50) |
| Leukopenia | 5 (50) |
| Thrombocytopenia | 5 (50) |
| Neutropenia | 4 (40) |
| Lymphopenia | 3 (30) |
| **All SAEs** | **5 (50)** |
| Grade 3-4 SAEs | 5 (50) |
| Aphasia | 1 (10) |
| Blood fibrinogen decreased | 1 (10) |
| Encephalopathy | 1 (10) |
| Febrile neutropenia | 1 (10) |
| Hyponatremia | 1 (10) |
| Lung infection | 2 (20) |
| **Cytokine release syndrome** | **8 (80)** |
| Grade 3-4 Events | 0 |
| **Neurologic Events** | **3 (30)** |
| Grade 3-4 Events | 2 (20) |
| **DLTs** | **0** |

### Example 1.C.2

Example 1.C.2 describes AEs based on the analysis time-point in Example 1.B.2. **Table E7** summarizes TEAEs in the subjects at this time point (only related serious AEs after ninety days are shown). TEAEs were observed to occur in ≥20% of subjects. One DLT of grade 4 hypertension was reported in DL2.

| **Table E7. Assessment of Presence or Absence of TEAEs for Example 1.B.2** | | | |
|---|---|---|---|
| | **All Grades** | **Grade ≥ 3** | **Treatment-related Grade ≥ 3** |
| Any TEAE, n (%) | 16 (100) | 16 (100) | 9 (56.3) |
| Anemia | 14 (87.5) | 11 (68.8) | 4 (25) |
| Thrombocytopenia | 13 (81.3) | 12 (75) | 5 (31.3) |
| Cytokine release syndrome | 12 (75) | 1 (6.3) | 1 (6.3) |
| Neutropenia | 10 (62.5) | 10 (62.5) | 6 (37.5) |
| Leukopenia | 9 (56.3) | 9 (56.3) | 5 (31.3) |
| Hypokalemia | 8 (50) | 0 | 0 |
| Pyrexia | 6 (37.5) | 0 | 0 |
| Lymphopenia | 5 (31.3) | 5 (31.3) | 5 (31.3) |
| Nausea | 5 (31.3) | 0 | 0 |
| Diarrhea | 4 (25) | 0 | 0 |
| Febrile neutropenia | 4 (25) | 3 (18.8) | 1 (6.3) |
| Headache | 4 (25) | 0 | 0 |
| Insomnia | 4 (25) | 0 | 0 |
| Tremor | 4 (25) | 0 | 0 |

**Table E8** summarizes SAEs in subjects at this time-point. All SAEs were of grade ≥ 3. One DLT of Grade 4 hypertension was reported in DL2.

| **Table E8. Assessment of Presence or Absence of SAEs for Example 1.B.2** | |
|---|---|
| | **All Patients (N=16)** |
| Any SAEs of any grade, n (%) | 7 (43.8) |
| Lung infection | 3 (18.8) |
| Aphasia | 1 (6.3) |
| Blood fibrinogen decreased | 1 (6.3) |
| Encephalopathy | 1 (6.3) |
| Febrile neutropenia | 1 (6.3) |
| Hypertension | 1 (6.3) |
| Hyponatremia | 1 (6.3) |

There were no Grade 4/5 adverse events of special interest observed in any of the subjects in Example 1.B.2 analysis. **Table E9** summarizes the incidence of cytokine release syndrome (CRS) and neurotoxicity adverse events in subjects. As shown in **Table E9,** low rates of grade 3 CRS (6.3%) and grade 3 neurologic events (18.8%) were observed at this time point in the study.

| **Table E9. Assessment of Presence or Absence of CRS and Neurotoxicity Adverse Events for Example 1.B.2** | | | |
|---|---|---|---|
| | **Total (N=16)** | **DL1 (N=6)** | **DL2 (N=10)** |
| **CRS- any grade, n (%)** | 12 (75) | 6 (100) | 6 (60) |
| Median time to first onset, d (range) | 6.5 (1-10) | 6.5 (1-9) | 5 (2-10) |
| Median duration, d (range) | 5.5 (2-30) | 5.5 (3-30) | 5.5 (2-13) |
| **Grade 3** | **1 (6.3)** | **0** | **1 (10)** |
| **Neurological events (NE)^{a}** - **any grade, n (%)** | 6 (37.5) | 2 (33.3) | 4 (40) |
| Median time to first onset, d (range) | 10 (4-21) | 16 (11-21) | 8 (4-11) |
| Median duration, d (range) | 6.5 (2-20) | 4 (2-6) | 8 (3-20) |
| **Grade 3^{b}** | **3 (18.8)** | **2 (33.3)** | **1 (10)** |
| **Any, n (%)** | | | |
| CRS or NE^{a} | 13 (81.3) | 6 (100) | 7 (70) |
| CRS or NE^{a} | 5 (31.3) | 2 (33.3) | 3 (30) |
| Tocilizumab and/or dexamethasone use | 11 (68.8) | 4 (66.7) | 7 (70) |
| **Tumor lysis syndrome - any grade, n (%)** | 2 (12.5) | 1 (16.7) | 1 (10) |
| **Grade 3** | **2 (12.5)** | **1 (16.7)** | **1 (10)** |

| | | | |
|---|---|---|---|
| ^{a}Neurologic events are treatment-related events. Encephalopathy n = 2; aphasia n =1; confusional state n = 1 | | | |

### D. Assessment of CAR+ T cells and Cytokine Analysis

Blood pharmacokinetics of the CAR-expressing T cells was determined using flow cytometry. Serum soluble chemokine and cytokine profiles for thirty-nine analytes were assessed using an electrochemiluminescence assay platform (e.g., V-PLEX immunoassays (MSD)). Analytes, including cytokines, were measured within the first thirty days of administration of the therapeutic anti-CD 19 CAR+ T cell compositions.

### Example 1.D.1

Based on data from the time-point described in Example 1.B.1 and 1.C.1, pharmacokinetic analysis was carried out to assess numbers of CAR⁺ T cells in peripheral blood post-treatment. Median Cₘₐₓ was 219 CAR⁺ T cells/µl with a range of 0.35 to 583.46. The median time to peak expansion was 15.5 days with a range of 13 to 19 days. The median area under the curve (AUC) was 1528 cells per day/µl with a range of 590 to 2847. In the one subject with a best response of progressive disease, minimal CAR⁺ C cell expansion was observed (**FIG. 1**). CAR⁺ T cells persisted in subjects maintaining their response at three months post-dose.

Serum analysis showed elevated levels of multiple biomarkers including CRP, IL-6, PLGF, IL-16, and IL-15 in conjunction with CRS and NE.

### Example 1.D.2

Based on data from the time-point described in Example 1.B.2 and 1.C.2, pharmacokinetic analysis was carried out to assess the numbers of CAR⁺ T cells in peripheral blood up to ninety days post-administration. As shown in **FIG. 2****,** CAR+ T cells persisted in subjects maintaining their response at three months post-administration.

Cytokine analysis revealed IL-16 and TNF as potential biomarkers of high-grade neurologic events (**FIG. 3A and 3B**)**.** Of 38 cytokines assessed, IL-16 and TNF were elevated in three of the four patients before the first onset of grade 2 or 3 neurologic events (**Table E9**).

### Example 2: Further Assessment of Response, Safety, Pharmacokinetics, Pharmacodynamics and Blood Analytes in Subjects with Relapsed and Refractory Chronic Lymphocytic Leukemia (CLL) or small lymphocytic lymphoma (SLL)

Response outcomes, safety outcomes, pharmacokinetic and pharmacodynamics parameters, and blood analytes were assessed in subjects at a subsequent point in time in the clinical study described in Example 1 above.

The analysis at this time point presented in this example is based on a total of sixteen subjects that had been administered the anti-CD19 CAR-expressing cells. Eligible subjects had received two or more prior lines of therapy including a Bruton's tyrosine kinase inhibitors (BTKi), e.g. ibrutinib, unless medically contraindicated, and had an Eastern Cooperative Oncology Group performance status (ECOG PS) of less than or equal to 1. After three days of lymphodepleting chemotherapy, subjects were administered a single dose of CAR-expressing T cells (each single dose via separate infusions of CD4+ CAR-expressing T cells and CD8+ CAR-expressing T cells, respectively) as follows: a single dose of dose level 1 (DL-1) containing 5 × 10⁷ total CAR-expressing T cells (n=6) or a single dose of dose level 2 (DL-2) containing 1 × 10⁸ total CAR-expressing T cells (n=10). The therapeutic T cell dose was administered as a defined cell composition by administering the formulated CD4+ CAR+ cell population and the formulated CD8+ CAR+ population administered at a target ratio of approximately 1:1, as described in Example 1.

Subjects were monitored for dose-limiting toxicities (DLTs) and response was assessed by iwCLL 2008 criteria. Minimal residual disease was assessed at 1×10⁻⁴ sensitivity by flow cytometry using peripheral blood and in bone marrow at 1×10⁻⁶ sensitivity using next generation sequencing (NGS) by clonoSEQ^{®} (Adaptive) deep sequencing of bone marrow (BM) aspirates.

At this analysis time point, 75% of subjects had high-risk features such as TP53 mutation, complex karyotype, or del17p, 100% had prior BTKi, e.g. ibrutinib, therapy, and 50% had prior venetoclax. The median number of prior lines of therapy was 4.5 (range 2-11).

There was one dose limiting toxicity of grade (G) 4 hypertension in a subject administered DL2. The most common Grade 3/4 treatment-emergent adverse events were cytopenias (thrombocytopenia, 75%; anemia, 69%; neutropenia, 63%; leukopenia, 56%). One subject had G3 cytokine release syndrome (CRS) and three subjects had G3 neurological events (NE).

Of 15 evaluable subjects, the best overall response rate (ORR) was 87% (13/15), with 83% (5/6) ORR at six months. Seven subjects (47%) achieved complete remission (CR) with or without complete blood count recovery (CR/CRi).

Ten of fifteen subjects (67%) achieved undetectable minimal residual disease (uMRD) in blood by day 30 and uMRD was achieved in seven of eight subjects (88%) in bone marrow aspirates. MRD-negative CRs were seen in subjects who had failed both BTKi and venetoclax. The median time to peak blood CAR+ T cell level was sixteen days (range 4-30).

These results demonstrate that in heavily pretreated subjects with standard- and high-risk CLL/SLL, including in subjects having received previous ibrutinib treatment, toxicities (i.e. CRS and NEs) following administration of an anti-CD19 CAR+ T cell composition were manageable. Further, of these subjects, CR/CRi and/or uMRD was rapidly achieved.

### Example 3: Administration of Anti-CD19 Expressing Cells to Subjects with Relapsed and Refractory Chronic Lymphocytic Leukemia (CLL) or small lymphocytic

### lymphoma (SLL)

Therapeutic CAR+ T cell compositions containing autologous T cells expressing a chimeric antigen-receptor (CAR) specific for CD19 were administered to human subjects with chronic lymphocytic leukemia (CLL) or small lymphocytic lymphoma (SLL) in a clinical study as described in Examples 1and 2 above. Response outcomes and safety outcomes were assessed in subjects at a subsequent point in time in the clinical study described in Examples 1 and 2 above. The anti-CD 19 therapeutic CAR-T cell compositions were produced as described in Example 1. Manufacturing of the anti-CD 19 therapeutic T cell composition was unsuccessful for one subject.

The analysis at this time point presented in this example is based on a total of 23 subjects that had been administered the anti-CD 19 CAR-expressing cells according to the eligibility for the clinical study as described in Example 1 and 2. Two subjects (9%) were intolerant to ibrutinib. Baseline characteristics of treated subjects is shown in **Table E10.**

| **Table E10: Baseline Characteristics** | | | | |
|---|---|---|---|---|
| | **Characteristic** | **All Subjects N=23** | **DL1** n=9 | **DL2** n=14 |
| | Median Age, years (range) | 66 (49-79) | 67 (49-76) | 66 (53-79) |
| | Male, n (%) | 11 (47.8) | 4 (44.4) | 7 (50.0) |
| | Bulky disease >5 cm, n (%)^{a} | 8 (34.8) | 3 (33.3) | 5 (35.7) |
| | SPD (cm²), median (range) | 24.7 (2-197) | 30.0 (2-119) | 24.7 (5-197) |
| | LDH (U/L), median (range) | 243 (119-634) | 227 (174-634) | 275 (119-527) |
| | Received bridging therapy, n (%) | 17 (73.9) | 5 (55.6) | 12 (85.7) |

| | **Stage, n (%)** | | | |
|---|---|---|---|---|
| | Rai Stage III/IV | 15 (65.2) | 6 (66.7) | 9 (64.3) |
| | Binet Stage C | 16 (69.6) | 7 (77.8) | 9 (64.3) |
| | **High-risk cytogenetics, n (%)** | **19 (82.6)** | **6 (66.7)** | **13 (92.9)** |
| | Del(17p), n (%) | 8 (34.8) | 3 (33.3) | 5 (35.7) |
| | TP53 mutation, n (%) | 14 (60.9) | 4 (44.4) | 10 (71.4) |
| | Complex karyotype, n (%)^{b} | 11 (47.8) | 5 (55.6) | 6 (42.9) |

| | **Patient Characteristics** | | | |
|---|---|---|---|---|
| | Prior lines of therapy, median (range) | 5 (2-11) | 5 (3-8) | 5 (2-11) |
| | Prior ibrutinib, n (%) | 23 (100) | 9 (100) | 14 (100) |
| | Ibrutinib relapsed/refractory, n (%) | 21 (91.3) | 9 (100) | 12 (85.7) |
| | Ibrutinib progression and prior venetoclax,^{c} n (%) | 13 (56.5) | 5 (55.6) | 8 (57.1) |

| | | | | |
|---|---|---|---|---|
| ^{a} Bulky disease defined as at least one lesion longest diameter >5cm. ^{b} At least three chromosomal aberrations. ^{c} Twelve subjects progressed on venetoclax; one subject had best response of stable disease after three months of treatment. | | | | |

Subjects received lymphodepleting chemotherapy with fludarabine (flu, 30 mg/m²) and cyclophosphamide (Cy, 300 mg/m²) for three days prior to CAR+ T cell infusion. Anti-CD19 CAR-expressing T cells were successfully manufactured for 96% of subjects. The cryopreserved anti-CD19 cell compositions were thawed prior to intravenous administration. Subjects were administered a single dose of CAR-expressing T cells (each single dose via separate infusion of CD4+ CAR-expressing T cells and CD8+ CAR-expressing T cells, respectively) as follows: a single dose of dose level 1 (DL-1) containing 5 × 10⁷ total CAR-expressing T cells (n=9 subjects) or a single dose of dose level 2 (DL-2) containing 1 × 10⁸ total CAR-expressing T cells (n=14 subjects). The therapeutic T cell dose was administered as a defined cell composition by administering the formulated CD4+ CAR+ cell population and the formulated CD8+ CAR+ population administered at a target ratio of approximately 1:1, as described in Example 1. Dose escalation followed a modified toxicity probability-interval-2 (mTIPI-2) algorithm (Guo et al. (2017) Contemp Clin Trials, 2017; 58:23-33). Dose-limiting toxicities (DLTs) were evaluated for twenty-eight days post administration.

### A. Assessment of Response

Responses were assessed by iwCLL criteria as described in Example 1 and 2. Minimal residual disease was assessed at 1×10⁻⁴ sensitivity by flow cytometry using peripheral blood and in bone marrow at 1×10⁻⁶ sensitivity using next generation sequencing (NGS) by clonoSEQ^{®} (Adaptive) deep sequencing of bone marrow (BM) aspirates. Subjects were monitored for response up to twenty-four months after administration of the CAR+ T cells. The median study follow-up was nine months, with a minimum follow-up of one month.

Response rates are shown in **FIGS. 4A-4B** and **FIG. 5****.** Twenty two subjects were evaluable for response defined as having a pretreatment assessment and at least one post-baseline assessment. One subject was not evaluable for response. The best overall response is shown in **FIG. 4A****.** As shown in **FIG. 4A****,** of the total number of evaluable subjects, 4.5% demonstrated progressive disease, 13.6% demonstrated stable disease, 36.4% demonstrated partial response or nodular partial response, and 45.5% demonstrated complete response or complete response with incomplete blood count recovery. Of the nine evaluable subjects that were administered DL1 (5 × 107 CAR-expressing T cells), 22.2% demonstrated stable disease, 11.1% demonstrated partial response or nodular partial response, and 66.7% demonstrated complete response or complete response with incomplete blood count recovery. Of thirteen subjects administered DL2 (1 × 10⁸ CAR-expressing T cells), 7.7% demonstrated progressive disease, 7.7% demonstrated stable disease, 53.8% demonstrated partial response or nodular partial response, and 30.8% demonstrated complete response or complete response with incomplete blood count recovery. The best overall response rate (ORR) was 82% (95% confidence interval: 59.7-94.8), and CR/CRi rate of 46% (one subject with nonevaluable MRD achieved a CR but later progressed). At one time point of assessment, sixty-eight percent (15/22) of subjects achieved an objective response by Day 30 after infusion. 78% (7/9) of responders with more than 9 months of post-infusion follow-up remained progression-free.

Twenty subjects were evaluable for minimal residual disease defined as having detectable minimal residual disease at baseline (**FIG. 4B**). As shown in **FIG. 4B****,** undetectable minimal residual disease (uMRD) was achieved in 75% peripheral blood and 65% bone marrow of subjects (one subject achieved uMRD in blood/BM then later had detectable MRD). Of 8 subjects administered DL1 (5 × 10⁷ CAR-expressing T cells) uMRD was achieved in 75% peripheral blood and bone marrow. Of 12 subjects administered DL2 (1 × 10⁸ CAR-expressing T cells), uMRD was achieved in 75% peripheral blood and 58% bone marrow.

The duration of response by progression-free time continued to improve over time (**FIG. 5**). Of twenty-two subjects, 27% (6/22) demonstrated deepening of response past day thirty: 3 subjects that exhibited partial response (PR) later achieved CR, 2 subjects went from stable disease (SD) to PR, and 1 subject went from SD to CR. Five of six subjects, 83%, with CR at six months remained in CR, including three subjects who maintained complete response beyond twelve months. Twelve of twenty evaluable subjects, 60% (12/20), had undetectable MRD by next generation sequencing at day thirty. Early uMRD seen in 60% of subjects was ongoing at 12 months and at a later time point of assessment.

### B. Assessment of Safety

The presence or absence of treatment-emergent adverse events (TEAE) following administration of anti-CD 19 CAR-T cell therapy was assessed as outlined in Example 1. TEAEs were reported in ≥ 25% of subjects at all dose levels (**Table E11**). At one time point of assessment, grade 3 or 4 anemia was observed in 96% of the subjects. Serious TEAEs occurred in some subjects (**Table E12**). **Table E13** reports incidence of certain adverse events, including cytokine release syndrome (CRS) and neurologic events (NE). No Grade 5 CRS or NE occurred. Neurological events were not mutually exclusive. Encephalopathy was seen in three subjects. Aphasia, confused state, muscular weakness, and somnolence each were seen in one subject. Dose-limiting toxicities occurred in two subjects receiving DL2 (1 × 10⁸ CAR-expressing T cells). One subject experienced Grade 4 hypertension, while one subject experienced Grade 3 encephalopathy, Grade 3 muscle weakness, and Grade 4 tumor lysis syndrome. One TEAE of Grade 5 respiratory failure was observed in a subject administered DL1 (5 × 10⁷ CAR-expressing T cells). For management of CRS and/or NE, 61% (n=14) of subjects received tocilizumab and 48% (n=1 1) received corticosteroids. Adverse events were manageable at both dose levels, with low rates of Grade 3 CRS (8.7%) and Grade 3 or 4 NE (21.7%). The higher rates of grades 1-2 CRS are consistent with a favorable safety profile of the anti-CD 19 CAR+ T cell composition in this group of subjects. Lymph node tumor burden was shown to correlate with NE (P=0.025).

Serum cytokine and chemokine levels were also assessed in the subjects. Cytokine analysis showed that NE onset was preceded by elevated TNFα and IL-16 early after anti-CD 19 CAR-expressing T cell infusion (P<0.01).

| **Table E11: Assessment of Presence or Absence of TEAEs** | | | | | |
|---|---|---|---|---|---|
| | | **All Grades** | **Grade ≥ 3** | **DL1 Grade ≥ 3** | **DL2 Grade ≥3** |
| | | **(N=23)** | **(N=23)** | **(n=9)** | **(n=14)** |
| | Any TEAE, n (%) | 23 (100.0) | 22 (95.7) | 8 (88.9) | 14 (100) |
| | Anemia | 19 (82.6) | 18 (78.3) | 7 (77.8) | 11 (78.6) |
| Cytokine release syndrome | | 17 (73.9) | 2 (8.7) | 0 | 2 (14.3) |
| | Thrombocytopenia | 17 (73.9) | 16 (69.6) | 4 (44.4) | 12 (85.7) |
| | Neutropenia | 13 (56.5) | 13 (56.5) | 4 (44.4) | 9 (64.3) |
| | Leukopenia | 11 (47.8) | 10 (43.5) | 4 (44.4) | 6 (42.9) |
| | Hypokalemia | 9 (39.1) | 0 | 0 | 0 |
| | Pyrexia | 9 (39.1) | 0 | 0 | 0 |
| | Nausea | 8 (34.8) | 0 | 0 | 0 |
| | Diarrhea | 7 (30.4) | 0 | 0 | 0 |
| | Hypophosphatemia | 7 (30.4) | 4 (17.4) | 0 | 4 (28.6) |
| | Tremor | 7 (30.4) | 0 | 0 | 0 |
| | Febrile neutropenia | 6 (26.1) | 5 (21.7) | 0 | 5 (35.7) |
| | Hypomagnesemia | 6 (26.1) | 0 | 0 | 0 |
| | Lymphopenia | 6 (26.1) | 6 (26.1) | 2 (22.2) | 4 (28.6) |

| **Table E12: Serious TEAEs Reported in > 1 Subject** | | | |
|---|---|---|---|
| | **All Subjects** | **DL1** | **DL2** |
| | **(N=23)** | **(n=9)** | **(n=14)** |
| Serious TEAEs of any grade, n (%) | 13 (56.5) | 4 (44.4) | 9 (64.3) |
| Cytokine release syndrome | 6 (26.1) | 1 (11.1) | 5 (35.7) |
| Pyrexia | 4 (17.4) | 3 (33.3) | 1 (7.1) |
| Encephalopathy | 3 (13.0) | 1 (11.1) | 2 (14.3) |
| Febrile neutropenia | 3 (13.0) | 0 | 3 (21.4) |
| Pneumonia | 3 (13.0) | 2 (22.2) | 1 (7.1) |
| Acute kidney injury | 2 (8.7) | 2 (22.2) | 0 |
| Aphasia | 2 (8.7) | 1 (11.1) | 1 (7.1) |
| Lung infection | 2 (8.7) | 1 (11.1) | 1 (7.1) |
| Tumor lysis syndrome | 2 (8.7) | 0 | 2 (14.3) |

| **Table E13: TEAEs of Special Interest** | | | |
|---|---|---|---|
| | **Total** | **DL1** | **DL2** |
| | **(N=23)** | **(n=9)** | **(n=14)** |
| CRS - any grade, n (%) | 17 (73.9) | 7 (77.8) | 10 (71.4) |
| Median time to first onset, day (range) | 4 (1-10) | 6 (1-9) | 3.5 (1-10) |
| Median duration, day (range) | 5 (2-30) | 5 (3-30) | 5.5 (2-27) |
| Grade 3, n (%) | 2 (8.7) | 0 | 2 (14.3) |
| **NE^{a} - any grade, n (%)** | **9 (39.1)** | **2 (22.2)** | **7 (50.0)** |
| Median time to first onset, day (range) | 4.0 (2-21) | 16 (11-21) | 4 (2-11) |
| Median duration, day (range) | 21.0 (6-169) | 8.5 (6-11) | 38 (9-169) |
| Grade ≥ 3, ^{b} n (%) | 5 (21.7) | 2 (22.2) | 3 (21.4) |

| **Any, n (%)** | | | |
|---|---|---|---|
| CRS or NE^{a} | 18 (78.3) | 7 (77.8) | 11 (78.6) |
| CRS and NE^{a} | 8 (34.8) | 2 (22.2) | 6 (42.9) |
| Tocilizumab and/or steroid use | 17 (73.9) | 5 (55.6) | 12 (85.7) |
| **Tumor lysis syndrome - any grade, n (%)** | **4 (17.4)** | **1 (11.1)** | **3 (21.4)** |
| Grade ≥ 3, n (%) | 4 (17.4) | 1 (11.1) | 3 (21.4) |
| Cardiac events, n (%) | 1 (4.3) | 1 (11.1) | 0 |

| | | | |
|---|---|---|---|
| ^{a} NE are treatment-related events ^{b} NE are not mutually exclusive | | | |

### C. Pharmacokinetics and Pharmacodynamics

Blood pharmacokinetics of the anti-CD 19 CAR-expressing T cells was determined using flow cytometry to assess numbers of CD3+ CAR+ T cells in peripheral blood post-treatment. Results are shown in **FIG. 6** where anti-CD 19 CAR+ T cells were given on study day 1. In **FIG. 6****,** the upper error bar represents the third quartile and the lower error bar represents the first quartile. A summary of PK/PD parameters is shown in **Table E14,** including maximum concentration (Cₘₐₓ) of cells in the blood, time to reach the maximum (peak) concentration (Tₘₐₓ), and the area under the curve (AUC).

| **Table E14: Pharmacokinetic (PK) and Pharmacodynamic (PD) Profile** | | | |
|---|---|---|---|
| **Median (Q1, Q3)** | **Total (N=23)** | **DL1 (N=9)** | **DL2 (N=14)** |
| Cₘₐₓ (cells/µL) | 125 (4.45, 320) | 111 (325, 313) | 130 (35.2, 305) |
| Tₘₐₓ (study day) | 15 (15,22) | 15 (15,22) | 15 (15,22) |
| AUC₁₋₂₉ (day*cells/ µL) | 1110 (34, 2120) | 1080 (26.2, 1790) | 1340 (215, 2230) |

The results were consistent with an observation that objective response, complete response and uMRD were achieved rapidly following anti-CD19 CAR-expressing T cell infusion, with durable responses observed past 6 months, for the heavily pretreated subjects with relapsed/refractory CLL/SLL (for example, including those that had received prior ibrutinib and those who have failed both prior venetoclax and ibrutinib) in a clinical study. Adverse events associated with anti-CD 19 CAR-expressing T cell administration, including cytokine release syndrome (CRS) and neurologic events (NE), were observed to be manageable at both dose levels tested.

### D. Assessment of Response, Safety and Pharmacokinetics in Subjects that have Failed Prior BTKi and Venetoclax Treatment

At a different analysis time point, response, safety and pharmacokinetics were assessed in a group of nine subjects, among the 23 total subjects, who have failed prior treatment with both a Bruton's tyrosine kinase inhibitor (BTKi) and venetoclax. **Table E15** shows the baseline characteristics of the 23 subjects, assessed in Example 3.A-3.C above, and those of the subjects that have failed BTKi and venetoclax.

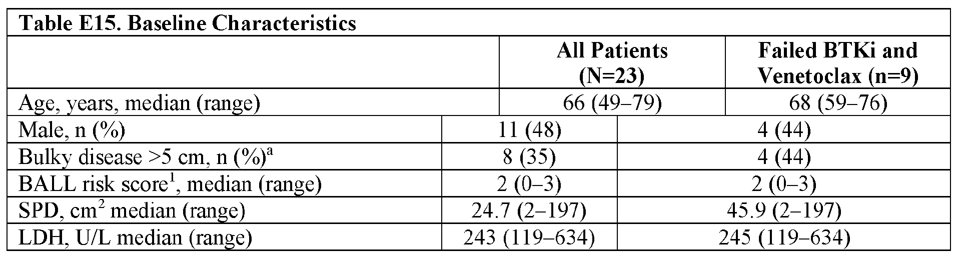

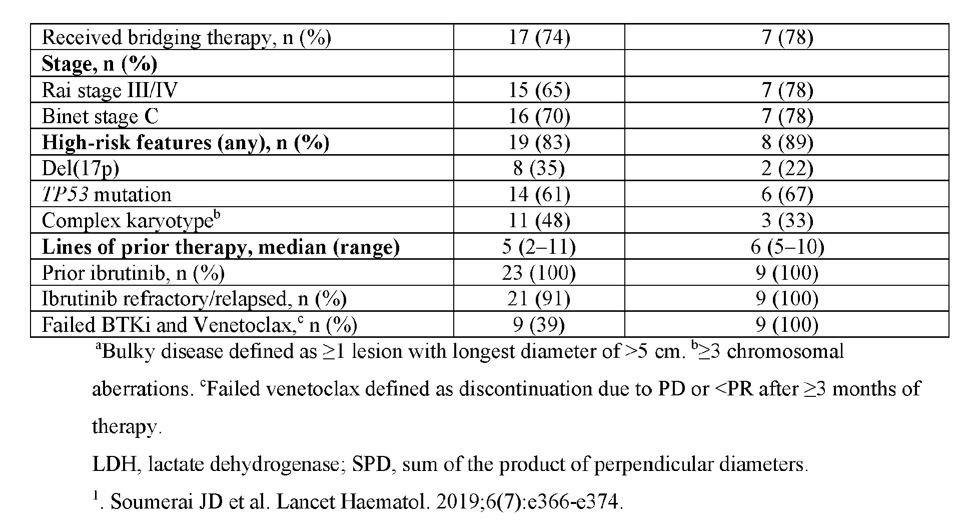

**Table E16** shows the treatment-emergent adverse events (TEAE) of the 23 total subjects or the nine subjects that failed both BTKi and venetoclax. Dose-limiting toxicities (DLTs) were observed in two subjects who received DL2 of the engineered cells: one subject exhibited grade 4 hypertension, and one subject, who were in the failed BTKi and venetoclax group, exhibited Grade 3 encephalopathy, Grade 3 muscle weakness, and Grade 4 tumor lysis syndrome.

| **Table E16. Safety Profile** | | |
|---|---|---|
| | **All Patients (N=23)** | **Failed BTKi and Venetoclax (n=9)** |
| **Serious TEAEs of any grade, n (%)** | **13 (56.5)** | **5 (55.6)** |
| CRS | 6 (26) | 2 (22) |
| Pyrexia | 4 (17) | 2 (22) |
| Encephalopathy | 3 (13) | 1 (11) |
| Febrile neutropenia | 3 (13) | 1 (11) |
| Pneumonia | 3 (13) | 0 |
| Acute kidney injury | 2 (9) | 0 |
| Aphasia | 2 (9) | 1 (11) |
| Lung infection | 2 (9) | 2 (22) |
| TLS | 2 (9) | 1 (11) |

**Table E17** shows the treatment-emergent adverse events (TEAE) of special interest in the 23 total subjects or the nine subjects that failed both BTKi and venetoclax.

| **Table E17. TEAEs of Special Interest** | | |
|---|---|---|
| | **All Patients^{a} (N=23)** | **Failed BTKi and Venetoclax (n=9)** |
| **CRS-any grade, n (%)** | 17 (74) | 6 (67) |
| Median time to first onset, days (range) | 4 (1-10) | 1.5 (1-4) |
| Median duration of first event, days (range) | 12 (2-50) | 21 (6-50) |
| Grade 3, n (%) | 2 (9) | 1 (11) |
| **NEs^{b}-any grade, n (%)** | 9 (39) | 4 (44) |
| Median time to first onset, days (range) | 6.5 (2-103) | 6.5 (2-21) |
| Median duration of first event, days (range) | 21 (6-312) | 23.5 (6-49) |
| Grade ≥3,^{c} n (%) | 5 (22) | 3 (33) |
| Any CRS or NEs,^{b} n (%) | 18 (78) | 7 (78) |
| Any CRS and NEs,^{b} n (%) | 8 (35) | 3 (33) |
| Tocilizumab and/or steroid use | 17 (74) | 6 (67) |
| **TLS, n (%)** | 4 (17) | 1 (11) |

| | | |
|---|---|---|
| ^{a}No differences in TEAE profile between dose levels. ^{b}NEs are treatment-related events defined by the investigator. ^{c}NEs are not mutually exclusive; encephalopathy (n=3); aphasia (n=1); confusional state (n=1); muscular weakness (n=1); somnolence (n=1). | | |

**FIGS. 7A** and **7B** show the best overall response (**FIG. 7A**) and undetectable minimal residual disease (uMRD) in blood by flow cytometry or in bone marrow by next generation sequencing (NGS) (**FIG. 7B**). The median study follow-up was 11 months. As shown, a high proportion of durable responses, including CRs, with a high best ORR of 81.5% in all evaluable subjects and 89% in subjects that have failed BTKi and venetoclax, were observed.

**FIG. 8** shows the individual response assessment of treated subjects that failed both BTKi and venetoclax and the other evaluable subjects. The results showed that most subjects achieved an early objective response by Day 30 (68%; n=15/22) and uMRD (75%; n=15/20), including 6 subjects that failed both BTKi and venetoclax (67%). The results showed that responses have deepened over time in 27% (n=6/22) of subjects overall and 33% of subjects that failed both BTKi and venetoclax (n=3/9). The results also showed that durable responses were maintained at 6 months post-administration in most subjects: 87% of all subjects and (n=13/15) 83% of subjects that failed both BTKi and venetoclax (n=5/6). 83% (n=5/6) of subjects with a CR at 6 months remain in CR at 9 months, with 3 subjects in CR past 12 months. Two subjects that failed both BTKi and venetoclax maintained responses past 12 months.

The median concentration of CD3+ CAR+ cells from the subjects over time after administration is shown in **FIG. 9****.** The results showed that the subset of subjects who failed both BTKi and venetoclax had a similar PK/PD profile as the full subject population.

### E. Conclusion

The administration of anti-CD 19 CAR-expressing cells to heavily pretreated subjects with high-risk CLL/SLL, all of whom had failed prior ibrutinib, with over half also having failed prior venetoclax treatment, resulted in manageable toxicity and good clinical response. Adverse events were manageable, including in the subset of subjects who had failed both a BTKi and venetoclax. Grade 3 CRS (9%) and grade 3 or 4 NEs (22%) were observed to be low. At a median follow-up of 11 months, administration of anti-CD 19 CAR-expressing cells resulted in a high proportion of durable responses, including CR, including in subjects that have failed both prior BTKi and venetoclax. Clinical responses were observed to be rapid, improved over time, and were deep and durable. For example, most initial responses were achieved by Day 30, and responses deepened over time in 27% of evaluated subjects and in 33% of subjects that have failed both prior BTKi and venetoclax. Durable objective responses were maintained 6 months after administration (87% of evaluated subjects, 83% of subjects that have failed both prior BTKi and venetoclax). 83% of subjects with a CR at 6 months remained in CR at 9 months, with 3 subjects in CR past 12 months. Subjects who failed both prior BTKi and venetoclax exhibited a similar PK/PD profile as the full subject population. The results support administration of the anti-CD19 CAR+ cells for subjects with CLL/SLL, including those with high-risk CLL/SLL and those who have failed multiple prior therapies such as a prior BTKi therapy and venetoclax.

The present invention is not intended to be limited in scope to the particular disclosed embodiments, which are provided, for example, to illustrate various aspects of the invention. Various modifications to the compositions and methods described will become apparent from the description and teachings herein.

### SEQUENCES

| **SEQ ID NO.** | **SEQUENCE** | **DESCRIPTION** |
|---|---|---|
| **1** | ESKYGPPCPPCP | spacer (IgG4hinge) (aa) Homo sapiens |
| **2** | GAATCTAAGTACGGACCGCCCTGCCCCCCTTGCCCT | spacer (IgG4hinge) (nt) homo sapiens |
| **3** | ESKYGPPCPPCPGQPREPQVYTLPPSQEEMTKNQVSLTCLVKGFYPSDIAVEWE SNGQPENNYKTTPPVLDSDGSFFLYSRLTVDKSRWQEGNVFSCSVMHEALHNHY TQKSLSLSLGK | Hinge-CH3 spacer Homo sapiens |
| **4** | ESKYGPPCPPCPAPEFLGGPSVFLFPPKPKDTLMISRTPEVTCVVVDVSQEDPE VQFNWYVDGVEVHNAKTKPREEQFNSTYRVVSVLTVLHQDWLNGKEYKCKVSNK GLPSSIEKTISKAKGQPREPQVYTLPPSQEEMTKNQVSLTCLVKGFYPSDIAVE WESNGQPENNYKTTPPVLDSDGSFFLYSRLTVDKSRWQEGNVFSCSVMHEALHN HYTQKSLSLSLGK | Hinge-CH2-CH3 spacer Homo sapiens |
| **5** | RWPESPKAQASSVPTAQPQAEGSLAKATTAPATTRNTGRGGEEKKKEKEKEEQE ERETKTPECPSHTQPLGVYLLTPAVQDLWLRDKATFTCFVVGSDLKDAHLTWEV AGKVPTGGVEEGLLERHSNGSQSQHSRLTLPRSLWNAGTSVTCTLNHPSLPPQR LMALREPAAQAPVKLSLNLLASSDPPEAASWLLCEVSGFSPPNILLMWLEDQRE VNTSGFAPARPPPQPGSTTFWAWSVLRVPAPPSPQPATYTCVVSHEDSRTLLNA SRSLEVSYVTDH | IgD-hinge-Fc Homo sapiens |
| **6** | LEGGEGRGSLLTCGDVEENPGPR | T2A artificial |
| **7** | MLLLVTSLLLCELPHPAFLLIPRKVCNGIGIGEFKDSLSINATNIKHFKNCTSI SGDLHILPVAFRGDSFTHTPPLDPQELDILKTVKEITGFLLIQAWPENRTDLHA FENLEIIRGRTKQHGQFSLAVVSLNITSLGLRSLKEISDGDVIISGNKNLCYAN TINWKKLFGTSGQKTKIISNRGENSCKATGQVCHALCSPEGCWGPEPRDCVSCR NVSRGRECVDKCNLLEGEPREFVENSECIQCHPECLPQAMNITCTGRGPDNCIQ CAHYIDGPHCVKTCPAGVMGENNTLVWKYADAGHVCHLCHPNCTYGCTGPGLEG CPTNGPKIPSIATGMVGALLLLLVVALGIGLFM | tEGFR artificial |
| **8** | FWVLVWGGVLACYS LLVTVAFII FWV | CD28 (amino acids 153-179 of Accession No. P 10747) Homo sapiens |
| **9** | IEVMYPPPYLDNEKSNGTIIHVKGKHLCPSPLFPGPSKPFWVLVVVGGVLACYS LLVTVAFIIFWV | CD28 (amino acids 114-179 of Accession No. P 10747) Homo sapiens |
| **10** | RSKRSRLLHSDYMNMTPRRPGPTRKHYQPYAPPRDFAAYRS | CD28 (amino acids 180-220 of P 10747) Homo sapiens |
| **11** | RSKRSRGGHSDYMNMTPRRPGPTRKHYQPYAPPRDFAAYRS | CD28 (LL to GG) Homo sapiens |
| **12** | KRGRKKLLYIFKQPFMRPVQTTQEEDGCSCRFPEEEEGGCEL | 4-1BB (amino acids 214-255 of Q07011.1) Homo sapiens |
| **13** | RVKFSRSADAPAYQQGQNQLYNELNLGRREEYDVLDKRRGRDPEMGGKPRRKNP QEGLYNELQKDKMAEAYSEIGMKGERRRGKGHDGLYQGLSTATKDTYDALHMQA LPPR | CD3 zeta Homo sapiens |
| **14** | RVKFSRSAEPPAYQQGQNQLYNELNLGRREEYDVLDKRRGRDPEMGGKPRRKNP QEGLYNELQKDKMAEAYSEIGMKGERRRGKGHDGLYQGLSTATKDTYDALHMQA LPPR | CD3 zeta Homo sapiens |
| **15** | RVKFSRSADAPAYKQGQNQLYNELNLGRREEYDVLDKRRGRDPEMGGKPRRKNP QEGLYNELQKDKMAEAYSEIGMKGERRRGKGHDGLYQGLSTATKDTYDALHMQA LPPR | CD3 zeta Homo sapiens |
| **16** | RKVCNGIGIGEFKDSLSINATNIKHFKNCTSISGDLHILPVAFRGDSFTHTPPL DPQELDILKTVKEITGFLLIQAWPENRTDLHAFENLEIIRGRTKQHGQFSLAVV SLNITSLGLRSLKEISDGDVIISGNKNLCYANTINWKKLFGTSGQKTKIISNRG ENSCKATGQVCHALCSPEGCWGPEPRDCVSCRNVSRGRECVDKCNLLEGEPREF VENSECIQCHPECLPQAMNITCTGRGPDNCIQCAHYIDGPHCVKTCPAGVMGEN NTLVWKYADAGHVCHLCHPNCTYGCTGPGLEGCPTNGPKIPSIATGMVGALLLL LVVALGIGLFM | tEGFR artificial |
| **17** | EGRGSLLTCGDVEENPGP | T2A artificial |
| **18** | GSGATNFSLLKQAGDVEENPGP | P2A |
| **19** | ATNFSLLKQAGDVEENPGP | P2A |
| **20** | QCTNYALLKLAGDVESNPGP | E2A |
| **21** | VKQTLNFDLLKLAGDVESNPGP | F2A |
| **22** | PGGG- (SGGGG) 5-P- wherein P is proline, G is glycine and S is serine | linker |
| **23** | GSADDAKKDAAKKDGKS | Linker |
| **24** | GSTSGSGKPGSGEGSTKG | Linker |
| **25** | gacatccagatgacccagaccacctccagcctgagcgccagcctgggcgaccgg gtgaccatcagctgccgggccagccaggacatcagcaagtacctgaactggtat cagcagaagcccgacggcaccgtcaagctgctgatctaccacaccagccggctg cacagcggcgtgcccagccggtttagcggcagcggctccggcaccgactacagc ctgaccatctccaacctggaacaggaagatatcgccacctacttttgccagcag ggcaacacactgccctacacctttggcggcggaacaaagctggaaatcaccggc agcacctccggcagcggcaagcctggcagcggcgagggcagcaccaagggcgag gtgaagctgcaggaaagcggccctggcctggtggcccccagccagagcctgagc gtgacctgcaccgtgagcggcgtgagcctgcccgactacggcgtgagctggatc cggcagccccccaggaagggcctggaatggctgggcgtgatctggggcagcgag accacctactacaacagcgccctgaagagccggctgaccatcatcaaggacaac agcaagagccaggtgttcctgaagatgaacagcctgcagaccgacgacaccgcc atctactactgcgccaagcactactactacggcggcagctacgccatggactac tggggccagggcaccagcgtgaccgtgagcagc | Sequence encoding scFv |
| **26** | X₁PPX₂P | Hinge |
| | X₁ is glycine, cysteine or arginine | |
| | X₂ is cysteine or threonine | |
| **27** | Glu Pro Lys Ser Cys Asp Lys Thr His Thr Cys Pro Pro Cys Pro | Hinge |
| **28** | Glu Arg Lys Cys Cys Val Glu Cys Pro Pro Cys Pro | Hinge |
| **29** | ELKTPLGDTHTCPRCPEPKSCDTPPPCPRCPEPKSCDTPPPCPRCPEPKSCDTP PPCPRCP | Hinge |
| **30** | Glu Ser Lys Tyr Gly Pro Pro Cys Pro Ser Cys Pro | Hinge |
| **31** | Glu Ser Lys Tyr Gly Pro Pro Cys Pro Pro Cys Pro | Hinge |
| **32** | Tyr Gly Pro Pro Cys Pro Pro Cys Pro | Hinge |
| **33** | Lys Tyr Gly Pro Pro Cys Pro Pro Cys Pro | Hinge |
| **34** | Glu Val Val Val Lys Tyr Gly Pro Pro Cys Pro Pro Cys Pro | Hinge |
| **35** | RASQDISKYLN | CDR L1 |
| **36** | SRLHSGV | CDR L2 |
| **37** | GNTLPYTFG | CDR L3 |
| **38** | DYGVS | CDR H1 |
| **39** | VIWGSETTYYNSALKS | CDR H2 |
| **40** | YAMDYWG | CDR H3 |
| **41** | EVKLQESGPGLVAPSQSLSVTCTVSGVSLPDYGVSWIRQPPRKGLEWLGVIWGS ETTYYNSALKSRLTIIKDNSKSQVFLKMNSLQTDDTAIYYCAKHYYYGGSYAMD YWGQGTSVTVSS | VH |
| **42** | DIQMTQTTSSLSASLGDRVTISCRASQDISKYLNWYQQKPDGTVKLLIYHTSRL HSGVPSRFSGSGSGTDYSLTISNLEQEDIATYFCQQGNTLPYTFGGGTKLEIT | VL |
| **43** | DIQMTQTTSSLSASLGDRVTISCRASQDISKYLNWYQQKPDGTVKLLIYHTSRL HSGVPSRFSGSGSGTDYSLTISNLEQEDIATYFCQQGNTLPYTFGGGTKLEITG STSGSGKPGSGEGSTKGEVKLQESGPGLVAPSQSLSVTCTVSGVSLPDYGVSWI RQPPRKGLEWLGVIWGSETTYYNSALKSRLTIIKDNSKSQVFLKMNSLQTDDTA IYYCAKHYYYGGSYAMDYWGQGTSVTVSS | scFv |
| **44** | KASQNVGTNVA | CDR L1 |
| **45** | SATYRNS | CDR L2 |
| **46** | QQYNRYPYT | CDR L3 |
| **47** | SYWMN | CDR H1 |
| **48** | QIYPGDGDTNYNGKFKG | CDR H2 |
| **49** | KTISSVVDFYFDY | CDR H3 |
| **50** | EVKLQQSGAELVRPGSSVKISCKASGYAFSSYWMNWVKQRPGQGLEWIGQIYPG DGDTNYNGKFKGQATLTADKSSSTAYMQLSGLTSEDSAVYFCARKTISSVVDFY FDYWGQGTTVTVSS | VH |
| **51** | DIELTQSPKFMSTSVGDRVSVTCKASQNVGTNVAWYQQKPGQSPKPLIYSATYR NSGVPDRFTGSGSGTDFTLTITNVQSKDLADYFCQQYNRYPYTSGGGTKLEIKR | VL |
| **52** | GGGGSGGGGSGGGGS | Linker |
| **53** | EVKLQQSGAELVRPGSSVKISCKASGYAFSSYWMNWVKQRPGQGLEWIGQIYPG DGDTNYNGKFKGQATLTADKSSSTAYMQLSGLTSEDSAVYFCARKTISSVVDFY FDYWGQGTTVTVSSGGGGSGGGGSGGGGSDIELTQSPKFMSTSVGDRVSVTCKA SQNVGTNVAWYQQKPGQSPKPLIYSATYRNSGVPDRFTGSGSGTDFTLTITNVQ SKDLADYFCQQYNRYPYTSGGGTKLEIKR | scFv |
| **54** | HYYYGGSYAMDY | CDR H3 |
| **55** | HTSRLHS | CDR L2 |
| **56** | QQGNTLPYT | CDR L3 |
| **57** | ACACGGCCTCGTGTATTACTGT | IGH primer |
| **58** | ACCTGAGGAGACGGTGACC | IGH Primer |

## Claims

1. A first composition comprising one of CD4+ T cells and CD8+ T cells for use with a second composition comprising the other of CD4+ T cells and CD8+ T cells in a method of treating a subject having chronic lymphocytic leukemia (CLL) or small lymphocytic lymphoma (SLL), wherein the method comprises administering to the subject a dose of engineered T cells comprising CD4⁺ and CD8⁺ T cells comprising a chimeric antigen receptor (CAR) that specifically binds to CD19, wherein:
(i) the subject has relapsed following remission after treatment with, become refractory to or failed treatment with and/or is intolerant to ibrutinib and venetoclax;
(ii) the administration comprises administering a plurality of separate compositions, wherein the plurality of separate compositions comprises the first composition comprising one of CD4⁺ T cells and CD8⁺ T cells and the second composition comprising the other of CD4⁺ T cells and CD8⁺ T cells; and
(iii) the dose of engineered T cells comprises a defined ratio of CD4+ cells expressing the CAR to CD8+ cells expressing the CAR, optionally wherein the ratio is between approximately 1:3 and approximately 3:1.

2. A plurality of separate compositions comprising a first composition comprising one of CD4+ T cells and CD8+ T cells and a second composition comprising the other of CD4+ T cells and CD8+ T cells for use in a method of treating subject having chronic lymphocytic leukemia (CLL) or small lymphocytic lymphoma (SLL), wherein the method comprises administering to the subject a dose of engineered T cells comprising CD4⁺ and CD8⁺ T cells comprising a chimeric antigen receptor (CAR) that specifically binds to CD 19, wherein:
(i) the subject has relapsed following remission after treatment with, become refractory to or failed treatment with and/or is intolerant to ibrutinib and venetoclax;
(ii) the administration comprises administering the plurality of separate compositions, wherein the plurality of separate compositions comprises the first composition comprising one of CD4⁺ T cells and CD8⁺ T cells and the second composition comprising the other of CD4⁺ T cells and CD8⁺ T cells; and
(iii) the dose of engineered T cells comprises a defined ratio of CD4+ cells expressing the CAR to CD8+ cells expressing the CAR, optionally wherein the ratio is between approximately 1:3 and approximately 3:1.

3. The first composition for use of claim 1 or the plurality of separate compositions for use of claim 2, wherein:
(i) the dose of engineered T cells is enriched for CD4+ and CD8+ primary human T cells;
(ii) the defined ratio of CD4⁺ cells expressing the CAR to CD8⁺ cells expressing the CAR is or is approximately 1:1; and/or
(iii) the dose of engineered T cells comprises:
(a) at or about 2.5 × 10⁷ total CAR-expressing cells to at or about 1.5 × 10⁸ total CAR-expressing cells;
(b) at or about 2.5 × 10⁷ total CAR-expressing cells to at or about 1.0 × 10⁸ total CAR-expressing cells; and/or
(c) at or about 2.5 × 10⁷ total CAR-expressing cells, at or about 5 × 10⁷ total cells or total CAR-expressing cells, or at or about 1 × 10⁸ total cells or total CAR-expressing cells.

4. The first composition for use of claim 1 or claim 3 or the plurality of separate compositions for use of claim 2 or claim 3, wherein the CAR comprised by the CD4⁺ T cells and/or the CAR comprised by the CD8⁺ T cells comprises a CAR that is the same and/or wherein the CD4⁺ T cells and/or the CD8⁺ T cells are genetically engineered to express a CAR that is the same.

5. The first composition for use of any of claims 1, 3, and 4 or the plurality of separate compositions for use of any of claims 2-4, wherein:
(i) the first composition comprises the CD8⁺ T cells and the second composition comprises the CD4+ T cells, optionally wherein the initiation of the administration of the first composition is carried out prior to the initiation of the administration of the second composition;
(ii) the administration of the first composition and the administration of the second composition are carried out no more than 48 hours apart;
(iii) the administration of the first composition and the administration of the second composition are carried out no more than 36 hours apart, no more than 24 hours apart, no more than 12 hours apart, no more than 6 hours apart, no more than 4 hours apart, no more than 2 hours apart, no more than 1 hour apart or no more than 30 minutes apart;
(iv)the administration of the first composition and the administration of the second composition are carried out on the same day, are carried out between about 0 and about 12 hours apart, between about 0 and about 6 hours apart or between about 0 and about 2 hours apart; or
the initiation of administration of the first composition and the initiation of administration of the second composition are carried out between about 1 minute and about 1 hour apart or between about 5 minutes and about 30 minutes apart; and/or
(v) the first composition and second composition are administered no more than 2 hours, no more than 1 hour, no more than 30 minutes, no more than 15 minutes, no more than 10 minutes or no more than 5 minutes apart.

6. The first composition for use of any of claims 1 and 3-5 or the plurality of separate compositions for use of any of claims 2-5, wherein:
(i) the subject has CLL or is suspected of having CLL or the subject is identified or selected as having CLL, optionally wherein the CLL is a relapsed or refractory CLL; and/or
(ii)
the subject has SLL or is suspected of having SLL or the subject is identified or selected as having SLL, optionally wherein the SLL is a relapsed or refractory SLL.

7. The first composition for use of any of claims 1 and 3-6 or the plurality of separate compositions for use of any of claims 2-6, wherein:
(i) prior to the administration of the dose of engineered T cells, the subject has been treated with one or more prior therapies for CLL or SLL, other than another dose of cells expressing CAR or a lymphodepleting therapy, optionally wherein the one or more prior therapies comprise at least two prior therapies, optionally 3, 4, 5, 6, 7, 8, 9 or more prior therapies;
(ii) at or immediately prior to the time of the administration of the dose of engineered T cells, the subject has relapsed following remission after treatment with, or become refractory to, failed and/or was intolerant to treatment with one or more prior therapies for CLL or SLL;
(iii) at or immediately prior to the time of the administration of the dose of engineered T cells, the subject has relapsed following remission after treatment with, or become refractory to, failed and/or was intolerant to treatment with two or more prior therapies; and/or
(iv) at or immediately prior to the time of the administration of the dose of engineered T cells, the subject has relapsed following remission after treatment with, or become refractory to, failed and/or was intolerant to treatment with three or more prior therapies.

8. The first composition for use of any of claims 1 and 3-7 or the plurality of separate compositions for use of any of claims 2-7, wherein at or prior to the administration of the dose of cells:
(i) the subject is or has been identified as having one or more cytogenetic abnormalities, optionally associated with high-risk CLL or SLL, optionally selected from among: complex karyotype or cytogenetic abnormalities, del 17p, unmutated IGVH gene, and TP53 mutation; and/or
the subject is or has been identified as having high-risk CLL or SLL;
(ii) the subject is or has been identified as having one or more cytogenetic abnormalities, optionally associated with high-risk CLL, optionally selected from among: complex karyotype or cytogenetic abnormalities, del 17p, unmutated IGVH gene, and TP53 mutation; and/or
the subject is or has been identified as having high-risk CLL, optionally wherein the subject has relapsed following remission after treatment with, or become refractory to, failed and/or was intolerant to treatment with two or more prior therapies;
(iii) the subject is or has been identified as having a standard-risk CLL or SLL, optionally wherein the subject has relapsed following remission after treatment with, or become refractory to, failed and/or was intolerant to treatment with three or more prior therapies; and/or
(iv) the subject is or has been identified as being intolerant to an inhibitor of Bruton's tyrosine kinase (BTK) and has received an inhibitor of BTK for a duration of less than at or about 6 months, and/or is ineligible for treatment with an inhibitor of BTK, optionally wherein:
(a) the subject is or has been identified as having high-risk CLL or SLL, and at or immediately prior to the time of the administration of the dose of cells, the subject has relapsed following remission after treatment with, or become refractory to, failed and/or was intolerant to treatment with one or more prior therapies other than the inhibitor of BTK; or
(b) the subject is or has been identified as having a standard-risk CLL or SLL, and at or immediately prior to the time of the administration of the dose of cells, the subject has relapsed following remission after treatment with, or become refractory to, failed and/or was intolerant to treatment with two or more prior therapies other than the inhibitor of BTK.

9. The first composition for use of any of claims 1 and 3-8 or the plurality of separate compositions for use of any of claims 2-8, wherein:
(i) the subject is or has been identified as having an ECOG status of 0 or 1; and/or
the subject does not have an ECOG status of >1;
(ii) at or immediately prior to the administration of the dose of engineered cells the subject does not have a Richter's transformation of CLL or SLL; and/or
(iii) the subject is an adult and/or is over at or about 50, 60, or 70 years of age.

10. The first composition for use of any of claims 1 and 3-9 or the plurality of separate compositions for use of any of claims 2-9, wherein:
(i) the engineered T cells are primary T cells obtained from a subject;
(ii) the engineered T cells are autologous to the subject; and/or
(iii) the dose of engineered cells are viable cells.

11. The first composition for use of any of claims 1 and 3-10 or the plurality of separate compositions for use of any of claims 2-10, wherein:
(i) the method further comprises, prior to the administration of the dose of engineered T cells, administering a lymphodepleting therapy to the subject and/or wherein the subject has been preconditioned with a lymphodepleting therapy, optionally wherein:
(a) the lymphodepleting therapy comprises the administration of fludarabine and/or cyclophosphamide;
(b) the lymphodepleting therapy comprises administration of cyclophosphamide at about 200-400 mg/m², optionally at or about 300 mg/m², inclusive, and/or fludarabine at about 20-40 mg/m², optionally 30 mg/m², daily for 2-4 days, optionally for 3 days; and/or
(c) the lymphodepleting therapy comprises administration of cyclophosphamide at or about 300 mg/m² and fludarabine at about 30 mg/m²daily for 3 days, optionally wherein the dose of cells is administered at least at or about 2-7 days after the lymphodepleting therapy or at least at or about 2-7 days after the initiation of the lymphodepleting therapy; and/or
(ii) the administration of the dose of engineered T cells and/or the lymphodepleting therapy is carried out via outpatient delivery.

12. The first composition for use of any of claims 1 and 3-11 or the plurality of separate compositions for use of any of claims 2-11, wherein, of a plurality of subjects treated according to the method:
(i) the response in at least 50%, at least 60%, at least 70%, at least 80%, at least 90% of the subjects treated is an objective response rate; and/or the response in at least 35%, at least 40 %, at least 50%, at least 60% or at least 70% of subjects treated is complete remission (CR), optionally wherein the duration of the response until progression is durable for greater than 3 months or greater than 6 months;
(ii) greater than 50%, greater than 60%, or greater than 70% had undetectable minimal residual disease (MRD) for at least one month, at least two months, at least three months or at least 6 months after administering the dose of engineered T cells;
(iii) no more than 10% of subjects exhibit a cytokine release syndrome (CRS) higher than grade 2; and/or
(iv) no more than 10%, no more than 20%, no more than 30% or no more than 40% of the subjects exhibit neurotoxicity higher than grade 2,
optionally wherein the plurality of subjects treated according to the method comprises a plurality of subjects that have relapsed following remission after treatment with, become refractory to, failed treatment with and/or is intolerant to ibrutinib and venetoclax.

13. The first composition for use of any of claims 1 and 3-12 or the plurality of separate compositions for use of any of claims 2-12, wherein:
the CAR comprises an extracellular antigen-binding domain specific for CD 19, a transmembrane domain, a cytoplasmic signaling domain derived from a costimulatory molecule, which optionally is a 4-1 BB, and a cytoplasmic signaling domain derived from a primary signaling ITAM-containing molecule, which optionally is a CD3zeta;
the CAR comprises, in order, an extracellular antigen-binding domain specific for CD19, a transmembrane domain, a cytoplasmic signaling domain derived from a costimulatory molecule, and a cytoplasmic signaling domain derived from a primary signaling ITAM-containing molecule.

14. The first composition for use of claim 13 or the plurality of separate compositions for use of claim 13, wherein the antigen-binding domain is an scFv, optionally wherein:
the scFv comprises a CDRL1 sequence of RASQDISKYLN (SEQ ID NO: 35), a CDRL2 sequence of SRLHSGV (SEQ ID NO: 36), and/or a CDRL3 sequence of GNTLPYTFG (SEQ ID NO: 37) and/or a CDRH1 sequence of DYGVS (SEQ ID NO: 38), a CDRH2 sequence of VIWGSETTYYNSALKS (SEQ ID NO: 39), and/or a CDRH3 sequence of YAMDYWG (SEQ ID NO: 40);
the scFv comprises a variable heavy chain region of FMC63 and a variable light chain region of FMC63 and/or a CDRL1 sequence of FMC63, a CDRL2 sequence of FMC63, a CDRL3 sequence of FMC63, a CDRH1 sequence of FMC63, a CDRH2 sequence of FMC63, and a CDRH3 sequence of FMC63 or binds to the same epitope as or competes for binding with any of the foregoing;
the scFv comprises a VH set forth in SEQ ID NO:41 and a VL set forth in SEQ ID NO: 42, optionally wherein the VH and VL are separated by a flexible linker, optionally wherein the flexible linker is or comprises the sequence set forth in SEQ ID NO:24; and/or
the scFv is or comprises the sequence set forth in SEQ ID NO:43.

15. The first composition for use of claim 13 or 14 or the plurality of separate compositions for use of claim 13 or claim 14, wherein:
(i) the costimulatory signaling region is a signaling domain of CD28 or 4-1BB;
(ii) the costimulatory signaling region is a signaling domain of 4-1BB;
(iii) the costimulatory domain comprises SEQ ID NO: 12 or a variant thereof having at least 85%, 86%, 87%, 88%, 89%, 90%, 91%, 92%, 93%, 94%, 95%, 96%, 97%, 98%, 99% or more sequence identity thereto;
(iv) the primary signaling domain is a CD3zeta signaling domain;
(v) the primary signaling domain comprises SEQ ID NO: 13 or 14 or 15 having at least 85%, 86%, 87%, 88%, 89%, 90%, 91%, 92%, 93%, 94%, 95%, 96%, 97%, 98%, 99% or more sequence identity thereto; and/or
(vi) the CAR further comprises a spacer between the transmembrane domain and the scFv, optionally wherein:
(a) the spacer is a polypeptide spacer that comprises or consists of all or a portion of an immunoglobulin hinge or a modified version thereof, optionally an IgG4 hinge, or a modified version thereof;
(b) the spacer is about 15 amino acids or less, and does not comprise a CD28 extracellular region or a CD8 extracellular region; and/or
(c) spacer is at or about 12 amino acids in length.

## Patentansprüche

1. Erste Zusammensetzung, umfassend entweder CD4+-T-Zellen oder CD8+-T-Zellen, zur Verwendung mit einer zweiten Zusammensetzung, die die jeweils anderen T-Zellen umfasst, bei einem Verfahren zur Behandlung eines Individuums, das an chronischer lymphatischer Leukämie (CLL) oder kleinzelligem lymphozytischem Lymphom (Small Lymphocytic Lymphoma, SLL) leidet, wobei das Verfahren Verabreichen einer Dosis konstruierter T-Zellen, die einen chimären Antigenrezeptor (CAR), der an CD19 spezifisch bindet, umfassende CD4⁺- und CD8⁺-T-Zellen umfassen, an das Individuum umfasst, wobei:
(i) das Individuum im Anschluss an eine Remission nach Behandlung mit Ibrutinib und Venetoclax einen Rückfall erlitten hat, gegen die Behandlung mit Ibrutinib und Venetoclax refraktär geworden ist bzw. darauf nicht angesprochen hat und/oder Ibrutinib und Venetoclax nicht verträgt;
(ii) die Verabreichung Verabreichen mehrerer getrennter Zusammensetzungen umfasst, wobei die mehreren getrennten Zusammensetzungen die erste Zusammensetzung, die entweder CD4⁺-T-Zellen oder CD8⁺-T-Zellen umfasst, und die zweite Zusammensetzung, die die jeweils anderen T-Zellen umfasst, umfassen; und
(iii) die Dosis konstruierter T-Zellen ein definiertes Verhältnis von den CAR exprimierenden CD4+-Zellen zu den CAR exprimierenden CD8+-Zellen umfasst, gegebenenfalls wobei das Verhältnis zwischen ungefähr 1:3 und ungefähr 3:1 liegt.

2. Mehrere getrennte Zusammensetzungen, umfassend eine erste Zusammensetzung, die entweder CD4+-T-Zellen oder CD8+-T-Zellen umfasst, und eine zweite Zusammensetzung, die die jeweils anderen T-Zellen umfasst, zur Verwendung bei einem Verfahren zur Behandlung eines Individuums, das an chronischer lymphatischer Leukämie (CLL) oder kleinzelligem lymphozytischem Lymphom (SLL) leidet, wobei das Verfahren Verabreichen einer Dosis konstruierter T-Zellen, die einen chimären Antigenrezeptor (CAR), der an CD19 spezifisch bindet, umfassende CD4⁺- und CD8⁺-T-Zellen umfassen, an das Individuum umfasst, wobei:
(i) das Individuum im Anschluss an eine Remission nach Behandlung mit Ibrutinib und Venetoclax einen Rückfall erlitten hat, gegen die Behandlung mit Ibrutinib und Venetoclax refraktär geworden ist bzw. darauf nicht angesprochen hat und/oder Ibrutinib und Venetoclax nicht verträgt;
(ii) die Verabreichung Verabreichen der mehreren getrennten Zusammensetzungen umfasst, wobei die mehreren getrennten Zusammensetzungen die erste Zusammensetzung, die entweder CD4⁺-T-Zellen oder CD8⁺-T-Zellen umfasst, und die zweite Zusammensetzung, die die jeweils anderen T-Zellen umfasst, umfassen; und
(iii) die Dosis konstruierter T-Zellen ein definiertes Verhältnis von den CAR exprimierenden CD4+-Zellen zu den CAR exprimierenden CD8+-Zellen umfasst, gegebenenfalls wobei das Verhältnis zwischen ungefähr 1:3 und ungefähr 3:1 liegt.

3. Erste Zusammensetzung zur Verwendung nach Anspruch 1 oder mehrere getrennte Zusammensetzungen zur Verwendung nach Anspruch 2, wobei:
(i) die Dosis konstruierter T-Zellen auf primäre menschliche CD4+- und CD8+-T-Zellen angereichert ist;
(ii) das definierte Verhältnis von den CAR exprimierenden CD4⁺-Zellen zu den CAR exprimierenden CD8⁺-Zellen bei oder bei ungefähr 1:1 liegt; und/oder
(iii) die Dosis konstruierter T-Zellen Folgendes umfasst:
(a) CAR exprimierende Zellen in einer Gesamtzahl von bzw. von etwa 2,5 × 10⁷ bis bzw. bis etwa 1,5 × 10⁸;
(b) CAR exprimierende Zellen in einer Gesamtzahl von bzw. von etwa 2,5 × 10⁷ bis bzw. bis etwa 1,0 × 10⁸; und/oder
(c) CAR exprimierende Zellen in einer Gesamtzahl von bzw. von etwa 2,5 × 10⁷, Zellen oder CAR exprimierende Zellen jeweils in einer Gesamtzahl von bzw. von etwa 5 × 10⁷ oder Zellen oder CAR exprimierende Zellen jeweils in einer Gesamtzahl von bzw. von etwa 1 × 10⁸.

4. Erste Zusammensetzung zur Verwendung nach Anspruch 1 oder Anspruch 3 oder mehrere getrennte Zusammensetzungen zur Verwendung nach Anspruch 2 oder Anspruch 3, wobei der auf den CD4⁺-T-Zellen enthaltene CAR und/oder der auf den CD8⁺-T-Zellen enthaltene CAR einen CAR umfasst, der identisch ist, und/oder wobei die CD4⁺-T-Zellen und/oder die CD8⁺-T-Zellen gentechnisch so konstruiert sind, dass sie einen CAR exprimieren, der identisch ist.

5. Erste Zusammensetzung zur Verwendung nach einem der Ansprüche 1, 3 und 4 oder mehrere getrennte Zusammensetzungen zur Verwendung nach einem der Ansprüche 2-4, wobei:
(i) die erste Zusammensetzung die CD8⁺-T-Zellen und die zweite Zusammensetzung die CD4+-T-Zellen umfasst, gegebenenfalls wobei die Einleitung der Verabreichung der ersten Zusammensetzung vor der Einleitung der Verabreichung der zweiten Zusammensetzung erfolgt;
(ii) die Verabreichung der ersten Zusammensetzung und die Verabreichung der zweiten Zusammensetzung in einem Abstand von nicht mehr als 48 Stunden durchgeführt werden;
(iii) die Verabreichung der ersten Zusammensetzung und die Verabreichung der zweiten Zusammensetzung in einem Abstand von nicht mehr als 36 Stunden, nicht mehr als 24 Stunden, nicht mehr als 12 Stunden, nicht mehr als 6 Stunden, nicht mehr als 4 Stunden, nicht mehr als 2 Stunden, nicht mehr als 1 Stunde oder nicht mehr als 30 Minuten durchgeführt werden;
(iv) die Verabreichung der ersten Zusammensetzung und die Verabreichung der zweiten Zusammensetzung am gleichen Tag, in einem Abstand zwischen etwa 0 und etwa 12 Stunden, zwischen etwa 0 und etwa 6 Stunden oder zwischen etwa 0 und etwa 2 Stunden durchgeführt werden; oder
die Einleitung der Verabreichung der ersten Zusammensetzung und die Einleitung der Verabreichung der zweiten Zusammensetzung in einem Abstand zwischen etwa 1 Minute und etwa 1 Stunde oder zwischen etwa 5 Minuten und etwa 30 Minuten erfolgen; und/oder
(v) die erste Zusammensetzung und die zweite Zusammensetzung in einem Abstand von nicht mehr als 2 Stunden, nicht mehr als 1 Stunde, nicht mehr als 30 Minuten, nicht mehr als 15 Minuten, nicht mehr als 10 Minuten oder nicht mehr als 5 Minuten verabreicht werden.

6. Erste Zusammensetzung zur Verwendung nach einem der Ansprüche 1 und 3-5 oder mehrere getrennte Zusammensetzungen zur Verwendung nach einem der Ansprüche 2-5, wobei:
(i) das Individuum an CLL leidet oder vermutlich an CLL leidet oder das Individuum als an CLL leidend identifiziert oder ausgewählt wird, gegebenenfalls wobei es sich bei der CLL um eine rezidivierte oder refraktäre CLL handelt; und/oder
(ii) das Individuum an SLL leidet oder vermutlich an SLL leidet oder das Individuum als an SLL leidend identifiziert oder ausgewählt ist, gegebenenfalls wobei es sich bei der SLL um eine rezidivierte oder refraktäre SLL handelt.

7. Erste Zusammensetzung zur Verwendung nach einem der Ansprüche 1 und 3-6 oder mehrere getrennte Zusammensetzungen zur Verwendung nach einem der Ansprüche 2-6, wobei:
(i) vor der Verabreichung der Dosis konstruierter T-Zellen das Individuum mit einer oder mehreren vorherigen Therapien gegen CLL oder SLL behandelt wurde, bei denen es sich weder um eine weitere Dosis CAR exprimierender CAR noch eine lymphodepletierende Therapie handelt, gegebenenfalls wobei die eine oder mehreren vorherigen Therapien wenigstens zwei vorherige Therapien, gegebenenfalls 3, 4, 5, 6, 7, 8, 9 oder mehr vorherige Therapien umfassen;
(ii) zu oder unmittelbar vor dem Zeitpunkt der Verabreichung der Dosis konstruierter T-Zellen das Individuum im Anschluss an eine Remission nach Behandlung mit einer oder mehreren vorherigen Therapien gegen CLL oder SLL einen Rückfall erlitten hat, gegen die Behandlung mit einer oder mehreren vorherigen Therapien gegen CLL oder SLL refraktär geworden ist, darauf nicht angesprochen hat und/oder sie nicht vertragen hat;
(iii) zu oder unmittelbar vor dem Zeitpunkt der Verabreichung der Dosis konstruierter T-Zellen das Individuum im Anschluss an eine Remission nach Behandlung mit zwei oder mehr vorherigen Therapien einen Rückfall erlitten hat, gegen die Behandlung mit zwei oder mehr vorherigen Therapien refraktär geworden ist, darauf nicht angesprochen hat und/oder sie nicht vertragen hat; und/oder
(iv) zu oder unmittelbar vor dem Zeitpunkt der Verabreichung der Dosis konstruierter T-Zellen das Individuum im Anschluss an eine Remission nach Behandlung mit drei oder mehr vorherigen Therapien einen Rückfall erlitten hat, gegen die Behandlung mit drei oder mehr vorherigen Therapien refraktär geworden ist, darauf nicht angesprochen hat und/oder sie nicht vertragen hat.

8. Erste Zusammensetzung zur Verwendung nach einem der Ansprüche 1 und 3-7 oder mehrere getrennte Zusammensetzungen zur Verwendung nach einem der Ansprüche 2-7, wobei bei oder vor der Verabreichung der Dosis Zellen:
(i) bei dem Individuum das Vorliegen einer oder mehrerer zytogenetischer Anomalien, gegebenenfalls in Zusammenhang mit Hochrisiko-CLL oder -SLL, identifiziert wird oder wurde, die gegebenenfalls ausgewählt sind unter: komplexen Karyotyp- oder zytogenetischen Anomalien, del 17p, unmutiertem IGVH-Gen und TP53-Mutation; und/oder
bei dem Individuum das Vorliegen von Hochrisiko-CLL oder -SLL identifiziert wird oder wurde;
(ii) bei dem Individuum das Vorliegen einer oder mehrerer zytogenetischer Anomalien, gegebenenfalls in Zusammenhang mit Hochrisiko-CLL, identifiziert wird oder wurde, die gegebenenfalls ausgewählt sind unter: komplexen Karyotyp- oder zytogenetischen Anomalien, del 17p, unmutiertem IGVH-Gen und TP53-Mutation; und/oder bei dem Individuum das Vorliegen von Hochrisiko-CLL identifiziert wird oder wurde, gegebenenfalls wobei das Individuum im Anschluss an eine Remission nach Behandlung mit zwei oder mehr vorherigen Therapien einen Rückfall erlitten hat, gegen die Behandlung mit zwei oder mehr vorherigen Therapien refraktär geworden ist, darauf nicht angesprochen hat und/oder sie nicht vertragen hat;
(iii) bei dem Individuum das Vorliegen einer Standardrisiko-CLL oder -SLL identifiziert wird oder wurde, gegebenenfalls wobei das Individuum im Anschluss an eine Remission nach Behandlung mit drei oder mehr vorherigen Therapien einen Rückfall erlitten hat, gegen die Behandlung mit drei oder mehr vorherigen Therapien refraktär geworden ist, darauf nicht angesprochen hat und/oder sie nicht vertragen hat; und/oder
(iv) bei dem Individuum das Vorliegen einer Unverträglichkeit gegenüber einem Inhibitor von Bruton-Tyrosinkinase (BTK) identifiziert wird oder wurde und das Individuum einen BTK-Inhibitor weniger als oder als etwa 6 Monate lang erhalten hat und/oder für eine Behandlung mit einem BTK-Inhibitor nicht in Frage kommt, gegebenenfalls wobei:
(a) bei dem Individuum das Vorliegen von Hochrisiko-CLL oder -SLL identifiziert wird oder wurde und zu oder unmittelbar vor dem Zeitpunkt der Verabreichung der Dosis Zellen das Individuum im Anschluss an eine Remission nach Behandlung mit einer oder mehreren vorherigen Therapien, bei denen es sich nicht um den BTK-Inhibitor handelt, einen Rückfall erlitten hat, gegen die Behandlung mit einer oder mehreren vorherigen Therapien, bei denen es sich nicht um den BTK-Inhibitor handelt, refraktär geworden ist, darauf nicht angesprochen hat und/oder sie nicht vertragen hat; oder
(b) bei dem Individuum das Vorliegen einer Standardrisiko-CLL oder -SLL identifiziert wird oder wurde und zu oder unmittelbar vor dem Zeitpunkt der Verabreichung der Dosis Zellen das Individuum im Anschluss an eine Remission nach Behandlung mit zwei oder mehr vorherigen Therapien, bei denen es sich nicht um den BTK-Inhibitor handelt, einen Rückfall erlitten hat, gegen die Behandlung mit zwei oder mehr vorherigen Therapien, bei denen es sich nicht um den BTK-Inhibitor handelt, refraktär geworden ist, darauf nicht angesprochen hat und/oder sie nicht vertragen hat.

9. Erste Zusammensetzung zur Verwendung nach einem der Ansprüche 1 und 3-8 oder mehrere getrennte Zusammensetzungen zur Verwendung nach einem der Ansprüche 2-8, wobei:
(i) bei dem Individuum das Vorliegen eines ECOG-Status von 0 oder 1 identifiziert wird oder wurde; und/oder bei dem Individuum kein ECOG-Status >1 vorliegt;
(ii) zu oder unmittelbar vor der Verabreichung der Dosis konstruierter Zellen bei dem Individuum keine Richter-Transformation von CLL oder SLL vorliegt; und/oder
(iii) es sich bei dem Individuum um eine erwachsene Person handelt und/oder das Individuum über oder über etwa 50, 60 oder 70 Jahre alt ist.

10. Erste Zusammensetzung zur Verwendung nach einem der Ansprüche 1 und 3-9 oder mehrere getrennte Zusammensetzungen zur Verwendung nach einem der Ansprüche 2-9, wobei:
(i) es sich bei den konstruierten T-Zellen um von einem Individuum erhaltene primäre T-Zellen handelt;
(ii) die konstruierten T-Zellen autolog für das Individuum sind; und/oder
(iii) es sich bei der Dosis konstruierter Zellen um lebensfähige Zellen handelt.

11. Erste Zusammensetzung zur Verwendung nach einem der Ansprüche 1 und 3-10 oder mehrere getrennte Zusammensetzungen zur Verwendung nach einem der Ansprüche 2-10, wobei:
(i) das Verfahren ferner vor der Verabreichung der Dosis konstruierter T-Zellen Verabreichen einer lymphodepletierenden Therapie an das Individuum umfasst und/oder wobei das Individuum mit einer lymphodepletierenden Therapie vorkonditioniert wurde, gegebenenfalls wobei:
(a) die lymphodepletierende Therapie die Verabreichung von Fludarabin und/oder Cyclophosphamid umfasst;
(b) die lymphodepletierende Therapie Verabreichung von etwa 200-400 mg/m², gegebenenfalls von oder von etwa 300 mg/m², einschließlich, Cyclophosphamid und/oder von etwa 20-40 mg/m², gegebenenfalls 30 mg/m² Fludarabin täglich über 2-4 Tage, gegebenenfalls über 3 Tage umfasst; und/oder
(c) die lymphodepletierende Therapie Verabreichung von oder von etwa 300 mg/m² Cyclophosphamid und/oder von etwa 30 mg/m² Fludarabin täglich über 3 Tage umfasst, gegebenenfalls wobei die Dosis Zellen wenigstens oder wenigstens etwa 2-7 Tage nach der lymphodepletierenden Therapie oder wenigstens oder wenigstens etwa 2-7 Tage nach der Einleitung der lymphodepletierenden Therapie verabreicht wird; und/oder
(ii) die Verabreichung der Dosis konstruierter T-Zellen und/oder der lymphodepletierenden Therapie ambulant erfolgt.

12. Erste Zusammensetzung zur Verwendung nach einem der Ansprüche 1 und 3-11 oder mehrere getrennte Zusammensetzungen zur Verwendung nach einem der Ansprüche 2-11, wobei bei mehreren gemäß dem Verfahren behandelten Individuen:
(i) das Ansprechen bei wenigstens 50 %, wenigstens 60 %, wenigstens 70 %, wenigstens 80 %, wenigstens 90 % der behandelten Individuen eine Zielansprechrate darstellt; und/oder das Ansprechen bei wenigstens 35 %, wenigstens 40 %, wenigstens 50 %, wenigstens 60 % oder wenigstens 70 % behandelter Individuen vollständige Remission (Complete Remission, CR) bedeutet, gegebenenfalls wobei der Ansprechzeitraum bis zum Fortschreiten länger als 3 Monate oder länger als 6 Monate beständig ist;
(ii) mehr als 50 %, mehr als 60 % oder mehr als 70 % nicht nachweisbare minimale Restkrankheit (Minimal Residual Disease, MRD) wenigstens einen Monat, wenigstens zwei Monate, wenigstens drei Monate oder wenigstens 6 Monate nach Verabreichen der Dosis konstruierter T-Zellen aufwiesen;
(iii) nicht mehr als 10 % der Individuen ein CRS (Cytokine Release Syndrome) höher als Grad 2 zeigen; und/oder
(iv) nicht mehr als 10 %, nicht mehr als 20 %, nicht mehr als 30 % oder nicht mehr als 40 % der Individuen Neurotoxizität höher als Grad 2 zeigen,
gegebenenfalls wobei die mehreren gemäß dem Verfahren behandelten Individuen mehrere Individuen umfassen, die im Anschluss an eine Remission nach Behandlung mit Ibrutinib und Venetoclax einen Rückfall erlitten haben, gegen die Behandlung mit Ibrutinib und Venetoclax refraktär geworden sind bzw. darauf nicht angesprochen haben und/oder Ibrutinib und Venetoclax nicht vertragen.

13. Erste Zusammensetzung zur Verwendung nach einem der Ansprüche 1 und 3-12 oder mehrere getrennte Zusammensetzungen zur Verwendung nach einem der Ansprüche 2-12, wobei:
der CAR eine extrazelluläre antigenbindende Domäne, die für CD19 spezifisch ist, eine Transmembrandomäne, eine cytoplasmatische Signalgebungsdomäne, die von einem Costimulatormolekül stammt, bei dem es sich gegebenenfalls um ein 4-1 BB handelt, und eine cytoplasmatische Signalgebungsdomäne, die von einem primären signalgebenden ITAM enthaltenden Molekül stammt, bei dem es sich gegebenenfalls um ein CD3zeta handelt, umfasst;
der CAR der Reihe nach eine extrazelluläre antigenbindende Domäne, die für CD19 spezifisch ist, eine Transmembrandomäne, eine cytoplasmatische Signalgebungsdomäne, die von einem Costimulatormolekül stammt, und eine cytoplasmatische Signalgebungsdomäne, die von einem primären signalgebenden ITAM enthaltenden Molekül stammt, umfasst.

14. Erste Zusammensetzung zur Verwendung nach Anspruch 13 oder mehrere getrennte Zusammensetzungen zur Verwendung nach Anspruch 13, wobei es sich bei der antigenbindenden Domäne um ein scFv handelt, gegebenenfalls wobei:
das scFv eine CDRL1-Sequenz RASQDISKYLN (SEQ ID NO: 35), eine CDRL2-Sequenz SRLHSGV (SEQ ID NO: 36) und/oder eine CDRL3-Sequenz GNTLPYTFG (SEQ ID NO: 37) und/oder eine CDRH1-Sequenz DYGVS (SEQ ID NO: 38), eine CDRH2-Sequenz VIWGSETTYYNSALKS (SEQ ID NO: 39) und/oder eine CDRH3-Sequenz YAMDYWG (SEQ ID NO: 40) umfasst;
das scFv eine variable Schwerkettenregion von FMC63 und eine variable Leichtkettenregion von FMC63 und/oder eine CDRL1-Sequenz von FMC63, eine CDRL2-Sequenz von FMC63, eine CDRL3-Sequenz von FMC63, eine CDRH1-Sequenz von FMC63, eine CDRH2-Sequenz von FMC63 und eine CDRH3-Sequenz von FMC63 umfasst oder an das gleiche Epitop bindet wie eines oder um die Bindung mit einem der vorstehenden konkurriert;
das scFv eine VH gemäß SEQ ID NO:41 und eine VL gemäß SEQ ID NO: 42 umfasst, gegebenenfalls wobei die VH und die VL durch einen flexiblen Linker getrennt sind, gegebenenfalls wobei der flexible Linker die Sequenz gemäß SEQ ID NO:24 ist oder umfasst; und/oder
das scFv die Sequenz gemäß SEQ ID NO:43 ist oder umfasst.

15. Erste Zusammensetzung zur Verwendung nach Anspruch 13 oder 14 oder mehrere getrennte Zusammensetzungen zur Verwendung nach Anspruch 13 oder Anspruch 14, wobei:
(i) es sich bei der Costimulator-Signalgebungsregion um eine Signalgebungsdomäne von CD28 oder 4-1BB handelt;
(ii) es sich bei der Costimulator-Signalgebungsregion um eine Signalgebungsdomäne von 4-1BB handelt;
(iii) die Costimulator-Domäne SEQ ID NO: 12 oder eine Variante davon mit einer Sequenzidentität von wenigstens 85 %, 86 %, 87 %, 88 %, 89 %, 90 %, 91 %, 92 %, 93 %, 94 %, 95 %, 96 %, 97 %, 98 %, 99 % oder mehr damit umfasst;
(iv) es sich bei der primären Signalgebungsdomäne um eine CD3zeta-Signalgebungsdomäne handelt;
(v) die primäre Signalgebungsdomäne SEQ ID NO: 13 oder 14 oder 15 mit einer Sequenzidentität von wenigstens 85 %, 86 %, 87 %, 88 %, 89 %, 90 %, 91 %, 92 %, 93 %, 94 %, 95 %, 96 %, 97 %, 98 %, 99 % oder mehr damit umfasst; und/oder
(vi) der CAR ferner einen Spacer zwischen der Transmembrandomäne und dem scFv umfasst, gegebenenfalls wobei:
(a) es sich bei dem Spacer um einen Polypeptid-Spacer handelt, der eine Immunglobulin-Hinge oder eine modifizierte Version davon, gegebenenfalls eine IgG4-Hinge oder eine modifizierte Version davon, ganz oder teilweise umfasst;
(b) der Spacer etwa 15 Aminosäuren lang oder kürzer ist und weder eine extrazelluläre CD28-Region noch eine extrazelluläre CD8-Region umfasst; und/oder
(c) Spacer eine Länge von oder von etwa 12 Aminosäuren aufweist.

## Revendications

1. Première composition comprenant l'un parmi des lymphocytes T CD4+ et des lymphocytes T CD8+ pour une utilisation avec une seconde composition comprenant l'autre parmi des lymphocytes T CD4+ et des lymphocytes T CD8+ dans un procédé de traitement d'un sujet atteint de leucémie lymphoïde chronique (LLC) ou de petit lymphome lymphocytaire (SLL), le procédé comprenant l'administration au sujet d'une dose de lymphocytes T modifiés comprenant des lymphocytes T CD4+ et CD8+ comprenant un récepteur antigénique chimérique (CAR) qui se lie spécifiquement à CD19, dans laquelle :
(i) le sujet a rechuté à la suite d'une rémission après un traitement avec, est devenu réfractaire à ou a échoué au traitement avec et/ou est intolérant à l'ibrutinib et au vénétoclax ;
(ii) l'administration comprend l'administration d'une pluralité de compositions distinctes, la pluralité de compositions distinctes comprenant la première composition comprenant l'un parmi des lymphocytes T CD4+ et des lymphocytes T CD8+ et la seconde composition comprenant l'autre parmi des lymphocytes T CD4+ et des lymphocytes T CD8+ ; et
(iii) la dose de lymphocytes T modifiés comprend un rapport défini de cellules CD4+ exprimant le CAR sur les cellules CD8+ exprimant le CAR, le rapport étant éventuellement compris entre environ 1:3 et environ 3:1.

2. Pluralité de compositions distinctes comprenant une première composition comprenant l'un parmi des lymphocytes T CD4+ et des lymphocytes T CD8+ et une seconde composition comprenant l'autre parmi des lymphocytes T CD4+ et des lymphocytes T CD8+ pour une utilisation dans un procédé de traitement d'un sujet atteint de leucémie lymphoïde chronique (LLC) ou d'un petit lymphome lymphocytaire (SLL), le procédé comprenant l'administration au sujet d'une dose de lymphocytes T modifiés comprenant des lymphocytes T CD4+ et CD8+ comprenant un récepteur antigénique chimérique (CAR) qui se lie spécifiquement à CD19, dans lesquelles :
(i) le sujet a récidivé après une rémission après le traitement par, est devenu réfractaire à ou a échoué au traitement par et/ou est intolérant à l'ibrutinib et au vénétoclax ;
(ii) l'administration comprend l'administration de la pluralité de compositions distinctes, la pluralité de compositions distinctes comprenant la première composition comprenant l'un parmi des lymphocytes T CD4+ et des lymphocytes T CD8+ et la seconde composition comprenant l'autre parmi des lymphocytes T CD4+ et des lymphocytes T CD8+ ; et
(iii) la dose de lymphocytes T modifiés comprend un rapport défini de cellules CD4+ exprimant le CAR sur les cellules CD8+ exprimant le CAR, le rapport étant éventuellement compris entre environ 1:3 et environ 3:1.

3. Première composition pour une utilisation selon la revendication 1 ou pluralité de compositions distinctes pour une utilisation selon la revendication 2, dans laquelle/lesquelles :
(i) la dose de lymphocytes T modifiés est enrichie en lymphocytes T humains primaires CD4+ et CD8+ ;
(ii) le rapport défini entre les cellules CD4+ exprimant le CAR et les cellules CD8+ exprimant le CAR est ou est d'environ 1:1 ; et/ou
(iii) la dose de lymphocytes T modifiés comprend :
(a) une quantité de ou d'environ 2,5 × 10⁷ cellules exprimant CAR au total jusqu'à une quantité de ou d'environ 1,5 × 10⁸ cellules au total exprimant CAR ;
(b) une quantité de ou d'environ 2,5 × 10⁷ cellules exprimant CAR au total jusqu'à une quantité de ou d'environ 1,0 × 10⁸ de cellules au total exprimant CAR ; et/ou
(c) une quantité de ou d'environ 2,5 × 10⁷ cellules exprimant CAR au total, une quantité de ou d'environ 5 × 10⁷ cellules au total exprimant CAR, ou une quantité de ou d'environ environ 1 × 10⁸ cellules au total exprimant CAR.

4. Première composition pour une utilisation selon la revendication 1 ou la revendication 3 ou pluralité de compositions distinctes pour une utilisation selon la revendication 2 ou la revendication 3, dans laquelle/lesquelles le CAR compris par les lymphocytes T CD4+ et/ou le CAR compris par les lymphocytes T CD8+ comprend un CAR qui est identique et/ou dans laquelle/lesquelles les lymphocytes T CD4+ et/ou les lymphocytes T CD8+ sont génétiquement modifiés pour exprimer un CAR qui est identique.

5. Première composition pour une utilisation selon l'une quelconque des revendications 1, 3 et 4 ou pluralité de compositions distinctes pour une utilisation selon l'une quelconque des revendications 2 à 4, dans laquelle :
(i) la première composition comprend les lymphocytes T CD8+ et la seconde composition comprend les lymphocytes T CD4+, éventuellement le début de l'administration de la première composition étant effectué avant le début de l'administration de la seconde composition ;
(ii) l'administration de la première composition et l'administration de la seconde composition sont réalisées à un intervalle maximum de 48 heures ;
(iii) l'administration de la première composition et l'administration de la seconde composition sont effectuées à pas plus de 36 heures d'intervalle, à pas plus de 24 heures d'intervalle, à pas plus de 12 heures d'intervalle, à pas plus de 6 heures d'intervalle, à pas plus de 4 heures d'intervalle, à pas plus de 2 heures d'intervalle, à pas plus d'une heure d'intervalle ou à pas plus de 30 minutes d'intervalle ;
(iv) l'administration de la première composition et l'administration de la deuxième composition sont effectuées le même jour, sont effectuées entre environ 0 et environ 12 heures d'intervalle, entre environ 0 et environ 6 heures d'intervalle ou entre environ 0 et environ 2 heures d'intervalle ; ou
le début de l'administration de la première composition et le début de l'administration de la deuxième composition sont effectués entre environ 1 minute et environ 1 heure d'intervalle ou entre environ 5 minutes et environ 30 minutes d'intervalle ; et/ou
(v) la première composition et la seconde composition sont administrées à pas plus de 2 heures, pas plus de 1 heure, pas plus de 30 minutes, pas plus de 15 minutes, pas plus de 10 minutes ou pas plus de 5 minutes d'intervalle.

6. Première composition pour une utilisation selon l'une quelconque des revendications 1 et 3 à 5 ou pluralité de compositions distinctes pour une utilisation selon l'une quelconque des revendications 2 à 5, dans laquelle/lesquelles :
(i) le sujet souffre de LLC ou est suspecté de souffrir de LLC ou le sujet est identifié ou sélectionné comme atteint de LLC, éventuellement la LLC étant une LLC récidivante ou réfractaire ; et/ou
(ii) le sujet souffre de SLL ou est suspecté de souffrir de SLL ou le sujet est identifié ou sélectionné comme souffrant de SLL, éventuellement dans lequel la SLL est une SLL en rechute ou réfractaire.

7. Première composition pour une utilisation selon l'une quelconque des revendications 1 et 3 à 6 ou pluralité de compositions distinctes pour une utilisation selon l'une quelconque des revendications 2 à 6, dans laquelle/lesquelles :
(i) avant l'administration de la dose de lymphocytes T modifiées, le sujet a été traité avec une ou plusieurs thérapies antérieures pour la LLC ou la SLL, autres qu'une autre dose de cellules exprimant CAR ou une thérapie de déplétion lymphatique, éventuellement dans laquelle/lesquelles la ou les thérapies antérieures comprennent au moins deux thérapies antérieures, éventuellement 3, 4, 5, 6, 7, 8, 9 thérapies antérieures ou plus ;
(ii) au moment ou immédiatement avant l'administration de la dose de lymphocytes T modifiés, le sujet a rechuté après une rémission après un traitement avec, ou est devenu réfractaire à, a échoué et/ou était intolérant au traitement avec un ou plusieurs traitements antérieurs pour LLC ou SLL ;
(iii) au moment ou immédiatement avant l'administration de la dose de lymphocytes T modifiés, le sujet a rechuté après une rémission après un traitement avec, ou est devenu réfractaire à, a échoué et/ou était intolérant au traitement avec deux ou plusieurs thérapies antérieures ; et/ou
(iv) au moment ou immédiatement avant l'administration de la dose de lymphocytes T modifiés, le sujet a rechuté après une rémission après un traitement avec, ou est devenu réfractaire à, a échoué et/ou était intolérant au traitement avec trois thérapies antérieures ou plus.

8. Première composition pour une utilisation selon l'une quelconque des revendications 1 et 3 à 7 ou pluralité de compositions distinctes pour une utilisation selon l'une quelconque des revendications 2 à 7, dans laquelle/lesquelles au moment ou avant l'administration de la dose de cellules :
(i) le sujet est ou a été identifié comme présentant une ou plusieurs anomalies cytogénétiques, éventuellement associées à une LLC ou une SLL à haut risque, éventuellement sélectionnées parmi : un caryotype complexe ou des anomalies cytogénétiques, del 17p, un gène IGVH non muté et une mutation TP53 ; et/ou le sujet souffre de ou a été identifié comme souffrant de LLC ou de SLL à haut risque ;
(ii) le sujet présente ou a été identifié comme présentant une ou plusieurs anomalies cytogénétiques, éventuellement associées à une LLC à haut risque, éventuellement sélectionnées parmi : un caryotype complexe ou des anomalies cytogénétiques, del 17p, un gène IGVH non muté et une mutation TP53 ; et/ou le sujet souffre de ou a été identifié comme souffrant de LLC à haut risque, éventuellement dans lequel le sujet a rechuté après une rémission après un traitement avec, ou est devenu réfractaire à, a échoué et/ou était intolérant au traitement avec deux thérapies antérieures ou plus ;
(iii) le sujet souffre de ou a été identifié comme souffrant d'une LLC ou d'une SLL à risque standard, éventuellement dans lequel le sujet a rechuté après une rémission après un traitement avec, ou est devenu réfractaire à, a échoué et/ou était intolérant au traitement avec trois thérapies antérieures ou plus ; et/ou
(iv) le sujet est ou a été identifié comme étant intolérant à un inhibiteur de la tyrosine kinase de Bruton (BTK) et a reçu un inhibiteur de la BTK pendant une durée inférieure à 6 mois environ, et/ou n'est pas éligible pour un traitement par un inhibiteur de BTK, éventuellement dans lequel :
(a) le sujet souffre de ou a été identifié comme souffrant de LLC ou de SLL à haut risque, et au moment ou immédiatement avant l'administration de la dose de cellules, le sujet a rechuté après une rémission après un traitement avec, ou est devenu réfractaire à, a échoué et/ou était intolérant au traitement avec une ou plusieurs thérapies antérieures autres que l'inhibiteur de BTK ; ou
(b) le sujet souffre de ou a été identifié comme souffrant d'une LLC ou d'une SLL à risque standard, et au moment ou immédiatement avant l'administration de la dose de cellules, le sujet a rechuté après une rémission après un traitement avec, ou est devenu réfractaire à, a échoué et/ou était intolérant au traitement avec deux ou plusieurs thérapies antérieures autres que l'inhibiteur de BTK.

9. Première composition pour une utilisation selon l'une quelconque des revendications 1 et 3 à 8 ou pluralité de compositions distinctes pour une utilisation selon l'une quelconque des revendications 2 à 8, dans laquelle/lesquelles :
(i) le sujet est ou a été identifié comme ayant un statut ECOG de 0 ou 1 ; et/ou
le sujet n'a pas un statut ECOG > 1 ;
(ii) au moment ou immédiatement avant l'administration de la dose de cellules modifiées, le sujet ne présente pas de transformation de Richter en LLC ou SLL ; et/ou
(iii) le sujet est un adulte et/ou a plus de 50, 60 ou 70 ans environ.

10. Première composition pour une utilisation selon l'une quelconque des revendications 1 et 3 à 9 ou pluralité de compositions distinctes pour une utilisation selon l'une quelconque des revendications 2 à 9, dans laquelle/lesquelles :
(i) les lymphocytes T modifiés sont des lymphocytes T primaires obtenus auprès d'un sujet ;
(ii) les lymphocytes T modifiés sont autologues au sujet ; et/ou
(iii) la dose de cellules modifiées est des cellules viables.

11. Première composition pour une utilisation selon l'une quelconque des revendications 1 et 3 à 10 ou pluralité de compositions distinctes pour une utilisation selon l'une quelconque des revendications 2 à 10, dans laquelle/lesquelles :
(i) le procédé comprend en outre, avant l'administration de la dose de lymphocytes T modifiés, l'administration d'une thérapie lymphodéplétive au sujet et/ou le sujet ayant été préconditionné avec une thérapie lymphodéplétive, éventuellement dans laquelle/lesquelles:
(a) la thérapie lymphodéplétive comprend l'administration de fludarabine et/ou de cyclophosphamide ;
(b) la thérapie lymphodéplétive comprend l'administration de cyclophosphamide à raison de ou d'environ 200 à 400 mg/m² , éventuellement de ou d'environ 300 mg/m², inclus, et/ou de fludarabine à raison de ou d'environ 20 à 40 mg/m² , éventuellement de 30 mg m², quotidiennement pendant 2 à 4 jours, éventuellement pendant 3 jours ; et/ou
(c) la thérapie lymphodéplétive comprend l'administration de cyclophosphamide à raison de ou d'environ 300 mg/m² et de fludarabine à raison de ou d'environ 30 mg/m² par jour pendant 3 jours, éventuellement, la dose de cellules étant administrée au moins environ 2 à 7 jours après la thérapie lymphodéplétive ou au moins environ 2 à 7 jours après le début de la thérapie lymphodéplétive ; et/ou
(ii) l'administration de la dose de lymphocytes T modifiés et/ou la thérapie lymphodéplétive est réalisée en ambulatoire.

12. Première composition pour une utilisation selon l'une quelconque des revendications 1 et 3 à 11 ou pluralité de compositions distinctes pour une utilisation selon l'une quelconque des revendications 2 à 11, dans laquelle/lesquelles, parmi une pluralité de sujets traités selon le procédé :
(i) la réponse chez au moins 50 %, au moins 60 %, au moins 70 %, au moins 80 %, au moins 90 % des sujets traités est un taux de réponse objectif ; et/ou la réponse chez au moins 35 %, au moins 40 %, au moins 50 %, au moins 60 % ou au moins 70 % des sujets traités est une rémission complète (RC), éventuellement dans laquelle la durée de la réponse jusqu'à la progression est durable pendant plus de 3 mois ou plus de 6 mois ;
(ii) plus de 50 %, plus de 60 % ou plus de 70 % présentaient une maladie résiduelle minimale (MRD) indétectable pendant au moins un mois, au moins deux mois, au moins trois mois ou au moins 6 mois après l'administration de la dose de lymphocytes T modifiés ;
(iii) pas plus de 10 % des sujets présentent un syndrome de libération de cytokines (CRS) supérieur au grade 2 ; et/ou
(iv) pas plus de 10 %, pas plus de 20 %, pas plus de 30 % ou pas plus de 40 % des sujets présentent une neurotoxicité supérieure au grade 2,
éventuellement, la pluralité de sujets traités selon le procédé comprend une pluralité de sujets qui ont rechuté après une rémission après un traitement avec, sont devenus réfractaires à, ont échoué au traitement avec et/ou sont intolérants à l'ibrutinib et au vénétoclax.

13. Première composition pour une utilisation selon l'une quelconque des revendications 1 et 3 à 12 ou pluralité de compositions distinctes pour une utilisation selon l'une quelconque des revendications 2 à 12, dans laquelle/lesquelles :
le CAR comprend un domaine extracellulaire de liaison à un antigène spécifique à CD19, un domaine transmembranaire, un domaine de signalisation cytoplasmique dérivé d'une molécule co-stimulatrice, qui est éventuellement un 4-1 BB, et un domaine de signalisation cytoplasmique dérivé d'une molécule de signalisation primaire contenant de l'ITAM , qui est éventuellement un CD3zêta ;
le CAR comprend, dans l'ordre, un domaine de liaison à un antigène extracellulaire spécifique à CD19, un domaine transmembranaire, un domaine de signalisation cytoplasmique dérivé d'une molécule co-stimulatrice et un domaine de signalisation cytoplasmique dérivé d'une molécule contenant de l'ITAM de signalisation primaire.

14. Première composition pour une utilisation selon la revendication 13 ou pluralité de compositions distinctes pour une utilisation selon la revendication 13, dans laquelle/lesquelles le domaine de liaison à un antigène est un scFv, éventuellement dans laquelle/lesquelles :
le scFv comprend une séquence CDRL1 de RASQDISKYLN (SEQ ID NO: 35), une séquence CDRL2 de SRLHSGV (SEQ ID NO: 36), et/ou une séquence CDRL3 de GNTLPYTFG (SEQ ID NO: 37) et/ou une séquence CDRH1 de DYGVS (SEQ ID NO: 38), une séquence CDRH2 de VIWGSETTYYNSALKS (SEQ ID NO: 39), et/ou une séquence CDRH3 de YAMDYWG (SEQ ID NO: 40);
le scFv comprend une région de chaîne lourde variable de FMC63 et une région de chaîne légère variable de FMC63 et/ou une séquence CDRL1 de FMC63, une séquence CDRL2 de FMC63, une séquence CDRL3 de FMC63, une séquence CDRH1 de FMC63, une séquence CDRH2 de FMC63, et une séquence CDRH3 de FMC63 ou se lie au même épitope que ou entre en compétition pour la liaison avec l'un des précédents ;
le scFv comprend une VH présentée dans SEQ ID NO: 41 et une VL présentée dans SEQ ID NO: 42, éventuellement dans lequel la VH et la VL sont séparées par un lieur flexible, éventuellement dans lequel le lieur flexible est ou comprend la séquence présentée dans SEQ ID NO: 24 ; et/ou
le scFv est ou comprend la séquence présentée dans SEQ ID NO: 43.

15. Première composition pour une utilisation selon la revendication 13 ou 14 ou pluralité de compositions distinctes pour une utilisation selon la revendication 13 ou la revendication 14, dans laquelle/lesquelles :
(i) la région de signalisation co-stimulatrice est un domaine de signalisation de CD28 ou 4-1BB ;
(ii) la région de signalisation co-stimulatrice est un domaine de signalisation de 4-1BB ;
(iii) le domaine co-stimulateur comprend SEQ ID NO: 12 ou un variant de celui-ci ayant au moins 85 %, 86 %, 87 %, 88 %, 89 %, 90 %, 91 %, 92 %, 93 %, 94 %, 95 %, 96 %, 97 %, 98 %, 99 % ou plus d'identité de séquence avec celle-ci ;
(iv) le domaine de signalisation principal est un domaine de signalisation CD3zêta ;
(v) le domaine de signalisation primaire comprend SEQ ID NO: 13 ou 14 ou 15 ayant au moins 85 %, 86 %, 87 %, 88 %, 89 %, 90 %, 91 %, 92 %, 93 %, 94 %, 95 %, 96 %, 97 %, 98 %, 99 % ou plus d'identité de séquence avec celle-ci ; et/ou
(vi) le CAR comprend en outre un espaceur entre le domaine transmembranaire et le scFv, éventuellement dans lequel :
(a) l'espaceur est un espaceur polypeptidique qui comprend ou est constitué de tout ou partie d'une charnière d'immunoglobuline ou d'une version modifiée de celle-ci, éventuellement une charnière d'IgG4, ou une version modifiée de celle-ci ;
(b) l'espaceur comporte environ 15 acides aminés ou moins, et ne comprend pas une région extracellulaire de CD28 ou une région extracellulaire de CD8 ; et/ou
(c) l'espaceur a une longueur de ou d'environ 12 acides aminés.
